# EUROPEAN PATENT APPLICATION

(11) **EP 1 676 861 A2**
(43) Date of publication of application: **05.07.2006**
(21) Application number: 05009877.1
(22) Date of filing: 13.10.1999
(51) Int. Cl.: C07K 14/72

(54) **Non-endogenous, constitutively activated human G protein-coupled receptors**

(30) Priority: 13.10.1998 US 170496; 12.11.1998 US 108029 P; 20.11.1998 US 109213 P; 27.11.1998 US 110060 P; 16.02.1999 US 120416 P; 26.02.1999 US 121852 P; 12.03.1999 US 123944 P; 12.03.1999 US 123945 P; 12.03.1999 US 123948 P; 12.03.1999 US 123946 P; 12.03.1999 US 123949 P; 12.03.1999 US 123951 P; 28.05.1999 US 136436 P; 28.05.1999 US 136437 P; 28.05.1999 US 136439 P; 28.05.1999 US 137567 P; 28.05.1999 US 137127 P; 28.05.1999 US 137131 P; 30.06.1999 US 141448 P; 27.08.1999 US 151114 P; 03.09.1999 US 152524 P; 29.09.1999 US 156653 P; 29.09.1999 US 156633 P; 29.09.1999 US 156555 P; 29.09.1999 US 156634 P; 01.10.1999 US 157280 P; 01.10.1999 US 157294 P; 01.10.1999 US 157281 P; 01.10.1999 US 157293 P; 01.10.1999 US 157282 P; 12.10.1999 US 417044; 12.10.1999 US 416760 P
(62) Divisional of application: 99950301.4
(71) Applicant: Arena Pharmaceuticals, Inc., San Diego, CA 92121 (US)
(72) Inventor: Chen, Ruoping, San Diego CA 92130 (US); Lowitz, Kevin, San Diego CA 92108 (US); Behan, Dominic P., San Diego CA 92131 (US); Liaw, Chen W., San Diego CA 92130 (US); Chalmers, Derek T., Greenwich, CT 06878 (US)
(74) Representative: Cripps, Joanna Elizabeth

(57) **Abstract**

The invention disclosed in this patent document relates to transmembrane receptors, more particularly to a human G protein-coupled receptor for which the endogenous ligand is unknown ("orphan GPCR receptors"), and most particularly to mutated (non-endogenous) versions of the human GPCRs for evidence of constitutive activity.

SEQUENCE LISTING

(1) GENERAL INFORMATION:

(i) APPLICANT: Behan, Dominic P.
Lehmann-Bruinsma, Karin
Chalmers, Derek T.
Lowitz, Kevin P.
Lin, I-Lin
Dang, Huong T.
Chen, Ruoping
Liaw, Chen W.

(ii) TITLE OF INVENTION: Non-Endogenous, Constitutively Activated Human
G Protein-Coupled Receptors

(iii) NUMBER OF SEQUENCES: 146

(iv) CORRESPONDENCE ADDRESS:
(A) ADDRESSEE: Arena Pharmaceuticals, Inc.
(B) STREET: 6166 Nancy Ridge Drive
(C) CITY: San Diego
(D) STATE: CA
(E) COUNTRY: USA
(F) ZIP: 92121

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US
(B) FILING DATE:
(C) CLASSIFICATION:

(viii) ATTORNEY/AGENT INFORMATION:
(A) NAME: Burgoon, Richard P.
(B) REGISTRATION NUMBER: 34,787

(ix) TELECOMMUNICATION INFORMATION:
(A) TELEPHONE: (858)453-7200
(B) TELEFAX: (858)453-7210

(2) INFORMATION FOR SEQ ID NO:1:

(i) SEQUENCE CHARACTERISTICS:
(A) LENGTH: 1260 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

ATGGTCTTCT CGGCAGTGTT GACTGCGTTC CATACCGGGA CATCCAACAC AACATTTGTC 60

GTGTATGAAA ACACCTACAT GAATATTACA CTCCCTCCAC CATTCCAGCA TCCTGACCTC 120

AGTCCATTGC TTAGATATAG TTTTGAAACC ATGGCTCCCA CTGGTTTGAG TTCCTTGACC 180

GTGAATAGTA CAGCTGTGCC CACAACACCA GCAGCATTTA AGAGCCTAAA CTTGCCTCTT 240

CAGATCACCC TTTCTGCTAT AATGATATTC ATTCTGTTTG TGTCTTTTCT TGGGAACTTG 300

GTTGTTTGCC TCATGGTTTA CCAAAAAGCT GCCATGAGGT CTGCAATTAA CATCCTCCTT 360

GCCAGCCTAG CTTTTGCAGA CATGTTGCTT GCAGTGCTGA ACATGCCCTT TGCCCTGGTA 420

ACTATTCTTA CTACCCGATG GATTTTTGGG AAATTCTTCT GTAGGGTATC TGCTATGTTT 480

TTCTGGTTAT TTGTGATAGA AGGAGTAGCC ATCCTGCTCA TCATTAGCAT AGATAGGTTC 540

CTTATTATAG TCCAGAGGCA GGATAAGCTA AACCCATATA GAGCTAAGGT TCTGATTGCA 600

GTTTCTTGGG CAACTTCCTT TTGTGTAGCT TTTCCTTTAG CCGTAGGAAA CCCCGACCTG 660

CAGATACCTT CCCGAGCTCC CCAGTGTGTG TTTGGGTACA CAACCAATCC AGGCTACCAG 720

GCTTATGTGA TTTTGATTTC TCTCATTTCT TTCTTCATAC CCTTCCTGGT AATACTGTAC 780

TCATTTATGG GCATACTCAA CACCCTTCGG CACAATGCCT TGAGGATCCA TAGCTACCCT 840

GAAGGTATAT GCCTCAGCCA GGCCAGCAAA CTGGGTCTCA TGAGTCTGCA GAGACCTTTC 900

CAGATGAGCA TTGACATGGG CTTTAAAACA CGTGCCTTCA CCACTATTTT GATTCTCTTT 960

GCTGTCTTCA TTGTCTGCTG GGCCCCATTC ACCACTTACA GCCTTGTGGC AACATTCAGT 1020

AAGCACTTTT ACTATCAGCA CAACTTTTTT GAGATTAGCA CCTGGCTACT GTGGCTCTGC 1080

TACCTCAAGT CTGCATTGAA TCCGCTGATC TACTACTGGA GGATTAAGAA ATTCCATGAT 1140

GCTTGCCTGG ACATGATGCC TAAGTCCTTC AAGTTTTTGC CGCAGCTCCC TGGTCACACA 1200

AAGCGACGGA TACGTCCTAG TGCTGTCTAT GTGTGTGGGG AACATCGGAC GGTGGTGTGA 1260

(3) INFORMATION FOR SEQ ID NO:2:

(i) SEQUENCE CHARACTERISTICS:
(A) LENGTH: 419 amino acids
(B) TYPE: amino acid
(C) STRANDEDNESS: single
(D) TOPOLOGY: linear

(ii) MOLECULE TYPE: DNA (genomic)

(xi) SEQUENCE DESCRIPTION: SEQ ID NO:2:

Met Val Phe Ser Ala Val Leu Thr Ala Phe His Thr Gly Thr Ser Asn
1               5                   10                  15

Thr Thr Phe Val Val Tyr Glu Asn Thr Tyr Met Asn Ile Thr Leu Pro
            20                  25                  30

Pro Pro Phe Gln His Pro Asp Leu Ser Pro Leu Leu Arg Tyr Ser Phe
        35                  40                  45

Glu Thr Met Ala Pro Thr Gly Leu Ser Ser Leu Thr Val Asn Ser Thr
    50                  55                  60

Ala Val Pro Thr Thr Pro Ala Ala Phe Lys Ser Leu Asn Leu Pro Leu
65                  70                  75                  80

Gln Ile Thr Leu Ser Ala Ile Met Ile Phe Ile Leu Phe Val Ser Phe
                85                  90                  95

Leu Gly Asn Leu Val Val Cys Leu Met Val Tyr Gln Lys Ala Ala Met
            100                 105                 110

Arg Ser Ala Ile Asn Ile Leu Leu Ala Ser Leu Ala Phe Ala Asp Met
        115                 120                 125

Leu Leu Ala Val Leu Asn Met Pro Phe Ala Leu Val Thr Ile Leu Thr
    130                 135                 140

Thr Arg Trp Ile Phe Gly Lys Phe Phe Cys Arg Val Ser Ala Met Phe
145                 150                 155                 160

Phe Trp Leu Phe Val Ile Glu Gly Val Ala Ile Leu Leu Ile Ile Ser
                165                 170                 175

Ile Asp Arg Phe Leu Ile Ile Val Gln Arg Gln Asp Lys Leu Asn Pro
            180                 185                 190

Tyr Arg Ala Lys Val Leu Ile Ala Val Ser Trp Ala Thr Ser Phe Cys
        195                 200                 205

Val Ala Phe Pro Leu Ala Val Gly Asn Pro Asp Leu Gln Ile Pro Ser
    210                 215                 220

Arg Ala Pro Gln Cys Val Phe Gly Tyr Thr Thr Asn Pro Gly Tyr Gln
225                 230                 235                 240

Ala Tyr Val Ile Leu Ile Ser Leu Ile Ser Phe Phe Ile Pro Phe Leu
                245                 250                 255

Val Ile Leu Tyr Ser Phe Met Gly Ile Leu Asn Thr Leu Arg His Asn
            260                 265                 270

Ala Leu Arg Ile His Ser Tyr Pro Glu Gly Ile Cys Leu Ser Gln Ala
        275                 280                 285

Ser Lys Leu Gly Leu Met Ser Leu Gln Arg Pro Phe Gln Met Ser Ile
    290                 295                 300

Asp Met Gly Phe Lys Thr Arg Ala Phe Thr Thr Ile Leu Ile Leu Phe
305                 310                 315                 320

Ala Val Phe Ile Val Cys Trp Ala Pro Phe Thr Thr Tyr Ser Leu Val
                325                 330                 335

Ala Thr Phe Ser Lys His Phe Tyr Tyr Gln His Asn Phe Phe Glu Ile
            340                 345                 350

Ser Thr Trp Leu Leu Trp Leu Cys Tyr Leu Lys Ser Ala Leu Asn Pro
        355                 360                 365

Leu Ile Tyr Tyr Trp Arg Ile Lys Lys Phe His Asp Ala Cys Leu Asp

370                 375                 380

Met Met Pro Lys Ser Phe Lys Phe Leu Pro Gln Leu Pro Gly His Thr
385                 390                 395                 400

Lys Arg Arg Ile Arg Pro Ser Ala Val Tyr Val Cys Gly Glu His Arg
                405                 410                 415

Thr Val Val

(4) INFORMATION FOR SEQ ID NO:3:

(i) SEQUENCE CHARACTERISTICS:
(A) LENGTH: 1119 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

ATGTTAGCCA ACAGCTCCTC AACCAACAGT TCTGTTCTCC CGTGTCCTGA CTACCGACCT 60

ACCCACCGCC TGCACTTGGT GGTCTACAGC TTGGTGCTGG CTGCCGGGCT CCCCCTCAAC 120

GCGCTAGCCC TCTGGGTCTT CCTGCGCGCG CTGCGCGTGC ACTCGGTGGT GAGCGTGTAC 180

ATGTGTAACC TGGCGGCCAG CGACCTGCTC TTCACCCTCT CGCTGCCCGT TCGTCTCTCC 240

TACTACGCAC TGCACCACTG GCCCTTCCCC GACCTCCTGT GCCAGACGAC GGGCGCCATC 300

TTCCAGATGA ACATGTACGG CAGCTGCATC TTCCTGATGC TCATCAACGT GGACCGCTAC 360

GCCGCCATCG TGCACCCGCT GCGACTGCGC CACCTGCGGC GGCCCCGCGT GGCGCGGCTG 420

CTCTGCCTGG GCGTGTGGGC GCTCATCCTG GTGTTTGCCG TGCCCGCCGC CCGCGTGCAC 480

AGGCCCTCGC GTTGCCGCTA CCGGGACCTC GAGGTGCGCC TATGCTTCGA GAGCTTCAGC 540

GACGAGCTGT GGAAAGGCAG GCTGCTGCCC CTCGTGCTGC TGGCCGAGGC GCTGGGCTTC 600

CTGCTGCCCC TGGCGGCGGT GGTCTACTCG TCGGGCCGAG TCTTCTGGAC GCTGGCGCGC 660

CCCGACGCCA CGCAGAGCCA GCGGCGGCGG AAGACCGTGC GCCTCCTGCT GGCTAACCTC 720

GTCATCTTCC TGCTGTGCTT CGTGCCCTAC AACAGCACGC TGGCGGTCTA CGGGCTGCTG 780

CGGAGCAAGC TGGTGGCGGC CAGCGTGCCT GCCCGCGATC GCGTGCGCGG GGTGCTGATG 840

GTGATGGTGC TGCTGGCCGG CGCCAACTGC GTGCTGGACC CGCTGGTGTA CTACTTTAGC 900

GCCGAGGGCT TCCGCAACAC CCTGCGCGGC CTGGGCACTC CGCACCGGGC CAGGACCTCG 960

GCCACCAACG GGACGCGGGC GGCGCTCGCG CAATCCGAAA GGTCCGCCGT CACCACCGAC 1020

GCCACCAGGC CGGATGCCGC CAGTCAGGGG CTGCTCCGAC CCTCCGACTC CCACTCTCTG 1080

TCTTCCTTCA CACAGTGTCC CCAGGATTCC GCCCTCTGA 1119

(5) INFORMATION FOR SEQ ID NO:4:

(i) SEQUENCE CHARACTERISTICS:
(A) LENGTH: 372 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:4:

(6) INFORMATION FOR SEQ ID NO:5:

(i) SEQUENCE CHARACTERISTICS:
(A) LENGTH: 1107 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

ATGGCCAACT CCACAGGGCT GAACGCCTCA GAAGTCGCAG GCTCGTTGGG GTTGATCCTG 60

GCAGCTGTCG TGGAGGTGGG GGCACTGCTG GGCAACGGCG CGCTGCTGGT CGTGGTGCTG 120

CGCACGCCGG GACTGCGCGA CGCGCTCTAC CTGGCGCACC TGTGCGTCGT GGACCTGCTG 180

GCGGCCGCCT CCATCATGCC GCTGGGCCTG CTGGCCGCAC CGCCGCCCGG GCTGGGCCGC 240

GTGCGCCTGG GCCCCGCGCC ATGCCGCGCC GCTCGCTTCC TCTCCGCCGC TCTGCTGCCG 300

GCCTGCACGC TCGGGGTGGC CGCACTTGGC CTGGCACGCT ACCGCCTCAT CGTGCACCCG 360

CTGCGGCCAG GCTCGCGGCC GCCGCCTGTG CTCGTGCTCA CCGCCGTGTG GGCCGCGGCG 420

GGACTGCTGG GCGCGCTCTC CCTGCTCGGC CCGCCGCCCG CACCGCCCCC TGCTCCTGCT 480

CGCTGCTCGG TCCTGGCTGG GGGCCTCGGG CCCTTCCGGC CGCTCTGGGC CCTGCTGGCC 540

TTCGCGCTGC CCGCCCTCCT GCTGCTCGGC GCCTACGGCG GCATCTTCGT GGTGGCGCGT 600

CGCGCTGCCC TGAGGCCCCC ACGGCCGGCG CGCGGGTCCC GACTCCGCTC GGACTCTCTG 660

GATAGCCGCC TTTCCATCTT GCCGCCGCTC CGGCCTCGCC TGCCCGGGGG CAAGGCGGCC 720

CTGGCCCCAG CGCTGGCCGT GGGCCAATTT GCAGCCTGCT GGCTGCCTTA TGGCTGCGCG 780

TGCCTGGCGC CCGCAGCGCG GGCCGCGGAA GCCGAAGCGG CTGTCACCTG GGTCGCCTAC 840

TCGGCCTTCG CGGCTCACCC CTTCCTGTAC GGGCTGCTGC AGCGCCCCGT GCGCTTGGCA 900

CTGGGCCGCC TCTCTCGCCG TGCACTGCCT GGACCTGTGC GGGCCTGCAC TCCGCAAGCC 960

TGGCACCCGC GGGCACTCTT GCAATGCCTC CAGAGACCCC CAGAGGGCCC TGCCGTAGGC 1020

CCTTCTGAGG CTCCAGAACA GACCCCCGAG TTGGCAGGAG GGCGGAGCCC CGCATACCAG 1080

GGGCCACCTG AGAGTTCTCT CTCCTGA 1107

(7) INFORMATION FOR SEQ ID NO:6:

(i) SEQUENCE CHARACTERISTICS:
(A) LENGTH: 368 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:6:

Met Ala Asn Ser Thr Gly Leu Asn Ala Ser Glu Val Ala Gly Ser Leu
1               5                   10                  15

Gly Leu Ile Leu Ala Ala Val Val Glu Val Gly Ala Leu Leu Gly Asn
            20                  25                  30

Gly Ala Leu Leu Val Val Val Leu Arg Thr Pro Gly Leu Arg Asp Ala
        35                  40                  45

Leu Tyr Leu Ala His Leu Cys Val Val Asp Leu Leu Ala Ala Ala Ser
    50                  55                  60

Ile Met Pro Leu Gly Leu Leu Ala Ala Pro Pro Pro Gly Leu Gly Arg
65                  70                  75                  80

Val Arg Leu Gly Pro Ala Pro Cys Arg Ala Ala Arg Phe Leu Ser Ala
                85                  90                  95

Ala Leu Leu Pro Ala Cys Thr Leu Gly Val Ala Ala Leu Gly Leu Ala
            100                 105                 110

Arg Tyr Arg Leu Ile Val His Pro Leu Arg Pro Gly Ser Arg Pro Pro
        115                 120                 125

Pro Val Leu Val Leu Thr Ala Val Trp Ala Ala Ala Gly Leu Leu Gly
    130                 135                 140

Ala Leu Ser Leu Leu Gly Pro Pro Pro Ala Pro Pro Pro Ala Pro Ala
145                 150                 155                 160

Arg Cys Ser Val Leu Ala Gly Gly Leu Gly Pro Phe Arg Pro Leu Trp
                165                 170                 175

Ala Leu Leu Ala Phe Ala Leu Pro Ala Leu Leu Leu Leu Gly Ala Tyr
            180                 185                 190

Gly Gly Ile Phe Val Val Ala Arg Arg Ala Ala Leu Arg Pro Pro Arg
        195                 200                 205

Pro Ala Arg Gly Ser Arg Leu Arg Ser Asp Ser Leu Asp Ser Arg Leu
    210                 215                 220

Ser Ile Leu Pro Pro Leu Arg Pro Arg Leu Pro Gly Gly Lys Ala Ala
225                 230                 235                 240

Leu Ala Pro Ala Leu Ala Val Gly Gln Phe Ala Ala Cys Trp Leu Pro
                245                 250                 255

Tyr Gly Cys Ala Cys Leu Ala Pro Ala Ala Arg Ala Ala Glu Ala Glu
            260                 265                 270

Ala Ala Val Thr Trp Val Ala Tyr Ser Ala Phe Ala Ala His Pro Phe
        275                 280                 285

Leu Tyr Gly Leu Leu Gln Arg Pro Val Arg Leu Ala Leu Gly Arg Leu
    290                 295                 300

Ser Arg Arg Ala Leu Pro Gly Pro Val Arg Ala Cys Thr Pro Gln Ala
305                 310                 315                 320

Trp His Pro Arg Ala Leu Leu Gln Cys Leu Gln Arg Pro Pro Glu Gly
                325                 330                 335

Pro Ala Val Gly Pro Ser Glu Ala Pro Glu Gln Thr Pro Glu Leu Ala
            340                 345                 350

Gly Gly Arg Ser Pro Ala Tyr Gln Gly Pro Pro Glu Ser Ser Leu Ser
        355                 360                 365

(8) INFORMATION FOR SEQ ID NO:7:

    (i) SEQUENCE CHARACTERISTICS:
        (A) LENGTH: 1008 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:7:

ATGGAATCAT CTTTCTCATT TGGAGTGATC CTTGCTGTCC TGGCCTCCCT CATCATTGCT      60

ACTAACACAC TAGTGGCTGT GGCTGTGCTG CTGTTGATCC ACAAGAATGA TGGTGTCAGT     120

CTCTGCTTCA CCTTGAATCT GGCTGTGGCT GACACCTTGA TTGGTGTGGC CATCTCTGGC     180

CTACTCACAG ACCAGCTCTC CAGCCCTTCT CGGCCCACAC AGAAGACCCT GTGCAGCCTG     240

CGGATGGCAT TTGTCACTTC CTCCGCAGCT GCCTCTGTCC TCACGGTCAT GCTGATCACC     300

TTTGACAGGT ACCTTGCCAT CAAGCAGCCC TTCCGCTACT TGAAGATCAT GAGTGGGTTC     360

GTGGCCGGGG CCTGCATTGC CGGGCTGTGG TTAGTGTCTT ACCTCATTGG CTTCCTCCCA 420

CTCGGAATCC CCATGTTCCA GCAGACTGCC TACAAAGGGC AGTGCAGCTT CTTTGCTGTA 480

TTTCACCCTC ACTTCGTGCT GACCCTCTCC TGCGTTGGCT TCTTCCCAGC CATGCTCCTC 540

TTTGTCTTCT TCTACTGCGA CATGCTCAAG ATTGCCTCCA TGCACAGCCA GCAGATTCGA 600

AAGATGGAAC ATGCAGGAGC CATGGCTGGA GGTTATCGAT CCCCACGGAC TCCCAGCGAC 660

TTCAAAGCTC TCCGTACTGT GTCTGTTCTC ATTGGGAGCT TTGCTCTATC CTGGACCCCC 720

TTCCTTATCA CTGGCATTGT GCAGGTGGCC TGCCAGGAGT GTCACCTCTA CCTAGTGCTG 780

GAACGGTACC TGTGGCTGCT CGGCGTGGGC AACTCCCTGC TCAACCCACT CATCTATGCC 840

TATTGGCAGA AGGAGGTGCG ACTGCAGCTC TACCACATGG CCCTAGGAGT GAAGAAGGTG 900

CTCACCTCAT TCCTCCTCTT TCTCTCGGCC AGGAATTGTG GCCCAGAGAG GCCCAGGGAA 960

AGTTCCTGTC ACATCGTCAC TATCTCCAGC TCAGAGTTTG ATGGCTAA 1008

(9) INFORMATION FOR SEQ ID NO:8:

(i) SEQUENCE CHARACTERISTICS:
(A) LENGTH: 335 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:8:

Met Glu Ser Ser Phe Ser Phe Gly Val Ile Leu Ala Val Leu Ala Ser
1               5                   10                  15

Leu Ile Ile Ala Thr Asn Thr Leu Val Ala Val Ala Val Leu Leu Leu
            20                  25                  30

Ile His Lys Asn Asp Gly Val Ser Leu Cys Phe Thr Leu Asn Leu Ala
        35                  40                  45

Val Ala Asp Thr Leu Ile Gly Val Ala Ile Ser Gly Leu Leu Thr Asp
    50                  55                  60

Gln Leu Ser Ser Pro Ser Arg Pro Thr Gln Lys Thr Leu Cys Ser Leu
65                  70                  75                  80

Arg Met Ala Phe Val Thr Ser Ser Ala Ala Ala Ser Val Leu Thr Val
                85                  90                  95

Met Leu Ile Thr Phe Asp Arg Tyr Leu Ala Ile Lys Gln Pro Phe Arg
            100                 105                 110

Tyr Leu Lys Ile Met Ser Gly Phe Val Ala Gly Ala Cys Ile Ala Gly
        115                 120                 125

Leu Trp Leu Val Ser Tyr Leu Ile Gly Phe Leu Pro Leu Gly Ile Pro
    130                 135                 140

Met Phe Gln Gln Thr Ala Tyr Lys Gly Gln Cys Ser Phe Phe Ala Val
145                 150                 155                 160

Phe His Pro His Phe Val Leu Thr Leu Ser Cys Val Gly Phe Phe Pro
                165                 170                 175

Ala Met Leu Leu Phe Val Phe Phe Tyr Cys Asp Met Leu Lys Ile Ala
            180                 185                 190

Ser Met His Ser Gln Gln Ile Arg Lys Met Glu His Ala Gly Ala Met
        195                 200                 205

Ala Gly Gly Tyr Arg Ser Pro Arg Thr Pro Ser Asp Phe Lys Ala Leu
    210                 215                 220

Arg Thr Val Ser Val Leu Ile Gly Ser Phe Ala Leu Ser Trp Thr Pro
225                 230                 235                 240

Phe Leu Ile Thr Gly Ile Val Gln Val Ala Cys Gln Glu Cys His Leu
                245                 250                 255

Tyr Leu Val Leu Glu Arg Tyr Leu Trp Leu Leu Gly Val Gly Asn Ser
            260                 265                 270

Leu Leu Asn Pro Leu Ile Tyr Ala Tyr Trp Gln Lys Glu Val Arg Leu
        275                 280                 285

Gln Leu Tyr His Met Ala Leu Gly Val Lys Lys Val Leu Thr Ser Phe
    290                 295                 300

Leu Leu Phe Leu Ser Ala Arg Asn Cys Gly Pro Glu Arg Pro Arg Glu
305                 310                 315                 320

Ser Ser Cys His Ile Val Thr Ile Ser Ser Ser Glu Phe Asp Gly
                325                 330                 335

(10) INFORMATION FOR SEQ ID NO:9:

     (i) SEQUENCE CHARACTERISTICS:
          (A) LENGTH: 1413 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:9:

ATGGACACTA CCATGGAAGC TGACCTGGGT GCCACTGGCC ACAGGCCCCG CACAGAGCTT      60

GATGATGAGG ACTCCTACCC CCAAGGTGGC TGGGACACGG TCTTCCTGGT GGCCCTGCTG     120

CTCCTTGGGC TGCCAGCCAA TGGGTTGATG GCGTGGCTGG CCGGCTCCCA GGCCCGGCAT     180

GGAGCTGGCA CGCGTCTGGC GCTGCTCCTG CTCAGCCTGG CCCTCTCTGA CTTCTTGTTC     240

CTGGCAGCAG CGGCCTTCCA GATCCTAGAG ATCCGGCATG GGGGACACTG GCCGCTGGGG     300

ACAGCTGCCT GCCGCTTCTA CTACTTCCTA TGGGGCGTGT CCTACTCCTC CGGCCTCTTC     360

CTGCTGGCCG CCCTCAGCCT CGACCGCTGC CTGCTGGCGC TGTGCCCACA CTGGTACCCT 420

GGGCACCGCC CAGTCCGCCT GCCCCTCTGG GTCTGCGCCG GTGTCTGGGT GCTGGCCACA 480

CTCTTCAGCG TGCCCTGGCT GGTCTTCCCC GAGGCTGCCG TCTGGTGGTA CGACCTGGTC 540

ATCTGCCTGG ACTTCTGGGA CAGCGAGGAG CTGTCGCTGA GGATGCTGGA GGTCCTGGGG 600

GGCTTCCTGC CTTTCCTCCT GCTGCTCGTC TGCCACGTGC TCACCCAGGC CACAGCCTGT 660

CGCACCTGCC ACCGCCAACA GCAGCCCGCA GCCTGCCGGG GCTTCGCCCG TGTGGCCAGG 720

ACCATTCTGT CAGCCTATGT GGTCCTGAGG CTGCCCTACC AGCTGGCCCA GCTGCTCTAC 780

CTGGCCTTCC TGTGGGACGT CTACTCTGGC TACCTGCTCT GGGAGGCCCT GGTCTACTCC 840

GACTACCTGA TCCTACTCAA CAGCTGCCTC AGCCCCTTCC TCTGCCTCAT GGCCAGTGCC 900

GACCTCCGGA CCCTGCTGCG CTCCGTGCTC TCGTCCTTCG CGGCAGCTCT CTGCGAGGAG 960

CGGCCGGGCA GCTTCACGCC CACTGAGCCA CAGACCCAGC TAGATTCTGA GGGTCCAACT 1020

CTGCCAGAGC CGATGGCAGA GGCCCAGTCA CAGATGGATC CTGTGGCCCA GCCTCAGGTG 1080

AACCCCACAC TCCAGCCACG ATCGGATCCC ACAGCTCAGC CACAGCTGAA CCCTACGGCC 1140

CAGCCACAGT CGGATCCCAC AGCCCAGCCA CAGCTGAACC TCATGGCCCA GCCACAGTCA 1200

GATTCTGTGG CCCAGCCACA GGCAGACACT AACGTCCAGA CCCCTGCACC TGCTGCCAGT 1260

TCTGTGCCCA GTCCCTGTGA TGAAGCTTCC CCAACCCCAT CCTCGCATCC TACCCCAGGG 1320

GCCCTTGAGG ACCCAGCCAC ACCTCCTGCC TCTGAAGGAG AAAGCCCCAG CAGCACCCCG 1380

CCAGAGGCGG CCCCGGGCGC AGGCCCCACG TGA 1413

(11) INFORMATION FOR SEQ ID NO:10:

(i) SEQUENCE CHARACTERISTICS:
(A) LENGTH: 468 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:10:

Met Asp Thr Thr Met Glu Ala Asp Leu Gly Ala Thr Gly His Arg Pro
1               5                   10                  15

Arg Thr Glu Leu Asp Asp Glu Asp Ser Tyr Pro Gln Gly Gly Trp Asp
            20                  25                  30

Thr Val Phe Leu Val Ala Leu Leu Leu Leu Gly Leu Pro Ala Asn Gly
        35                  40                  45

Leu Met Ala Trp Leu Ala Gly Ser Gln Ala Arg His Gly Ala Gly Thr
    50                  55                  60

Arg Leu Ala Leu Leu Leu Leu Ser Leu Ala Leu Ser Asp Phe Leu Phe
65                  70                  75                  80

Leu Ala Ala Ala Ala Phe Gln Ile Leu Glu Ile Arg His Gly Gly His
                85                  90                  95

Trp Pro Leu Gly Thr Ala Ala Cys Arg Phe Tyr Tyr Phe Leu Trp Gly
            100                 105                 110

Val Ser Tyr Ser Ser Gly Leu Phe Leu Leu Ala Ala Leu Ser Leu Asp
        115                 120                 125

Arg Cys Leu Leu Ala Leu Cys Pro His Trp Tyr Pro Gly His Arg Pro
    130                 135                 140

Val Arg Leu Pro Leu Trp Val Cys Ala Gly Val Trp Val Leu Ala Thr
145                 150                 155                 160

Leu Phe Ser Val Pro Trp Leu Val Phe Pro Glu Ala Ala Val Trp Trp
                165                 170                 175

Tyr Asp Leu Val Ile Cys Leu Asp Phe Trp Asp Ser Glu Glu Leu Ser
            180                 185                 190

Leu Arg Met Leu Glu Val Leu Gly Gly Phe Leu Pro Phe Leu Leu Leu
        195                 200                 205

Leu Val Cys His Val Leu Thr Gln Ala Thr Arg Thr Cys His Arg Gln
    210                 215                 220

Gln Gln Pro Ala Ala Cys Arg Gly Phe Ala Arg Val Ala Arg Thr Ile
225                 230                 235                 240

Leu Ser Ala Tyr Val Val Leu Arg Leu Pro Tyr Gln Leu Ala Gln Leu
                245                 250                 255

Leu Tyr Leu Ala Phe Leu Trp Asp Val Tyr Ser Gly Tyr Leu Leu Trp
            260                 265                 270

Glu Ala Leu Val Tyr Ser Asp Tyr Leu Ile Leu Leu Asn Ser Cys Leu
        275                 280                 285

Ser Pro Phe Leu Cys Leu Met Ala Ser Ala Asp Leu Arg Thr Leu Leu
    290                 295                 300

Arg Ser Val Leu Ser Ser Phe Ala Ala Ala Leu Cys Glu Glu Arg Pro
305                 310                 315                 320

Gly Ser Phe Thr Pro Thr Glu Pro Gln Thr Gln Leu Asp Ser Glu Gly
                325                 330                 335

Pro Thr Leu Pro Glu Pro Met Ala Glu Ala Gln Ser Gln Met Asp Pro
            340                 345                 350

Val Ala Gln Pro Gln Val Asn Pro Thr Leu Gln Pro Arg Ser Asp Pro
        355                 360                 365

Thr Ala Gln Pro Gln Leu Asn Pro Thr Ala Gln Pro Gln Ser Asp Pro
    370                 375                 380

Thr Ala Gln Pro Gln Leu Asn Leu Met Ala Gln Pro Gln Ser Asp Ser
385                 390                 395                 400

Val Ala Gln Pro Gln Ala Asp Thr Asn Val Gln Thr Pro Ala Pro Ala
                405                 410                 415

Ala Ser Ser Val Pro Ser Pro Cys Asp Glu Ala Ser Pro Thr Pro Ser
            420                 425                 430

Ser His Pro Thr Pro Gly Ala Leu Glu Asp Pro Ala Thr Pro Pro Ala
        435                 440                 445

Ser Glu Gly Glu Ser Pro Ser Ser Thr Pro Pro Glu Ala Ala Pro Gly
    450                 455                 460

Ala Gly Pro Thr
465

(12) INFORMATION FOR SEQ ID NO:11:

(i) SEQUENCE CHARACTERISTICS:
(A) LENGTH: 1248 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

ATGTCAGGGA TGGAAAAACT TCAGAATGCT TCCTGGATCT ACCAGCAGAA ACTAGAAGAT 60

CCATTCCAGA AACACCTGAA CAGCACCGAG GAGTATCTGG CCTTCCTCTG CGGACCTCGG 120

CGCAGCCACT TCTTCCTCCC CGTGTCTGTG GTGTATGTGC CAATTTTTGT GGTGGGGGTC 180

ATTGGCAATG TCCTGGTGTG CCTGGTGATT CTGCAGCACC AGGCTATGAA GACGCCCACC 240

AACTACTACC TCTTCAGCCT GGCGGTCTCT GACCTCCTGG TCCTGCTCCT TGGAATGCCC 300

CTGGAGGTCT ATGAGATGTG GCGCAACTAC CCTTTCTTGT TCGGGCCCGT GGGCTGCTAC 360

TTCAAGACGG CCCTCTTTGA GACCGTGTGC TTCGCCTCCA TCCTCAGCAT CACCACCGTC 420

AGCGTGGAGC GCTACGTGGC CATCCTACAC CCGTTCCGCG CCAAACTGCA GAGCACCCGG 480

CGCCGGGCCC TCAGGATCCT CGGCATCGTC TGGGGCTTCT CCGTGCTCTT CTCCCTGCCC 540

AACACCAGCA TCCATGGCAT CAAGTTCCAC TACTTCCCCA ATGGGTCCCT GGTCCCAGGT 600

TCGGCCACCT GTACGGTCAT CAAGCCCATG TGGATCTACA ATTTCATCAT CCAGGTCACC 660

TCCTTCCTAT TCTACCTCCT CCCCATGACT GTCATCAGTG TCCTCTACTA CCTCATGGCA 720

CTCAGACTAA AGAAAGACAA ATCTCTTGAG GCAGATGAAG GGAATGCAAA TATTCAAAGA 780

CCCTGCAGAA AATCAGTCAA CAAGATGCTG TTTGTCTTGG TCTTAGTGTT TGCTATCTGT 840

TGGGCCCCGT TCCACATTGA CCGACTCTTC TTCAGCTTTG TGGAGGAGTG GAGTGAATCC 900

CTGGCTGCTG TGTTCAACCT CGTCCATGTG GTGTCAGGTG TCTTCTTCTA CCTGAGCTCA 960

GCTGTCAACC CCATTATCTA TAACCTACTG TCTCGCCGCT TCCAGGCAGC ATTCCAGAAT 1020

GTGATCTCTT CTTTCCACAA ACAGTGGCAC TCCCAGCATG ACCCACAGTT GCCACCTGCC 1080

CAGCGGAACA TCTTCCTGAC AGAATGCCAC TTTGTGGAGC TGACCGAAGA TATAGGTCCC 1140

CAATTCCCAT GTCAGTCATC CATGCACAAC TCTCACCTCC CAACAGCCCT CTCTAGTGAA 1200

CAGATGTCAA GAACAAACTA TCAAAGCTTC CACTTTAACA AAACCTGA 1248

(13) INFORMATION FOR SEQ ID NO:12:

(i) SEQUENCE CHARACTERISTICS:
(A) LENGTH: 415 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:12:

(14) INFORMATION FOR SEQ ID NO:13:

(i) SEQUENCE CHARACTERISTICS:
(A) LENGTH: 1173 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

ATGCCAGATA CTAATAGCAC AATCAATTTA TCACTAAGCA CTCGTGTTAC TTTAGCATTT 60

TTTATGTCCT TAGTAGCTTT TGCTATAATG CTAGGAAATG CTTTGGTCAT TTTAGCTTTT 120

GTGGTGGACA AAAACCTTAG ACATCGAAGT AGTTATTTTT TTCTTAACTT GGCCATCTCT 180

GACTTCTTTG TGGGTGTGAT CTCCATTCCT TTGTACATCC CTCACACGCT GTTCGAATGG 240

GATTTTGGAA AGGAAATCTG TGTATTTTGG CTCACTACTG ACTATCTGTT ATGTACAGCA 300

TCTGTATATA ACATTGTCCT CATCAGCTAT GATCGATACC TGTCAGTCTC AAATGCTGTG 360

TCTTATAGAA CTCAACATAC TGGGGTCTTG AAGATTGTTA CTCTGATGGT GGCCGTTTGG 420

GTGCTGGCCT TCTTAGTGAA TGGGCCAATG ATTCTAGTTT CAGAGTCTTG GAAGGATGAA 480

GGTAGTGAAT GTGAACCTGG ATTTTTTTCG GAATGGTACA TCCTTGCCAT CACATCATTC 540

TTGGAATTCG TGATCCCAGT CATCTTAGTC GCTTATTTCA ACATGAATAT TTATTGGAGC 600

CTGTGGAAGC GTGATCATCT CAGTAGGTGC CAAAGCCATC CTGGACTGAC TGCTGTCTCT 660

TCCAACATCT GTGGACACTC ATTCAGAGGT AGACTATCTT CAAGGAGATC TCTTTCTGCA 720

TCGACAGAAG TTCCTGCATC CTTTCATTCA GAGAGACAGA GGAGAAAGAG TAGTCTCATG 780

TTTTCCTCAA GAACCAAGAT GAATAGCAAT ACAATTGCTT CCAAAATGGG TTCCTTCTCC 840

CAATCAGATT CTGTAGCTCT TCACCAAAGG GAACATGTTG AACTGCTTAG AGCCAGGAGA 900

TTAGCCAAGT CACTGGCCAT TCTCTTAGGG GTTTTTGCTG TTTGCTGGGC TCCATATTCT 960

CTGTTCACAA TTGTCCTTTC ATTTTATTCC TCAGCAACAG GTCCTAAATC AGTTTGGTAT 1020

AGAATTGCAT TTTGGCTTCA GTGGTTCAAT TCCTTTGTCA ATCCTCTTTT GTATCCATTG 1080

TGTCACAAGC GCTTTCAAAA GGCTTTCTTG AAAATATTTT GTATAAAAAA GCAACCTCTA 1140

CCATCACAAC ACAGTCGGTC AGTATCTTCT TAA 1173

(15) INFORMATION FOR SEQ ID NO:14:

(i) SEQUENCE CHARACTERISTICS:
(A) LENGTH: 390 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:14:

Met Pro Asp Thr Asn Ser Thr Ile Asn Leu Ser Leu Ser Thr Arg Val
1               5                   10                  15

Thr Leu Ala Phe Phe Met Ser Leu Val Ala Phe Ala Ile Met Leu Gly
            20                  25                  30

Asn Ala Leu Val Ile Leu Ala Phe Val Val Asp Lys Asn Leu Arg His
        35                  40                  45

Arg Ser Ser Tyr Phe Phe Leu Asn Leu Ala Ile Ser Asp Phe Phe Val
    50                  55                  60

Gly Val Ile Ser Ile Pro Leu Tyr Ile Pro His Thr Leu Phe Glu Trp
65                  70                  75                  80

Asp Phe Gly Lys Glu Ile Cys Val Phe Trp Leu Thr Thr Asp Tyr Leu
                85                  90                  95

Leu Cys Thr Ala Ser Val Tyr Asn Ile Val Leu Ile Ser Tyr Asp Arg
            100                 105                 110

Tyr Leu Ser Val Ser Asn Ala Val Ser Tyr Arg Thr Gln His Thr Gly
        115                 120                 125

Val Leu Lys Ile Val Thr Leu Met Val Ala Val Trp Val Leu Ala Phe
    130                 135                 140

Leu Val Asn Gly Pro Met Ile Leu Val Ser Glu Ser Trp Lys Asp Glu
145                 150                 155                 160

Gly Ser Glu Cys Glu Pro Gly Phe Phe Ser Glu Trp Tyr Ile Leu Ala
                165                 170                 175

Ile Thr Ser Phe Leu Glu Phe Val Ile Pro Val Ile Leu Val Ala Tyr
            180                 185                 190

Phe Asn Met Asn Ile Tyr Trp Ser Leu Trp Lys Arg Asp His Leu Ser
        195                 200                 205

Arg Cys Gln Ser His Pro Gly Leu Thr Ala Val Ser Ser Asn Ile Cys
    210                 215                 220

Gly His Ser Phe Arg Gly Arg Leu Ser Ser Arg Arg Ser Leu Ser Ala
225                 230                 235                 240

Ser Thr Glu Val Pro Ala Ser Phe His Ser Glu Arg Gln Arg Arg Lys
                245                 250                 255

Ser Ser Leu Met Phe Ser Ser Arg Thr Lys Met Asn Ser Asn Thr Ile
            260                 265                 270

Ala Ser Lys Met Gly Ser Phe Ser Gln Ser Asp Ser Val Ala Leu His
        275                 280                 285

Gln Arg Glu His Val Glu Leu Leu Arg Ala Arg Arg Leu Ala Lys Ser
    290                 295                 300

Leu Ala Ile Leu Leu Gly Val Phe Ala Val Cys Trp Ala Pro Tyr Ser
305                 310                 315                 320

Leu Phe Thr Ile Val Leu Ser Phe Tyr Ser Ser Ala Thr Gly Pro Lys
                325                 330                 335

Ser Val Trp Tyr Arg Ile Ala Phe Trp Leu Gln Trp Phe Asn Ser Phe
            340                 345                 350

Val Asn Pro Leu Leu Tyr Pro Leu Cys His Lys Arg Phe Gln Lys Ala
        355                 360                 365

Phe Leu Lys Ile Phe Cys Ile Lys Lys Gln Pro Leu Pro Ser Gln His
    370                 375                 380

Ser Arg Ser Val Ser Ser
385                 390

(16) INFORMATION FOR SEQ ID NO:15:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:15:

GGAAAGCTTA ACGATCCCCA GGAGCAACAT 30

(17) INFORMATION FOR SEQ ID NO:16:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: protein

(iv) ANTI-SENSE: YES

(xi) SEQUENCE DESCRIPTION: SEQ ID NO:16:

CTGGGATCCT ACGAGAGCAT TTTTCACACA G 31

(18) INFORMATION FOR SEQ ID NO:17:

(i) SEQUENCE CHARACTERISTICS:
(A) LENGTH: 1128 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

ATGGCGAACG CGAGCGAGCC GGGTGGCAGC GGCGGCGGCG AGGCGGCCGC CCTGGGCCTC 60

AAGCTGGCCA CGCTCAGCCT GCTGCTGTGC GTGAGCCTAG CGGGCAACGT GCTGTTCGCG 120

CTGCTGATCG TGCGGGAGCG CAGCCTGCAC CGCGCCCCGT ACTACCTGCT GCTCGACCTG 180

TGCCTGGCCG ACGGGCTGCG CGCGCTCGCC TGCCTCCCGG CCGTCATGCT GGCGGCGCGG 240

CGTGCGGCGG CCGCGGCGGG GGCGCCGCCG GGCGCGCTGG GCTGCAAGCT GCTCGCCTTC 300

CTGGCCGCGC TCTTCTGCTT CCACGCCGCC TTCCTGCTGC TGGGCGTGGG CGTCACCCGC 360

TACCTGGCCA TCGCGCACCA CCGCTTCTAT GCAGAGCGCC TGGCCGGCTG GCCGTGCGCC 420

GCCATGCTGG TGTGCGCCGC CTGGGCGCTG GCGCTGGCCG CGGCCTTCCC GCCAGTGCTG 480

GACGGCGGTG GCGACGACGA GGACGCGCCG TGCGCCCTGG AGCAGCGGCC CGACGGCGCC 540

CCCGGCGCGC TGGGCTTCCT GCTGCTGCTG GCCGTGGTGG TGGGCGCCAC GCACCTCGTC 600

TACCTCCGCC TGCTCTTCTT CATCCACGAC CGCCGCAAGA TGCGGCCCGC GCGCCTGGTG 660

CCCGCCGTCA GCCACGACTG GACCTTCCAC GGCCCGGGCG CCACCGGCCA GGCGGCCGCC 720

AACTGGACGG CGGGCTTCGG CCGCGGGCCC ACGCCGCCCG CGCTTGTGGG CATCCGGCCC 780

GCAGGGCCGG GCCGCGGCGC GCGCCGCCTC CTCGTGCTGG AAGAATTCAA GACGGAGAAG 840

AGGCTGTGCA AGATGTTCTA CGCCGTCACG CTGCTCTTCC TGCTCCTCTG GGGGCCCTAC 900

GTCGTGGCCA GCTACCTGCG GGTCCTGGTG CGGCCCGGCG CCGTCCCCCA GGCCTACCTG 960

ACGGCCTCCG TGTGGCTGAC CTTCGCGCAG GCCGGCATCA ACCCCGTCGT GTGCTTCCTC 1020

TTCAACAGGG AGCTGAGGGA CTGCTTCAGG GCCCAGTTCC CCTGCTGCCA GAGCCCCCGG 1080

ACCACCCAGG CGACCCATCC CTGCGACCTG AAAGGCATTG GTTTATGA 1128

(19) INFORMATION FOR SEQ ID NO:18:

(i) SEQUENCE CHARACTERISTICS:
(A) LENGTH: 375 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:18:

Met Ala Asn Ala Ser Glu Pro Gly Gly Ser Gly Gly Gly Glu Ala Ala
1               5                   10                  15

Ala Leu Gly Leu Lys Leu Ala Thr Leu Ser Leu Leu Leu Cys Val Ser
            20                  25                  30

Leu Ala Gly Asn Val Leu Phe Ala Leu Leu Ile Val Arg Glu Arg Ser
        35                  40                  45

Leu His Arg Ala Pro Tyr Tyr Leu Leu Leu Asp Leu Cys Leu Ala Asp
    50                  55                  60

Gly Leu Arg Ala Leu Ala Cys Leu Pro Ala Val Met Leu Ala Ala Arg
65                  70                  75                  80

Arg Ala Ala Ala Ala Ala Gly Ala Pro Pro Gly Ala Leu Gly Cys Lys
                85                  90                  95

Leu Leu Ala Phe Leu Ala Ala Leu Phe Cys Phe His Ala Ala Phe Leu
            100                 105                 110

Leu Leu Gly Val Gly Val Thr Arg Tyr Leu Ala Ile Ala His His Arg
        115                 120                 125

Phe Tyr Ala Glu Arg Leu Ala Gly Trp Pro Cys Ala Ala Met Leu Val

130                 135                 140

Cys Ala Ala Trp Ala Leu Ala Leu Ala Ala Ala Phe Pro Pro Val Leu
145                 150                 155                 160

Asp Gly Gly Gly Asp Asp Glu Asp Ala Pro Cys Ala Leu Glu Gln Arg
                165                 170                 175

Pro Asp Gly Ala Pro Gly Ala Leu Gly Phe Leu Leu Leu Leu Ala Val
            180                 185                 190

Val Val Gly Ala Thr His Leu Val Tyr Leu Arg Leu Leu Phe Phe Ile
        195                 200                 205

His Asp Arg Arg Lys Met Arg Pro Ala Arg Leu Val Pro Ala Val Ser
    210                 215                 220

His Asp Trp Thr Phe His Gly Pro Gly Ala Thr Gly Gln Ala Ala Ala
225                 230                 235                 240

Asn Trp Thr Ala Gly Phe Gly Arg Gly Pro Thr Pro Pro Ala Leu Val
                245                 250                 255

Gly Ile Arg Pro Ala Gly Pro Gly Arg Gly Ala Arg Arg Leu Leu Val
            260                 265                 270

Leu Glu Glu Phe Lys Thr Glu Lys Arg Leu Cys Lys Met Phe Tyr Ala
        275                 280                 285

Val Thr Leu Leu Phe Leu Leu Leu Trp Gly Pro Tyr Val Val Ala Ser
    290                 295                 300

Tyr Leu Arg Val Leu Val Arg Pro Gly Ala Val Pro Gln Ala Tyr Leu
305                 310                 315                 320

Thr Ala Ser Val Trp Leu Thr Phe Ala Gln Ala Gly Ile Asn Pro Val
                325                 330                 335

Val Cys Phe Leu Phe Asn Arg Glu Leu Arg Asp Cys Phe Arg Ala Gln
            340                 345                 350

Phe Pro Cys Cys Gln Ser Pro Arg Thr Thr Gln Ala Thr His Pro Cys
        355                 360                 365

Asp Leu Lys Gly Ile Gly Leu
    370                 375

(20) INFORMATION FOR SEQ ID NO:19:

(i) SEQUENCE CHARACTERISTICS:
(A) LENGTH: 1002 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

ATGAACACCA CAGTGATGCA AGGCTTCAAC AGATCTGAGC GGTGCCCCAG AGACACTCGG     60

ATAGTACAGC TGGTATTCCC AGCCCTCTAC ACAGTGGTTT TCTTGACCGG CATCCTGCTG 120

AATACTTTGG CTCTGTGGGT GTTTGTTCAC ATCCCCAGCT CCTCCACCTT CATCATCTAC 180

CTCAAAAACA CTTTGGTGGC CGACTTGATA ATGACACTCA TGCTTCCTTT CAAAATCCTC 240

TCTGACTCAC ACCTGGCACC CTGGCAGCTC AGAGCTTTTG TGTGTCGTTT TTCTTCGGTG 300

ATATTTTATG AGACCATGTA TGTGGGCATC GTGCTGTTAG GGCTCATAGC CTTTGACAGA 360

TTCCTCAAGA TCATCAGACC TTTGAGAAAT ATTTTTCTAA AAAAACCTGT TTTTGCAAAA 420

ACGGTCTCAA TCTTCATCTG GTTCTTTTTG TTCTTCATCT CCCTGCCAAA TACGATCTTG 480

AGCAACAAGG AAGCAACACC ATCGTCTGTG AAAAAGTGTG CTTCCTTAAA GGGGCCTCTG 540

GGGCTGAAAT GGCATCAAAT GGTAAATAAC ATATGCCAGT TTATTTTCTG GACTGTTTTT 600

ATCCTAATGC TTGTGTTTTA TGTGGTTATT GCAAAAAAAG TATATGATTC TTATAGAAAG 660

TCCAAAAGTA AGGACAGAAA AAACAACAAA AAGCTGGAAG GCAAAGTATT TGTTGTCGTG 720

GCTGTCTTCT TTGTGTGTTT TGCTCCATTT CATTTTGCCA GAGTTCCATA TACTCACAGT 780

CAAACCAACA ATAAGACTGA CTGTAGACTG CAAAATCAAC TGTTTATTGC TAAAGAAACA 840

ACTCTCTTTT TGGCAGCAAC TAACATTTGT ATGGATCCCT TAATATACAT ATTCTTATGT 900

AAAAAATTCA CAGAAAAGCT ACCATGTATG CAAGGGAGAA AGACCACAGC ATCAAGCCAA 960

GAAAATCATA GCAGTCAGAC AGACAACATA ACCTTAGGCT GA 1002

(21) INFORMATION FOR SEQ ID NO:20:

(i) SEQUENCE CHARACTERISTICS:
(A) LENGTH: 333 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:20:

Met Asn Thr Thr Val Met Gln Gly Phe Asn Arg Ser Glu Arg Cys Pro
1               5                   10                  15

Arg Asp Thr Arg Ile Val Gln Leu Val Phe Pro Ala Leu Tyr Thr Val
            20                  25                  30

Val Phe Leu Thr Gly Ile Leu Leu Asn Thr Leu Ala Leu Trp Val Phe
        35                  40                  45

Val His Ile Pro Ser Ser Ser Thr Phe Ile Ile Tyr Leu Lys Asn Thr
    50                  55                  60

Leu Val Ala Asp Leu Ile Met Thr Leu Met Leu Pro Phe Lys Ile Leu
65                  70                  75                  80

Ser Asp Ser His Leu Ala Pro Trp Gln Leu Arg Ala Phe Val Cys Arg
                85                  90                  95

Phe Ser Ser Val Ile Phe Tyr Glu Thr Met Tyr Val Gly Ile Val Leu
            100                 105                 110

Leu Gly Leu Ile Ala Phe Asp Arg Phe Leu Lys Ile Ile Arg Pro Leu
        115                 120                 125

Arg Asn Ile Phe Leu Lys Lys Pro Val Phe Ala Lys Thr Val Ser Ile
    130                 135                 140

Phe Ile Trp Phe Phe Leu Phe Phe Ile Ser Leu Pro Asn Thr Ile Leu
145                 150                 155                 160

Ser Asn Lys Glu Ala Thr Pro Ser Ser Val Lys Lys Cys Ala Ser Leu
                165                 170                 175

Lys Gly Pro Leu Gly Leu Lys Trp His Gln Met Val Asn Asn Ile Cys
            180                 185                 190

Gln Phe Ile Phe Trp Thr Val Phe Ile Leu Met Leu Val Phe Tyr Val
        195                 200                 205

Val Ile Ala Lys Lys Val Tyr Asp Ser Tyr Arg Lys Ser Lys Ser Lys
    210                 215                 220

Asp Arg Lys Asn Asn Lys Lys Leu Glu Gly Lys Val Phe Val Val Val
225                 230                 235                 240

Ala Val Phe Phe Val Cys Phe Ala Pro Phe His Phe Ala Arg Val Pro
                245                 250                 255

Tyr Thr His Ser Gln Thr Asn Asn Lys Thr Asp Cys Arg Leu Gln Asn
            260                 265                 270

Gln Leu Phe Ile Ala Lys Glu Thr Thr Leu Phe Leu Ala Ala Thr Asn
        275                 280                 285

Ile Cys Met Asp Pro Leu Ile Tyr Ile Phe Leu Cys Lys Lys Phe Thr
    290                 295                 300

Glu Lys Leu Pro Cys Met Gln Gly Arg Lys Thr Thr Ala Ser Ser Gln
305                 310                 315                 320

Glu Asn His Ser Ser Gln Thr Asp Asn Ile Thr Leu Gly
                325                 330

(22) INFORMATION FOR SEQ ID NO:21:

     (i) SEQUENCE CHARACTERISTICS:
          (A) LENGTH: 1122 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:21:

ATGGCCAACA CTACCGGAGA GCCTGAGGAG GTGAGCGGCG CTCTGTCCCC ACCGTCCGCA 60

TCAGCTTATG TGAAGCTGGT ACTGCTGGGA CTGATTATGT GCGTGAGCCT GGCGGGTAAC 120

GCCATCTTGT CCCTGCTGGT GCTCAAGGAG CGTGCCCTGC ACAAGGCTCC TTACTACTTC 180

CTGCTGGACC TGTGCCTGGC CGATGGCATA CGCTCTGCCG TCTGCTTCCC CTTTGTGCTG 240

GCTTCTGTGC GCCACGGCTC TTCATGGACC TTCAGTGCAC TCAGCTGCAA GATTGTGGCC 300

TTTATGGCCG TGCTCTTTTG CTTCCATGCG GCCTTCATGC TGTTCTGCAT CAGCGTCACC 360

CGCTACATGG CCATCGCCCA CCACCGCTTC TACGCCAAGC GCATGACACT CTGGACATGC 420

GCGGCTGTCA TCTGCATGGC CTGGACCCTG TCTGTGGCCA TGGCCTTCCC ACCTGTCTTT 480

GACGTGGGCA CCTACAAGTT TATTCGGGAG GAGGACCAGT GCATCTTTGA GCATCGCTAC 540

TTCAAGGCCA ATGACACGCT GGGCTTCATG CTTATGTTGG CTGTGCTCAT GGCAGCTACC 600

CATGCTGTCT ACGGCAAGCT GCTCCTCTTC GAGTATCGTC ACCGCAAGAT GAAGCCAGTG 660

CAGATGGTGC CAGCCATCAG CCAGAACTGG ACATTCCATG GTCCCGGGGC CACCGGCCAG 720

GCTGCTGCCA ACTGGATCGC CGGCTTTGGC CGTGGGCCCA TGCCACCAAC CCTGCTGGGT 780

ATCCGGCAGA ATGGGCATGC AGCCAGCCGG CGGCTACTGG GCATGGACGA GGTCAAGGGT 840

GAAAAGCAGC TGGGCCGCAT GTTCTACGCG ATCACACTGC TCTTTCTGCT CCTCTGGTCA 900

CCCTACATCG TGGCCTGCTA CTGGCGAGTG TTTGTGAAAG CCTGTGCTGT GCCCCACCGC 960

TACCTGGCCA CTGCTGTTTG GATGAGCTTC GCCCAGGCTG CCGTCAACCC AATTGTCTGC 1020

TTCCTGCTCA ACAAGGACCT CAAGAAGTGC CTGACCACTC ACGCCCCCTG CTGGGGCACA 1080

GGAGGTGCCC CGGCTCCCAG AGAACCCTAC TGTGTCATGT GA 1122

(23) INFORMATION FOR SEQ ID NO:22:

(i) SEQUENCE CHARACTERISTICS:
(A) LENGTH: 373 amino acids
(B) TYPE: amino acid
(C) STRANDEDNESS:
(D) TOPOLOGY: not relevant

(ii) MOLECULE TYPE: DNA (genomic)

(xi) SEQUENCE DESCRIPTION: SEQ ID NO:22:

Met Ala Asn Thr Thr Gly Glu Pro Glu Glu Val Ser Gly Ala Leu Ser
1               5                   10                  15

Pro Pro Ser Ala Ser Ala Tyr Val Lys Leu Val Leu Leu Gly Leu Ile
            20                  25                  30

Met Cys Val Ser Leu Ala Gly Asn Ala Ile Leu Ser Leu Leu Val Leu
        35                  40                  45

Lys Glu Arg Ala Leu His Lys Ala Pro Tyr Tyr Phe Leu Leu Asp Leu

    50                  55                  60

Cys Leu Ala Asp Gly Ile Arg Ser Ala Val Cys Phe Pro Phe Val Leu
65                  70                  75                  80

Ala Ser Val Arg His Gly Ser Ser Trp Thr Phe Ser Ala Leu Ser Cys
                85                  90                  95

Lys Ile Val Ala Phe Met Ala Val Leu Phe Cys Phe His Ala Ala Phe
            100                 105                 110

Met Leu Phe Cys Ile Ser Val Thr Arg Tyr Met Ala Ile Ala His His
        115                 120                 125

Arg Phe Tyr Ala Lys Arg Met Thr Leu Trp Thr Cys Ala Ala Val Ile
    130                 135                 140

Cys Met Ala Trp Thr Leu Ser Val Ala Met Ala Phe Pro Pro Val Phe
145                 150                 155                 160

Asp Val Gly Thr Tyr Lys Phe Ile Arg Glu Glu Asp Gln Cys Ile Phe
                165                 170                 175

Glu His Arg Tyr Phe Lys Ala Asn Asp Thr Leu Gly Phe Met Leu Met
            180                 185                 190

Leu Ala Val Leu Met Ala Ala Thr His Ala Val Tyr Gly Lys Leu Leu
        195                 200                 205

Leu Phe Glu Tyr Arg His Arg Lys Met Lys Pro Val Gln Met Val Pro
    210                 215                 220

Ala Ile Ser Gln Asn Trp Thr Phe His Gly Pro Gly Ala Thr Gly Gln
225                 230                 235                 240

Ala Ala Ala Asn Trp Ile Ala Gly Phe Gly Arg Gly Pro Met Pro Pro
                245                 250                 255

Thr Leu Leu Gly Ile Arg Gln Asn Gly His Ala Ala Ser Arg Arg Leu
            260                 265                 270

Leu Gly Met Asp Glu Val Lys Gly Glu Lys Gln Leu Gly Arg Met Phe
        275                 280                 285

Tyr Ala Ile Thr Leu Leu Phe Leu Leu Leu Trp Ser Pro Tyr Ile Val
    290                 295                 300

Ala Cys Tyr Trp Arg Val Phe Val Lys Ala Cys Ala Val Pro His Arg
305                 310                 315                 320

Tyr Leu Ala Thr Ala Val Trp Met Ser Phe Ala Gln Ala Ala Val Asn
                325                 330                 335

Pro Ile Val Cys Phe Leu Leu Asn Lys Asp Leu Lys Lys Cys Leu Thr
            340                 345                 350

Thr His Ala Pro Cys Trp Gly Thr Gly Gly Ala Pro Ala Pro Arg Glu
        355                 360                 365

Pro Tyr Cys Val Met
    370

(24) INFORMATION FOR SEQ ID NO:23:

(i) SEQUENCE CHARACTERISTICS:
(A) LENGTH: 1053 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:23:

ATGGCTTTGG AACAGAACCA GTCAACAGAT TATTATTATG AGGAAAATGA AATGAATGGC 60

ACTTATGACT ACAGTCAATA TGAATTGATC TGTATCAAAG AAGATGTCAG AGAATTTGCA 120

AAAGTTTTCC TCCCTGTATT CCTCACAATA GCTTTCGTCA TTGGACTTGC AGGCAATTCC 180

ATGGTAGTGG CAATTTATGC CTATTACAAG AAACAGAGAA CCAAAACAGA TGTGTACATC 240

CTGAATTTGG CTGTAGCAGA TTTACTCCTT CTATTCACTC TGCCTTTTTG GGCTGTTAAT 300

GCAGTTCATG GGTGGGTTTT AGGGAAAATA ATGTGCAAAA TAACTTCAGC CTTGTACACA 360

CTAAACTTTG TCTCTGGAAT GCAGTTTCTG GCTTGCATCA GCATAGACAG ATATGTGGCA 420

GTAACTAATG TCCCCAGCCA ATCAGGAGTG GGAAAACCAT GCTGGATCAT CTGTTTCTGT 480

GTCTGGATGG CTGCCATCTT GCTGAGCATA CCCCAGCTGG TTTTTTATAC AGTAAATGAC 540

AATGCTAGGT GCATTCCCAT TTTCCCCCGC TACCTAGGAA CATCAATGAA AGCATTGATT 600

CAAATGCTAG AGATCTGCAT TGGATTTGTA GTACCCTTTC TTATTATGGG GGTGTGCTAC 660

TTTATCACGG CAAGGACACT CATGAAGATG CCAAACATTA AAATATCTCG ACCCCTAAAA 720

GTTCTGCTCA CAGTCGTTAT AGTTTTCATT GTCACTCAAC TGCCTTATAA CATTGTCAAG 780

TTCTGCCGAG CCATAGACAT CATCTACTCC CTGATCACCA GCTGCAACAT GAGCAAACGC 840

ATGGACATCG CCATCCAAGT CACAGAAAGC ATTGCACTCT TTCACAGCTG CCTCAACCCA 900

ATCCTTTATG TTTTTATGGG AGCATCTTTC AAAAACTACG TTATGAAAGT GGCCAAGAAA 960

TATGGGTCCT GGAGAAGACA GAGACAAAGT GTGGAGGAGT TTCCTTTTGA TTCTGAGGGT 1020

CCTACAGAGC CAACCAGTAC TTTTAGCATT TAA 1053

(25) INFORMATION FOR SEQ ID NO:24:

(i) SEQUENCE CHARACTERISTICS:
(A) LENGTH: 350 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:24:

(26) INFORMATION FOR SEQ ID NO:25:

(i) SEQUENCE CHARACTERISTICS:
(A) LENGTH: 1116 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:25:

ATGCCAGGAA ACGCCACCCC AGTGACCACC ACTGCCCCGT GGGCCTCCCT GGGCCTCTCC 60

GCCAAGACCT GCAACAACGT GTCCTTCGAA GAGAGCAGGA TAGTCCTGGT CGTGGTGTAC 120

AGCGCGGTGT GCACGCTGGG GGTGCCGGCC AACTGCCTGA CTGCGTGGCT GGCGCTGCTG 180

CAGGTACTGC AGGGCAACGT GCTGGCCGTC TACCTGCTCT GCCTGGCACT CTGCGAACTG 240

CTGTACACAG GCACGCTGCC ACTCTGGGTC ATCTATATCC GCAACCAGCA CCGCTGGACC 300

CTAGGCCTGC TGGCCTCGAA GGTGACCGCC TACATCTTCT TCTGCAACAT CTACGTCAGC 360

ATCCTCTTCC TGTGCTGCAT CTCCTGCGAC CGCTTCGTGG CCGTGGTGTA CGCGCTGGAG 420

AGTCGGGGCC GCCGCCGCCG GAGGACCGCC ATCCTCATCT CCGCCTGCAT CTTCATCCTC 480

GTCGGGATCG TTCACTACCC GGTGTTCCAG ACGGAAGACA AGGAGACCTG CTTTGACATG 540

CTGCAGATGG ACAGCAGGAT TGCCGGGTAC TACTACGCCA GGTTCACCGT TGGCTTTGCC 600

ATCCCTCTCT CCATCATCGC CTTCACCAAC CACCGGATTT TCAGGAGCAT CAAGCAGAGC 660

ATGGGCTTAA GCGCTGCCCA GAAGGCCAAG GTGAAGCACT CGGCCATCGC GGTGGTTGTC 720

ATCTTCCTAG TCTGCTTCGC CCCGTACCAC CTGGTTCTCC TCGTCAAAGC CGCTGCCTTT 780

TCCTACTACA GAGGAGACAG GAACGCCATG TGCGGCTTGG AGGAAAGGCT GTACACAGCC 840

TCTGTGGTGT TTCTGTGCCT GTCCACGGTG AACGGCGTGG CTGACCCCAT TATCTACGTG 900

CTGGCCACGG ACCATTCCCG CCAAGAAGTG TCCAGAATCC ATAAGGGGTG GAAAGAGTGG 960

TCCATGAAGA CAGACGTCAC CAGGCTCACC CACAGCAGGG ACACCGAGGA GCTGCAGTCG 1020

CCCGTGGCCC TTGCAGACCA CTACACCTTC TCCAGGCCCG TGCACCCACC AGGGTCACCA 1080

TGCCCTGCAA AGAGGCTGAT TGAGGAGTCC TGCTGA 1116

(28) INFORMATION FOR SEQ ID NO:26:

(i) SEQUENCE CHARACTERISTICS:

(A) LENGTH: 371 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:26:

Met Pro Gly Asn Ala Thr Pro Val Thr Thr Thr Ala Pro Trp Ala Ser
1               5                   10                  15

Leu Gly Leu Ser Ala Lys Thr Cys Asn Asn Val Ser Phe Glu Glu Ser
            20                  25                  30

Arg Ile Val Leu Val Val Val Tyr Ser Ala Val Cys Thr Leu Gly Val
        35                  40                  45

Pro Ala Asn Cys Leu Thr Ala Trp Leu Ala Leu Leu Gln Val Leu Gln
    50                  55                  60

Gly Asn Val Leu Ala Val Tyr Leu Leu Cys Leu Ala Leu Cys Glu Leu
65                  70                  75                  80

Leu Tyr Thr Gly Thr Leu Pro Leu Trp Val Ile Tyr Ile Arg Asn Gln
                85                  90                  95

His Arg Trp Thr Leu Gly Leu Leu Ala Ser Lys Val Thr Ala Tyr Ile
            100                 105                 110

Phe Phe Cys Asn Ile Tyr Val Ser Ile Leu Phe Leu Cys Cys Ile Ser
        115                 120                 125

Cys Asp Arg Phe Val Ala Val Val Tyr Ala Leu Glu Ser Arg Gly Arg
    130                 135                 140

Arg Arg Arg Arg Thr Ala Ile Leu Ile Ser Ala Cys Ile Phe Ile Leu
145                 150                 155                 160

Val Gly Ile Val His Tyr Pro Val Phe Gln Thr Glu Asp Lys Glu Thr
                165                 170                 175

Cys Phe Asp Met Leu Gln Met Asp Ser Arg Ile Ala Gly Tyr Tyr Tyr
            180                 185                 190

Ala Arg Phe Thr Val Gly Phe Ala Ile Pro Leu Ser Ile Ile Ala Phe
        195                 200                 205

Thr Asn His Arg Ile Phe Arg Ser Ile Lys Gln Ser Met Gly Leu Ser
    210                 215                 220

Ala Ala Gln Lys Ala Lys Val Lys His Ser Ala Ile Ala Val Val Val
225                 230                 235                 240

Ile Phe Leu Val Cys Phe Ala Pro Tyr His Leu Val Leu Leu Val Lys
                245                 250                 255

Ala Ala Ala Phe Ser Tyr Tyr Arg Gly Asp Arg Asn Ala Met Cys Gly
            260                 265                 270

Leu Glu Glu Arg Leu Tyr Thr Ala Ser Val Val Phe Leu Cys Leu Ser
        275                 280                 285

Thr Val Asn Gly Val Ala Asp Pro Ile Ile Tyr Val Leu Ala Thr Asp
    290                 295                 300

His Ser Arg Gln Glu Val Ser Arg Ile His Lys Gly Trp Lys Glu Trp
305                 310                 315                 320

Ser Met Lys Thr Asp Val Thr Arg Leu Thr His Ser Arg Asp Thr Glu
                325                 330                 335

Glu Leu Gln Ser Pro Val Ala Leu Ala Asp His Tyr Thr Phe Ser Arg
            340                 345                 350

Pro Val His Pro Pro Gly Ser Pro Cys Pro Ala Lys Arg Leu Ile Glu
        355                 360                 365

Glu Ser Cys
    370

(28) INFORMATION FOR SEQ ID NO:27:

(i) SEQUENCE CHARACTERISTICS:
(A) LENGTH: 1113 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:27:

ATGGCGAACT ATAGCCATGC AGCTGACAAC ATTTTGCAAA ATCTCTCGCC TCTAACAGCC 60

TTTCTGAAAC TGACTTCCTT GGGTTTCATA ATAGGAGTCA GCGTGGTGGG CAACCTCCTG 120

ATCTCCATTT TGCTAGTGAA AGATAAGACC TTGCATAGAG CACCTTACTA CTTCCTGTTG 180

GATCTTTGCT GTTCAGATAT CCTCAGATCT GCAATTTGTT TCCCATTTGT GTTCAACTCT 240

GTCAAAAATG GCTCTACCTG GACTTATGGG ACTCTGACTT GCAAAGTGAT TGCCTTTCTG 300

GGGGTTTTGT CCTGTTTCCA CACTGCTTTC ATGCTCTTCT GCATCAGTGT CACCAGATAC 360

TTAGCTATCG CCCATCACCG CTTCTATACA AAGAGGCTGA CCTTTTGGAC GTGTCTGGCT 420

GTGATCTGTA TGGTGTGGAC TCTGTCTGTG GCCATGGCAT TTCCCCCGGT TTTAGACGTG 480

GGCACTTACT CATTCATTAG GGAGGAAGAT CAATGCACCT TCCAACACCG CTCCTTCAGG 540

GCTAATGATT CCTTAGGATT TATGCTGCTT CTTGCTCTCA TCCTCCTAGC CACACAGCTT 600

GTCTACCTCA AGCTGATATT TTTCGTCCAC GATCGAAGAA AAATGAAGCC AGTCCAGTTT 660

GTAGCAGCAG TCAGCCAGAA CTGGACTTTT CATGGTCCTG GAGCCAGTGG CCAGGCAGCT 720

GCCAATTGGC TAGCAGGATT TGGAAGGGGT CCCACACCAC CCACCTTGCT GGGCATCAGG 780

CAAAATGCAA ACACCACAGG CAGAAGAAGG CTATTGGTCT TAGACGAGTT CAAAATGGAG 840

AAAAGAATCA GCAGAATGTT CTATATAATG ACTTTTCTGT TTCTAACCTT GTGGGGCCCC 900

TACCTGGTGG CCTGTTATTG GAGAGTTTTT GCAAGAGGGC CTGTAGTACC AGGGGGATTT 960

CTAACAGCTG CTGTCTGGAT GAGTTTTGCC CAAGCAGGAA TCAATCCTTT TGTCTGCATT 1020

TTCTCAAACA GGGAGCTGAG GCGCTGTTTC AGCACAACCC TTCTTTACTG CAGAAAATCC 1080

AGGTTACCAA GGGAACCTTA CTGTGTTATA TGA 1113

(29) INFORMATION FOR SEQ ID NO:28:

(i) SEQUENCE CHARACTERISTICS:
(A) LENGTH: 370 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:28:

Met Ala Asn Tyr Ser His Ala Ala Asp Asn Ile Leu Gln Asn Leu Ser
1               5                   10                  15

Pro Leu Thr Ala Phe Leu Lys Leu Thr Ser Leu Gly Phe Ile Ile Gly
            20                  25                  30

Val Ser Val Val Gly Asn Leu Leu Ile Ser Ile Leu Leu Val Lys Asp
        35                  40                  45

Lys Thr Leu His Arg Ala Pro Tyr Tyr Phe Leu Leu Asp Leu Cys Cys
    50                  55                  60

Ser Asp Ile Leu Arg Ser Ala Ile Cys Phe Pro Phe Val Phe Asn Ser
65                  70                  75                  80

Val Lys Asn Gly Ser Thr Trp Thr Tyr Gly Thr Leu Thr Cys Lys Val
                85                  90                  95

Ile Ala Phe Leu Gly Val Leu Ser Cys Phe His Thr Ala Phe Met Leu
            100                 105                 110

Phe Cys Ile Ser Val Thr Arg Tyr Leu Ala Ile Ala His His Arg Phe
        115                 120                 125

Tyr Thr Lys Arg Leu Thr Phe Trp Thr Cys Leu Ala Val Ile Cys Met
    130                 135                 140

Val Trp Thr Leu Ser Val Ala Met Ala Phe Pro Pro Val Leu Asp Val
145                 150                 155                 160

Gly Thr Tyr Ser Phe Ile Arg Glu Glu Asp Gln Cys Thr Phe Gln His
                165                 170                 175

Arg Ser Phe Arg Ala Asn Asp Ser Leu Gly Phe Met Leu Leu Leu Ala
            180                 185                 190

Leu Ile Leu Leu Ala Thr Gln Leu Val Tyr Leu Lys Leu Ile Phe Phe
        195                 200                 205

Val His Asp Arg Arg Lys Met Lys Pro Val Gln Phe Val Ala Ala Val
    210                 215                 220

Ser Gln Asn Trp Thr Phe His Gly Pro Gly Ala Ser Gly Gln Ala Ala
225                 230                 235                 240

Ala Asn Trp Leu Ala Gly Phe Gly Arg Gly Pro Thr Pro Pro Thr Leu
                245                 250                 255

Leu Gly Ile Arg Gln Asn Ala Asn Thr Thr Gly Arg Arg Arg Leu Leu
            260                 265                 270

Val Leu Asp Glu Phe Lys Met Glu Lys Arg Ile Ser Arg Met Phe Tyr
        275                 280                 285

Ile Met Thr Phe Leu Phe Leu Thr Leu Trp Gly Pro Tyr Leu Val Ala
    290                 295                 300

Cys Tyr Trp Arg Val Phe Ala Arg Gly Pro Val Val Pro Gly Gly Phe
305                 310                 315                 320

Leu Thr Ala Ala Val Trp Met Ser Phe Ala Gln Ala Gly Ile Asn Pro
                325                 330                 335

Phe Val Cys Ile Phe Ser Asn Arg Glu Leu Arg Arg Cys Phe Ser Thr
            340                 345                 350

Thr Leu Leu Tyr Cys Arg Lys Ser Arg Leu Pro Arg Glu Pro Tyr Cys
        355                 360                 365

Val Ile
    370

(30) INFORMATION FOR SEQ ID NO:29:

(i) SEQUENCE CHARACTERISTICS:
(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:29:

ATGCAGGTCC CGAACAGCAC CGGCCCGGAC AACGCGACGC TGCAGATGCT GCGGAACCCG 60

GCGATCGCGG TGGCCCTGCC CGTGGTGTAC TCGCTGGTGG CGGCGGTCAG CATCCCGGGC 120

AACCTCTTCT CTCTGTGGGT GCTGTGCCGG CGCATGGGGC CCAGATCCCC GTCGGTCATC 180

TTCATGATCA ACCTGAGCGT CACGGACCTG ATGCTGGCCA GCGTGTTGCC TTTCCAAATC 240

TACTACCATT GCAACCGCCA CCACTGGGTA TTCGGGGTGC TGCTTTGCAA CGTGGTGACC 300

GTGGCCTTTT ACGCAAACAT GTATTCCAGC ATCCTCACCA TGACCTGTAT CAGCGTGGAG 360

CGCTTCCTGG GGGTCCTGTA CCCGCTCAGC TCCAAGCGCT GGCGCCGCCG TCGTTACGCG 420

GTGGCCGCGT GTGCAGGGAC CTGGCTGCTG CTCCTGACCG CCCTGTGCCC GCTGGCGCGC 480

ACCGATCTCA CCTACCCGGT GCACGCCCTG GGCATCATCA CCTGCTTCGA CGTCCTCAAG 540

TGGACGATGC TCCCCAGCGT GGCCATGTGG GCCGTGTTCC TCTTCACCAT CTTCATCCTG 600

CTGTTCCTCA TCCCGTTCGT GATCACCGTG GCTTGTTACA CGGCCACCAT CCTCAAGCTG 660

TTGCGCACGG AGGAGGCGCA CGGCCGGGAG CAGCGGAGGC GCGCGGTGGG CCTGGCCGCG 720

GTGGTCTTGC TGGCCTTTGT CACCTGCTTC GCCCCCAACA ACTTCGTGCT CCTGGCGCAC 780

ATCGTGAGCC GCCTGTTCTA CGGCAAGAGC TACTACCACG TGTACAAGCT CACGCTGTGT 840

CTCAGCTGCC TCAACAACTG TCTGGACCCG TTTGTTTATT ACTTTGCGTC CCGGGAATTC 900

CAGCTGCGCC TGCGGGAATA TTTGGGCTGC CGCCGGGTGC CCAGAGACAC CCTGGACACG 960

CGCCGCGAGA GCCTCTTCTC CGCCAGGACC ACGTCCGTGC GCTCCGAGGC CGGTGCGCAC 1020

CCTGAAGGGA TGGAGGGAGC CACCAGGCCC GGCCTCCAGA GGCAGGAGAG TGTGTTCTGA 1080

(31) INFORMATION FOR SEQ ID NO:30:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:30:

Met Gln Val Pro Asn Ser Thr Gly Pro Asp Asn Ala Thr Leu Gln Met
1               5                   10                  15

Leu Arg Asn Pro Ala Ile Ala Val Ala Leu Pro Val Val Tyr Ser Leu
            20                  25                  30

Val Ala Ala Val Ser Ile Pro Gly Asn Leu Phe Ser Leu Trp Val Leu
        35                  40                  45

Cys Arg Arg Met Gly Pro Arg Ser Pro Ser Val Ile Phe Met Ile Asn
    50                  55                  60

Leu Ser Val Thr Asp Leu Met Leu Ala Ser Val Leu Pro Phe Gln Ile
65                  70                  75                  80

Tyr Tyr His Cys Asn Arg His His Trp Val Phe Gly Val Leu Leu Cys
                85                  90                  95

Asn Val Val Thr Val Ala Phe Tyr Ala Asn Met Tyr Ser Ser Ile Leu
            100                 105                 110

Thr Met Thr Cys Ile Ser Val Glu Arg Phe Leu Gly Val Leu Tyr Pro

115                 120                 125

Leu Ser Ser Lys Arg Trp Arg Arg Arg Arg Tyr Ala Val Ala Ala Cys
    130                 135                 140

Ala Gly Thr Trp Leu Leu Leu Leu Thr Ala Leu Cys Pro Leu Ala Arg
145                 150                 155                 160

Thr Asp Leu Thr Tyr Pro Val His Ala Leu Gly Ile Ile Thr Cys Phe
                165                 170                 175

Asp Val Leu Lys Trp Thr Met Leu Pro Ser Val Ala Met Trp Ala Val
            180                 185                 190

Phe Leu Phe Thr Ile Phe Ile Leu Leu Phe Leu Ile Pro Phe Val Ile
        195                 200                 205

Thr Val Ala Cys Tyr Thr Ala Thr Ile Leu Lys Leu Leu Arg Thr Glu
    210                 215                 220

Glu Ala His Gly Arg Glu Gln Arg Arg Arg Ala Val Gly Leu Ala Ala
225                 230                 235                 240

Val Val Leu Leu Ala Phe Val Thr Cys Phe Ala Pro Asn Asn Phe Val
                245                 250                 255

Leu Leu Ala His Ile Val Ser Arg Leu Phe Tyr Gly Lys Ser Tyr Tyr
            260                 265                 270

His Val Tyr Lys Leu Thr Leu Cys Leu Ser Cys Leu Asn Asn Cys Leu
        275                 280                 285

Asp Pro Phe Val Tyr Tyr Phe Ala Ser Arg Glu Phe Gln Leu Arg Leu
    290                 295                 300

Arg Glu Tyr Leu Gly Cys Arg Arg Val Pro Arg Asp Thr Leu Asp Thr
305                 310                 315                 320

Arg Arg Glu Ser Leu Phe Ser Ala Arg Thr Thr Ser Val Arg Ser Glu
                325                 330                 335

Ala Gly Ala His Pro Glu Gly Met Glu Gly Ala Thr Arg Pro Gly Leu
            340                 345                 350

Gln Arg Gln Glu Ser Val Phe
        355

(32) INFORMATION FOR SEQ ID NO:31:

(i) SEQUENCE CHARACTERISTICS:
(A) LENGTH: 1503 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

ATGGAGCGTC CCTGGGAGGA CAGCCCAGGC CCGGAGGGGG CAGCTGAGGG CTCGCCTGTG      60

CCAGTCGCCG CCGGGGCGCG CTCCGGTGCC GCGGCGAGTG GCACAGGCTG GCAGCCATGG 120
GCTGAGTGCC CGGGACCCAA GGGGAGGGGG CAACTGCTGG CGACCGCCGG CCCTTTGCGT 180
CGCTGGCCCG CCCCCTCGCC TGCCAGCTCC AGCCCCGCCC CCGGAGCGGC GTCCGCTCAC 240
TCGGTTCAAG GCAGCGCGAC TGCGGGTGGC GCACGACCAG GGCGCAGACC TTGGGGCGCG 300
CGGCCCATGG AGTCGGGGCT GCTGCGGCCG GCGCCGGTGA GCGAGGTCAT CGTCCTGCAT 360
TACAACTACA CCGGCAAGCT CCGCGGTGCG AGCTACCAGC CGGGTGCCGG CCTGCGCGCC 420
GACGCCGTGG TGTGCCTGGC GGTGTGCGCC TTCATCGTGC TAGAGAATCT AGCCGTGTTG 480
TTGGTGCTCG GACGCCACCC GCGCTTCCAC GCTCCCATGT TCCTGCTCCT GGGCAGCCTC 540
ACGTTGTCGG ATCTGCTGGC AGGCGCCGCC TACGCCGCCA ACATCCTACT GTCGGGGCCG 600
CTCACGCTGA AACTGTCCCC CGCGCTCTGG TTCGCACGGG AGGGAGGCGT CTTCGTGGCA 660
CTCACTGCGT CCGTGCTGAG CCTCCTGGCC ATCGCGCTGG AGCGCAGCCT CACCATGGCG 720
CGCAGGGGGC CCGCGCCCGT CTCCAGTCGG GGGCGCACGC TGGCGATGGC AGCCGCGGCC 780
TGGGGCGTGT CGCTGCTCCT CGGGCTCCTG CCAGCGCTGG GCTGGAATTG CCTGGGTCGC 840
CTGGACGCTT GCTCCACTGT CTTGCCGCTC TACGCCAAGG CCTACGTGCT CTTCTGCGTG 900
CTCGCCTTCG TGGGCATCCT GGCCGCGATC TGTGCACTCT ACGCGCGCAT CTACTGCCAG 960
GTACGCGCCA ACGCGCGGCG CCTGCCGGCA CGGCCCGGGA CTGCGGGGAC CACCTCGACC 1020
CGGGCGCGTC GCAAGCCGCG CTCTCTGGCC TTGCTGCGCA CGCTCAGCGT GGTGCTCCTG 1080
GCCTTTGTGG CATGTTGGGG CCCCCTCTTC CTGCTGCTGT TGCTCGACGT GGCGTGCCCG 1140
GCGCGCACCT GTCCTGTACT CCTGCAGGCC GATCCCTTCC TGGGACTGGC CATGGCCAAC 1200
TCACTTCTGA ACCCCATCAT CTACACGCTC ACCAACCGCG ACCTGCGCCA CGCGCTCCTG 1260
CGCCTGGTCT GCTGCGGACG CCACTCCTGC GGCAGAGACC CGAGTGGCTC CCAGCAGTCG 1320
GCGAGCGCGG CTGAGGCTTC CGGGGGCCTG CGCCGCTGCC TGCCCCCGGG CCTTGATGGG 1380
AGCTTCAGCG GCTCGGAGCG CTCATCGCCC CAGCGCGACG GGCTGGACAC CAGCGGCTCC 1440
ACAGGCAGCC CCGGTGCACC CACAGCCGCC CGGACTCTGG TATCAGAACC GGCTGCAGAC 1500
TGA 1503

(33) INFORMATION FOR SEQ ID NO:32:

(i) SEQUENCE CHARACTERISTICS:
(A) LENGTH: 500 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:32:

Met Glu Arg Pro Trp Glu Asp Ser Pro Gly Pro Glu Gly Ala Ala Glu
1               5                   10                  15

Gly Ser Pro Val Pro Val Ala Ala Gly Ala Arg Ser Gly Ala Ala Ala
            20                  25                  30

Ser Gly Thr Gly Trp Gln Pro Trp Ala Glu Cys Pro Gly Pro Lys Gly
        35                  40                  45

Arg Gly Gln Leu Leu Ala Thr Ala Gly Pro Leu Arg Arg Trp Pro Ala
    50                  55                  60

Pro Ser Pro Ala Ser Ser Ser Pro Ala Pro Gly Ala Ala Ser Ala His
65                  70                  75                  80

Ser Val Gln Gly Ser Ala Thr Ala Gly Gly Ala Arg Pro Gly Arg Arg
                85                  90                  95

Pro Trp Gly Ala Arg Pro Met Glu Ser Gly Leu Leu Arg Pro Ala Pro
            100                 105                 110

Val Ser Glu Val Ile Val Leu His Tyr Asn Tyr Thr Gly Lys Leu Arg
        115                 120                 125

Gly Ala Ser Tyr Gln Pro Gly Ala Gly Leu Arg Ala Asp Ala Val Val
    130                 135                 140

Cys Leu Ala Val Cys Ala Phe Ile Val Leu Glu Asn Leu Ala Val Leu
145                 150                 155                 160

Leu Val Leu Gly Arg His Pro Arg Phe His Ala Pro Met Phe Leu Leu
                165                 170                 175

Leu Gly Ser Leu Thr Leu Ser Asp Leu Leu Ala Gly Ala Ala Tyr Ala
            180                 185                 190

Ala Asn Ile Leu Leu Ser Gly Pro Leu Thr Leu Lys Leu Ser Pro Ala
        195                 200                 205

Leu Trp Phe Ala Arg Glu Gly Gly Val Phe Val Ala Leu Thr Ala Ser
    210                 215                 220

Val Leu Ser Leu Leu Ala Ile Ala Leu Glu Arg Ser Leu Thr Met Ala
225                 230                 235                 240

Arg Arg Gly Pro Ala Pro Val Ser Ser Arg Gly Arg Thr Leu Ala Met
                245                 250                 255

Ala Ala Ala Ala Trp Gly Val Ser Leu Leu Leu Gly Leu Leu Pro Ala
            260                 265                 270

Leu Gly Trp Asn Cys Leu Gly Arg Leu Asp Ala Cys Ser Thr Val Leu
        275                 280                 285

Pro Leu Tyr Ala Lys Ala Tyr Val Leu Phe Cys Val Leu Ala Phe Val
    290                 295                 300

Gly Ile Leu Ala Ala Ile Cys Ala Leu Tyr Ala Arg Ile Tyr Cys Gln

305                 310                 315                 320

Val Arg Ala Asn Ala Arg Arg Leu Pro Ala Arg Pro Gly Thr Ala Gly
                325                 330                 335

Thr Thr Ser Thr Arg Ala Arg Arg Lys Pro Arg Ser Leu Ala Leu Leu
            340                 345                 350

Arg Thr Leu Ser Val Val Leu Leu Ala Phe Val Ala Cys Trp Gly Pro
        355                 360                 365

Leu Phe Leu Leu Leu Leu Leu Asp Val Ala Cys Pro Ala Arg Thr Cys
    370                 375                 380

Pro Val Leu Leu Gln Ala Asp Pro Phe Leu Gly Leu Ala Met Ala Asn
385                 390                 395                 400

Ser Leu Leu Asn Pro Ile Ile Tyr Thr Leu Thr Asn Arg Asp Leu Arg
                405                 410                 415

His Ala Leu Leu Arg Leu Val Cys Cys Gly Arg His Ser Cys Gly Arg
            420                 425                 430

Asp Pro Ser Gly Ser Gln Gln Ser Ala Ser Ala Ala Glu Ala Ser Gly
        435                 440                 445

Gly Leu Arg Arg Cys Leu Pro Pro Gly Leu Asp Gly Ser Phe Ser Gly
    450                 455                 460

Ser Glu Arg Ser Ser Pro Gln Arg Asp Gly Leu Asp Thr Ser Gly Ser
465                 470                 475                 480

Thr Gly Ser Pro Gly Ala Pro Thr Ala Ala Arg Thr Leu Val Ser Glu
                485                 490                 495

Pro Ala Ala Asp
            500

(34) INFORMATION FOR SEQ ID NO:33:

     (i) SEQUENCE CHARACTERISTICS:
          (A) LENGTH: 1029 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:33:

ATGCAAGCCG TCGACAATCT CACCTCTGCG CCTGGGAACA CCAGTCTGTG CACCAGAGAC      60

TACAAAATCA CCCAGGTCCT CTTCCCACTG CTCTACACTG TCCTGTTTTT TGTTGGACTT     120

ATCACAAATG GCCTGGCGAT GAGGATTTTC TTTCAAATCC GGAGTAAATC AAACTTTATT     180

ATTTTTCTTA AGAACACAGT CATTTCTGAT CTTCTCATGA TTCTGACTTT TCCATTCAAA     240

ATTCTTAGTG ATGCCAAACT GGGAACAGGA CCACTGAGAA CTTTTGTGTG TCAAGTTACC     300

TCCGTCATAT TTTATTTCAC AATGTATATC AGTATTTCAT TCCTGGGACT GATAACTATC 360

GATCGCTACC AGAAGACCAC CAGGCCATTT AAAACATCCA ACCCCAAAAA TCTCTTGGGG 420

GCTAAGATTC TCTCTGTTGT CATCTGGGCA TTCATGTTCT TACTCTCTTT GCCTAACATG 480

ATTCTGACCA ACAGGCAGCC GAGAGACAAG AATGTGAAGA AATGCTCTTT CCTTAAATCA 540

GAGTTCGGTC TAGTCTGGCA TGAAATAGTA AATTACATCT GTCAAGTCAT TTTCTGGATT 600

AATTTCTTAA TTGTTATTGT ATGTTATACA CTCATTACAA AAGAACTGTA CCGGTCATAC 660

GTAAGAACGA GGGGTGTAGG TAAAGTCCCC AGGAAAAAGG TGAACGTCAA AGTTTTCATT 720

ATCATTGCTG TATTCTTTAT TTGTTTTGTT CCTTTCCATT TTGCCCGAAT TCCTTACACC 780

CTGAGCCAAA CCCGGGATGT CTTTGACTGC ACTGCTGAAA ATACTCTGTT CTATGTGAAA 840

GAGAGCACTC TGTGGTTAAC TTCCTTAAAT GCATGCCTGG ATCCGTTCAT CTATTTTTTC 900

CTTTGCAAGT CCTTCAGAAA TTCCTTGATA AGTATGCTGA AGTGCCCCAA TTCTGCAACA 960

TCTCTGTCCC AGGACAATAG GAAAAAAGAA CAGGATGGTG GTGACCCAAA TGAAGAGACT 1020

CCAATGTAA 1029

(35) INFORMATION FOR SEQ ID NO:34:

(i) SEQUENCE CHARACTERISTICS:
(A) LENGTH: 342 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:34:

(36) INFORMATION FOR SEQ ID NO:35:

     (i) SEQUENCE CHARACTERISTICS:
          (A) LENGTH: 1077 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:35:

ATGTCGGTCT GCTACCGTCC CCCAGGGAAC GAGACACTGC TGAGCTGGAA GACTTCGCGG      60

GCCACAGGCA CAGCCTTCCT GCTGCTGGCG GCGCTGCTGG GGCTGCCTGG CAACGGCTTC     120

GTGGTGTGGA GCTTGGCGGG CTGGCGGCCT GCACGGGGGC GACCGCTGGC GGCCACGCTT 180

GTGCTGCACC TGGCGCTGGC CGACGGCGCG GTGCTGCTGC TCACGCCGCT CTTTGTGGCC 240

TTCCTGACCC GGCAGGCCTG GCCGCTGGGC CAGGCGGGCT GCAAGGCGGT GTACTACGTG 300

TGCGCGCTCA GCATGTACGC CAGCGTGCTG CTCACCGGCC TGCTCAGCCT GCAGCGCTGC 360

CTCGCAGTCA CCCGCCCCTT CCTGGCGCCT CGGCTGCGCA GCCCGGCCCT GGCCCGCCGC 420

CTGCTGCTGG CGGTCTGGCT GGCCGCCCTG TTGCTCGCCG TCCCGGCCGC CGTCTACCGC 480

CACCTGTGGA GGGACCGCGT ATGCCAGCTG TGCCACCCGT CGCCGGTCCA CGCCGCCGCC 540

CACCTGAGCC TGGAGACTCT GACCGCTTTC GTGCTTCCTT TCGGGCTGAT GCTCGGCTGC 600

TACAGCGTGA CGCTGGCACG GCTGCGGGGC GCCCGCTGGG GCTCCGGGCG GCACGGGGCG 660

CGGGTGGGCC GGCTGGTGAG CGCCATCGTG CTTGCCTTCG GCTTGCTCTG GGCCCCCTAC 720

CACGCAGTCA ACCTTCTGCA GGCGGTCGCA GCGCTGGCTC CACCGGAAGG GGCCTTGGCG 780

AAGCTGGGCG GAGCCGGCCA GGCGGCGCGA GCGGGAACTA CGGCCTTGGC CTTCTTCAGT 840

TCTAGCGTCA ACCCGGTGCT CTACGTCTTC ACCGCTGGAG ATCTGCTGCC CCGGGCAGGT 900

CCCCGTTTCC TCACGCGGCT CTTCGAAGGC TCTGGGGAGG CCCGAGGGGG CGGCCGCTCT 960

AGGGAAGGGA CCATGGAGCT CCGAACTACC CCTCAGCTGA AAGTGGTGGG GCAGGGCCGC 1020

GGCAATGGAG ACCCGGGGGG TGGGATGGAG AAGGACGGTC CGGAATGGGA CCTTTGA 1077

(37) INFORMATION FOR SEQ ID NO:36:

(i) SEQUENCE CHARACTERISTICS:
(A) LENGTH: 358 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:36:

Met Ser Val Cys Tyr Arg Pro Pro Gly Asn Glu Thr Leu Leu Ser Trp
1               5                   10                  15

Lys Thr Ser Arg Ala Thr Gly Thr Ala Phe Leu Leu Leu Ala Ala Leu
            20                  25                  30

Leu Gly Leu Pro Gly Asn Gly Phe Val Val Trp Ser Leu Ala Gly Trp
        35                  40                  45

Arg Pro Ala Arg Gly Arg Pro Leu Ala Ala Thr Leu Val Leu His Leu
    50                  55                  60

Ala Leu Ala Asp Gly Ala Val Leu Leu Leu Thr Pro Leu Phe Val Ala
65                  70                  75                  80

Phe Leu Thr Arg Gln Ala Trp Pro Leu Gly Gln Ala Gly Cys Lys Ala

85                  90                  95

Val Tyr Tyr Val Cys Ala Leu Ser Met Tyr Ala Ser Val Leu Leu Thr
            100                 105                 110

Gly Leu Leu Ser Leu Gln Arg Cys Leu Ala Val Thr Arg Pro Phe Leu
        115                 120                 125

Ala Pro Arg Leu Arg Ser Pro Ala Leu Ala Arg Arg Leu Leu Leu Ala
    130                 135                 140

Val Trp Leu Ala Ala Leu Leu Leu Ala Val Pro Ala Ala Val Tyr Arg
145                 150                 155                 160

His Leu Trp Arg Asp Arg Val Cys Gln Leu Cys His Pro Ser Pro Val
                165                 170                 175

His Ala Ala Ala His Leu Ser Leu Glu Thr Leu Thr Ala Phe Val Leu
            180                 185                 190

Pro Phe Gly Leu Met Leu Gly Cys Tyr Ser Val Thr Leu Ala Arg Leu
        195                 200                 205

Arg Gly Ala Arg Trp Gly Ser Gly Arg His Gly Ala Arg Val Gly Arg
    210                 215                 220

Leu Val Ser Ala Ile Val Leu Ala Phe Gly Leu Leu Trp Ala Pro Tyr
225                 230                 235                 240

His Ala Val Asn Leu Leu Gln Ala Val Ala Ala Leu Ala Pro Pro Glu
                245                 250                 255

Gly Ala Leu Ala Lys Leu Gly Gly Ala Gly Gln Ala Ala Arg Ala Gly
            260                 265                 270

Thr Thr Ala Leu Ala Phe Phe Ser Ser Ser Val Asn Pro Val Leu Tyr
        275                 280                 285

Val Phe Thr Ala Gly Asp Leu Leu Pro Arg Ala Gly Pro Arg Phe Leu
    290                 295                 300

Thr Arg Leu Phe Glu Gly Ser Gly Glu Ala Arg Gly Gly Gly Arg Ser
305                 310                 315                 320

Arg Glu Gly Thr Met Glu Leu Arg Thr Thr Pro Gln Leu Lys Val Val
                325                 330                 335

Gly Gln Gly Arg Gly Asn Gly Asp Pro Gly Gly Gly Met Glu Lys Asp
            340                 345                 350

Gly Pro Glu Trp Asp Leu
        355

(38) INFORMATION FOR SEQ ID NO:37:

     (i) SEQUENCE CHARACTERISTICS:
          (A) LENGTH: 1005 base pairs
          (B) TYPE: nucleic acid
          (C) STRANDEDNESS: single
          (D) TOPOLOGY: linear

(ii) MOLECULE TYPE: DNA (genomic)

(xi) SEQUENCE DESCRIPTION: SEQ ID NO:37:

ATGCTGGGGA TCATGGCATG GAATGCAACT TGCAAAAACT GGCTGGCAGC AGAGGCTGCC 60

CTGGAAAAGT ACTACCTTTC CATTTTTTAT GGGATTGAGT TCGTTGTGGG AGTCCTTGGA 120

AATACCATTG TTGTTTACGG CTACATCTTC TCTCTGAAGA ACTGGAACAG CAGTAATATT 180

TATCTCTTTA ACCTCTCTGT CTCTGACTTA GCTTTTCTGT GCACCCTCCC CATGCTGATA 240

AGGAGTTATG CCAATGGAAA CTGGATATAT GGAGACGTGC TCTGCATAAG CAACCGATAT 300

GTGCTTCATG CCAACCTCTA TACCAGCATT CTCTTTCTCA CTTTTATCAG CATAGATCGA 360

TACTTGATAA TTAAGTATCC TTTCCGAGAA CACCTTCTGC AAAAGAAAGA GTTTGCTATT 420

TTAATCTCCT TGGCCATTTG GGTTTTAGTA ACCTTAGAGT TACTACCCAT ACTTCCCCTT 480

ATAAATCCTG TTATAACTGA CAATGGCACC ACCTGTAATG ATTTTGCAAG TTCTGGAGAC 540

CCCAACTACA ACCTCATTTA CAGCATGTGT CTAACACTGT TGGGGTTCCT TATTCCTCTT 600

TTTGTGATGT GTTTCTTTTA TTACAAGATT GCTCTCTTCC TAAAGCAGAG GAATAGGCAG 660

GTTGCTACTG CTCTGCCCCT TGAAAAGCCT CTCAACTTGG TCATCATGGC AGTGGTAATC 720

TTCTCTGTGC TTTTTACACC CTATCACGTC ATGCGGAATG TGAGGATCGC TTCACGCCTG 780

GGGAGTTGGA AGCAGTATCA GTGCACTCAG GTCGTCATCA ACTCCTTTTA CATTGTGACA 840

CGGCCTTTGG CCTTTCTGAA CAGTGTCATC AACCCTGTCT TCTATTTTCT TTTGGGAGAT 900

CACTTCAGGG ACATGCTGAT GAATCAACTG AGACACAACT TCAAATCCCT TACATCCTTT 960

AGCAGATGGG CTCATGAACT CCTACTTTCA TTCAGAGAAA AGTGA 1005

(39) INFORMATION FOR SEQ ID NO:38:

(i) SEQUENCE CHARACTERISTICS:
(A) LENGTH: 334 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:38:

Met Leu Gly Ile Met Ala Trp Asn Ala Thr Cys Lys Asn Trp Leu Ala
1               5                   10                  15

Ala Glu Ala Ala Leu Glu Lys Tyr Tyr Leu Ser Ile Phe Tyr Gly Ile
            20                  25                  30

Glu Phe Val Val Gly Val Leu Gly Asn Thr Ile Val Val Tyr Gly Tyr
        35                  40                  45

Ile Phe Ser Leu Lys Asn Trp Asn Ser Ser Asn Ile Tyr Leu Phe Asn
    50                  55                  60

Leu Ser Val Ser Asp Leu Ala Phe Leu Cys Thr Leu Pro Met Leu Ile
65                  70                  75                  80

Arg Ser Tyr Ala Asn Gly Asn Trp Ile Tyr Gly Asp Val Leu Cys Ile
                85                  90                  95

Ser Asn Arg Tyr Val Leu His Ala Asn Leu Tyr Thr Ser Ile Leu Phe
            100                 105                 110

Leu Thr Phe Ile Ser Ile Asp Arg Tyr Leu Ile Ile Lys Tyr Pro Phe
        115                 120                 125

Arg Glu His Leu Leu Gln Lys Lys Glu Phe Ala Ile Leu Ile Ser Leu
    130                 135                 140

Ala Ile Trp Val Leu Val Thr Leu Glu Leu Leu Pro Ile Leu Pro Leu
145                 150                 155                 160

Ile Asn Pro Val Ile Thr Asp Asn Gly Thr Thr Cys Asn Asp Phe Ala
                165                 170                 175

Ser Ser Gly Asp Pro Asn Tyr Asn Leu Ile Tyr Ser Met Cys Leu Thr
            180                 185                 190

Leu Leu Gly Phe Leu Ile Pro Leu Phe Val Met Cys Phe Phe Tyr Tyr
        195                 200                 205

Lys Ile Ala Leu Phe Leu Lys Gln Arg Asn Arg Gln Val Ala Thr Ala
    210                 215                 220

Leu Pro Leu Glu Lys Pro Leu Asn Leu Val Ile Met Ala Val Val Ile
225                 230                 235                 240

Phe Ser Val Leu Phe Thr Pro Tyr His Val Met Arg Asn Val Arg Ile
                245                 250                 255

Ala Ser Arg Leu Gly Ser Trp Lys Gln Tyr Gln Cys Thr Gln Val Val
            260                 265                 270

Ile Asn Ser Phe Tyr Ile Val Thr Arg Pro Leu Ala Phe Leu Asn Ser
        275                 280                 285

Val Ile Asn Pro Val Phe Tyr Phe Leu Leu Gly Asp His Phe Arg Asp
    290                 295                 300

Met Leu Met Asn Gln Leu Arg His Asn Phe Lys Ser Leu Thr Ser Phe
305                 310                 315                 320

Ser Arg Trp Ala His Glu Leu Leu Leu Ser Phe Arg Glu Lys
                325                 330

(40) INFORMATION FOR SEQ ID NO:39:

     (i) SEQUENCE CHARACTERISTICS:
          (A) LENGTH: 1296 base pairs
          (B) TYPE: nucleic acid
          (C) STRANDEDNESS: single

(D) TOPOLOGY: linear

(ii) MOLECULE TYPE: DNA (genomic)

(xi) SEQUENCE DESCRIPTION: SEQ ID NO:39:

ATGCAGGCGC TTAACATTAC CCCGGAGCAG TTCTCTCGGC TGCTGCGGGA CCACAACCTG 60

ACGCGGGAGC AGTTCATCGC TCTGTACCGG CTGCGACCGC TCGTCTACAC CCCAGAGCTG 120

CCGGGACGCG CCAAGCTGGC CCTCGTGCTC ACCGGCGTGC TCATCTTCGC CCTGGCGCTC 180

TTTGGCAATG CTCTGGTGTT CTACGTGGTG ACCCGCAGCA AGGCCATGCG CACCGTCACC 240

AACATCTTTA TCTGCTCCTT GGCGCTCAGT GACCTGCTCA TCACCTTCTT CTGCATTCCC 300

GTCACCATGC TCCAGAACAT TTCCGACAAC TGGCTGGGGG GTGCTTTCAT TTGCAAGATG 360

GTGCCATTTG TCCAGTCTAC CGCTGTTGTG ACAGAAATGC TCACTATGAC CTGCATTGCT 420

GTGGAAAGGC ACCAGGGACT TGTGCATCCT TTTAAAATGA AGTGGCAATA CACCAACCGA 480

AGGGCTTTCA CAATGCTAGG TGTGGTCTGG CTGGTGGCAG TCATCGTAGG ATCACCCATG 540

TGGCACGTGC AACAACTTGA GATCAAATAT GACTTCCTAT ATGAAAAGGA ACACATCTGC 600

TGCTTAGAAG AGTGGACCAG CCCTGTGCAC CAGAAGATCT ACACCACCTT CATCCTTGTC 660

ATCCTCTTCC TCCTGCCTCT TATGGTGATG CTTATTCTGT ACAGTAAAAT TGGTTATGAA 720

CTTTGGATAA AGAAAAGAGT TGGGGATGGT TCAGTGCTTC GAACTATTCA TGGAAAAGAA 780

ATGTCCAAAA TAGCCAGGAA GAAGAAACGA GCTGTCATTA TGATGGTGAC AGTGGTGGCT 840

CTCTTTGCTG TGTGCTGGGC ACCATTCCAT GTTGTCCATA TGATGATTGA ATACAGTAAT 900

TTTGAAAAGG AATATGATGA TGTCACAATC AAGATGATTT TTGCTATCGT GCAAATTATT 960

GGATTTTCCA ACTCCATCTG TAATCCCATT GTCTATGCAT TTATGAATGA AAACTTCAAA 1020

AAAAATGTTT TGTCTGCAGT TTGTTATTGC ATAGTAAATA AAACCTTCTC TCCAGCACAA 1080

AGGCATGGAA ATTCAGGAAT TACAATGATG CGGAAGAAAG CAAAGTTTTC CCTCAGAGAG 1140

AATCCAGTGG AGGAAACCAA AGGAGAAGCA TTCAGTGATG GCAACATTGA AGTCAAATTG 1200

TGTGAACAGA CAGAGGAGAA GAAAAAGCTC AAACGACATC TTGCTCTCTT TAGGTCTGAA 1260

CTGGCTGAGA ATTCTCCTTT AGACAGTGGG CATTAA 1296

(41) INFORMATION FOR SEQ ID NO:40:

(i) SEQUENCE CHARACTERISTICS:
(A) LENGTH: 431 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:40:

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu
            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Val
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile

305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

(42) INFORMATION FOR SEQ ID NO:41:

     (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:41:

CTGTGTACAG CAGTTCGCAG AGTG                                          24

(43) INFORMATION FOR SEQ ID NO:42:

     (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:42:

GAGTGCCAGG CAGAGCAGGT AGAC                                          24

(44) INFORMATION FOR SEQ ID NO:43:

     (i) SEQUENCE CHARACTERISTICS:
          (A) LENGTH: 31 base pairs
          (B) TYPE: nucleic acid
          (C) STRANDEDNESS: single
          (D) TOPOLOGY: linear

(ii) MOLECULE TYPE: DNA (genomic)

(iv) ANTI-SENSE: NO

(xi) SEQUENCE DESCRIPTION: SEQ ID NO:43:

CCCGAATTCC TGCTTGCTCC CAGCTTGGCC C 31

(45) INFORMATION FOR SEQ ID NO:44:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:44:

TGTGGATCCT GCTGTCAAAG GTCCCATTCC GG 32

(46) INFORMATION FOR SEQ ID NO:45:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:45:

TCACAATGCT AGGTGTGGTC 20

(47) INFORMATION FOR SEQ ID NO:46:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:46:

TGCATAGACA ATGGGATTAC AG 22

(48) INFORMATION FOR SEQ ID NO:47:

(i) SEQUENCE CHARACTERISTICS:
(A) LENGTH: 511 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:47:

TCACAATGCT AGGTGTGGTC TGGCTGGTGG CAGTCATCGT AGGATCACCC ATGTGGCACG 60

TGCAACAACT TGAGATCAAA TATGACTTCC TATATGAAAA GGAACACATC TGCTGCTTAG 120

AAGAGTGGAC CAGCCCTGTG CACCAGAAGA TCTACACCAC CTTCATCCTT GTCATCCTCT 180

TCCTCCTGCC TCTTATGGTG ATGCTTATTC TGTACGTAAA ATTGGTTATG AACTTTGGAT 240

AAAGAAAAGA GTTGGGGATG GTTCAGTGCT TCGAACTATT CATGGAAAAG AAATGTCCAA 300

AATAGCCAGG AAGAAGAAAC GAGCTGTCAT TATGATGGTG ACAGTGGTGG CTCTCTTTGC 360

TGTGTGCTGG GCACCATTCC ATGTTGTCCA TATGATGATT GAATACAGTA ATTTTGAAAA 420

GGAATATGAT GATGTCACAA TCAAGATGAT TTTTGCTATC GTGCAAATTA TTGGATTTTC 480

CAACTCCATC TGTAATCCCA TTGTCTATGC A 511

(49) INFORMATION FOR SEQ ID NO:48:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:48:

CTGCTTAGAA GAGTGGACCA G 21

(50) INFORMATION FOR SEQ ID NO:49:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:49:

CTGTGCACCA GAAGATCTAC AC 22

(51) INFORMATION FOR SEQ ID NO:50:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:50:

CAAGGATGAA GGTGGTGTAG A 21

(52) INFORMATION FOR SEQ ID NO:51:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:51:

GTGTAGATCT TCTGGTGCAC AGG 23

(53) INFORMATION FOR SEQ ID NO:52:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:52:

GCAATGCAGG TCATAGTGAG C 21

(54) INFORMATION FOR SEQ ID NO:53:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: YES

(xi) SEQUENCE DESCRIPTION: SEQ ID NO:53:

TGGAGCATGG TGACGGGAAT GCAGAAG 27

(55) INFORMATION FOR SEQ ID NO:54:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:54:

GTGATGAGCA GGTCACTGAG CGCCAAG 27

(56) INFORMATION FOR SEQ ID NO:55:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:55:

GCAATGCAGG CGCTTAACAT TAC 23

(57) INFORMATION FOR SEQ ID NO:56:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:56:

TTGGGTTACA ATCTGAAGGG CA 22

(58) INFORMATION FOR SEQ ID NO:57:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:57:

ACTCCGTGTC CAGCAGGACT CTG 23

(58) INFORMATION FOR SEQ ID NO:58:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:58:

TGCGTGTTCC TGGACCCTCA CGTG 24

(58) INFORMATION FOR SEQ ID NO:59:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:59:

CAGGCCTTGG ATTTTAATGT CAGGGATGG 29

(61) INFORMATION FOR SEQ ID NO:60:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:60:

GGAGAGTCAG CTCTGAAAGA ATTCAGG 27

(62) INFORMATION FOR SEQ ID NO:61:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:61:

TGATGTGATG CCAGATACTA ATAGCAC 27

(63) INFORMATION FOR SEQ ID NO:62:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:62:

CCTGATTCAT TTAGGTGAGA TTGAGAC 27

(64) INFORMATION FOR SEQ ID NO:63:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:63:

CCCAAGCTTC CCCAGGTGTA TTTGAT 26

(3) INFORMATION FOR SEQ ID NO:63:

(i) SEQUENCE CHARACTERISTICS:
(A) LENGTH: 26 base pairs
(B) TYPE: nucleic acid

(C) STRANDEDNESS: single
(D) TOPOLOGY: linear

(ii) MOLECULE TYPE: DNA (genomic)

(xi) SEQUENCE DESCRIPTION: SEQ ID NO:64:

GTTGGATCCA CATAATGCAT TTTCTC 26

(66) INFORMATION FOR SEQ ID NO:65:

(i) SEQUENCE CHARACTERISTICS:
(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:65:

ATGATTCTCA ACTCTTCTAC TGAAGATGGT ATTAAAAGAA TCCAAGATGA TTGTCCCAAA 60

GCTGGAAGGC ATAATTACAT ATTTGTCATG ATTCCTACTT TATACAGTAT CATCTTTGTG 120

GTGGGAATAT TTGGAAACAG CTTGGTGGTG ATAGTCATTT ACTTTTATAT GAAGCTGAAG 180

ACTGTGGCCA GTGTTTTTCT TTTGAATTTA GCACTGGCTG ACTTATGCTT TTTACTGACT 240

TTGCCACTAT GGGCTGTCTA CACAGCTATG GAATACCGCT GGCCCTTTGG CAATTACCTA 300

TGTAAGATTG CTTCAGCCAG CGTCAGTTTC AACCTGTACG CTAGTGTGTT TCTACTCACG 360

TGTCTCAGCA TTGATCGATA CCTGGCTATT GTTCACCCAA TGAAGTCCCG CCTTCGACGC 420

ACAATGCTTG TAGCCAAAGT CACCTGCATC ATCATTTGGC TGCTGGCAGG CTTGGCCAGT 480

TTGCCAGCTA TAATCCATCG AAATGTATTT TTCATTGAGA ACACCAATAT TACAGTTTGT 540

GCTTTCCATT ATGAGTCCCA AAATTCAACC CTTCCGATAG GGCTGGGCCT GACCAAAAAT 600

ATACTGGGTT TCCTGTTTCC TTTTCTGATC ATTCTTACAA GTTATACTCT TATTTGGAAG 660

GCCCTAAAGA AGGCTTATGA AATTCAGAAG AACAAACCAA GAAATGATGA TATTTTTAAG 720

ATAATTATGG CAATTGTGCT TTTCTTTTTC TTTTCCTGGA TTCCCCACCA AATATTCACT 780

TTTCTGGATG TATTGATTCA ACTAGGCATC ATACGTGACT GTAGAATTGC AGATATTGTG 840

GACACGGCCA TGCCTATCAC CATTTGTATA GCTTATTTTA ACAATTGCCT GAATCCTCTT 900

TTTTATGGCT TTCTGGGGAA AAAATTTAAA AGATATTTTC TCCAGCTTCT AAAATATATT 960

CCCCCAAAAG CCAAATCCCA CTCAAACCTT TCAACAAAAA TGAGCACGCT TTCCTACCGC 1020

CCCTCAGATA ATGTAAGCTC ATCCACCAAG AAGCCTGCAC CATGTTTTGA GGTTGAGTGA 1080

(67) INFORMATION FOR SEQ ID NO:66:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:66:

(68) INFORMATION FOR SEQ ID NO:67:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:67:

ACCATGGGCA GCCCCTGGAA CGGCAGC 27

(69) INFORMATION FOR SEQ ID NO:68:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:68:

AGAACCACCA CCAGCAGGAC GCGGACGGTC TGCCGGTGG 39

(70) INFORMATION FOR SEQ ID NO:69:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:69:

GTCCGCGTCC TGCTGGTGGT GGTTCTGGCA TTTATAATT 39

(71) INFORMATION FOR SEQ ID NO:70:

(i) SEQUENCE CHARACTERISTICS:
(A) LENGTH: 33 base pairs
(B) TYPE: nucleic acid
(C) STRANDEDNESS: single
(D) TOPOLOGY: not relevant

(ii) MOLECULE TYPE: DNA (genomic)

(xi) SEQUENCE DESCRIPTION: SEQ ID NO:70:

CCTGGATCCT TATCCCATCG TCTTCACGTT AGC 33

(72) INFORMATION FOR SEQ ID NO:71:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:71:

CTGGAATTCT CCTGCCAGCA TGGTGA 26

(73) INFORMATION FOR SEQ ID NO:72:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:72:

GCAGGATCCT ATATTGCGTG CTCTGTCCCC 30

(74) INFORMATION FOR SEQ ID NO:73:

(i) SEQUENCE CHARACTERISTICS:
(A) LENGTH: 999 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:73:

ATGGTGAACT CCACCCACCG TGGGATGCAC ACTTCTCTGC ACCTCTGGAA CCGCAGCAGT 60

TACAGACTGC ACAGCAATGC CAGTGAGTCC CTTGGAAAAG GCTACTCTGA TGGAGGGTGC 120

TACGAGCAAC TTTTTGTCTC TCCTGAGGTG TTTGTGACTC TGGGTGTCAT CAGCTTGTTG 180

GAGAATATCT TAGTGATTGT GGCAATAGCC AAGAACAAGA ATCTGCATTC ACCCATGTAC 240

TTTTTCATCT GCAGCTTGGC TGTGGCTGAT ATGCTGGTGA GCGTTTCAAA TGGATCAGAA 300

ACCATTATCA TCACCCTATT AAACAGTACA GATACGGATG CACAGAGTTT CACAGTGAAT 360

ATTGATAATG TCATTGACTC GGTGATCTGT AGCTCCTTGC TTGCATCCAT TTGCAGCCTG 420

CTTTCAATTG CAGTGGACAG GTACTTTACT ATCTTCTATG CTCTCCAGTA CCATAACATT 480

ATGACAGTTA AGCGGGTTGG GATCAGCATA AGTTGTATCT GGGCAGCTTG CACGGTTTCA 540

GGCATTTTGT TCATCATTTA CTCAGATAGT AGTGCTGTCA TCATCTGCCT CATCACCATG 600

TTCTTCACCA TGCTGGCTCT CATGGCTTCT CTCTATGTCC ACATGTTCCT GATGGCCAGG 660

CTTCACATTA AGAGGATTGC TGTCCTCCCC GGCACTGGTG CCATCCGCCA AGGTGCCAAT 720

ATGAAGGGAG CGATTACCTT GACCATCCTG ATTGGCGTCT TTGTTGTCTG CTGGGCCCCA 780

TTCTTCCTCC ACTTAATATT CTACATCTCT TGTCCTCAGA ATCCATATTG TGTGTGCTTC 840

ATGTCTCACT TTAACTTGTA TCTCATACTG ATCATGTGTA ATTCAATCAT CGATCCTCTG 900

ATTTATGCAC TCCGGAGTCA AGAACTGAGG AAAACCTTCA AAGAGATCAT CTGTTGCTAT 960

CCCCTGGGAG GCCTTTGTGA CTTGTCTAGC AGATATTAA 999

(75) INFORMATION FOR SEQ ID NO:74:

(i) SEQUENCE CHARACTERISTICS:
(A) LENGTH: 332 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:74:

(76) INFORMATION FOR SEQ ID NO:75:

     (i) SEQUENCE CHARACTERISTICS:
          (A) LENGTH: 32 base pairs
          (B) TYPE: nucleic acid
          (C) STRANDEDNESS: single
          (D) TOPOLOGY: linear

(ii) MOLECULE TYPE: DNA (genomic)

(xi) SEQUENCE DESCRIPTION: SEQ ID NO:75:

CCGAAGCTTC GAGCTGAGTA AGGCGGCGGG CT 32

(77) INFORMATION FOR SEQ ID NO:76:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:76:

GTGGAATTCA TTTGCCCTGC CTCAACCCCC A 31

(78) INFORMATION FOR SEQ ID NO:77:

(i) SEQUENCE CHARACTERISTICS:
(A) LENGTH: 1344 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:77:

ATGGAGCTGC TAAAGCTGAA CCGGAGCGTG CAGGGAACCG GACCCGGGCC GGGGGCTTCC 60

CTGTGCCGCC CGGGGGCGCC TCTCCTCAAC AGCAGCAGTG TGGGCAACCT CAGCTGCGAG 120

CCCCCTCGCA TTCGCGGAGC CGGGACACGA GAATTGGAGC TGGCCATTAG AATCACTCTT 180

TACGCAGTGA TCTTCCTGAT GAGCGTTGGA GGAAATATGC TCATCATCGT GGTCCTGGGA 240

CTGAGCCGCC GCCTGAGGAC TGTCACCAAT GCCTTCCTCC TCTCACTGGC AGTCAGCGAC 300

CTCCTGCTGG CTGTGGCTTG CATGCCCTTC ACCCTCCTGC CCAATCTCAT GGGCACATTC 360

ATCTTTGGCA CCGTCATCTG CAAGGCGGTT TCCTACCTCA TGGGGGTGTC TGTGAGTGTG 420

TCCACGCTAA GCCTCGTGGC CATCGCACTG GAGCGATATA GCGCCATCTG CCGACCACTG 480

CAGGCACGAG TGTGGCAGAC GCGCTCCCAC GCGGCTCGCG TGATTGTAGC CACGTGGCTG 540

CTGTCCGGAC TACTCATGGT GCCCTACCCC GTGTACACTG TCGTGCAACC AGTGGGGCCT 600

CGTGTGCTGC AGTGCGTGCA TCGCTGGCCC AGTGCGCGGG TCCGCCAGAC CTGGTCCGTA 660

CTGCTGCTTC TGCTCTTGTT CTTCATCCCA GGTGTGGTTA TGGCCGTGGC CTACGGGCTT 720

ATCTCTCGCG AGCTCTACTT AGGGCTTCGC TTTGACGGCG ACAGTGACAG CGACAGCCAA 780

AGCAGGGTCC GAAACCAAGG CGGGCTGCCA GGGGCTGTTC ACCAGAACGG GCGTTGCCGG 840

CCTGAGACTG GCGCGGTTGG CAAAGACAGC GATGGCTGCT ACGTGCAACT TCCACGTTCC 900

CGGCCTGCCC TGGAGCTGAC GGCGCTGACG GCTCCTGGGC CGGGATCCGG CTCCCGGCCC 960

ACCCAGGCCA AGCTGCTGGC TAAGAAGCGC GTGGTGCGAA TGTTGCTGGT GATCGTTGTG 1020

CTTTTTTTTC TGTGTTGGTT GCCAGTTTAT AGTGCCAACA CGTGGCGCGC CTTTGATGGC 1080

CCGGGTGCAC ACCGAGCACT CTCGGGTGCT CCTATCTCCT TCATTCACTT GCTGAGCTAC 1140

GCCTCGGCCT GTGTCAACCC CCTGGTCTAC TGCTTCATGC ACCGTCGCTT TCGCCAGGCC 1200

TGCCTGGAAA CTTGCGCTCG CTGCTGCCCC CGGCCTCCAC GAGCTCGCCC CAGGGCTCTT 1260

CCCGATGAGG ACCCTCCCAC TCCCTCCATT GCTTCGCTGT CCAGGCTTAG CTACACCACC 1320

ATCAGCACAC TGGGCCCTGG CTGA 1344

(79) INFORMATION FOR SEQ ID NO:78:

(i) SEQUENCE CHARACTERISTICS:
(A) LENGTH: 447 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:78:

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu
            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu

145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Val Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

(80) INFORMATION FOR SEQ ID NO:79:

     (i) SEQUENCE CHARACTERISTICS:
          (A) LENGTH: 30 base pairs
          (B) TYPE: nucleic acid

(C) STRANDEDNESS: single
(D) TOPOLOGY: linear

(ii) MOLECULE TYPE: DNA (genomic)

(xi) SEQUENCE DESCRIPTION: SEQ ID NO:79:

TGCAAGCTTA AAAAGGAAAA AATGAACAGC 30

(81) INFORMATION FOR SEQ ID NO:80:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:80:

TAAGGATCCC TTCCCTTCAA AACATCCTTG 30

(82) INFORMATION FOR SEQ ID NO:81:

(i) SEQUENCE CHARACTERISTICS:
(A) LENGTH: 1014 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:81:

ATGAACAGCA CATGTATTGA AGAACAGCAT GACCTGGATC ACTATTTGTT TCCCATTGTT 60

TACATCTTTG TGATTATAGT CAGCATTCCA GCCAATATTG GATCTCTGTG TGTGTCTTTC 120

CTGCAACCCA AGAAGGAAAG TGAACTAGGA ATTTACCTCT TCAGTTTGTC ACTATCAGAT 180

TTACTCTATG CATTAACTCT CCCTTTATGG ATTGATTATA CTTGGAATAA AGACAACTGG 240

ACTTTCTCTC CTGCCTTGTG CAAAGGGAGT GCTTTTCTCA TGTACATGAA GTTTTACAGC 300

AGCACAGCAT TCCTCACCTG CATTGCCGTT GATCGGTATT TGGCTGTTGT CTACCCTTTG 360

AAGTTTTTTT TCCTAAGGAC AAGAAGAATT GCACTCATGG TCAGCCTGTC CATCTGGATA 420

TTGGAAACCA TCTTCAATGC TGTCATGTTG TGGGAAGATG AAACAGTTGT TGAATATTGC 480

GATGCCGAAA AGTCTAATTT TACTTTATGC TATGACAAAT ACCCTTTAGA GAAATGGCAA 540

ATCAACCTCA ACTTGTTCAG GACGTGTACA GGCTATGCAA TACCTTTGGT CACCATCCTG 600

ATCTGTAACC GGAAAGTCTA CCAAGCTGTG CGGCACAATA AAGCCACGGA AAACAAGGAA 660

AAGAAGAGAA TCATAAAACT ACTTGTCAGC ATCACAGTTA CTTTTGTCTT ATGCTTTACT 720

CCCTTTCATG TGATGTTGCT GATTCGCTGC ATTTTAGAGC ATGCTGTGAA CTTCGAAGAC 780

CACAGCAATT CTGGGAAGCG AACTTACACA ATGTATAGAA TCACGGTTGC ATTAACAAGT 840

TTAAATTGTG TTGCTGATCC AATTCTGTAC TGTTTTGTTA CCGAAACAGG AAGATATGAT 900

ATGTGGAATA TATTAAAATT CTGCACTGGG AGGTGTAATA CATCACAAAG ACAAAGAAAA 960

CGCATACTTT CTGTGTCTAC AAAAGATACT ATGGAATTAG AGGTCCTTGA GTAG 1014

(83) INFORMATION FOR SEQ ID NO:82:

(i) SEQUENCE CHARACTERISTICS:
(A) LENGTH: 337 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:82:

Met Asn Ser Thr Cys Ile Glu Glu Gln His Asp Leu Asp His Tyr Leu
1               5                   10                  15

Phe Pro Ile Val Tyr Ile Phe Val Ile Ile Val Ser Ile Pro Ala Asn
            20                  25                  30

Ile Gly Ser Leu Cys Val Ser Phe Leu Gln Pro Lys Lys Glu Ser Glu
        35                  40                  45

Leu Gly Ile Tyr Leu Phe Ser Leu Ser Leu Ser Asp Leu Leu Tyr Ala
    50                  55                  60

Leu Thr Leu Pro Leu Trp Ile Asp Tyr Thr Trp Asn Lys Asp Asn Trp
65                  70                  75                  80

Thr Phe Ser Pro Ala Leu Cys Lys Gly Ser Ala Phe Leu Met Tyr Met
                85                  90                  95

Lys Phe Tyr Ser Ser Thr Ala Phe Leu Thr Cys Ile Ala Val Asp Arg
            100                 105                 110

Tyr Leu Ala Val Val Tyr Pro Leu Lys Phe Phe Phe Leu Arg Thr Arg
        115                 120                 125

Arg Ile Ala Leu Met Val Ser Leu Ser Ile Trp Ile Leu Glu Thr Ile
    130                 135                 140

Phe Asn Ala Val Met Leu Trp Glu Asp Glu Thr Val Val Glu Tyr Cys
145                 150                 155                 160

Asp Ala Glu Lys Ser Asn Phe Thr Leu Cys Tyr Asp Lys Tyr Pro Leu
                165                 170                 175

Glu Lys Trp Gln Ile Asn Leu Asn Leu Phe Arg Thr Cys Thr Gly Tyr
            180                 185                 190

Ala Ile Pro Leu Val Thr Ile Leu Ile Cys Asn Arg Lys Val Tyr Gln

195                 200                 205

Ala Val Arg His Asn Lys Ala Thr Glu Asn Lys Glu Lys Lys Arg Ile
    210                 215                 220

Ile Lys Leu Leu Val Ser Ile Thr Val Thr Phe Val Leu Cys Phe Thr
225                 230                 235                 240

Pro Phe His Val Met Leu Leu Ile Arg Cys Ile Leu Glu His Ala Val
                245                 250                 255

Asn Phe Glu Asp His Ser Asn Ser Gly Lys Arg Thr Tyr Thr Met Tyr
            260                 265                 270

Arg Ile Thr Val Ala Leu Thr Ser Leu Asn Cys Val Ala Asp Pro Ile
        275                 280                 285

Leu Tyr Cys Phe Val Thr Glu Thr Gly Arg Tyr Asp Met Trp Asn Ile
    290                 295                 300

Leu Lys Phe Cys Thr Gly Arg Cys Asn Thr Ser Gln Arg Gln Arg Lys
305                 310                 315                 320

Arg Ile Leu Ser Val Ser Thr Lys Asp Thr Met Glu Leu Glu Val Leu
                325                 330                 335

Glu

(84) INFORMATION FOR SEQ ID NO:83:

     (i) SEQUENCE CHARACTERISTICS:
          (A) LENGTH: 40 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:83:

CAGGAAGAAG AAACGAGCTG TCATTATGAT GGTGACAGTG                                40

(85) INFORMATION FOR SEQ ID NO:84:

     (i) SEQUENCE CHARACTERISTICS:
          (A) LENGTH: 40 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:84:

CACTGTCACC ATCATAATGA CAGCTCGTTT CTTCTTCCTG                                40

(86) INFORMATION FOR SEQ ID NO:85:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:85:

GGCCACCGGC AGACCAAACG CGTCCTGCTG 30

(87) INFORMATION FOR SEQ ID NO:86:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:86:

CTCCTTCGGT CCTCCTATCG TTGTCAGAAG T 31

(88) INFORMATION FOR SEQ ID NO:87:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:87:

GGAAAAGAAG AGAATCAAAA AACTACTTGT CAGCATC 37

(89) INFORMATION FOR SEQ ID NO:88:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:88:

CTCCTTCGGT CCTCCTATCG TTGTCAGAAG T 31

(90) INFORMATION FOR SEQ ID NO:89:

(i) SEQUENCE CHARACTERISTICS:

(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:89:

ATGATTCTCA ACTCTTCTAC TGAAGATGGT ATTAAAAGAA TCCAAGATGA TTGTCCCAAA 60

GCTGGAAGGC ATAATTACAT ATTTGTCATG ATTCCTACTT TATACAGTAT CATCTTTGTG 120

GTGGGAATAT TTGGAAACAG CTTGGTGGTG ATAGTCATTT ACTTTTATAT GAAGCTGAAG 180

ACTGTGGCCA GTGTTTTTCT TTTGAATTTA GCACTGGCTG ACTTATGCTT TTTACTGACT 240

TTGCCACTAT GGGCTGTCTA CACAGCTATG GAATACCGCT GGCCCTTTGG CAATTACCTA 300

TGTAAGATTG CTTCAGCCAG CGTCAGTTTC AACCTGTACG CTAGTGTGTT TCTACTCACG 360

TGTCTCAGCA TTGATCGATA CCTGGCTATT GTTCACCCAA TGAAGTCCCG CCTTCGACGC 420

ACAATGCTTG TAGCCAAAGT CACCTGCATC ATCATTTGGC TGCTGGCAGG CTTGGCCAGT 480

TTGCCAGCTA TAATCCATCG AAATGTATTT TTCATTGAGA ACACCAATAT TACAGTTTGT 540

GCTTTCCATT ATGAGTCCCA AAATTCAACC CTTCCGATAG GGCTGGGCGT GACCAAAAAT 600

ATACTGGGTT TCCTGTTTCC TTTTCTGATC ATTCTTACAA GTTATACTCT TATTTGGAAG 660

GCCCTAAAGA AGGCTTATGA AATTCAGAAG AACAAACCAA GAAATGATGA TATTAAAAAG 720

ATAATTATGG CAATTGTGCT TTTCTTTTTC TTTTCCTGGA TTCCCCACCA AATATTCACT 780

TTTCTGGATG TATTGATTCA ACTAGGCATC ATACGTGACT GTAGAATTGC AGATATTGTG 840

GACACGGCCA TGCCTATCAC CATTTGTATA GCTTATTTTA ACAATTGCCT GAATCCTCTT 900

TTTTATGGCT TTCTGGGGAA AAAATTTAAA AGATATTTTC TCCAGCTTCT AAAATATATT 960

CCCCCAAAAG CCAAATCCCA CTCAAACCTT TCAACAAAAA TGAGCACGCT TTCCTACCGC 1020

CCCTCAGATA ATGTAAGCTC ATCCACCAAG AAGCCTGCAC CATGTTTTGA GGTTGAGTGA 1080

(91) INFORMATION FOR SEQ ID NO:90:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:90:

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Lys Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu
        355

(92) INFORMATION FOR SEQ ID NO:91:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:91:

CCAAGAAATG ATGATATTAA AAAGATAATT ATGGC 35

(93) INFORMATION FOR SEQ ID NO:92:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:92:

CTCCTTCGGT CCTCCTATCG TTGTCAGAAG T 31

(94) INFORMATION FOR SEQ ID NO:93:

(i) SEQUENCE CHARACTERISTICS:
(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:93:

ATGATTCTCA ACTCTTCTAC TGAAGATGGT ATTAAAAGAA TCCAAGATGA TTGTCCCAAA 60

GCTGGAAGGC ATAATTACAT ATTTGTCATG ATTCCTACTT TATACAGTAT CATCTTTGTG 120

GTGGGAATAT TTGGAAACAG CTTGGTGGTG ATAGTCATTT ACTTTTATAT GAAGCTGAAG 180

ACTGTGGCCA GTGTTTTTCT TTTGAATTTA GCACTGGCTG ACTTATGCTT TTTACTGACT 240

TTGCCACTAT GGGCTGTCTA CACAGCTATG GAATACCGCT GGCCCTTTGG CAATTACCTA 300

TGTAAGATTG CTTCAGCCAG CGTCAGTTTC GCCCTGTACG CTAGTGTGTT TCTACTCACG 360

TGTCTCAGCA TTGATCGATA CCTGGCTATT GTTCACCCAA TGAAGTCCCG CCTTCGACGC 420

ACAATGCTTG TAGCCAAAGT CACCTGCATC ATCATTTGGC TGCTGGCAGG CTTGGCCAGT 480

TTGCCAGCTA TAATCCATCG AAATGTATTT TTCATTGAGA ACACCAATAT TACAGTTTGT 540

GCTTTCCATT ATGAGTCCCA AAATTCAACC CTTCCGATAG GGCTGGGCCT GACCAAAAAT 600

ATACTGGGTT TCCTGTTTCC TTTTCTGATC ATTCTTACAA GTTATACTCT TATTTGGAAG 660

GCCCTAAAGA AGGCTTATGA AATTCAGAAG AACAAACCAA GAAATGATGA TATTTTTAAG 720

ATAATTATGG CAATTGTGCT TTTCTTTTTC TTTTCCTGGA TTCCCCACCA AATATTCACT 780

TTTCTGGATG TATTGATTCA ACTAGGCATC ATACGTGACT GTAGAATTGC AGATATTGTG 840

GACACGGCCA TGCCTATCAC CATTTGTATA GCTTATTTTA ACAATTGCCT GAATCCTCTT 900

TTTTATGGCT TTCTGGGGAA AAAATTTAAA AGATATTTTC TCCAGCTTCT AAAATATATT 960

CCCCCAAAAG CCAAATCCCA CTCAAACCTT TCAACAAAAA TGAGCACGCT TTCCTACCGC 1020

CCCTCAGATA ATGTAAGCTC ATCCACCAAG AAGCCTGCAC CATGTTTTGA GGTTGAGTGA 1080

(95) INFORMATION FOR SEQ ID NO:94:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:94:

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Ala Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Phe Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu
        355

(97) INFORMATION FOR SEQ ID NO:95:

     (i) SEQUENCE CHARACTERISTICS:
          (A) LENGTH: 26 base pairs
          (B) TYPE: nucleic acid
          (C) STRANDEDNESS: single
          (D) TOPOLOGY: linear

    (ii) MOLECULE TYPE: DNA (genomic)

    (iv) ANTI-SENSE: NO

    (xi) SEQUENCE DESCRIPTION: SEQ ID NO:95:

CCCAAGCTTC CCCAGGTGTA TTTGAT 26

(97) INFORMATION FOR SEQ ID NO:96:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:96:

CCTGCAGGCG AAACTGACTC TGGCTGAAG 29

(98) INFORMATION FOR SEQ ID NO:97:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:97:

CTGTACGCTA GTGTGTTTCT ACTCACGTGT CTCAGCATTG AT 42

(99) INFORMATION FOR SEQ ID NO:98:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:98:

GTTGGATCCA CATAATGCAT TTTCTC 26

(100) INFORMATION FOR SEQ ID NO:99:

(i) SEQUENCE CHARACTERISTICS:
(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:99:

ATGATTCTCA ACTCTTCTAC TGAAGATGGT ATTAAAAGAA TCCAAGATGA TTGTCCCAAA 60

GCTGGAAGGC ATAATTACAT ATTTGTCATG ATTCCTACTT TATACAGTAT CATCTTTGTG 120

GTGGGAATAT TTGGAAACAG CTTGGTGGTG ATAGTCATTT ACTTTTATAT GAAGCTGAAG 180

ACTGTGGCCA GTGTTTTTCT TTTGAATTTA GCACTGGCTG ACTTATGCTT TTTACTGACT 240

TTGCCACTAT GGGCTGTCTA CACAGCTATG GAATACCGCT GGCCCTTTGG CAATTACCTA 300

TGTAAGATTG CTTCAGCCAG CGTCAGTTTC AACCTGTACG CTAGTGTGTT TCTACTCACG 360

TGTCTCAGCA TTGATCGATA CCTGGCTATT GTTCACCCAA TGAAGTCCCG CCTTCGACGC 420

ACAATGCTTG TAGCCAAAGT CACCTGCATC ATCATTTGGC TGCTGGCAGG CTTGGCCAGT 480

TTGCCAGCTA TAATCCATCG AAATGTATTT TTCATTGAGA ACACCAATAT TACAGTTTGT 540

GCTTTCCATT ATGAGTCCCA AAATTCAACC CTTCCGATAG GGCTGGGCCT GACCAAAAAT 600

ATACTGGGTT TCCTGTTTCC TTTTCTGATC ATTCTTACAA GTTATTTTGG AATTCGAAAA 660

CACTTACTGA AGACGAATAG CTATGGGAAG AACAGGATAA CCCGTGACCA AGTTAAGAAG 720

ATAATTATGG CAATTGTGCT TTTCTTTTTC TTTTCCTGGA TTCCCCACCA AATATTCACT 780

TTTCTGGATG TATTGATTCA ACTAGGCATC ATACGTGACT GTAGAATTGC AGATATTGTG 840

GACACGGCCA TGCCTATCAC CATTTGTATA GCTTATTTTA ACAATTGCCT GAATCCTCTT 900

TTTTATGGCT TTCTGGGGAA AAAATTTAAA AGATATTTTC TCCAGCTTCT AAAATATATT 960

CCCCCAAAAG CCAAATCCCA CTCAAACCTT TCAACAAAAA TGAGCACGCT TTCCTACCGC 1020

CCCTCAGATA ATGTAAGCTC ATCCACCAAG AAGCCTGCAC CATGTTTTGA GGTTGAGTGA 1080

(101) INFORMATION FOR SEQ ID NO:100:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:100:

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Phe Gly Ile Arg Lys His Leu Leu Lys
    210                 215                 220

Thr Asn Ser Tyr Gly Lys Asn Arg Ile Thr Arg Asp Gln Val Lys Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu

        355

(102) INFORMATION FOR SEQ ID NO:101:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:101:

TCCGAATTCC AAAATAACTT GTAAGAATGA TCAGAAA 37

(103) INFORMATION FOR SEQ ID NO:102:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:102:

AGATCTTAAG AAGATAATTA TGGCAATTGT GCT 33

(104) INFORMATION FOR SEQ ID NO:103:

(i) SEQUENCE CHARACTERISTICS:
(A) LENGTH: 62 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:103:

AATTCGAAAA CACTTACTGA AGACGAATAG CTATGGGAAG AACAGGATAA CCCGTGACCA 60

AG 62

(105) INFORMATION FOR SEQ ID NO:104:

(i) SEQUENCE CHARACTERISTICS:
(A) LENGTH: 62 base pairs
(B) TYPE: nucleic acid
(C) STRANDEDNESS: single

(D) TOPOLOGY: linear

(ii) MOLECULE TYPE: DNA (genomic)

(iv) ANTI-SENSE: YES

(xi) SEQUENCE DESCRIPTION: SEQ ID NO:104:

TTAACTTGGT CACGGGTTAT CCTGTTCTTC CCATAGCTAT TCGTCTTCAG TAAGTGTTTT 60

CG 62

(106) INFORMATION FOR SEQ ID NO:105:

(i) SEQUENCE CHARACTERISTICS:
(A) LENGTH: 1083 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:105:

ATGATTCTCA ACTCTTCTAC TGAAGATGGT ATTAAAAGAA TCCAAGATGA TTGTCCCAAA 60

GCTGGAAGGC ATAATTACAT ATTTGTCATG ATTCCTACTT TATACAGTAT CATCTTTGTG 120

GTGGGAATAT TTGGAAACAG CTTGGTGGTG ATAGTCATTT ACTTTTATAT GAAGCTGAAG 180

ACTGTGGCCA GTGTTTTTCT TTTGAATTTA GCACTGGCTG ACTTATGCTT TTTACTGACT 240

TTGCCACTAT GGGCTGTCTA CACAGCTATG GAATACCGCT GGCCCTTTGG CAATTACCTA 300

TGTAAGATTG CTTCAGCCAG CGTCAGTTTC AACCTGTACG CTAGTGTGTT TCTACTCACG 360

TGTCTCAGCA TTGATCGATA CCTGGCTATT GTTCACCCAA TGAAGTCCCG CCTTCGACGC 420

ACAATGCTTG TAGCCAAAGT CACCTGCATC ATCATTTGGC TGCTGGCAGG CTTGGCCAGT 480

TTGCCAGCTA TAATCCATCG AAATGTATTT TTCATTGAGA ACACCAATAT TACAGTTTGT 540

GCTTTCCATT ATGAGTCCCA AAATTCAACC CTTCCGATAG GGCTGGGCCT GACCAAAAAT 600

ATACTGGGTT TCCTGTTTCC TTTTCTGATC ATTCTTACAA GTTATACTCT TATTTGGAAG 660

GCCCTAAAGA AGGCTTATGA AATTCAGAAG AACAAACCAA GAAATGATGA TATTTTTAAG 720

ATAATTATGG CAGCAATTGT GCTTTTCTTT TTCTTTTCCT GGATTCCCCA CCAAATATTC 780

ACTTTTCTGG ATGTATTGAT TCAACTAGGC ATCATACGTG ACTGTAGAAT TGCAGATATT 840

GTGGACACGG CCATGCCTAT CACCATTTGT ATAGCTTATT TTAACAATTG CCTGAATCCT 900

CTTTTTTATG GCTTTCTGGG GAAAAAATTT AAAAGATATT TTCTCCAGCT TCTAAAATAT 960

ATTCCCCCAA AAGCCAAATC CCACTCAAAC CTTTCAACAA AAATGAGCAC GCTTTCCTAC 1020

CGCCCCTCAG ATAATGTAAG CTCATCCACC AAGAAGCCTG CACCATGTTT TGAGGTTGAG 1080

TGA                                                                    1083

(107) INFORMATION FOR SEQ ID NO:106:

     (i) SEQUENCE CHARACTERISTICS:
          (A) LENGTH: 360 amino acids
          (B) TYPE: amino acid
          (C) STRANDEDNESS:
          (D) TOPOLOGY: not relevant

    (ii) MOLECULE TYPE: protein

    (xi) SEQUENCE DESCRIPTION: SEQ ID NO:106:

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Phe Lys
225                 230                 235                 240

Ile Ile Met Ala Ala Ile Val Leu Phe Phe Phe Phe Ser Trp Ile Pro
                245                 250                 255

His Gln Ile Phe Thr Phe Leu Asp Val Leu Ile Gln Leu Gly Ile Ile
            260                 265                 270

Arg Asp Cys Arg Ile Ala Asp Ile Val Asp Thr Ala Met Pro Ile Thr
        275                 280                 285

Ile Cys Ile Ala Tyr Phe Asn Asn Cys Leu Asn Pro Leu Phe Tyr Gly
    290                 295                 300

Phe Leu Gly Lys Lys Phe Lys Arg Tyr Phe Leu Gln Leu Leu Lys Tyr
305                 310                 315                 320

Ile Pro Pro Lys Ala Lys Ser His Ser Asn Leu Ser Thr Lys Met Ser
                325                 330                 335

Thr Leu Ser Tyr Arg Pro Ser Asp Asn Val Ser Ser Ser Thr Lys Lys
            340                 345                 350

Pro Ala Pro Cys Phe Glu Val Glu
        355                 360

(108) INFORMATION FOR SEQ ID NO:107:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:107:

CCCAAGCTTC CCCAGGTGTA TTTGAT                                        26

(109) INFORMATION FOR SEQ ID NO:108:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:108:

AAGCACAATT GCTGCATAAT TATCTTAAAA ATATCATC                           38

(110) INFORMATION FOR SEQ ID NO:109:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:109:

AAGATAATTA TGGCAGCAAT TGTGCTTTTC TTTTTCTTT 39

(111) INFORMATION FOR SEQ ID NO:110:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:110:

GTTGGATCCA CATAATGCAT TTTCTC 26

(112) INFORMATION FOR SEQ ID NO:111:

(i) SEQUENCE CHARACTERISTICS:
(A) LENGTH: 1344 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:111:

ATGGAGCTGC TAAAGCTGAA CCGGAGCGTG CAGGGAACCG GACCCGGGCC GGGGGCTTCC 60

CTGTGCCGCC CGGGGGCGCC TCTCCTCAAC AGCAGCAGTG TGGGCAACCT CAGCTGCGAG 120

CCCCCTCGCA TTCGCGGAGC CGGGACACGA GAATTGGAGC TGGCCATTAG AATCACTCTT 180

TACGCAGTGA TCTTCCTGAT GAGCGTTGGA GGAAATATGC TCATCATCGT GGTCCTGGGA 240

CTGAGCCGCC GCCTGAGGAC TGTCACCAAT GCCTTCCTCC TCTCACTGGC AGTCAGCGAC 300

CTCCTGCTGG CTGTGGCTTG CATGCCCTTC ACCCTCCTGC CCAATCTCAT GGGCACATTC 360

ATCTTTGGCA CCGTCATCTG CAAGGCGGTT TCCTACCTCA TGGGGGTGTC TGTGAGTGTG 420

TCCACGCTAA GCCTCGTGGC CATCGCACTG GAGCGATATA GCGCCATCTG CCGACCACTG 480

CAGGCACGAG TGTGGCAGAC GCGCTCCCAC GCGGCTCGCG TGATTGTAGC CACGTGGCTG 540

CTGTCCGGAC TACTCATGGT GCCCTACCCC GTGTACACTG TCGTGCAACC AGTGGGGCCT 600

CGTGTGCTGC AGTGCGTGCA TCGCTGGCCC AGTGCGCGGG TCCGCCAGAC CTGGTCCGTA 660

CTGCTGCTTC TGCTCTTGTT CTTCATCCCA GGTGTGGTTA TGGCCGTGGC CTACGGGCTT 720

ATCTCTCGCG AGCTCTACTT AGGGCTTCGC TTTGACGGCG ACAGTGACAG CGACAGCCAA 780

AGCAGGGTCC GAAACCAAGG CGGGCTGCCA GGGGCTGTTC ACCAGAACGG GCGTTGCCGG 840

CCTGAGACTG GCGCGGTTGG CAAAGACAGC GATGGCTGCT ACGTGCAACT TCCACGTTCC 900

CGGCCTGCCC TGGAGCTGAC GGCGCTGACG GCTCCTGGGC CGGGATCCGG CTCCCGGCCC 960

ACCCAGGCCA AGCTGCTGGC TAAGAAGCGC GTGAAACGAA TGTTGCTGGT GATCGTTGTG 1020

CTTTTTTTTC TGTGTTGGTT GCCAGTTTAT AGTGCCAACA CGTGGCGCGC CTTTGATGGC 1080

CCGGGTGCAC ACCGAGCACT CTCGGGTGCT CCTATCTCCT TCATTCACTT GCTGAGCTAC 1140

GCCTCGGCCT GTGTCAACCC CCTGGTCTAC TGCTTCATGC ACCGTCGCTT TCGCCAGGCC 1200

TGCCTGGAAA CTTGCGCTCG CTGCTGCCCC CGGCCTCCAC GAGCTCGCCC CAGGGCTCTT 1260

CCCGATGAGG ACCCTCCCAC TCCCTCCATT GCTTCGCTGT CCAGGCTTAG CTACACCACC 1320

ATCAGCACAC TGGGCCCTGG CTGA 1344

(113) INFORMATION FOR SEQ ID NO:112:

(i) SEQUENCE CHARACTERISTICS:
(A) LENGTH: 447 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:112:

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu

            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu
145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Lys Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

(114) INFORMATION FOR SEQ ID NO:113:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:113:

CAGCAGCATG CGCTTCACGC GCTTCTTAGC CCAG 34

(115) INFORMATION FOR SEQ ID NO:114:

(i) SEQUENCE CHARACTERISTICS:
(A) LENGTH: 33 base pairs
(B) TYPE: nucleic acid
(C) STRANDEDNESS: single
(D) TOPOLOGY: not relevant

(ii) MOLECULE TYPE: DNA (genomic)

(xi) SEQUENCE DESCRIPTION: SEQ ID NO:114:

AGAAGCGCGT GAAGCGCATG CTGCTGGTGA TCGTT 35

(116) INFORMATION FOR SEQ ID NO:115:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:115:

ATGGAGAAAA GAATCAAAAG AATGTTCTAT ATA 33

(117) INFORMATION FOR SEQ ID NO:116:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:116:

TATATAGAAC ATTCTTTTGA TTCTTTTCTC CAT 33

(118) INFORMATION FOR SEQ ID NO:117:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:117:

CGCTCTCTGG CCTTGAAGCG CACGCTCAGC 30

(119) INFORMATION FOR SEQ ID NO:118:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:118:

GCTGAGCGTG CGCTTCAAGG CCAGAGAGCG 30

(120) INFORMATION FOR SEQ ID NO:119:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:119:

CCCAGGAAAA AGGTGAAAGT CAAAGTTTTC 30

(121) INFORMATION FOR SEQ ID NO:120:

(i) SEQUENCE CHARACTERISTICS:
(A) LENGTH: 30 base pairs

(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:120:

GAAAACTTTG ACTTTCACCT TTTTCCTGGG 30

(122) INFORMATION FOR SEQ ID NO:121:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:121:

GGGGCGCGGG TGAAACGGCT GGTGAGC 27

(123) INFORMATION FOR SEQ ID NO:122:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:122:

GCTCACCAGC CGTTTCACCC GCGCCCC 27

(124) INFORMATION FOR SEQ ID NO:123:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:123:

CCCCTTGAAA AGCCTAAGAA CTTGGTCATC 30

(125) INFORMATION FOR SEQ ID NO:124:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:124:

GATGACCAAG TTCTTAGGCT TTTCAAGGGG 30

(126) INFORMATION FOR SEQ ID NO:125:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:125:

GATCTCTAGA ATGAACAGCA CATGTATTGA AG 32

(127) INFORMATION FOR SEQ ID NO:126:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:126:

CTAGGGTACC CGCTCAAGGA CCTCTAATTC CATAG 35

(128) INFORMATION FOR SEQ ID NO:127:

(i) SEQUENCE CHARACTERISTICS:
(A) LENGTH: 1296 base pairs

(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:127:

ATGCAGGCGC TTAACATTAC CCCGGAGCAG TTCTCTCGGC TGCTGCGGGA CCACAACCTG 60

ACGCGGGAGC AGTTCATCGC TCTGTACCGG CTGCGACCGC TCGTCTACAC CCCAGAGCTG 120

CCGGGACGCG CCAAGCTGGC CCTCGTGCTC ACCGGCGTGC TCATCTTCGC CCTGGCGCTC 180

TTTGGCAATG CTCTGGTGTT CTACGTGGTG ACCCGCAGCA AGGCCATGCG CACCGTCACC 240

AACATCTTTA TCTGCTCCTT GGCGCTCAGT GACCTGCTCA TCACCTTCTT CTGCATTCCC 300

GTCACCATGC TCCAGAACAT TTCCGACAAC TGGCTGGGGG GTGCTTTCAT TTGCAAGATG 360

GTGCCATTTG TCCAGTCTAC CGCTGTTGTG ACAGAAATGC TCACTATGAC CTGCATTGCT 420

GTGGAAAGGC ACCAGGGACT TGTGCATCCT TTTAAAATGA AGTGGCAATA CACCAACCGA 480

AGGGCTTTCA CAATGCTAGG TGTGGTCTGG CTGGTGGCAG TCATCGTAGG ATCACCCATG 540

TGGCACGTGC AACAACTTGA GATCAAATAT GACTTCCTAT ATGAAAAGGA ACACATCTGC 600

TGCTTAGAAG AGTGGACCAG CCCTGTGCAC CAGAAGATCT ACACCACCTT CATCCTTGTC 660

ATCCTCTTCC TCCTGCCTCT TATGGTGATG CTTATTCTGT ACAGTAAAAT TGGTTATGAA 720

CTTTGGATAA AGAAAAGAGT TGGGGATGGT TCAGTGCTTC GAACTATTCA TGGAAAAGAA 780

ATGTCCAAAA TAGCCAGGAA GAAGAAACGA GCTAAGATTA TGATGGTGAC AGTGGTGGCT 840

CTCTTTGCTG TGTGCTGGGC ACCATTCCAT GTTGTCCATA TGATGATTGA ATACAGTAAT 900

TTTGAAAAGG AATATGATGA TGTCACAATC AAGATGATTT TTGCTATCGT GCAAATTATT 960

GGATTTTCCA ACTCCATCTG TAATCCCATT GTCTATGCAT TTATGAATGA AAACTTCAAA 1020

AAAAATGTTT TGTCTGCAGT TTGTTATTGC ATAGTAAATA AAACCTTCTC TCCAGCACAA 1080

AGGCATGGAA ATTCAGGAAT TACAATGATG CGGAAGAAAG CAAAGTTTTC CCTCAGAGAG 1140

AATCCAGTGG AGGAAACCAA AGGAGAAGCA TTCAGTGATG GCAACATTGA AGTCAAATTG 1200

TGTGAACAGA CAGAGGAGAA GAAAAAGCTC AAACGACATC TTGCTCTCTT TAGGTCTGAA 1260

CTGGCTGAGA ATTCTCCTTT AGACAGTGGG CATTAA 1296

(129) INFORMATION FOR SEQ ID NO:128:

(i) SEQUENCE CHARACTERISTICS:
(A) LENGTH: 431 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:128:

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu
            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Lys
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile
305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

(130) INFORMATION FOR SEQ ID NO:129:

     (i) SEQUENCE CHARACTERISTICS:
          (A) LENGTH: 2040 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:129:

ATGGGCAGCC CCTGGAACGG CAGCGACGGC CCCGAGGGGG CGCGGGAGCC GCCGTGGCCC    60

GCGCTGCCGC CTTGCGACGA GCGCCGCTGC TCGCCCTTTC CCCTGGGGGC GCTGGTGCCG   120

GTGACCGCTG TGTGCCTGTG CCTGTTCGTC GTCGGGGTGA GCGGCAACGT GGTGACCGTG   180

ATGCTGATCG GGCGCTACCG GGACATGCGG ACCACCACCA ACTTGTACCT GGGCAGCATG   240

GCCGTGTCCG ACCTACTCAT CCTGCTCGGG CTGCCGTTCG ACCTGTACCG CCTCTGGCGC   300

TCGCGGCCCT GGGTGTTCGG GCCGCTGCTC TGCCGCCTGT CCCTCTACGT GGGCGAGGGC   360

TGCACCTACG CCACGCTGCT GCACATGACC GCGCTCAGCG TCGAGCGCTA CCTGGCCATC   420

TGCCGCCCGC TCCGCGCCCG CGTCTTGGTC ACCCGGCGCC GCGTCCGCGC GCTCATCGCT   480

GTGCTCTGGG CCGTGGCGCT GCTCTCTGCC GGTCCCTTCT TGTTCCTGGT GGGCGTCGAG   540

CAGGACCCCG GCATCTCCGT AGTCCCGGGC CTCAATGGCA CCGCGCGGAT CGCCTCCTCG   600

CCTCTCGCCT CGTCGCCGCC TCTCTGGCTC TCGCGGGCGC CACCGCCGTC CCCGCCGTCG   660

GGGCCCGAGA CCGCGGAGGC CGCGGCGCTG TTCAGCCGCG AATGCCGGCC GAGCCCCGCG 720

CAGCTGGGCG CGCTGCGTGT CATGCTGTGG GTCACCACCG CCTACTTCTT CCTGCCCTTT 780

CTGTGCCTCA GCATCCTCTA CGGGCTCATC GGGCGGGAGC TGTGGAGCAG CCGGCGGCCG 840

CTGCGAGGCC CGGCCGCCTC GGGGCGGGAG AGAGGCCACC GGCAGACCAA ACGCGTCCTG 900

CGTAAGTGGA GCCGCCGTGG TTCCAAAGAC GCCTGCCTGC AGTCCGCCCC GCCGGGGACC 960

GCGCAAACGC TGGGTCCCCT TCCCCTGCTC GCCCAGCTCT GGGCGCCGCT TCCAGCTCCC 1020

TTTCCTATTT CGATTCCAGC CTCCACCCGC CGGTACTTCC CATCCCCCGA GAAAACCATG 1080

TCCTGTCCCC CAGGAGCTCT GGGGGACCCC AGGGCGCTTT GAGGGTGGGA TCCCCGGATC 1140

CGATTCAGTA ACCAGCAGTG CTTTTCCAGA GCCTCTGAGA CCAGAAAGGA GAGTTGGTAA 1200

TTCTTAATCC AACCACCTGT TAGATGCCAC AAATGAGGAG TCCTCACAGT GCTCTTGAGA 1260

AGACGAGGGA GATTTCATTA AGCTAAAATT TTTTATTTAA TGTTAAGTGA TGCTGAAGGC 1320

TAAAGTAAAC CTTGCTCGTA TCAAAAAGTA AAGATTGTGC AGACCTGTTG TAGAATTCTT 1380

TTCAACAGAG AACAGAAAAC TTGTCTCCGA AGTGGGTTTG TGGAAGGAAG CCTGCCAAGG 1440

CGGCTTGTTC AGAGAAATTG CTCCTTCTGG TTTATGTCCA GCCTTGATAA CACATATGGG 1500

AGCCTACTAT GCAGTTTTAA AGCAAGTATC CATGCAGCCT GCAGCCTGGT CATTTTTTCT 1560

GGGGTGAGGA TCTGCCTAGG TAGAAGTTTT CTCTAATTTA TTTTGCTGTT ACTTGTTATT 1620

GCAGATGGTT CCTTGTCGGG GTGGGGGGTT TATTTGCTTC CCAATGCTTT TGTTAATCCC 1680

GGTGCTGTGT CTTATGTTGC AGTGGTGGTG GTTCTGGCAT TTATAATTTG CTGGTTGCCC 1740

TTCCACGTTG GCAGAATCAT TTACATAAAC ACGGAAGATT CGCGGATGAT GTACTTCTCT 1800

CAGTACTTTA ACATCGTCGC TCTGCAACTT TTCTATCTGA GCGCATCTAT CAACCCAATC 1860

CTCTACAACC TCATTTCAAA GAAGTACAGA GCGGCGGCCT TTAAACTGCT GCTCGCAAGG 1920

AAGTCCAGGC CGAGAGGCTT CCACAGAAGC AGGGACACTG CGGGGGAAGT TGCAGGGGAC 1980

ACTGGAGGAG ACACGGTGGG CTACACCGAG ACAAGCGCTA ACGTGAAGAC GATGGGATAA 2040

(131) INFORMATION FOR SEQ ID NO:130:

(i) SEQUENCE CHARACTERISTICS:
(A) LENGTH: 412 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:130:

Met Gly Ser Pro Trp Asn Gly Ser Asp Gly Pro Glu Gly Ala Arg Glu
1               5                   10                  15

Pro Pro Trp Pro Ala Leu Pro Pro Cys Asp Glu Arg Arg Cys Ser Pro
            20                  25                  30

Phe Pro Leu Gly Ala Leu Val Pro Val Thr Ala Val Cys Leu Cys Leu
        35                  40                  45

Phe Val Val Gly Val Ser Gly Asn Val Val Thr Val Met Leu Ile Gly
    50                  55                  60

Arg Tyr Arg Asp Met Arg Thr Thr Thr Asn Leu Tyr Leu Gly Ser Met
65                  70                  75                  80

Ala Val Ser Asp Leu Leu Ile Leu Leu Gly Leu Pro Phe Asp Leu Tyr
                85                  90                  95

Arg Leu Trp Arg Ser Arg Pro Trp Val Phe Gly Pro Leu Leu Cys Arg
            100                 105                 110

Leu Ser Leu Tyr Val Gly Glu Gly Cys Thr Tyr Ala Thr Leu Leu His
        115                 120                 125

Met Thr Ala Leu Ser Val Glu Arg Tyr Leu Ala Ile Cys Arg Pro Leu
    130                 135                 140

Arg Ala Arg Val Leu Val Thr Arg Arg Arg Val Arg Ala Leu Ile Ala
145                 150                 155                 160

Val Leu Trp Ala Val Ala Leu Leu Ser Ala Gly Pro Phe Leu Phe Leu
                165                 170                 175

Val Gly Val Glu Gln Asp Pro Gly Ile Ser Val Val Pro Gly Leu Asn
            180                 185                 190

Gly Thr Ala Arg Ile Ala Ser Ser Pro Leu Ala Ser Ser Pro Pro Leu
        195                 200                 205

Trp Leu Ser Arg Ala Pro Pro Pro Ser Pro Pro Ser Gly Pro Glu Thr
    210                 215                 220

Ala Glu Ala Ala Ala Leu Phe Ser Arg Glu Cys Arg Pro Ser Pro Ala
225                 230                 235                 240

Gln Leu Gly Ala Leu Arg Val Met Leu Trp Val Thr Thr Ala Tyr Phe
                245                 250                 255

Phe Leu Pro Phe Leu Cys Leu Ser Ile Leu Tyr Gly Leu Ile Gly Arg
            260                 265                 270

Glu Leu Trp Ser Ser Arg Arg Pro Leu Arg Gly Pro Ala Ala Ser Gly
        275                 280                 285

Arg Glu Arg Gly His Arg Gln Thr Lys Arg Val Leu Leu Val Val Val
    290                 295                 300

Leu Ala Phe Ile Ile Cys Trp Leu Pro Phe His Val Gly Arg Ile Ile
305                 310                 315                 320

Tyr Ile Asn Thr Glu Asp Ser Arg Met Met Tyr Phe Ser Gln Tyr Phe
                325                 330                 335

Asn Ile Val Ala Leu Gln Leu Phe Tyr Leu Ser Ala Ser Ile Asn Pro
            340                 345                 350

Ile Leu Tyr Asn Leu Ile Ser Lys Lys Tyr Arg Ala Ala Ala Phe Lys
        355                 360                 365

Leu Leu Leu Ala Arg Lys Ser Arg Pro Arg Gly Phe His Arg Ser Arg
    370                 375                 380

Asp Thr Ala Gly Glu Val Ala Gly Asp Thr Gly Gly Asp Thr Val Gly
385                 390                 395                 400

Tyr Thr Glu Thr Ser Ala Asn Val Lys Thr Met Gly
                405                 410

(132) INFORMATION FOR SEQ ID NO:131:

(i) SEQUENCE CHARACTERISTICS:
(A) LENGTH: 1344 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:131:

ATGGAGCTGC TAAAGCTGAA CCGGAGCGTG CAGGGAACCG GACCCGGGCC GGGGGCTTCC      60

CTGTGCCGCC CGGGGGCGCC TCTCCTCAAC AGCAGCAGTG TGGGCAACCT CAGCTGCGAG     120

CCCCCTCGCA TTCGCGGAGC CGGGACACGA GAATTGGAGC TGGCCATTAG AATCACTCTT     180

TACGCAGTGA TCTTCCTGAT GAGCGTTGGA GGAAATATGC TCATCATCGT GGTCCTGGGA     240

CTGAGCCGCC GCCTGAGGAC TGTCACCAAT GCCTTCCTCC TCTCACTGGC AGTCAGCGAC     300

CTCCTGCTGG CTGTGGCTTG CATGCCCTTC ACCCTCCTGC CCAATCTCAT GGGCACATTC     360

ATCTTTGGCA CCGTCATCTG CAAGGCGGTT TCCTACCTCA TGGGGGTGTC TGTGAGTGTG     420

TCCACGCTAA GCCTCGTGGC CATCGCACTG GAGCGATATA GCGCCATCTG CCGACCACTG     480

CAGGCACGAG TGTGGCAGAC GCGCTCCCAC GCGGCTCGCG TGATTGTAGC CACGTGGCTG     540

CTGTCCGGAC TACTCATGGT GCCCTACCCC GTGTACACTG TCGTGCAACC AGTGGGGCCT     600

CGTGTGCTGC AGTGCGTGCA TCGCTGGCCC AGTGCGCGGG TCCGCCAGAC CTGGTCCGTA     660

CTGCTGCTTC TGCTCTTGTT CTTCATCCCA GGTGTGGTTA TGGCCGTGGC CTACGGGCTT     720

ATCTCTCGCG AGCTCTACTT AGGGCTTCGC TTTGACGGCG ACAGTGACAG CGACAGCCAA     780

AGCAGGGTCC GAAACCAAGG CGGGCTGCCA GGGGCTGTTC ACCAGAACGG GCGTTGCCGG     840

CCTGAGACTG GCGCGGTTGG CAAAGACAGC GATGGCTGCT ACGTGCAACT TCCACGTTCC     900

CGGCCTGCCC TGGAGCTGAC GGCGCTGACG GCTCCTGGGC CGGGATCCGG CTCCCGGCCC 960

ACCCAGGCCA AGCTGCTGGC TAAGAAGCGC GTGAAACGAA TGTTGCTGGT GATCGTTGTG 1020

CTTTTTTTTC TGTGTTGGTT GCCAGTTTAT AGTGCCAACA CGTGGCGCGC CTTTGATGGC 1080

CCGGGTGCAC ACCGAGCACT CTCGGGTGCT CCTATCTCCT TCATTCACTT GCTGAGCTAC 1140

GCCTCGGCCT GTGTCAACCC CCTGGTCTAC TGCTTCATGC ACCGTCGCTT TCGCCAGGCC 1200

TGCCTGGAAA CTTGCGCTCG CTGCTGCCCC CGGCCTCCAC GAGCTCGCCC CAGGGCTCTT 1260

CCCGATGAGG ACCCTCCCAC TCCCTCCATT GCTTCGCTGT CCAGGCTTAG CTACACCACC 1320

ATCAGCACAC TGGGCCCTGG CTGA 1344

(133) INFORMATION FOR SEQ ID NO:132:

(i) SEQUENCE CHARACTERISTICS:
(A) LENGTH: 447 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:132:

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu
            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu
145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Lys Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

(134) INFORMATION FOR SEQ ID NO:133:

     (i) SEQUENCE CHARACTERISTICS:
          (A) LENGTH: 1014 base pairs
          (B) TYPE: nucleic acid
          (C) STRANDEDNESS: single
          (D) TOPOLOGY: linear

    (ii) MOLECULE TYPE: DNA (genomic)

(xi) SEQUENCE DESCRIPTION: SEQ ID NO:133:

ATGAACAGCA CATGTATTGA AGAACAGCAT GACCTGGATC ACTATTTGTT TCCCATTGTT 60

TACATCTTTG TGATTATAGT CAGCATTCCA GCCAATATTG GATCTCTGTG TGTGTCTTTC 120

CTGCAAGCAA AGAAGGAAAG TGAACTAGGA ATTTACCTCT TCAGTTTGTC ACTATCAGAT 180

TTACTCTATG CATTAACTCT CCCTTTATGG ATTGATTATA CTTGGAATAA AGACAACTGG 240

ACTTTCTCTC CTGCCTTGTG CAAAGGGAGT GCTTTTCTCA TGTACATGAA TTTTTACAGC 300

AGCACAGCAT TCCTCACCTG CATTGCCGTT GATCGGTATT TGGCTGTTGT CTACCCTTTG 360

AAGTTTTTTT TCCTAAGGAC AAGAAGATTT GCACTCATGG TCAGCCTGTC CATCTGGATA 420

TTGGAAACCA TCTTCAATGC TGTCATGTTG TGGGAAGATG AAACAGTTGT TGAATATTGC 480

GATGCCGAAA AGTCTAATTT TACTTTATGC TATGACAAAT ACCCTTTAGA GAAATGGCAA 540

ATCAACCTCA ACTTGTTCAG GACGTGTACA GGCTATGCAA TACCTTTGGT CACCATCCTG 600

ATCTGTAACC GGAAAGTCTA CCAAGCTGTG CGGCACAATA AAGCCACGGA AAACAAGGAA 660

AAGAAGAGAA TCAAAAAACT ACTTGTCAGC ATCACAGTTA CTTTTGTCTT ATGCTTTACT 720

CCCTTTCATG TGATGTTGCT GATTCGCTGC ATTTTAGAGC ATGCTGTGAA CTTCGAAGAC 780

CACAGCAATT CTGGGAAGCG AACTTACACA ATGTATAGAA TCACGGTTGC ATTAACAAGT 840

TTAAATTGTG TTGCTGATCC AATTCTGTAC TGTTTTGTTA CCGAAACAGG AAGATATGAT 900

ATGTGGAATA TATTAAAATT CTGCACTGGG AGGTGTAATA CATCACAAAG ACAAAGAAAA 960

CGCATACTTT CTGTGTCTAC AAAAGATACT ATGGAATTAG AGGTCCTTGA GTAG 1014

(135) INFORMATION FOR SEQ ID NO:134:

(i) SEQUENCE CHARACTERISTICS:
(A) LENGTH: 337 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:134:

Met Asn Ser Thr Cys Ile Glu Glu Gln His Asp Leu Asp His Tyr Leu
1               5                   10                  15

Phe Pro Ile Val Tyr Ile Phe Val Ile Ile Val Ser Ile Pro Ala Asn
            20                  25                  30

Ile Gly Ser Leu Cys Val Ser Phe Leu Gln Ala Lys Lys Glu Ser Glu
        35                  40                  45

Leu Gly Ile Tyr Leu Phe Ser Leu Ser Leu Ser Asp Leu Leu Tyr Ala
    50                  55                  60

Leu Thr Leu Pro Leu Trp Ile Asp Tyr Thr Trp Asn Lys Asp Asn Trp
65                  70                  75                  80

Thr Phe Ser Pro Ala Leu Cys Lys Gly Ser Ala Phe Leu Met Tyr Met
                85                  90                  95

Asn Phe Tyr Ser Ser Thr Ala Phe Leu Thr Cys Ile Ala Val Asp Arg
            100                 105                 110

Tyr Leu Ala Val Val Tyr Pro Leu Lys Phe Phe Phe Leu Arg Thr Arg
        115                 120                 125

Arg Phe Ala Leu Met Val Ser Leu Ser Ile Trp Ile Leu Glu Thr Ile
    130                 135                 140

Phe Asn Ala Val Met Leu Trp Glu Asp Glu Thr Val Val Glu Tyr Cys
145                 150                 155                 160

Asp Ala Glu Lys Ser Asn Phe Thr Leu Cys Tyr Asp Lys Tyr Pro Leu
                165                 170                 175

Glu Lys Trp Gln Ile Asn Leu Asn Leu Phe Arg Thr Cys Thr Gly Tyr
            180                 185                 190

Ala Ile Pro Leu Val Thr Ile Leu Ile Cys Asn Arg Lys Val Tyr Gln
        195                 200                 205

Ala Val Arg His Asn Lys Ala Thr Glu Asn Lys Glu Lys Lys Arg Ile
    210                 215                 220

Lys Lys Leu Leu Val Ser Ile Thr Val Thr Phe Val Leu Cys Phe Thr
225                 230                 235                 240

Pro Phe His Val Met Leu Leu Ile Arg Cys Ile Leu Glu His Ala Val
                245                 250                 255

Asn Phe Glu Asp His Ser Asn Ser Gly Lys Arg Thr Tyr Thr Met Tyr
            260                 265                 270

Arg Ile Thr Val Ala Leu Thr Ser Leu Asn Cys Val Ala Asp Pro Ile
        275                 280                 285

Leu Tyr Cys Phe Val Thr Glu Thr Gly Arg Tyr Asp Met Trp Asn Ile
    290                 295                 300

Leu Lys Phe Cys Thr Gly Arg Cys Asn Thr Ser Gln Arg Gln Arg Lys
305                 310                 315                 320

Arg Ile Leu Ser Val Ser Thr Lys Asp Thr Met Glu Leu Glu Val Leu
                325                 330                 335

Glu

(136) INFORMATION FOR SEQ ID NO:135:

     (i) SEQUENCE CHARACTERISTICS:
          (A) LENGTH: 999 base pairs

(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:135:

ATGGTGAACT CCACCCACCG TGGGATGCAC ACTTCTCTGC ACCTCTGGAA CCGCAGCAGT 60

TACAGACTGC ACAGCAATGC CAGTGAGTCC CTTGGAAAAG GCTACTCTGA TGGAGGGTGC 120

TACGAGCAAC TTTTTGTCTC TCCTGAGGTG TTTGTGACTC TGGGTGTCAT CAGCTTGTTG 180

GAGAATATCT TAGTGATTGT GGCAATAGCC AAGAACAAGA ATCTGCATTC ACCCATGTAC 240

TTTTTCATCT GCAGCTTGGC TGTGGCTGAT ATGCTGGTGA GCGTTTCAAA TGGATCAGAA 300

ACCATTATCA TCACCCTATT AAACAGTACA GATACGGATG CACAGAGTTT CACAGTGAAT 360

ATTGATAATG TCATTGACTC GGTGATCTGT AGCTCCTTGC TTGCATCCAT TTGCAGCCTG 420

CTTTCAATTG CAGTGGACAG GTACTTTACT ATCTTCTATG CTCTCCAGTA CCATAACATT 480

ATGACAGTTA AGCGGGTTGG GATCAGCATA AGTTGTATCT GGGCAGCTTG CACGGTTTCA 540

GGCATTTTGT TCATCATTTA CTCAGATAGT AGTGCTGTCA TCATCTGCCT CATCACCATG 600

TTCTTCACCA TGCTGGCTCT CATGGCTTCT CTCTATGTCC ACATGTTCCT GATGGCCAGG 660

CTTCACATTA AGAGGATTGC TGTCCTCCCC GGCACTGGTG CCATCCGCCA AGGTGCCAAT 720

ATGAAGGGAA AAATTACCTT GACCATCCTG ATTGGCGTCT TTGTTGTCTG CTGGGCCCCA 780

TTCTTCCTCC ACTTAATATT CTACATCTCT TGTCCTCAGA ATCCATATTG TGTGTGCTTC 840

ATGTCTCACT TTAACTTGTA TCTCATACTG ATCATGTGTA ATTCAATCAT CGATCCTCTG 900

ATTTATGCAC TCCGGAGTCA AGAACTGAGG AAAACCTTCA AAGAGATCAT CTGTTGCTAT 960

CCCCTGGGAG GCCTTTGTGA CTTGTCTAGC AGATATTAA 999

(137) INFORMATION FOR SEQ ID NO:136:

(i) SEQUENCE CHARACTERISTICS:
(A) LENGTH: 332 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:136:

Met Val Asn Ser Thr His Arg Gly Met His Thr Ser Leu His Leu Trp
1               5                   10                  15

Asn Arg Ser Ser Tyr Arg Leu His Ser Asn Ala Ser Glu Ser Leu Gly
            20                  25                  30

Lys Gly Tyr Ser Asp Gly Gly Cys Tyr Glu Gln Leu Phe Val Ser Pro
        35                  40                  45

Glu Val Phe Val Thr Leu Gly Val Ile Ser Leu Leu Glu Asn Ile Leu
    50                  55                  60

Val Ile Val Ala Ile Ala Lys Asn Lys Asn Leu His Ser Pro Met Tyr
65                  70                  75                  80

Phe Phe Ile Cys Ser Leu Ala Val Ala Asp Met Leu Val Ser Val Ser
                85                  90                  95

Asn Gly Ser Glu Thr Ile Ile Ile Thr Leu Leu Asn Ser Thr Asp Thr
            100                 105                 110

Asp Ala Gln Ser Phe Thr Val Asn Ile Asp Asn Val Ile Asp Ser Val
        115                 120                 125

Ile Cys Ser Ser Leu Leu Ala Ser Ile Cys Ser Leu Leu Ser Ile Ala
    130                 135                 140

Val Asp Arg Tyr Phe Thr Ile Phe Tyr Ala Leu Gln Tyr His Asn Ile
145                 150                 155                 160

Met Thr Val Lys Arg Val Gly Ile Ser Ile Ser Cys Ile Trp Ala Ala
                165                 170                 175

Cys Thr Val Ser Gly Ile Leu Phe Ile Ile Tyr Ser Asp Ser Ser Ala
            180                 185                 190

Val Ile Ile Cys Leu Ile Thr Met Phe Phe Thr Met Leu Ala Leu Met
        195                 200                 205

Ala Ser Leu Tyr Val His Met Phe Leu Met Ala Arg Leu His Ile Lys
    210                 215                 220

Arg Ile Ala Val Leu Pro Gly Thr Gly Ala Ile Arg Gln Gly Ala Asn
225                 230                 235                 240

Met Lys Gly Lys Ile Thr Leu Thr Ile Leu Ile Gly Val Phe Val Val
                245                 250                 255

Cys Trp Ala Pro Phe Phe Leu His Leu Ile Phe Tyr Ile Ser Cys Pro
            260                 265                 270

Gln Asn Pro Tyr Cys Val Cys Phe Met Ser His Phe Asn Leu Tyr Leu
        275                 280                 285

Ile Leu Ile Met Cys Asn Ser Ile Ile Asp Pro Leu Ile Tyr Ala Leu
    290                 295                 300

Arg Ser Gln Glu Leu Arg Lys Thr Phe Lys Glu Ile Ile Cys Cys Tyr
305                 310                 315                 320

Pro Leu Gly Gly Leu Cys Asp Leu Ser Ser Arg Tyr
                325                 330

(138) INFORMATION FOR SEQ ID NO:137:

    (i) SEQUENCE CHARACTERISTICS:

(A) LENGTH: 33 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:137:

GCCAATATGA AGGGAAAAAT TACCTTGACC ATC 33

(137) INFORMATION FOR SEQ ID NO:138:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:138:

CTCCTTCGGT CCTCCTATCG TTGTCAGAAG T 31

(140) INFORMATION FOR SEQ ID NO:139:

(i) SEQUENCE CHARACTERISTICS:
(A) LENGTH: 1842 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:139:

ATGGGGCCCA CCCTAGCGGT TCCCACCCCC TATGGCTGTA TTGGCTGTAA GCTACCCCAG 60

CCAGAATACC CACCGGCTCT AATCATCTTT ATGTTCTGCG CGATGGTTAT CACCATCGTT 120

GTAGACCTAA TCGGCAACTC CATGGTCATT TTGGCTGTGA CGAAGAACAA GAAGCTCCGG 180

AATTCTGGCA ACATCTTCGT GGTCAGTCTC TCTGTGGCCG ATATGCTGGT GGCCATCTAC 240

CCATACCCTT TGATGCTGCA TGCCATGTCC ATTGGGGGCT GGGATCTGAG CCAGTTACAG 300

TGCCAGATGG TCGGGTTCAT CACAGGGCTG AGTGTGGTCG GCTCCATCTT CAACATCGTG 360

GCAATCGCTA TCAACCGTTA CTGCTACATC TGCCACAGCC TCCAGTACGA ACGGATCTTC 420

AGTGTGCGCA ATACCTGCAT CTACCTGGTC ATCACCTGGA TCATGACCGT CCTGGCTGTC 480

CTGCCCAACA TGTACATTGG CACCATCGAG TACGATCCTC GCACCTACAC CTGCATCTTC 540

AACTATCTGA ACAACCCTGT CTTCACTGTT ACCATCGTCT GCATCCACTT CGTCCTCCCT 600

CTCCTCATCG TGGGTTTCTG CTACGTGAGG ATCTGGACCA AAGTGCTGGC GGCCCGTGAC 660

CCTGCAGGGC AGAATCCTGA CAACCAACTT GCTGAGGTTC GCAATTTTCT AACCATGTTT 720

GTGATCTTCC TCCTCTTTGC AGTGTGCTGG TGCCCTATCA ACGTGCTCAC TGTCTTGGTG 780

GCTGTCAGTC CGAAGGAGAT GGCAGGCAAG ATCCCCAACT GGCTTTATCT TGCAGCCTAC 840

TTCATAGCCT ACTTCAACAG CTGCCTCAAC GCTGTGATCT ACGGGCTCCT CAATGAGAAT 900

TTCCGAAGAG AATACTGGAC CATCTTCCAT GCTATGCGGC ACCCTATCAT ATTCTTCCCT 960

GGCCTCATCA GTGATATTCG TGAGATGCAG GAGGCCCGTA CCCTGGCCCG CGCCCGTGCC 1020

CATGCTCGCG ACCAAGCTCG TGAACAAGAC CGTGCCCATG CCTGTCCTGC TGTGGAGGAA 1080

ACCCCGATGA ATGTCCGGAA TGTTCCATTA CCTGGTGATG CTGCAGCTGG CCACCCCGAC 1140

CGTGCCTCTG GCCACCCTAA GCCCCATTCC AGATCCTCCT CTGCCTATCG CAAATCTGCC 1200

TCTACCCACC ACAAGTCTGT CTTTAGCCAC TCCAAGGCTG CCTCTGGTCA CCTCAAGCCT 1260

GTCTCTGGCC ACTCCAAGCC TGCCTCTGGT CACCCCAAGT CTGCCACTGT CTACCCTAAG 1320

CCTGCCTCTG TCCATTTCAA GGGTGACTCT GTCCATTTCA AGGGTGACTC TGTCCATTTC 1380

AAGCCTGACT CTGTTCATTT CAAGCCTGCT TCCAGCAACC CCAAGCCCAT CACTGGCCAC 1440

CATGTCTCTG CTGGCAGCCA CTCCAAGTCT GCCTTCAGTG CTGCCACCAG CCACCCTAAA 1500

CCCATCAAGC CAGCTACCAG CCATGCTGAG CCCACCACTG CTGACTATCC CAAGCCTGCC 1560

ACTACCAGCC ACCCTAAGCC CGCTGCTGCT GACAACCCTG AGCTCTCTGC CTCCCATTGC 1620

CCCGAGATCC CTGCCATTGC CCACCCTGTG TCTGACGACA GTGACCTCCC TGAGTCGGCC 1680

TCTAGCCCTG CCGCTGGGCC CACCAAGCCT GCTGCCAGCC AGCTGGAGTC TGACACCATC 1740

GCTGACCTTC CTGACCCTAC TGTAGTCACT ACCAGTACCA ATGATTACCA TGATGTCGTG 1800

GTTGTTGATG TTGAAGATGA TCCTGATGAA ATGGCTGTGT GA 1842

(141) INFORMATION FOR SEQ ID NO:140:

(i) SEQUENCE CHARACTERISTICS:
(A) LENGTH: 613 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:140:

Met Gly Pro Thr Leu Ala Val Pro Thr Pro Tyr Gly Cys Ile Gly Cys
1               5                   10                  15

Lys Leu Pro Gln Pro Glu Tyr Pro Pro Ala Leu Ile Ile Phe Met Phe
            20                  25                  30

Cys Ala Met Val Ile Thr Ile Val Val Asp Leu Ile Gly Asn Ser Met

        35                  40                  45

Val Ile Leu Ala Val Thr Lys Asn Lys Lys Leu Arg Asn Ser Gly Asn
    50                  55                  60

Ile Phe Val Val Ser Leu Ser Val Ala Asp Met Leu Val Ala Ile Tyr
65                  70                  75                  80

Pro Tyr Pro Leu Met Leu His Ala Met Ser Ile Gly Gly Trp Asp Leu
                85                  90                  95

Ser Gln Leu Gln Cys Gln Met Val Gly Phe Ile Thr Gly Leu Ser Val
            100                 105                 110

Val Gly Ser Ile Phe Asn Ile Val Ala Ile Ala Ile Asn Arg Tyr Cys
        115                 120                 125

Tyr Ile Cys His Ser Leu Gln Tyr Glu Arg Ile Phe Ser Val Arg Asn
    130                 135                 140

Thr Cys Ile Tyr Leu Val Ile Thr Trp Ile Met Thr Val Leu Ala Val
145                 150                 155                 160

Leu Pro Asn Met Tyr Ile Gly Thr Ile Glu Tyr Asp Pro Arg Thr Tyr
                165                 170                 175

Thr Cys Ile Phe Asn Tyr Leu Asn Asn Pro Val Phe Thr Val Thr Ile
            180                 185                 190

Val Cys Ile His Phe Val Leu Pro Leu Leu Ile Val Gly Phe Cys Tyr
        195                 200                 205

Val Arg Ile Trp Thr Lys Val Leu Ala Ala Arg Asp Pro Ala Gly Gln
    210                 215                 220

Asn Pro Asp Asn Gln Leu Ala Glu Val Arg Asn Phe Leu Thr Met Phe
225                 230                 235                 240

Val Ile Phe Leu Leu Phe Ala Val Cys Trp Cys Pro Ile Asn Val Leu
                245                 250                 255

Thr Val Leu Val Ala Val Ser Pro Lys Glu Met Ala Gly Lys Ile Pro
            260                 265                 270

Asn Trp Leu Tyr Leu Ala Ala Tyr Phe Ile Ala Tyr Phe Asn Ser Cys
        275                 280                 285

Leu Asn Ala Val Ile Tyr Gly Leu Leu Asn Glu Asn Phe Arg Arg Glu
    290                 295                 300

Tyr Trp Thr Ile Phe His Ala Met Arg His Pro Ile Ile Phe Phe Pro
305                 310                 315                 320

Gly Leu Ile Ser Asp Ile Arg Glu Met Gln Glu Ala Arg Thr Leu Ala
                325                 330                 335

Arg Ala Arg Ala His Ala Arg Asp Gln Ala Arg Glu Gln Asp Arg Ala
            340                 345                 350

His Ala Cys Pro Ala Val Glu Glu Thr Pro Met Asn Val Arg Asn Val
        355                 360                 365

Pro Leu Pro Gly Asp Ala Ala Ala Gly His Pro Asp Arg Ala Ser Gly
    370                 375                 380

His Pro Lys Pro His Ser Arg Ser Ser Ser Ala Tyr Arg Lys Ser Ala
385                 390                 395                 400

Ser Thr His His Lys Ser Val Phe Ser His Ser Lys Ala Ala Ser Gly
                405                 410                 415

His Leu Lys Pro Val Ser Gly His Ser Lys Pro Ala Ser Gly His Pro
            420                 425                 430

Lys Ser Ala Thr Val Tyr Pro Lys Pro Ala Ser Val His Phe Lys Gly
        435                 440                 445

Asp Ser Val His Phe Lys Gly Asp Ser Val His Phe Lys Pro Asp Ser
    450                 455                 460

Val His Phe Lys Pro Ala Ser Ser Asn Pro Lys Pro Ile Thr Gly His
465                 470                 475                 480

His Val Ser Ala Gly Ser His Ser Lys Ser Ala Phe Ser Ala Ala Thr
                485                 490                 495

Ser His Pro Lys Pro Ile Lys Pro Ala Thr Ser His Ala Glu Pro Thr
            500                 505                 510

Thr Ala Asp Tyr Pro Lys Pro Ala Thr Thr Ser His Pro Lys Pro Ala
        515                 520                 525

Ala Ala Asp Asn Pro Glu Leu Ser Ala Ser His Cys Pro Glu Ile Pro
    530                 535                 540

Ala Ile Ala His Pro Val Ser Asp Asp Ser Asp Leu Pro Glu Ser Ala
545                 550                 555                 560

Ser Ser Pro Ala Ala Gly Pro Thr Lys Pro Ala Ala Ser Gln Leu Glu
                565                 570                 575

Ser Asp Thr Ile Ala Asp Leu Pro Asp Pro Thr Val Val Thr Thr Ser
            580                 585                 590

Thr Asn Asp Tyr His Asp Val Val Val Val Asp Val Glu Asp Asp Pro
        595                 600                 605

Asp Glu Met Ala Val
    610

(142) INFORMATION FOR SEQ ID NO:141:

     (i) SEQUENCE CHARACTERISTICS:
          (A) LENGTH: 1842 base pairs
          (B) TYPE: nucleic acid
          (C) STRANDEDNESS: single
          (D) TOPOLOGY: linear

    (ii) MOLECULE TYPE: DNA (genomic)

(xi) SEQUENCE DESCRIPTION: SEQ ID NO:141:

ATGGGGCCCA CCCTAGCGGT TCCCACCCCC TATGGCTGTA TTGGCTGTAA GCTACCCCAG 60

CCAGAATACC CACCGGCTCT AATCATCTTT ATGTTCTGCG CGATGGTTAT CACCATCGTT 120

GTAGACCTAA TCGGCAACTC CATGGTCATT TTGGCTGTGA CGAAGAACAA GAAGCTCCGG 180

AATTCTGGCA ACATCTTCGT GGTCAGTCTC TCTGTGGCCG ATATGCTGGT GGCCATCTAC 240

CCATACCCTT TGATGCTGCA TGCCATGTCC ATTGGGGGCT GGGATCTGAG CCAGTTACAG 300

TGCCAGATGG TCGGGTTCAT CACAGGGCTG AGTGTGGTCG GCTCCATCTT CAACATCGTG 360

GCAATCGCTA TCAACCGTTA CTGCTACATC TGCCACAGCC TCCAGTACGA ACGGATCTTC 420

AGTGTGCGCA ATACCTGCAT CTACCTGGTC ATCACCTGGA TCATGACCGT CCTGGCTGTC 480

CTGCCCAACA TGTACATTGG CACCATCGAG TACGATCCTC GCACCTACAC CTGCATCTTC 540

AACTATCTGA ACAACCCTGT CTTCACTGTT ACCATCGTCT GCATCCACTT CGTCCTCCCT 600

CTCCTCATCG TGGGTTTCTG CTACGTGAGG ATCTGGACCA AAGTGCTGGC GGCCCGTGAC 660

CCTGCAGGGC AGAATCCTGA CAACCAACTT GCTGAGGTTC GCAATAAACT AACCATGTTT 720

GTGATCTTCC TCCTCTTTGC AGTGTGCTGG TGCCCTATCA ACGTGCTCAC TGTCTTGGTG 780

GCTGTCAGTC CGAAGGAGAT GGCAGGCAAG ATCCCCAACT GGCTTTATCT TGCAGCCTAC 840

TTCATAGCCT ACTTCAACAG CTGCCTCAAC GCTGTGATCT ACGGGCTCCT CAATGAGAAT 900

TTCCGAAGAG AATACTGGAC CATCTTCCAT GCTATGCGGC ACCCTATCAT ATTCTTCTCT 960

GGCCTCATCA GTGATATTCG TGAGATGCAG GAGGCCCGTA CCCTGGCCCG CGCCCGTGCC 1020

CATGCTCGCG ACCAAGCTCG TGAACAAGAC CGTGCCCATG CCTGTCCTGC TGTGGAGGAA 1080

ACCCCGATGA ATGTCCGGAA TGTTCCATTA CCTGGTGATG CTGCAGCTGG CCACCCCGAC 1140

CGTGCCTCTG GCCACCCTAA GCCCCATTCC AGATCCTCCT CTGCCTATCG CAAATCTGCC 1200

TCTACCCACC ACAAGTCTGT CTTTAGCCAC TCCAAGGCTG CCTCTGGTCA CCTCAAGCCT 1260

GTCTCTGGCC ACTCCAAGCC TGCCTCTGGT CACCCCAAGT CTGCCACTGT CTACCCTAAG 1320

CCTGCCTCTG TCCATTTCAA GGCTGACTCT GTCCATTTCA AGGGTGACTC TGTCCATTTC 1380

AAGCCTGACT CTGTTCATTT CAAGCCTGCT TCCAGCAACC CCAAGCCCAT CACTGGCCAC 1440

CATGTCTCTG CTGGCAGCCA CTCCAAGTCT GCCTTCAATG CTGCCACCAG CCACCCTAAA 1500

CCCATCAAGC CAGCTACCAG CCATGCTGAG CCCACCACTG CTGACTATCC CAAGCCTGCC 1560

ACTACCAGCC ACCCTAAGCC CGCTGCTGCT GACAACCCTG AGCTCTCTGC CTCCCATTGC 1620

CCCGAGATCC CTGCCATTGC CCACCCTGTG TCTGACGACA GTGACCTCCC TGAGTCGGCC 1680

TCTAGCCCTG CCGCTGGGCC CACCAAGCCT GCTGCCAGCC AGCTGGAGTC TGACACCATC 1740

GCTGACCTTC CTGACCCTAC TGTAGTCACT ACCAGTACCA ATGATTACCA TGATGTCGTG 1800

GTTGTTGATG TTGAAGATGA TCCTGATGAA ATGGCTGTGT GA                    1842

(143) INFORMATION FOR SEQ ID NO:142:

     (i) SEQUENCE CHARACTERISTICS:
          (A) LENGTH: 613 amino acids
          (B) TYPE: amino acid
          (C) STRANDEDNESS:
          (D) TOPOLOGY: not relevant

    (ii) MOLECULE TYPE: protein

    (xi) SEQUENCE DESCRIPTION: SEQ ID NO:142:

Met Gly Pro Thr Leu Ala Val Pro Thr Pro Tyr Gly Cys Ile Gly Cys
1               5                   10                  15

Lys Leu Pro Gln Pro Glu Tyr Pro Pro Ala Leu Ile Ile Phe Met Phe
            20                  25                  30

Cys Ala Met Val Ile Thr Ile Val Val Asp Leu Ile Gly Asn Ser Met
        35                  40                  45

Val Ile Leu Ala Val Thr Lys Asn Lys Lys Leu Arg Asn Ser Gly Asn
    50                  55                  60

Ile Phe Val Val Ser Leu Ser Val Ala Asp Met Leu Val Ala Ile Tyr
65                  70                  75                  80

Pro Tyr Pro Leu Met Leu His Ala Met Ser Ile Gly Gly Trp Asp Leu
                85                  90                  95

Ser Gln Leu Gln Cys Gln Met Val Gly Phe Ile Thr Gly Leu Ser Val
            100                 105                 110

Val Gly Ser Ile Phe Asn Ile Val Ala Ile Ala Ile Asn Arg Tyr Cys
        115                 120                 125

Tyr Ile Cys His Ser Leu Gln Tyr Glu Arg Ile Phe Ser Val Arg Asn
    130                 135                 140

Thr Cys Ile Tyr Leu Val Ile Thr Trp Ile Met Thr Val Leu Ala Val
145                 150                 155                 160

Leu Pro Asn Met Tyr Ile Gly Thr Ile Glu Tyr Asp Pro Arg Thr Tyr
                165                 170                 175

Thr Cys Ile Phe Asn Tyr Leu Asn Asn Pro Val Phe Thr Val Thr Ile
            180                 185                 190

Val Cys Ile His Phe Val Leu Pro Leu Leu Ile Val Gly Phe Cys Tyr
        195                 200                 205

Val Arg Ile Trp Thr Lys Val Leu Ala Ala Arg Asp Pro Ala Gly Gln
    210                 215                 220

Asn Pro Asp Asn Gln Leu Ala Glu Val Arg Asn Lys Leu Thr Met Phe
225                 230                 235                 240

Val Ile Phe Leu Leu Phe Ala Val Cys Trp Cys Pro Ile Asn Val Leu
                245                 250                 255

Thr Val Leu Val Ala Val Ser Pro Lys Glu Met Ala Gly Lys Ile Pro
            260                 265                 270

Asn Trp Leu Tyr Leu Ala Ala Tyr Phe Ile Ala Tyr Phe Asn Ser Cys
        275                 280                 285

Leu Asn Ala Val Ile Tyr Gly Leu Leu Asn Glu Asn Phe Arg Arg Glu
    290                 295                 300

Tyr Trp Thr Ile Phe His Ala Met Arg His Pro Ile Ile Phe Phe Ser
305                 310                 315                 320

Gly Leu Ile Ser Asp Ile Arg Glu Met Gln Glu Ala Arg Thr Leu Ala
                325                 330                 335

Arg Ala Arg Ala His Ala Arg Asp Gln Ala Arg Glu Gln Asp Arg Ala
            340                 345                 350

His Ala Cys Pro Ala Val Glu Glu Thr Pro Met Asn Val Arg Asn Val
        355                 360                 365

Pro Leu Pro Gly Asp Ala Ala Ala Gly His Pro Asp Arg Ala Ser Gly
    370                 375                 380

His Pro Lys Pro His Ser Arg Ser Ser Ser Ala Tyr Arg Lys Ser Ala
385                 390                 395                 400

Ser Thr His His Lys Ser Val Phe Ser His Ser Lys Ala Ala Ser Gly
                405                 410                 415

His Leu Lys Pro Val Ser Gly His Ser Lys Pro Ala Ser Gly His Pro
            420                 425                 430

Lys Ser Ala Thr Val Tyr Pro Lys Pro Ala Ser Val His Phe Lys Ala
        435                 440                 445

Asp Ser Val His Phe Lys Gly Asp Ser Val His Phe Lys Pro Asp Ser
    450                 455                 460

Val His Phe Lys Pro Ala Ser Ser Asn Pro Lys Pro Ile Thr Gly His
465                 470                 475                 480

His Val Ser Ala Gly Ser His Ser Lys Ser Ala Phe Asn Ala Ala Thr
                485                 490                 495

Ser His Pro Lys Pro Ile Lys Pro Ala Thr Ser His Ala Glu Pro Thr
            500                 505                 510

Thr Ala Asp Tyr Pro Lys Pro Ala Thr Thr Ser His Pro Lys Pro Ala
        515                 520                 525

Ala Ala Asp Asn Pro Glu Leu Ser Ala Ser His Cys Pro Glu Ile Pro
    530                 535                 540

Ala Ile Ala His Pro Val Ser Asp Asp Ser Asp Leu Pro Glu Ser Ala
545                 550                 555                 560

Ser Ser Pro Ala Ala Gly Pro Thr Lys Pro Ala Ala Ser Gln Leu Glu

565                 570                 575

Ser Asp Thr Ile Ala Asp Leu Pro Asp Pro Thr Val Val Thr Thr Ser
        580                 585                 590

Thr Asn Asp Tyr His Asp Val Val Val Val Asp Val Glu Asp Asp Pro
    595                 600                 605

Asp Glu Met Ala Val
    610

(144) INFORMATION FOR SEQ ID NO:143:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:143:

GCTGAGGTTC GCAATAAACT AACCATGTTT GTG                                33

(145) INFORMATION FOR SEQ ID NO:144:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:144:

CTCCTTCGGT CCTCCTATCG TTGTCAGAAG T                                  31

(146) INFORMATION FOR SEQ ID NO:145:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:145:

TTAGATATCG GGGCCCACCC TAGCGGT                                       27

(147) INFORMATION FOR SEQ ID NO:146:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:146:

GGTACCCCCA CAGCCATTTC ATCAGGATC 29

### Annex to the application documents - subsequently filed sequences listing

SEQUENCE LISTING

<110> Arena Pharmaceuticals, Inc.

<120> Non-Endogenous, Constitutively Activated Human G Protein-Coupled
      Receptors

<130> JEC/FP6292718

<140> EP 05009877.1
<141> 2005-05-06

<150> EP 99950301.4
<151> 1999-10-13

<150> PCT/US99/24065
<151> 1999-10-13

<150> US 09/170,496
<151> 1998-10-13

<150> US 60/109,213
<151> 1998-11-20

<150> US 60/110,060
<151> 1998-11-27

<150> US 60/108,029
<151> 1998-11-12

<150> US 60/120,416
<151> 1999-02-16

<150> US 60/121,852
<151> 1999-02-26

<150> US 60/123,944
<151> 1999-03-12

<150> US 60/123,945
<151> 1999-03-12

<150> US 60/123,948
<151> 1999-03-12

<150> US 60/123,946
<151> 1999-03-12

<150> US 60/123,949
<151> 1999-03-12

<150> US 60/123,951
<151> 1999-03-12

<150> US 60/136,436
<151> 1999-05-28

<150> US 60/136,437
<151> 1999-05-28

<150> US 60/136,439
<151> 1999-05-28

<150> US 60/136,567
<151> 1999-05-28

<150> US 60/137,127
<151> 1999-05-28

<150> US 60/137,131
<151> 1999-05-28

<150> US 60/141,448
<151> 1999-06-29

<150> US 60/151,114
<151> 1999-08-27

<150> US 60/152,524
<151> 1999-09-03

<150> US 60/156,633
<151> 1999-09-29

<150> US 60/156,555
<151> 1999-09-29

<150> US 60/156,634
<151> 1999-09-29

<150>  US 60/156,653
<151>  1999-09-09

<150>  US 60/157,280
<151>  1999-10-01

<150>  US 60/157,294
<151>  1999-10-01

<150>  US 60/157,281
<151>  1999-10-01

<150>  US 60/157,293
<151>  1999-10-01

<150>  US 60/157,282
<151>  1999-10-01

<150>  US 09/417,044
<151>  1999-10-12

<150>  US 09/416,760
<151>  1999-10-12

<160>  146

<170>  PatentIn version 3.1

<210>  1
<211>  1260
<212>  DNA
<213>  Homo sapiens

<400>  1
atggtcttct cggcagtgtt gactgcgttc cataccggga catccaacac aacatttgtc      60

gtgtatgaaa acacctacat gaatattaca ctccctccac cattccagca tcctgacctc     120

agtccattgc ttagatatag ttttgaaacc atggctccca ctggtttgag ttccttgacc     180

gtgaatagta cagctgtgcc cacaacacca gcagcattta agagcctaaa cttgcctctt     240

cagatcaccc tttctgctat aatgatattc attctgtttg tgtcttttct tgggaacttg     300

gttgtttgcc tcatggttta ccaaaaagct gccatgaggt ctgcaattaa catcctcctt     360

gccagcctag cttttgcaga catgttgctt gcagtgctga acatgccctt tgccctggta    420

actattctta ctacccgatg gatttttggg aaattcttct gtagggtatc tgctatgttt    480

ttctggttat ttgtgataga aggagtagcc atcctgctca tcattagcat agataggttc    540

cttattatag tccagaggca ggataagcta aacccatata gagctaaggt tctgattgca    600

gtttcttggg caacttcctt ttgtgtagct tttcctttag ccgtaggaaa ccccgacctg    660

cagatacctt cccgagctcc ccagtgtgtg tttgggtaca caaccaatcc aggctaccag    720

gcttatgtga ttttgatttc tctcatttct ttcttcatac ccttcctggt aatactgtac    780

tcatttatgg gcatactcaa cacccttcgg cacaatgcct tgaggatcca tagctaccct    840

gaaggtatat gcctcagcca ggccagcaaa ctgggtctca tgagtctgca gagacctttc    900

cagatgagca ttgacatggg ctttaaaaca cgtgccttca ccactatttt gattctcttt    960

gctgtcttca ttgtctgctg ggccccattc accacttaca gccttgtggc aacattcagt   1020

aagcactttt actatcagca caactttttt gagattagca cctggctact gtggctctgc   1080

tacctcaagt ctgcattgaa tccgctgatc tactactgga ggattaagaa attccatgat   1140

gcttgcctgg acatgatgcc taagtccttc aagtttttgc cgcagctccc tggtcacaca   1200

aagcgacgga tacgtcctag tgctgtctat gtgtgtgggg aacatcggac ggtggtgtga   1260

<210>  2
<211>  419
<212>  PRT
<213>  Homo sapiens

<400>  2

Met Val Phe Ser Ala Val Leu Thr Ala Phe His Thr Gly Thr Ser Asn
1               5                   10                  15

Thr Thr Phe Val Val Tyr Glu Asn Thr Tyr Met Asn Ile Thr Leu Pro
            20                  25                  30

Pro Pro Phe Gln His Pro Asp Leu Ser Pro Leu Leu Arg Tyr Ser Phe
        35                  40                  45

Glu Thr Met Ala Pro Thr Gly Leu Ser Ser Leu Thr Val Asn Ser Thr
    50                  55                  60

Ala Val Pro Thr Thr Pro Ala Ala Phe Lys Ser Leu Asn Leu Pro Leu
65                  70                  75                  80

Gln Ile Thr Leu Ser Ala Ile Met Ile Phe Ile Leu Phe Val Ser Phe
                85                  90                  95

Leu Gly Asn Leu Val Val Cys Leu Met Val Tyr Gln Lys Ala Ala Met
            100                 105                 110

Arg Ser Ala Ile Asn Ile Leu Leu Ala Ser Leu Ala Phe Ala Asp Met
        115                 120                 125

Leu Leu Ala Val Leu Asn Met Pro Phe Ala Leu Val Thr Ile Leu Thr
    130                 135                 140

Thr Arg Trp Ile Phe Gly Lys Phe Phe Cys Arg Val Ser Ala Met Phe
145                 150                 155                 160

Phe Trp Leu Phe Val Ile Glu Gly Val Ala Ile Leu Leu Ile Ile Ser
                165                 170                 175

Ile Asp Arg Phe Leu Ile Ile Val Gln Arg Gln Asp Lys Leu Asn Pro
            180                 185                 190

Tyr Arg Ala Lys Val Leu Ile Ala Val Ser Trp Ala Thr Ser Phe Cys
        195                 200                 205

Val Ala Phe Pro Leu Ala Val Gly Asn Pro Asp Leu Gln Ile Pro Ser
    210                 215                 220

Arg Ala Pro Gln Cys Val Phe Gly Tyr Thr Thr Asn Pro Gly Tyr Gln
225                 230                 235                 240

Ala Tyr Val Ile Leu Ile Ser Leu Ile Ser Phe Phe Ile Pro Phe Leu
                245                 250                 255

Val Ile Leu Tyr Ser Phe Met Gly Ile Leu Asn Thr Leu Arg His Asn
            260                 265                 270

Ala Leu Arg Ile His Ser Tyr Pro Glu Gly Ile Cys Leu Ser Gln Ala
        275                 280                 285

Ser Lys Leu Gly Leu Met Ser Leu Gln Arg Pro Phe Gln Met Ser Ile
    290                 295                 300

Asp Met Gly Phe Lys Thr Arg Ala Phe Thr Thr Ile Leu Ile Leu Phe
305                 310                 315                 320

Ala Val Phe Ile Val Cys Trp Ala Pro Phe Thr Thr Tyr Ser Leu Val
                325                 330                 335

Ala Thr Phe Ser Lys His Phe Tyr Tyr Gln His Asn Phe Phe Glu Ile
            340                 345                 350

Ser Thr Trp Leu Leu Trp Leu Cys Tyr Leu Lys Ser Ala Leu Asn Pro
        355                 360                 365

Leu Ile Tyr Tyr Trp Arg Ile Lys Lys Phe His Asp Ala Cys Leu Asp
    370                 375                 380

Met Met Pro Lys Ser Phe Lys Phe Leu Pro Gln Leu Pro Gly His Thr
385                 390                 395                 400

Lys Arg Arg Ile Arg Pro Ser Ala Val Tyr Val Cys Gly Glu His Arg
                405                 410                 415

Thr Val Val

<210> 3
<211> 1119
<212> DNA
<213> Homo sapiens

<400> 3
atgttagcca acagctcctc aaccaacagt tctgttctcc cgtgtcctga ctaccgacct      60

acccaccgcc tgcacttggt ggtctacagc ttggtgctgg ctgccgggct ccccctcaac     120

gcgctagccc tctgggtctt cctgcgcgcg ctgcgcgtgc actcggtggt gagcgtgtac     180

atgtgtaacc tggcggccag cgacctgctc ttcaccctct cgctgcccgt tcgtctctcc     240

tactacgcac tgcaccactg gcccttcccc gacctcctgt gccagacgac gggcgccatc     300

ttccagatga acatgtacgg cagctgcatc ttcctgatgc tcatcaacgt ggaccgctac     360

gccgccatcg tgcacccgct gcgactgcgc cacctgcggc ggccccgcgt ggcgcggctg     420

ctctgcctgg gcgtgtgggc gctcatcctg gtgtttgccg tgcccgccgc ccgcgtgcac     480

aggccctcgc gttgccgcta ccgggacctc gaggtgcgcc tatgcttcga gagcttcagc     540

gacgagctgt ggaaaggcag gctgctgccc ctcgtgctgc tggccgaggc gctgggcttc     600

ctgctgcccc tggcggcggt ggtctactcg tcgggccgag tcttctggac gctggcgcgc     660

cccgacgcca cgcagagcca gcggcggcgg aagaccgtgc gcctcctgct ggctaacctc     720

gtcatcttcc tgctgtgctt cgtgccctac aacagcacgc tggcggtcta cgggctgctg     780

cggagcaagc tggtggcggc cagcgtgcct gcccgcgatc gcgtgcgcgg ggtgctgatg     840

gtgatggtgc tgctggccgg cgccaactgc gtgctggacc cgctggtgta ctactttagc 900

gccgagggct tccgcaacac cctgcgcggc ctgggcactc cgcaccgggc caggacctcg 960

gccaccaacg ggacgcgggc ggcgctcgcg caatccgaaa ggtccgccgt caccaccgac 1020

gccaccaggc cggatgccgc cagtcagggg ctgctccgac cctccgactc ccactctctg 1080

tcttccttca cacagtgtcc ccaggattcc gccctctga 1119

<210> 4
<211> 372
<212> PRT
<213> Homo sapiens

<400> 4

<210>  5
<211>  1107
<212>  DNA
<213>  Homo sapiens

<400>  5
atggccaact ccacagggct gaacgcctca gaagtcgcag gctcgttggg gttgatcctg      60

gcagctgtcg tggaggtggg ggcactgctg ggcaacggcg cgctgctggt cgtggtgctg     120

cgcacgccgg gactgcgcga cgcgctctac ctggcgcacc tgtgcgtcgt ggacctgctg     180

gcggccgcct ccatcatgcc gctgggcctg ctggccgcac cgccgcccgg gctgggccgc     240

gtgcgcctgg gccccgcgcc atgccgcgcc gctcgcttcc tctccgccgc tctgctgccg     300

gcctgcacgc tcggggtggc cgcacttggc ctggcacgct accgcctcat cgtgcacccg     360

ctgcggccag gctcgcggcc gccgcctgtg ctcgtgctca ccgccgtgtg ggccgcggcg     420

ggactgctgg gcgcgctctc cctgctcggc ccgccgcccg caccgccccc tgctcctgct     480

cgctgctcgg tcctggctgg gggcctcggg cccttccggc cgctctgggc cctgctggcc 540

ttcgcgctgc ccgccctcct gctgctcggc gcctacggcg gcatcttcgt ggtggcgcgt 600

cgcgctgccc tgaggccccc acggccggcg cgcgggtccc gactccgctc ggactctctg 660

gatagccgcc tttccatctt gccgccgctc cggcctcgcc tgcccggggg caaggcggcc 720

ctggccccag cgctggccgt gggccaattt gcagcctgct ggctgcctta tggctgcgcg 780

tgcctggcgc ccgcagcgcg ggccgcggaa gccgaagcgg ctgtcacctg ggtcgcctac 840

tcggccttcg cggctcaccc cttcctgtac gggctgctgc agcgccccgt gcgcttggca 900

ctgggccgcc tctctcgccg tgcactgcct ggacctgtgc gggcctgcac tccgcaagcc 960

tggcacccgc gggcactctt gcaatgcctc cagagacccc cagagggccc tgccgtaggc 1020

ccttctgagg ctccagaaca gacccccgag ttggcaggag ggcggagccc cgcataccag 1080

gggccacctg agagttctct ctcctga 1107

<210> 6
<211> 368
<212> PRT
<213> Homo sapiens

<400> 6

Met Ala Asn Ser Thr Gly Leu Asn Ala Ser Glu Val Ala Gly Ser Leu
1               5                   10                  15

Gly Leu Ile Leu Ala Ala Val Val Glu Val Gly Ala Leu Leu Gly Asn
            20                  25                  30

Gly Ala Leu Leu Val Val Val Leu Arg Thr Pro Gly Leu Arg Asp Ala
        35                  40                  45

Leu Tyr Leu Ala His Leu Cys Val Val Asp Leu Leu Ala Ala Ala Ser
    50                  55                  60

Ile Met Pro Leu Gly Leu Leu Ala Ala Pro Pro Pro Gly Leu Gly Arg
65                  70                  75                  80

Val Arg Leu Gly Pro Ala Pro Cys Arg Ala Ala Arg Phe Leu Ser Ala
                85                  90                  95

Ala Leu Leu Pro Ala Cys Thr Leu Gly Val Ala Ala Leu Gly Leu Ala
            100                 105                 110

Arg Tyr Arg Leu Ile Val His Pro Leu Arg Pro Gly Ser Arg Pro Pro
        115                 120                 125

Pro Val Leu Val Leu Thr Ala Val Trp Ala Ala Ala Gly Leu Leu Gly
    130                 135                 140

Ala Leu Ser Leu Leu Gly Pro Pro Pro Ala Pro Pro Pro Ala Pro Ala
145                 150                 155                 160

Arg Cys Ser Val Leu Ala Gly Gly Leu Gly Pro Phe Arg Pro Leu Trp
                165                 170                 175

Ala Leu Leu Ala Phe Ala Leu Pro Ala Leu Leu Leu Leu Gly Ala Tyr
            180                 185                 190

Gly Gly Ile Phe Val Val Ala Arg Arg Ala Ala Leu Arg Pro Pro Arg
        195                 200                 205

Pro Ala Arg Gly Ser Arg Leu Arg Ser Asp Ser Leu Asp Ser Arg Leu
    210                 215                 220

Ser Ile Leu Pro Pro Leu Arg Pro Arg Leu Pro Gly Gly Lys Ala Ala
225                 230                 235                 240

Leu Ala Pro Ala Leu Ala Val Gly Gln Phe Ala Ala Cys Trp Leu Pro
                245                 250                 255

Tyr Gly Cys Ala Cys Leu Ala Pro Ala Ala Arg Ala Ala Glu Ala Glu
            260                 265                 270

Ala Ala Val Thr Trp Val Ala Tyr Ser Ala Phe Ala Ala His Pro Phe
        275                 280                 285

Leu Tyr Gly Leu Leu Gln Arg Pro Val Arg Leu Ala Leu Gly Arg Leu
    290                 295                 300

Ser Arg Arg Ala Leu Pro Gly Pro Val Arg Ala Cys Thr Pro Gln Ala
305                 310                 315                 320

Trp His Pro Arg Ala Leu Leu Gln Cys Leu Gln Arg Pro Pro Glu Gly
                325                 330                 335

Pro Ala Val Gly Pro Ser Glu Ala Pro Glu Gln Thr Pro Glu Leu Ala
            340                 345                 350

Gly Gly Arg Ser Pro Ala Tyr Gln Gly Pro Pro Glu Ser Ser Leu Ser
        355                 360                 365

<210>  7
<211>  1008
<212>  DNA
<213>  Homo sapiens

<400>  7
atggaatcat ctttctcatt tggagtgatc cttgctgtcc tggcctccct catcattgct      60

actaacacac tagtggctgt ggctgtgctg ctgttgatcc acaagaatga tggtgtcagt     120

ctctgcttca ccttgaatct ggctgtggct gacaccttga ttggtgtggc catctctggc     180

ctactcacag accagctctc cagcccttct cggcccacac agaagaccct gtgcagcctg     240

cggatggcat ttgtcacttc ctccgcagct gcctctgtcc tcacggtcat gctgatcacc 300

tttgacaggt accttgccat caagcagccc ttccgctact tgaagatcat gagtgggttc 360

gtggccgggg cctgcattgc cgggctgtgg ttagtgtctt acctcattgg cttcctccca 420

ctcggaatcc ccatgttcca gcagactgcc tacaaagggc agtgcagctt ctttgctgta 480

tttcaccctc acttcgtgct gaccctctcc tgcgttggct tcttcccagc catgctcctc 540

tttgtcttct tctactgcga catgctcaag attgcctcca tgcacagcca gcagattcga 600

aagatggaac atgcaggagc catggctgga ggttatcgat ccccacggac tcccagcgac 660

ttcaaagctc tccgtactgt gtctgttctc attgggagct ttgctctatc ctggaccccc 720

ttccttatca ctggcattgt gcaggtggcc tgccaggagt gtcacctcta cctagtgctg 780

gaacggtacc tgtggctgct cggcgtgggc aactccctgc tcaacccact catctatgcc 840

tattggcaga aggaggtgcg actgcagctc taccacatgg ccctaggagt gaagaaggtg 900

ctcacctcat tcctcctctt tctctcggcc aggaattgtg gcccagagag gcccagggaa 960

agttcctgtc acatcgtcac tatctccagc tcagagtttg atggctaa 1008

<210> 8
<211> 335
<212> PRT
<213> Homo sapiens

<400> 8

Met Glu Ser Ser Phe Ser Phe Gly Val Ile Leu Ala Val Leu Ala Ser
1               5                   10                  15

Leu Ile Ile Ala Thr Asn Thr Leu Val Ala Val Ala Val Leu Leu Leu
            20                  25                  30

Ile His Lys Asn Asp Gly Val Ser Leu Cys Phe Thr Leu Asn Leu Ala
        35                  40                  45

Val Ala Asp Thr Leu Ile Gly Val Ala Ile Ser Gly Leu Leu Thr Asp
    50                  55                  60

Gln Leu Ser Ser Pro Ser Arg Pro Thr Gln Lys Thr Leu Cys Ser Leu
65                  70                  75                  80

Arg Met Ala Phe Val Thr Ser Ser Ala Ala Ala Ser Val Leu Thr Val
                85                  90                  95

Met Leu Ile Thr Phe Asp Arg Tyr Leu Ala Ile Lys Gln Pro Phe Arg
            100                 105                 110

Tyr Leu Lys Ile Met Ser Gly Phe Val Ala Gly Ala Cys Ile Ala Gly
        115                 120                 125

Leu Trp Leu Val Ser Tyr Leu Ile Gly Phe Leu Pro Leu Gly Ile Pro
    130                 135                 140

Met Phe Gln Gln Thr Ala Tyr Lys Gly Gln Cys Ser Phe Phe Ala Val
145                 150                 155                 160

Phe His Pro His Phe Val Leu Thr Leu Ser Cys Val Gly Phe Phe Pro
                165                 170                 175

Ala Met Leu Leu Phe Val Phe Phe Tyr Cys Asp Met Leu Lys Ile Ala
            180                 185                 190

Ser Met His Ser Gln Gln Ile Arg Lys Met Glu His Ala Gly Ala Met
        195                 200                 205

Ala Gly Gly Tyr Arg Ser Pro Arg Thr Pro Ser Asp Phe Lys Ala Leu
    210                 215                 220

Arg Thr Val Ser Val Leu Ile Gly Ser Phe Ala Leu Ser Trp Thr Pro
225                 230                 235                 240

Phe Leu Ile Thr Gly Ile Val Gln Val Ala Cys Gln Glu Cys His Leu
                245                 250                 255

Tyr Leu Val Leu Glu Arg Tyr Leu Trp Leu Leu Gly Val Gly Asn Ser
            260                 265                 270

Leu Leu Asn Pro Leu Ile Tyr Ala Tyr Trp Gln Lys Glu Val Arg Leu
        275                 280                 285

Gln Leu Tyr His Met Ala Leu Gly Val Lys Lys Val Leu Thr Ser Phe
    290                 295                 300

Leu Leu Phe Leu Ser Ala Arg Asn Cys Gly Pro Glu Arg Pro Arg Glu
305                 310                 315                 320

Ser Ser Cys His Ile Val Thr Ile Ser Ser Ser Glu Phe Asp Gly
                325                 330                 335

<210> 9
<211> 1413
<212> DNA
<213> Homo sapiens

<400> 9
atggacacta ccatggaagc tgacctgggt gccactggcc acaggccccg cacagagctt      60

gatgatgagg actcctaccc ccaaggtggc tgggacacgg tcttcctggt ggccctgctg     120

ctccttgggc tgccagccaa tgggttgatg gcgtggctgg ccggctccca ggcccggcat     180

ggagctggca cgcgtctggc gctgctcctg ctcagcctgg ccctctctga cttcttgttc     240

ctggcagcag cggccttcca gatcctagag atccggcatg ggggacactg gccgctgggg     300

acagctgcct gccgcttcta ctacttccta tggggcgtgt cctactcctc cggcctcttc     360

ctgctggccg ccctcagcct cgaccgctgc ctgctggcgc tgtgcccaca ctggtaccct 420

gggcaccgcc cagtccgcct gcccctctgg gtctgcgccg gtgtctgggt gctggccaca 480

ctcttcagcg tgccctggct ggtcttcccc gaggctgccg tctggtggta cgacctggtc 540

atctgcctgg acttctggga cagcgaggag ctgtcgctga ggatgctgga ggtcctgggg 600

ggcttcctgc ctttcctcct gctgctcgtc tgccacgtgc tcacccaggc cacagcctgt 660

cgcacctgcc accgccaaca gcagcccgca gcctgccggg gcttcgcccg tgtggccagg 720

accattctgt cagcctatgt ggtcctgagg ctgccctacc agctggccca gctgctctac 780

ctggccttcc tgtgggacgt ctactctggc tacctgctct gggaggccct ggtctactcc 840

gactacctga tcctactcaa cagctgcctc agccccttcc tctgcctcat ggccagtgcc 900

gacctccgga ccctgctgcg ctccgtgctc tcgtccttcg cggcagctct ctgcgaggag 960

cggccgggca gcttcacgcc cactgagcca cagacccagc tagattctga gggtccaact 1020

ctgccagagc cgatggcaga ggcccagtca cagatggatc ctgtggccca gcctcaggtg 1080

aaccccacac tccagccacg atcggatccc acagctcagc cacagctgaa ccctacggcc 1140

cagccacagt cggatcccac agcccagcca cagctgaacc tcatggccca gccacagtca 1200

gattctgtgg cccagccaca ggcagacact aacgtccaga cccctgcacc tgctgccagt 1260

tctgtgccca gtccctgtga tgaagcttcc ccaaccccat cctcgcatcc taccccaggg 1320

gcccttgagg acccagccac acctcctgcc tctgaaggag aaagccccag cagcaccccg 1380

ccagaggcgg ccccgggcgc aggccccacg tga 1413

<210> 10
<211> 468
<212> PRT
<213> Homo sapiens

<400> 10

Met Asp Thr Thr Met Glu Ala Asp Leu Gly Ala Thr Gly His Arg Pro
1               5                   10                  15

Arg Thr Glu Leu Asp Asp Glu Asp Ser Tyr Pro Gln Gly Gly Trp Asp
            20                  25                  30

Thr Val Phe Leu Val Ala Leu Leu Leu Leu Gly Leu Pro Ala Asn Gly
        35                  40                  45

Leu Met Ala Trp Leu Ala Gly Ser Gln Ala Arg His Gly Ala Gly Thr
    50                  55                  60

Arg Leu Ala Leu Leu Leu Leu Ser Leu Ala Leu Ser Asp Phe Leu Phe
65                  70                  75                  80

Leu Ala Ala Ala Ala Phe Gln Ile Leu Glu Ile Arg His Gly Gly His
                85                  90                  95

Trp Pro Leu Gly Thr Ala Ala Cys Arg Phe Tyr Tyr Phe Leu Trp Gly
            100                 105                 110

Val Ser Tyr Ser Ser Gly Leu Phe Leu Leu Ala Ala Leu Ser Leu Asp
        115                 120                 125

Arg Cys Leu Leu Ala Leu Cys Pro His Trp Tyr Pro Gly His Arg Pro
    130                 135                 140

Val Arg Leu Pro Leu Trp Val Cys Ala Gly Val Trp Val Leu Ala Thr
145                 150                 155                 160

Leu Phe Ser Val Pro Trp Leu Val Phe Pro Glu Ala Ala Val Trp Trp
                165                 170                 175

Tyr Asp Leu Val Ile Cys Leu Asp Phe Trp Asp Ser Glu Glu Leu Ser
            180                 185                 190

Leu Arg Met Leu Glu Val Leu Gly Gly Phe Leu Pro Phe Leu Leu Leu
        195                 200                 205

Leu Val Cys His Val Leu Thr Gln Ala Thr Arg Thr Cys His Arg Gln
    210                 215                 220

Gln Gln Pro Ala Ala Cys Arg Gly Phe Ala Arg Val Ala Arg Thr Ile
225                 230                 235                 240

Leu Ser Ala Tyr Val Val Leu Arg Leu Pro Tyr Gln Leu Ala Gln Leu
                245                 250                 255

Leu Tyr Leu Ala Phe Leu Trp Asp Val Tyr Ser Gly Tyr Leu Leu Trp
            260                 265                 270

Glu Ala Leu Val Tyr Ser Asp Tyr Leu Ile Leu Leu Asn Ser Cys Leu
        275                 280                 285

Ser Pro Phe Leu Cys Leu Met Ala Ser Ala Asp Leu Arg Thr Leu Leu
    290                 295                 300

Arg Ser Val Leu Ser Ser Phe Ala Ala Ala Leu Cys Glu Glu Arg Pro
305                 310                 315                 320

Gly Ser Phe Thr Pro Thr Glu Pro Gln Thr Gln Leu Asp Ser Glu Gly
                325                 330                 335

Pro Thr Leu Pro Glu Pro Met Ala Glu Ala Gln Ser Gln Met Asp Pro
            340                 345                 350

Val Ala Gln Pro Gln Val Asn Pro Thr Leu Gln Pro Arg Ser Asp Pro
        355                 360                 365

Thr Ala Gln Pro Gln Leu Asn Pro Thr Ala Gln Pro Gln Ser Asp Pro
    370                 375                 380

Thr Ala Gln Pro Gln Leu Asn Leu Met Ala Gln Pro Gln Ser Asp Ser
385                 390                 395                 400

Val Ala Gln Pro Gln Ala Asp Thr Asn Val Gln Thr Pro Ala Pro Ala
                405                 410                 415

Ala Ser Ser Val Pro Ser Pro Cys Asp Glu Ala Ser Pro Thr Pro Ser
            420                 425                 430

Ser His Pro Thr Pro Gly Ala Leu Glu Asp Pro Ala Thr Pro Pro Ala
        435                 440                 445

Ser Glu Gly Glu Ser Pro Ser Ser Thr Pro Pro Glu Ala Ala Pro Gly
    450                 455                 460

Ala Gly Pro Thr
465

<210> 11
<211> 1248
<212> DNA
<213> Homo sapiens

<400> 11
atgtcaggga tggaaaaact tcagaatgct tcctggatct accagcagaa actagaagat      60

ccattccaga aacacctgaa cagcaccgag gagtatctgg ccttcctctg cggacctcgg     120

cgcagccact tcttcctccc cgtgtctgtg gtgtatgtgc caatttttgt ggtgggggtc 180

attggcaatg tcctggtgtg cctggtgatt ctgcagcacc aggctatgaa gacgcccacc 240

aactactacc tcttcagcct ggcggtctct gacctcctgg tcctgctcct tggaatgccc 300

ctggaggtct atgagatgtg gcgcaactac cctttcttgt tcgggcccgt gggctgctac 360

ttcaagacgg ccctctttga gaccgtgtgc ttcgcctcca tcctcagcat caccaccgtc 420

agcgtggagc gctacgtggc catcctacac ccgttccgcg ccaaactgca gagcacccgg 480

cgccgggccc tcaggatcct cggcatcgtc tggggcttct ccgtgctctt ctccctgccc 540

aacaccagca tccatggcat caagttccac tacttcccca atgggtccct ggtcccaggt 600

tcggccacct gtacggtcat caagcccatg tggatctaca atttcatcat ccaggtcacc 660

tccttcctat tctacctcct ccccatgact gtcatcagtg tcctctacta cctcatggca 720

ctcagactaa agaaagacaa atctcttgag gcagatgaag ggaatgcaaa tattcaaaga 780

ccctgcagaa aatcagtcaa caagatgctg tttgtcttgg tcttagtgtt tgctatctgt 840

tgggccccgt tccacattga ccgactcttc ttcagctttg tggaggagtg gagtgaatcc 900

ctggctgctg tgttcaacct cgtccatgtg gtgtcaggtg tcttcttcta cctgagctca 960

gctgtcaacc ccattatcta taacctactg tctcgccgct tccaggcagc attccagaat 1020

gtgatctctt ctttccacaa acagtggcac tcccagcatg acccacagtt gccacctgcc 1080

cagcggaaca tcttcctgac agaatgccac tttgtggagc tgaccgaaga tataggtccc 1140

caattcccat gtcagtcatc catgcacaac tctcacctcc caacagccct ctctagtgaa 1200

cagatgtcaa gaacaaacta tcaaagcttc cactttaaca aaacctga 1248

<210> 12
<211> 415
<212> PRT
<213> Homo sapiens

<400> 12

<210> 13
<211> 1173
<212> DNA
<213> Homo sapiens

<400> 13
atgccagata ctaatagcac aatcaattta tcactaagca ctcgtgttac tttagcattt      60

tttatgtcct tagtagcttt tgctataatg ctaggaaatg ctttggtcat tttagctttt     120

gtggtggaca aaaaccttag acatcgaagt agttattttt ttcttaactt ggccatctct     180

gacttctttg tgggtgtgat ctccattcct ttgtacatcc ctcacacgct gttcgaatgg     240

gattttggaa aggaaatctg tgtattttgg ctcactactg actatctgtt atgtacagca     300

tctgtatata acattgtcct catcagctat gatcgatacc tgtcagtctc aaatgctgtg     360

tcttatagaa ctcaacatac tggggtcttg aagattgtta ctctgatggt ggccgtttgg     420

gtgctggcct tcttagtgaa tgggccaatg attctagttt cagagtcttg gaaggatgaa     480

ggtagtgaat gtgaacctgg atttttttcg gaatggtaca tccttgccat cacatcattc     540

ttggaattcg tgatcccagt catcttagtc gcttatttca acatgaatat ttattggagc     600

ctgtggaagc gtgatcatct cagtaggtgc caaagccatc ctggactgac tgctgtctct 660

tccaacatct gtggacactc attcagaggt agactatctt caaggagatc tctttctgca 720

tcgacagaag ttcctgcatc ctttcattca gagagacaga ggagaaagag tagtctcatg 780

ttttcctcaa gaaccaagat gaatagcaat acaattgctt ccaaaatggg ttccttctcc 840

caatcagatt ctgtagctct tcaccaaagg gaacatgttg aactgcttag agccaggaga 900

ttagccaagt cactggccat tctcttaggg gtttttgctg tttgctgggc tccatattct 960

ctgttcacaa ttgtcctttc attttattcc tcagcaacag gtcctaaatc agtttggtat 1020

agaattgcat tttggcttca gtggttcaat tcctttgtca atcctctttt gtatccattg 1080

tgtcacaagc gctttcaaaa ggctttcttg aaaatatttt gtataaaaaa gcaacctcta 1140

ccatcacaac acagtcggtc agtatcttct taa 1173

<210> 14
<211> 390
<212> PRT
<213> Homo sapiens

<400> 14

Met Pro Asp Thr Asn Ser Thr Ile Asn Leu Ser Leu Ser Thr Arg Val
1               5                   10                  15

Thr Leu Ala Phe Phe Met Ser Leu Val Ala Phe Ala Ile Met Leu Gly
            20                  25                  30

Asn Ala Leu Val Ile Leu Ala Phe Val Val Asp Lys Asn Leu Arg His
        35                  40                  45

Arg Ser Ser Tyr Phe Phe Leu Asn Leu Ala Ile Ser Asp Phe Phe Val
    50                  55                  60

Gly Val Ile Ser Ile Pro Leu Tyr Ile Pro His Thr Leu Phe Glu Trp
65                  70                  75                  80

Asp Phe Gly Lys Glu Ile Cys Val Phe Trp Leu Thr Thr Asp Tyr Leu
                85                  90                  95

Leu Cys Thr Ala Ser Val Tyr Asn Ile Val Leu Ile Ser Tyr Asp Arg
            100                 105                 110

Tyr Leu Ser Val Ser Asn Ala Val Ser Tyr Arg Thr Gln His Thr Gly
        115                 120                 125

Val Leu Lys Ile Val Thr Leu Met Val Ala Val Trp Val Leu Ala Phe
    130                 135                 140

Leu Val Asn Gly Pro Met Ile Leu Val Ser Glu Ser Trp Lys Asp Glu
145                 150                 155                 160

Gly Ser Glu Cys Glu Pro Gly Phe Phe Ser Glu Trp Tyr Ile Leu Ala
                165                 170                 175

Ile Thr Ser Phe Leu Glu Phe Val Ile Pro Val Ile Leu Val Ala Tyr
            180                 185                 190

Phe Asn Met Asn Ile Tyr Trp Ser Leu Trp Lys Arg Asp His Leu Ser
        195                 200                 205

Arg Cys Gln Ser His Pro Gly Leu Thr Ala Val Ser Ser Asn Ile Cys
    210                 215                 220

Gly His Ser Phe Arg Gly Arg Leu Ser Ser Arg Arg Ser Leu Ser Ala
225                 230                 235                 240

Ser Thr Glu Val Pro Ala Ser Phe His Ser Glu Arg Gln Arg Arg Lys
                245                 250                 255

Ser Ser Leu Met Phe Ser Ser Arg Thr Lys Met Asn Ser Asn Thr Ile
            260                 265                 270

Ala Ser Lys Met Gly Ser Phe Ser Gln Ser Asp Ser Val Ala Leu His
        275                 280                 285

Gln Arg Glu His Val Glu Leu Leu Arg Ala Arg Arg Leu Ala Lys Ser
    290                 295                 300

Leu Ala Ile Leu Leu Gly Val Phe Ala Val Cys Trp Ala Pro Tyr Ser
305                 310                 315                 320

Leu Phe Thr Ile Val Leu Ser Phe Tyr Ser Ser Ala Thr Gly Pro Lys
                325                 330                 335

Ser Val Trp Tyr Arg Ile Ala Phe Trp Leu Gln Trp Phe Asn Ser Phe
            340                 345                 350

Val Asn Pro Leu Leu Tyr Pro Leu Cys His Lys Arg Phe Gln Lys Ala
        355                 360                 365

Phe Leu Lys Ile Phe Cys Ile Lys Lys Gln Pro Leu Pro Ser Gln His
    370                 375                 380

Ser Arg Ser Val Ser Ser
385                 390

<210>  15
<211>  30
<212>  DNA
<213>  Homo sapiens

<400>  15
ggaaagctta acgatcccca ggagcaacat                                        30


<210>  16
<211>  31
<212>  DNA
<213>  Homo sapiens

<400>  16
ctgggatcct acgagagcat ttttcacaca g                                       31


<210>  17
<211>  1128
<212>  DNA
<213>  Homo sapiens

<400>  17
atggcgaacg cgagcgagcc gggtggcagc ggcggcggcg aggcggccgc cctgggcctc      60

aagctggcca cgctcagcct gctgctgtgc gtgagcctag cgggcaacgt gctgttcgcg     120

ctgctgatcg tgcgggagcg cagcctgcac cgcgccccgt actacctgct gctcgacctg     180

tgcctggccg acgggctgcg cgcgctcgcc tgcctcccgg ccgtcatgct ggcggcgcgg     240

cgtgcggcgg ccgcggcggg ggcgccgccg ggcgcgctgg gctgcaagct gctcgccttc     300

ctggccgcgc tcttctgctt ccacgccgcc ttcctgctgc tgggcgtggg cgtcacccgc     360

tacctggcca tcgcgcacca ccgcttctat gcagagcgcc tggccggctg gccgtgcgcc     420

gccatgctgg tgtgcgccgc ctgggcgctg gcgctggccg cggccttccc gccagtgctg     480

gacggcggtg gcgacgacga ggacgcgccg tgcgccctgg agcagcggcc cgacggcgcc     540

cccggcgcgc tgggcttcct gctgctgctg gccgtggtgg tgggcgccac gcacctcgtc     600

tacctccgcc tgctcttctt catccacgac cgccgcaaga tgcggcccgc gcgcctggtg     660

cccgccgtca gccacgactg gaccttccac ggcccgggcg ccaccggcca ggcggccgcc 720

aactggacgg cgggcttcgg ccgcgggccc acgccgcccg cgcttgtggg catccggccc 780

gcagggccgg gccgcggcgc gcgccgcctc ctcgtgctgg aagaattcaa gacggagaag 840

aggctgtgca agatgttcta cgccgtcacg ctgctcttcc tgctcctctg ggggccctac 900

gtcgtggcca gctacctgcg ggtcctggtg cggcccggcg ccgtccccca ggcctacctg 960

acggcctccg tgtggctgac cttcgcgcag gccggcatca accccgtcgt gtgcttcctc 1020

ttcaacaggg agctgaggga ctgcttcagg gcccagttcc cctgctgcca gagcccccgg 1080

accacccagg cgacccatcc ctgcgacctg aaaggcattg gtttatga 1128

<210> 18
<211> 375
<212> PRT
<213> Homo sapiens

<400> 18

Met Ala Asn Ala Ser Glu Pro Gly Gly Ser Gly Gly Gly Glu Ala Ala
1               5                   10                  15

Ala Leu Gly Leu Lys Leu Ala Thr Leu Ser Leu Leu Leu Cys Val Ser
            20                  25                  30

Leu Ala Gly Asn Val Leu Phe Ala Leu Leu Ile Val Arg Glu Arg Ser
        35                  40                  45

Leu His Arg Ala Pro Tyr Tyr Leu Leu Leu Asp Leu Cys Leu Ala Asp
    50                  55                  60

Gly Leu Arg Ala Leu Ala Cys Leu Pro Ala Val Met Leu Ala Ala Arg
65                  70                  75                  80

Arg Ala Ala Ala Ala Ala Gly Ala Pro Pro Gly Ala Leu Gly Cys Lys
                    85                  90                  95

Leu Leu Ala Phe Leu Ala Ala Leu Phe Cys Phe His Ala Ala Phe Leu
                100                 105                 110

Leu Leu Gly Val Gly Val Thr Arg Tyr Leu Ala Ile Ala His His Arg
            115                 120                 125

Phe Tyr Ala Glu Arg Leu Ala Gly Trp Pro Cys Ala Ala Met Leu Val
    130                 135                 140

Cys Ala Ala Trp Ala Leu Ala Leu Ala Ala Ala Phe Pro Pro Val Leu
145                 150                 155                 160

Asp Gly Gly Gly Asp Asp Glu Asp Ala Pro Cys Ala Leu Glu Gln Arg
                165                 170                 175

Pro Asp Gly Ala Pro Gly Ala Leu Gly Phe Leu Leu Leu Leu Ala Val
            180                 185                 190

Val Val Gly Ala Thr His Leu Val Tyr Leu Arg Leu Leu Phe Phe Ile
        195                 200                 205

His Asp Arg Arg Lys Met Arg Pro Ala Arg Leu Val Pro Ala Val Ser
    210                 215                 220

His Asp Trp Thr Phe His Gly Pro Gly Ala Thr Gly Gln Ala Ala Ala
225                 230                 235                 240

Asn Trp Thr Ala Gly Phe Gly Arg Gly Pro Thr Pro Pro Ala Leu Val
                245                 250                 255

Gly Ile Arg Pro Ala Gly Pro Gly Arg Gly Ala Arg Arg Leu Leu Val
            260                 265                 270

Leu Glu Glu Phe Lys Thr Glu Lys Arg Leu Cys Lys Met Phe Tyr Ala
        275                 280                 285

Val Thr Leu Leu Phe Leu Leu Leu Trp Gly Pro Tyr Val Val Ala Ser
    290                 295                 300

Tyr Leu Arg Val Leu Val Arg Pro Gly Ala Val Pro Gln Ala Tyr Leu
305                 310                 315                 320

Thr Ala Ser Val Trp Leu Thr Phe Ala Gln Ala Gly Ile Asn Pro Val
                325                 330                 335

Val Cys Phe Leu Phe Asn Arg Glu Leu Arg Asp Cys Phe Arg Ala Gln
            340                 345                 350

Phe Pro Cys Cys Gln Ser Pro Arg Thr Thr Gln Ala Thr His Pro Cys
        355                 360                 365

Asp Leu Lys Gly Ile Gly Leu
    370                 375

<210>  19
<211>  1002
<212>  DNA
<213>  Homo sapiens

<400>  19
atgaacacca cagtgatgca aggcttcaac agatctgagc ggtgccccag agacactcgg      60

atagtacagc tggtattccc agccctctac acagtggttt tcttgaccgg catcctgctg     120

aatactttgg ctctgtgggt gtttgttcac atccccagct cctccacctt catcatctac     180

ctcaaaaaca ctttggtggc cgacttgata atgacactca tgcttccttt caaaatcctc     240

tctgactcac acctggcacc ctggcagctc agagcttttg tgtgtcgttt ttcttcggtg 300

atattttatg agaccatgta tgtgggcatc gtgctgttag ggctcatagc ctttgacaga 360

ttcctcaaga tcatcagacc tttgagaaat atttttctaa aaaaacctgt ttttgcaaaa 420

acggtctcaa tcttcatctg gttctttttg ttcttcatct ccctgccaaa tacgatcttg 480

agcaacaagg aagcaacacc atcgtctgtg aaaaagtgtg cttccttaaa ggggcctctg 540

gggctgaaat ggcatcaaat ggtaaataac atatgccagt ttattttctg gactgttttt 600

atcctaatgc ttgtgtttta tgtggttatt gcaaaaaaag tatatgattc ttatagaaag 660

tccaaaagta aggacagaaa aaacaacaaa aagctggaag gcaaagtatt tgttgtcgtg 720

gctgtcttct ttgtgtgttt tgctccattt cattttgcca gagttccata tactcacagt 780

caaaccaaca ataagactga ctgtagactg caaaatcaac tgtttattgc taaagaaaca 840

actctctttt tggcagcaac taacatttgt atggatccct taatatacat attcttatgt 900

aaaaaattca cagaaaagct accatgtatg caagggagaa agaccacagc atcaagccaa 960

gaaaatcata gcagtcagac agacaacata accttaggct ga 1002

<210> 20
<211> 333
<212> PRT
<213> Homo sapiens

<400> 20

Met Asn Thr Thr Val Met Gln Gly Phe Asn Arg Ser Glu Arg Cys Pro
1               5                   10                  15

Arg Asp Thr Arg Ile Val Gln Leu Val Phe Pro Ala Leu Tyr Thr Val
            20                  25                  30

Val Phe Leu Thr Gly Ile Leu Leu Asn Thr Leu Ala Leu Trp Val Phe
        35                  40                  45

Val His Ile Pro Ser Ser Ser Thr Phe Ile Ile Tyr Leu Lys Asn Thr
    50                  55                  60

Leu Val Ala Asp Leu Ile Met Thr Leu Met Leu Pro Phe Lys Ile Leu
65                  70                  75                  80

Ser Asp Ser His Leu Ala Pro Trp Gln Leu Arg Ala Phe Val Cys Arg
                85                  90                  95

Phe Ser Ser Val Ile Phe Tyr Glu Thr Met Tyr Val Gly Ile Val Leu
            100                 105                 110

Leu Gly Leu Ile Ala Phe Asp Arg Phe Leu Lys Ile Ile Arg Pro Leu
        115                 120                 125

Arg Asn Ile Phe Leu Lys Lys Pro Val Phe Ala Lys Thr Val Ser Ile
    130                 135                 140

Phe Ile Trp Phe Phe Leu Phe Phe Ile Ser Leu Pro Asn Thr Ile Leu
145                 150                 155                 160

Ser Asn Lys Glu Ala Thr Pro Ser Ser Val Lys Lys Cys Ala Ser Leu
                165                 170                 175

Lys Gly Pro Leu Gly Leu Lys Trp His Gln Met Val Asn Asn Ile Cys
            180                 185                 190

Gln Phe Ile Phe Trp Thr Val Phe Ile Leu Met Leu Val Phe Tyr Val
        195                 200                 205

Val Ile Ala Lys Lys Val Tyr Asp Ser Tyr Arg Lys Ser Lys Ser Lys
    210                 215                 220

Asp Arg Lys Asn Asn Lys Lys Leu Glu Gly Lys Val Phe Val Val Val
225                 230                 235                 240

Ala Val Phe Phe Val Cys Phe Ala Pro Phe His Phe Ala Arg Val Pro
                245                 250                 255

Tyr Thr His Ser Gln Thr Asn Asn Lys Thr Asp Cys Arg Leu Gln Asn
            260                 265                 270

Gln Leu Phe Ile Ala Lys Glu Thr Thr Leu Phe Leu Ala Ala Thr Asn
        275                 280                 285

Ile Cys Met Asp Pro Leu Ile Tyr Ile Phe Leu Cys Lys Lys Phe Thr
    290                 295                 300

Glu Lys Leu Pro Cys Met Gln Gly Arg Lys Thr Thr Ala Ser Ser Gln
305                 310                 315                 320

Glu Asn His Ser Ser Gln Thr Asp Asn Ile Thr Leu Gly
                325                 330

<210>  21
<211>  1122
<212>  DNA
<213>  Homo sapiens

<400>  21
atggccaaca ctaccggaga gcctgaggag gtgagcggcg ctctgtcccc accgtccgca      60

tcagcttatg tgaagctggt actgctggga ctgattatgt gcgtgagcct ggcgggtaac     120

gccatcttgt ccctgctggt gctcaaggag cgtgccctgc acaaggctcc ttactacttc     180

ctgctggacc tgtgcctggc cgatggcata cgctctgccg tctgcttccc ctttgtgctg     240

gcttctgtgc gccacggctc ttcatggacc ttcagtgcac tcagctgcaa gattgtggcc     300

tttatggccg tgctcttttg cttccatgcg gccttcatgc tgttctgcat cagcgtcacc     360

cgctacatgg ccatcgccca ccaccgcttc tacgccaagc gcatgacact ctggacatgc 420

gcggctgtca tctgcatggc ctggaccctg tctgtggcca tggccttccc acctgtcttt 480

gacgtgggca cctacaagtt tattcgggag gaggaccagt gcatctttga gcatcgctac 540

ttcaaggcca atgacacgct gggcttcatg cttatgttgg ctgtgctcat ggcagctacc 600

catgctgtct acggcaagct gctcctcttc gagtatcgtc accgcaagat gaagccagtg 660

cagatggtgc cagccatcag ccagaactgg acattccatg gtcccggggc caccggccag 720

gctgctgcca actggatcgc cggctttggc cgtgggccca tgccaccaac cctgctgggt 780

atccggcaga atgggcatgc agccagccgg cggctactgg gcatggacga ggtcaagggt 840

gaaaagcagc tgggccgcat gttctacgcg atcacactgc tctttctgct cctctggtca 900

ccctacatcg tggcctgcta ctggcgagtg tttgtgaaag cctgtgctgt gccccaccgc 960

tacctggcca ctgctgtttg gatgagcttc gcccaggctg ccgtcaaccc aattgtctgc 1020

ttcctgctca acaaggacct caagaagtgc ctgaccactc acgccccctg ctggggcaca 1080

ggaggtgccc cggctcccag agaaccctac tgtgtcatgt ga 1122

<210> 22
<211> 373
<212> PRT
<213> Homo sapiens

<400> 22

Met Ala Asn Thr Thr Gly Glu Pro Glu Glu Val Ser Gly Ala Leu Ser
1               5                   10                  15

Pro Pro Ser Ala Ser Ala Tyr Val Lys Leu Val Leu Leu Gly Leu Ile
            20                  25                  30

Met Cys Val Ser Leu Ala Gly Asn Ala Ile Leu Ser Leu Leu Val Leu
        35                  40                  45

Lys Glu Arg Ala Leu His Lys Ala Pro Tyr Tyr Phe Leu Leu Asp Leu
    50                  55                  60

Cys Leu Ala Asp Gly Ile Arg Ser Ala Val Cys Phe Pro Phe Val Leu
65                  70                  75                  80

Ala Ser Val Arg His Gly Ser Ser Trp Thr Phe Ser Ala Leu Ser Cys
                85                  90                  95

Lys Ile Val Ala Phe Met Ala Val Leu Phe Cys Phe His Ala Ala Phe
            100                 105                 110

Met Leu Phe Cys Ile Ser Val Thr Arg Tyr Met Ala Ile Ala His His
        115                 120                 125

Arg Phe Tyr Ala Lys Arg Met Thr Leu Trp Thr Cys Ala Ala Val Ile
    130                 135                 140

Cys Met Ala Trp Thr Leu Ser Val Ala Met Ala Phe Pro Pro Val Phe
145                 150                 155                 160

Asp Val Gly Thr Tyr Lys Phe Ile Arg Glu Glu Asp Gln Cys Ile Phe
                165                 170                 175

Glu His Arg Tyr Phe Lys Ala Asn Asp Thr Leu Gly Phe Met Leu Met
            180                 185                 190

Leu Ala Val Leu Met Ala Ala Thr His Ala Val Tyr Gly Lys Leu Leu
        195                 200                 205

Leu Phe Glu Tyr Arg His Arg Lys Met Lys Pro Val Gln Met Val Pro
    210                 215                 220

Ala Ile Ser Gln Asn Trp Thr Phe His Gly Pro Gly Ala Thr Gly Gln
225                 230                 235                 240

Ala Ala Ala Asn Trp Ile Ala Gly Phe Gly Arg Gly Pro Met Pro Pro
                245                 250                 255

Thr Leu Leu Gly Ile Arg Gln Asn Gly His Ala Ala Ser Arg Arg Leu
            260                 265                 270

Leu Gly Met Asp Glu Val Lys Gly Glu Lys Gln Leu Gly Arg Met Phe
        275                 280                 285

Tyr Ala Ile Thr Leu Leu Phe Leu Leu Leu Trp Ser Pro Tyr Ile Val
    290                 295                 300

Ala Cys Tyr Trp Arg Val Phe Val Lys Ala Cys Ala Val Pro His Arg
305                 310                 315                 320

Tyr Leu Ala Thr Ala Val Trp Met Ser Phe Ala Gln Ala Ala Val Asn
                325                 330                 335

Pro Ile Val Cys Phe Leu Leu Asn Lys Asp Leu Lys Lys Cys Leu Thr
            340                 345                 350

Thr His Ala Pro Cys Trp Gly Thr Gly Gly Ala Pro Ala Pro Arg Glu
        355                 360                 365

Pro Tyr Cys Val Met
    370

<210> 23
<211> 1053
<212> DNA
<213> Homo sapiens

<400> 23
atggctttgg aacagaacca gtcaacagat tattattatg aggaaaatga aatgaatggc 60

acttatgact acagtcaata tgaattgatc tgtatcaaag aagatgtcag agaatttgca 120

aaagttttcc tccctgtatt cctcacaata gctttcgtca ttggacttgc aggcaattcc 180

atggtagtgg caatttatgc ctattacaag aaacagagaa ccaaaacaga tgtgtacatc 240

ctgaatttgg ctgtagcaga tttactcctt ctattcactc tgcctttttg ggctgttaat 300

gcagttcatg ggtgggtttt agggaaaata atgtgcaaaa taacttcagc cttgtacaca 360

ctaaactttg tctctggaat gcagtttctg gcttgcatca gcatagacag atatgtggca 420

gtaactaatg tccccagcca atcaggagtg ggaaaaccat gctggatcat ctgtttctgt 480

gtctggatgg ctgccatctt gctgagcata ccccagctgg ttttttatac agtaaatgac 540

aatgctaggt gcattcccat tttcccccgc tacctaggaa catcaatgaa agcattgatt 600

caaatgctag agatctgcat tggatttgta gtaccctttc ttattatggg ggtgtgctac 660

tttatcacgg caaggacact catgaagatg ccaaacatta aaatatctcg acccctaaaa 720

gttctgctca cagtcgttat agttttcatt gtcactcaac tgccttataa cattgtcaag 780

ttctgccgag ccatagacat catctactcc ctgatcacca gctgcaacat gagcaaacgc 840

atggacatcg ccatccaagt cacagaaagc attgcactct ttcacagctg cctcaaccca 900

atcctttatg tttttatggg agcatctttc aaaaactacg ttatgaaagt ggccaagaaa 960

tatgggtcct ggagaagaca gagacaaagt gtggaggagt ttccttttga ttctgagggt 1020

cctacagagc caaccagtac ttttagcatt taa 1053

<210>  24
<211>  350
<212>  PRT
<213>  Homo sapiens

<400>  24

<210> 25
<211> 1116
<212> DNA
<213> Homo sapiens

<400> 25
atgccaggaa acgccacccc agtgaccacc actgccccgt gggcctccct gggcctctcc 60

gccaagacct gcaacaacgt gtccttcgaa gagagcagga tagtcctggt cgtggtgtac 120

agcgcggtgt gcacgctggg ggtgccggcc aactgcctga ctgcgtggct ggcgctgctg 180

caggtactgc agggcaacgt gctggccgtc tacctgctct gcctggcact ctgcgaactg 240

ctgtacacag gcacgctgcc actctgggtc atctatatcc gcaaccagca ccgctggacc 300

ctaggcctgc tggcctcgaa ggtgaccgcc tacatcttct tctgcaacat ctacgtcagc 360

atcctcttcc tgtgctgcat ctcctgcgac cgcttcgtgg ccgtggtgta cgcgctggag 420

agtcggggcc gccgccgccg gaggaccgcc atcctcatct ccgcctgcat cttcatcctc 480

gtcgggatcg ttcactaccc ggtgttccag acggaagaca aggagacctg ctttgacatg 540

ctgcagatgg acagcaggat tgccgggtac tactacgcca ggttcaccgt tggctttgcc 600

atccctctct ccatcatcgc cttcaccaac caccggattt tcaggagcat caagcagagc 660

atgggcttaa gcgctgccca gaaggccaag gtgaagcact cggccatcgc ggtggttgtc 720

atcttcctag tctgcttcgc cccgtaccac ctggttctcc tcgtcaaagc cgctgccttt 780

tcctactaca gaggagacag gaacgccatg tgcggcttgg aggaaaggct gtacacagcc 840

tctgtggtgt ttctgtgcct gtccacggtg aacggcgtgg ctgaccccat tatctacgtg 900

ctggccacgg accattcccg ccaagaagtg tccagaatcc ataaggggtg gaaagagtgg 960

tccatgaaga cagacgtcac caggctcacc cacagcaggg acaccgagga gctgcagtcg 1020

cccgtggccc ttgcagacca ctacaccttc tccaggcccg tgcacccacc agggtcacca 1080

tgccctgcaa agaggctgat tgaggagtcc tgctga                                    1116

<210>  26
<211>  371
<212>  PRT
<213>  Homo sapiens

<400>  26

Met Pro Gly Asn Ala Thr Pro Val Thr Thr Thr Ala Pro Trp Ala Ser
1               5                   10                  15

Leu Gly Leu Ser Ala Lys Thr Cys Asn Asn Val Ser Phe Glu Glu Ser
            20                  25                  30

Arg Ile Val Leu Val Val Val Tyr Ser Ala Val Cys Thr Leu Gly Val
        35                  40                  45

Pro Ala Asn Cys Leu Thr Ala Trp Leu Ala Leu Leu Gln Val Leu Gln
    50                  55                  60

Gly Asn Val Leu Ala Val Tyr Leu Leu Cys Leu Ala Leu Cys Glu Leu
65                  70                  75                  80

Leu Tyr Thr Gly Thr Leu Pro Leu Trp Val Ile Tyr Ile Arg Asn Gln
                85                  90                  95

His Arg Trp Thr Leu Gly Leu Leu Ala Ser Lys Val Thr Ala Tyr Ile
            100                 105                 110

Phe Phe Cys Asn Ile Tyr Val Ser Ile Leu Phe Leu Cys Cys Ile Ser
        115                 120                 125

Cys Asp Arg Phe Val Ala Val Val Tyr Ala Leu Glu Ser Arg Gly Arg
    130                 135                 140

Arg Arg Arg Arg Thr Ala Ile Leu Ile Ser Ala Cys Ile Phe Ile Leu
145                 150                 155                 160

Val Gly Ile Val His Tyr Pro Val Phe Gln Thr Glu Asp Lys Glu Thr
                165                 170                 175

Cys Phe Asp Met Leu Gln Met Asp Ser Arg Ile Ala Gly Tyr Tyr Tyr
            180                 185                 190

Ala Arg Phe Thr Val Gly Phe Ala Ile Pro Leu Ser Ile Ile Ala Phe
        195                 200                 205

Thr Asn His Arg Ile Phe Arg Ser Ile Lys Gln Ser Met Gly Leu Ser
    210                 215                 220

Ala Ala Gln Lys Ala Lys Val Lys His Ser Ala Ile Ala Val Val Val
225                 230                 235                 240

Ile Phe Leu Val Cys Phe Ala Pro Tyr His Leu Val Leu Leu Val Lys
                245                 250                 255

Ala Ala Ala Phe Ser Tyr Tyr Arg Gly Asp Arg Asn Ala Met Cys Gly
            260                 265                 270

Leu Glu Glu Arg Leu Tyr Thr Ala Ser Val Val Phe Leu Cys Leu Ser
        275                 280                 285

Thr Val Asn Gly Val Ala Asp Pro Ile Ile Tyr Val Leu Ala Thr Asp
    290                 295                 300

His Ser Arg Gln Glu Val Ser Arg Ile His Lys Gly Trp Lys Glu Trp
305                 310                 315                 320

Ser Met Lys Thr Asp Val Thr Arg Leu Thr His Ser Arg Asp Thr Glu
                325                 330                 335

Glu Leu Gln Ser Pro Val Ala Leu Ala Asp His Tyr Thr Phe Ser Arg
            340                 345                 350

Pro Val His Pro Pro Gly Ser Pro Cys Pro Ala Lys Arg Leu Ile Glu
        355                 360                 365

Glu Ser Cys
    370

<210> 27
<211> 1113
<212> DNA
<213> Homo sapiens

<400> 27
atggcgaact atagccatgc agctgacaac attttgcaaa atctctcgcc tctaacagcc 60

tttctgaaac tgacttcctt gggtttcata ataggagtca gcgtggtggg caacctcctg 120

atctccattt tgctagtgaa agataagacc ttgcatagag caccttacta cttcctgttg 180

gatctttgct gttcagatat cctcagatct gcaatttgtt tcccatttgt gttcaactct 240

gtcaaaaatg gctctacctg gacttatggg actctgactt gcaaagtgat tgcctttctg 300

gggtttttgt cctgtttcca cactgctttc atgctcttct gcatcagtgt caccagatac 360

ttagctatcg cccatcaccg cttctataca aagaggctga ccttttggac gtgtctggct 420

gtgatctgta tggtgtggac tctgtctgtg gccatggcat ttcccccggt tttagacgtg 480

ggcacttact cattcattag ggaggaagat caatgcacct tccaacaccg ctccttcagg 540

gctaatgatt ccttaggatt tatgctgctt cttgctctca tcctcctagc cacacagctt 600

gtctacctca agctgatatt tttcgtccac gatcgaagaa aaatgaagcc agtccagttt 660

gtagcagcag tcagccagaa ctggactttt catggtcctg gagccagtgg ccaggcagct 720

gccaattggc tagcaggatt tggaaggggt cccacaccac ccaccttgct gggcatcagg 780

caaaatgcaa acaccacagg cagaagaagg ctattggtct tagacgagtt caaaatggag 840

aaaagaatca gcagaatgtt ctatataatg acttttctgt ttctaacctt gtggggcccc 900

tacctggtgg cctgttattg gagagttttt gcaagagggc ctgtagtacc agggggattt 960

ctaacagctg ctgtctggat gagttttgcc caagcaggaa tcaatccttt tgtctgcatt 1020

ttctcaaaca gggagctgag gcgctgtttc agcacaaccc ttctttactg cagaaaatcc 1080

aggttaccaa gggaacctta ctgtgttata tga 1113

<210> 28
<211> 370
<212> PRT
<213> Homo sapiens

<400> 28

Met Ala Asn Tyr Ser His Ala Ala Asp Asn Ile Leu Gln Asn Leu Ser
1               5                   10                  15

Pro Leu Thr Ala Phe Leu Lys Leu Thr Ser Leu Gly Phe Ile Ile Gly
            20                  25                  30

Val Ser Val Val Gly Asn Leu Leu Ile Ser Ile Leu Leu Val Lys Asp
        35                  40                  45

Lys Thr Leu His Arg Ala Pro Tyr Tyr Phe Leu Leu Asp Leu Cys Cys
    50                  55                  60

Ser Asp Ile Leu Arg Ser Ala Ile Cys Phe Pro Phe Val Phe Asn Ser
65                  70                  75                  80

Val Lys Asn Gly Ser Thr Trp Thr Tyr Gly Thr Leu Thr Cys Lys Val
                85                  90                  95

Ile Ala Phe Leu Gly Val Leu Ser Cys Phe His Thr Ala Phe Met Leu
            100                 105                 110

Phe Cys Ile Ser Val Thr Arg Tyr Leu Ala Ile Ala His His Arg Phe
        115                 120                 125

Tyr Thr Lys Arg Leu Thr Phe Trp Thr Cys Leu Ala Val Ile Cys Met
    130                 135                 140

Val Trp Thr Leu Ser Val Ala Met Ala Phe Pro Pro Val Leu Asp Val
145                 150                 155                 160

Gly Thr Tyr Ser Phe Ile Arg Glu Glu Asp Gln Cys Thr Phe Gln His
                165                 170                 175

Arg Ser Phe Arg Ala Asn Asp Ser Leu Gly Phe Met Leu Leu Leu Ala
            180                 185                 190

Leu Ile Leu Leu Ala Thr Gln Leu Val Tyr Leu Lys Leu Ile Phe Phe
        195                 200                 205

Val His Asp Arg Arg Lys Met Lys Pro Val Gln Phe Val Ala Ala Val
    210                 215                 220

Ser Gln Asn Trp Thr Phe His Gly Pro Gly Ala Ser Gly Gln Ala Ala
225                 230                 235                 240

Ala Asn Trp Leu Ala Gly Phe Gly Arg Gly Pro Thr Pro Pro Thr Leu
                245                 250                 255

Leu Gly Ile Arg Gln Asn Ala Asn Thr Thr Gly Arg Arg Arg Leu Leu
            260                 265                 270

Val Leu Asp Glu Phe Lys Met Glu Lys Arg Ile Ser Arg Met Phe Tyr
      275                 280                 285

Ile Met Thr Phe Leu Phe Leu Thr Leu Trp Gly Pro Tyr Leu Val Ala
    290                 295                 300

Cys Tyr Trp Arg Val Phe Ala Arg Gly Pro Val Val Pro Gly Gly Phe
305                 310                 315                 320

Leu Thr Ala Ala Val Trp Met Ser Phe Ala Gln Ala Gly Ile Asn Pro
                325                 330                 335

Phe Val Cys Ile Phe Ser Asn Arg Glu Leu Arg Arg Cys Phe Ser Thr
            340                 345                 350

Thr Leu Leu Tyr Cys Arg Lys Ser Arg Leu Pro Arg Glu Pro Tyr Cys
      355                 360                 365

Val Ile
    370

<210>  29
<211>  1080
<212>  DNA
<213>  Homo sapiens

<400>  29
atgcaggtcc cgaacagcac cggcccggac aacgcgacgc tgcagatgct gcggaacccg      60

gcgatcgcgg tggccctgcc cgtggtgtac tcgctggtgg cggcggtcag catcccgggc     120

aacctcttct ctctgtgggt gctgtgccgg cgcatggggc ccagatcccc gtcggtcatc     180

ttcatgatca acctgagcgt cacggacctg atgctggcca gcgtgttgcc tttccaaatc     240

tactaccatt gcaaccgcca ccactgggta ttcggggtgc tgctttgcaa cgtggtgacc     300

gtggcctttt acgcaaacat gtattccagc atcctcacca tgacctgtat cagcgtggag     360

cgcttcctgg gggtcctgta cccgctcagc tccaagcgct ggcgccgccg tcgttacgcg 420

gtggccgcgt gtgcagggac ctggctgctg ctcctgaccg ccctgtgccc gctggcgcgc 480

accgatctca cctacccggt gcacgccctg ggcatcatca cctgcttcga cgtcctcaag 540

tggacgatgc tccccagcgt ggccatgtgg gccgtgttcc tcttcaccat cttcatcctg 600

ctgttcctca tcccgttcgt gatcaccgtg gcttgttaca cggccaccat cctcaagctg 660

ttgcgcacgg aggaggcgca cggccgggag cagcggaggc gcgcggtggg cctggccgcg 720

gtggtcttgc tggcctttgt cacctgcttc gcccccaaca acttcgtgct cctggcgcac 780

atcgtgagcc gcctgttcta cggcaagagc tactaccacg tgtacaagct cacgctgtgt 840

ctcagctgcc tcaacaactg tctggacccg tttgtttatt actttgcgtc ccgggaattc 900

cagctgcgcc tgcgggaata tttgggctgc cgccgggtgc ccagagacac cctggacacg 960

cgccgcgaga gcctcttctc cgccaggacc acgtccgtgc gctccgaggc cggtgcgcac 1020

cctgaaggga tggagggagc caccaggccc ggcctccaga ggcaggagag tgtgttctga 1080

<210> 30
<211> 359
<212> PRT
<213> Homo sapiens

<400> 30

Met Gln Val Pro Asn Ser Thr Gly Pro Asp Asn Ala Thr Leu Gln Met
1               5                   10                  15

Leu Arg Asn Pro Ala Ile Ala Val Ala Leu Pro Val Val Tyr Ser Leu
            20                  25                  30

Val Ala Ala Val Ser Ile Pro Gly Asn Leu Phe Ser Leu Trp Val Leu
        35                  40                  45

Cys Arg Arg Met Gly Pro Arg Ser Pro Ser Val Ile Phe Met Ile Asn
    50                  55                  60

Leu Ser Val Thr Asp Leu Met Leu Ala Ser Val Leu Pro Phe Gln Ile
65                  70                  75                  80

Tyr Tyr His Cys Asn Arg His His Trp Val Phe Gly Val Leu Leu Cys
                85                  90                  95

Asn Val Val Thr Val Ala Phe Tyr Ala Asn Met Tyr Ser Ser Ile Leu
            100                 105                 110

Thr Met Thr Cys Ile Ser Val Glu Arg Phe Leu Gly Val Leu Tyr Pro
        115                 120                 125

Leu Ser Ser Lys Arg Trp Arg Arg Arg Arg Tyr Ala Val Ala Ala Cys
    130                 135                 140

Ala Gly Thr Trp Leu Leu Leu Leu Thr Ala Leu Cys Pro Leu Ala Arg
145                 150                 155                 160

Thr Asp Leu Thr Tyr Pro Val His Ala Leu Gly Ile Ile Thr Cys Phe
                165                 170                 175

Asp Val Leu Lys Trp Thr Met Leu Pro Ser Val Ala Met Trp Ala Val
            180                 185                 190

Phe Leu Phe Thr Ile Phe Ile Leu Leu Phe Leu Ile Pro Phe Val Ile
        195                 200                 205

Thr Val Ala Cys Tyr Thr Ala Thr Ile Leu Lys Leu Leu Arg Thr Glu
    210                 215                 220

Glu Ala His Gly Arg Glu Gln Arg Arg Arg Ala Val Gly Leu Ala Ala
225                 230                 235                 240

Val Val Leu Leu Ala Phe Val Thr Cys Phe Ala Pro Asn Asn Phe Val
                245                 250                 255

Leu Leu Ala His Ile Val Ser Arg Leu Phe Tyr Gly Lys Ser Tyr Tyr
            260                 265                 270

His Val Tyr Lys Leu Thr Leu Cys Leu Ser Cys Leu Asn Asn Cys Leu
        275                 280                 285

Asp Pro Phe Val Tyr Tyr Phe Ala Ser Arg Glu Phe Gln Leu Arg Leu
    290                 295                 300

Arg Glu Tyr Leu Gly Cys Arg Arg Val Pro Arg Asp Thr Leu Asp Thr
305                 310                 315                 320

Arg Arg Glu Ser Leu Phe Ser Ala Arg Thr Thr Ser Val Arg Ser Glu
                325                 330                 335

Ala Gly Ala His Pro Glu Gly Met Glu Gly Ala Thr Arg Pro Gly Leu
            340                 345                 350

Gln Arg Gln Glu Ser Val Phe
        355

<210> 31
<211> 1503
<212> DNA
<213> Homo sapiens

<400> 31
atggagcgtc cctgggagga cagcccaggc ccggaggggg cagctgaggg ctcgcctgtg 60

ccagtcgccg ccggggcgcg ctccggtgcc gcggcgagtg gcacaggctg gcagccatgg 120

gctgagtgcc cgggacccaa ggggaggggg caactgctgg cgaccgccgg ccctttgcgt 180

cgctggcccg ccccctcgcc tgccagctcc agccccgccc ccggagcggc gtccgctcac 240

tcggttcaag gcagcgcgac tgcgggtggc gcacgaccag ggcgcagacc ttggggcgcg 300

cggcccatgg agtcggggct gctgcggccg gcgccggtga gcgaggtcat cgtcctgcat 360

tacaactaca ccggcaagct ccgcggtgcg agctaccagc cgggtgccgg cctgcgcgcc 420

gacgccgtgg tgtgcctggc ggtgtgcgcc ttcatcgtgc tagagaatct agccgtgttg 480

ttggtgctcg gacgccaccc gcgcttccac gctcccatgt tcctgctcct gggcagcctc 540

acgttgtcgg atctgctggc aggcgccgcc tacgccgcca acatcctact gtcggggccg 600

ctcacgctga aactgtcccc cgcgctctgg ttcgcacggg agggaggcgt cttcgtggca 660

ctcactgcgt ccgtgctgag cctcctggcc atcgcgctgg agcgcagcct caccatggcg 720

cgcagggggc ccgcgcccgt ctccagtcgg gggcgcacgc tggcgatggc agccgcggcc 780

tggggcgtgt cgctgctcct cgggctcctg ccagcgctgg gctggaattg cctgggtcgc 840

ctggacgctt gctccactgt cttgccgctc tacgccaagg cctacgtgct cttctgcgtg 900

ctcgccttcg tgggcatcct ggccgcgatc tgtgcactct acgcgcgcat ctactgccag 960

gtacgcgcca acgcgcggcg cctgccggca cggcccggga ctgcggggac cacctcgacc 1020

cgggcgcgtc gcaagccgcg ctctctggcc ttgctgcgca cgctcagcgt ggtgctcctg 1080

gcctttgtgg catgttgggg ccccctcttc ctgctgctgt tgctcgacgt ggcgtgcccg 1140

gcgcgcacct gtcctgtact cctgcaggcc gatcccttcc tgggactggc catggccaac 1200

tcacttctga accccatcat ctacacgctc accaaccgcg acctgcgcca cgcgctcctg 1260

cgcctggtct gctgcggacg ccactcctgc ggcagagacc cgagtggctc ccagcagtcg 1320

gcgagcgcgg ctgaggcttc cgggggcctg cgccgctgcc tgcccccggg ccttgatggg 1380

agcttcagcg gctcggagcg ctcatcgccc cagcgcgacg ggctggacac cagcggctcc 1440

acaggcagcc ccggtgcacc cacagccgcc cggactctgg tatcagaacc ggctgcagac 1500

tga 1503

<210> 32
<211> 500
<212> PRT
<213> Homo sapiens

<400> 32

Met Glu Arg Pro Trp Glu Asp Ser Pro Gly Pro Glu Gly Ala Ala Glu
1               5                   10                  15

Gly Ser Pro Val Pro Val Ala Ala Gly Ala Arg Ser Gly Ala Ala Ala
            20                  25                  30

Ser Gly Thr Gly Trp Gln Pro Trp Ala Glu Cys Pro Gly Pro Lys Gly
        35                  40                  45

Arg Gly Gln Leu Leu Ala Thr Ala Gly Pro Leu Arg Arg Trp Pro Ala
    50                  55                  60

Pro Ser Pro Ala Ser Ser Ser Pro Ala Pro Gly Ala Ala Ser Ala His
65                  70                  75                  80

Ser Val Gln Gly Ser Ala Thr Ala Gly Gly Ala Arg Pro Gly Arg Arg
                85                  90                  95

Pro Trp Gly Ala Arg Pro Met Glu Ser Gly Leu Leu Arg Pro Ala Pro
            100                 105                 110

Val Ser Glu Val Ile Val Leu His Tyr Asn Tyr Thr Gly Lys Leu Arg
        115                 120                 125

Gly Ala Ser Tyr Gln Pro Gly Ala Gly Leu Arg Ala Asp Ala Val Val
    130                 135                 140

Cys Leu Ala Val Cys Ala Phe Ile Val Leu Glu Asn Leu Ala Val Leu
145                 150                 155                 160

Leu Val Leu Gly Arg His Pro Arg Phe His Ala Pro Met Phe Leu Leu
                165                 170                 175

Leu Gly Ser Leu Thr Leu Ser Asp Leu Leu Ala Gly Ala Ala Tyr Ala
            180                 185                 190

Ala Asn Ile Leu Leu Ser Gly Pro Leu Thr Leu Lys Leu Ser Pro Ala
        195                 200                 205

Leu Trp Phe Ala Arg Glu Gly Gly Val Phe Val Ala Leu Thr Ala Ser
    210                 215                 220

Val Leu Ser Leu Leu Ala Ile Ala Leu Glu Arg Ser Leu Thr Met Ala
225                 230                 235                 240

Arg Arg Gly Pro Ala Pro Val Ser Ser Arg Gly Arg Thr Leu Ala Met
                245                 250                 255

Ala Ala Ala Ala Trp Gly Val Ser Leu Leu Leu Gly Leu Leu Pro Ala
            260                 265                 270

Leu Gly Trp Asn Cys Leu Gly Arg Leu Asp Ala Cys Ser Thr Val Leu
        275                 280                 285

Pro Leu Tyr Ala Lys Ala Tyr Val Leu Phe Cys Val Leu Ala Phe Val
    290                 295                 300

Gly Ile Leu Ala Ala Ile Cys Ala Leu Tyr Ala Arg Ile Tyr Cys Gln
305                 310                 315                 320

Val Arg Ala Asn Ala Arg Arg Leu Pro Ala Arg Pro Gly Thr Ala Gly
                325                 330                 335

Thr Thr Ser Thr Arg Ala Arg Arg Lys Pro Arg Ser Leu Ala Leu Leu
            340                 345                 350

Arg Thr Leu Ser Val Val Leu Leu Ala Phe Val Ala Cys Trp Gly Pro
        355                 360                 365

Leu Phe Leu Leu Leu Leu Leu Asp Val Ala Cys Pro Ala Arg Thr Cys
    370                 375                 380

Pro Val Leu Leu Gln Ala Asp Pro Phe Leu Gly Leu Ala Met Ala Asn
385                 390                 395                 400

Ser Leu Leu Asn Pro Ile Ile Tyr Thr Leu Thr Asn Arg Asp Leu Arg
                405                 410                 415

His Ala Leu Leu Arg Leu Val Cys Cys Gly Arg His Ser Cys Gly Arg
            420                 425                 430

Asp Pro Ser Gly Ser Gln Gln Ser Ala Ser Ala Ala Glu Ala Ser Gly
        435                 440                 445

Gly Leu Arg Arg Cys Leu Pro Pro Gly Leu Asp Gly Ser Phe Ser Gly
    450                 455                 460

Ser Glu Arg Ser Ser Pro Gln Arg Asp Gly Leu Asp Thr Ser Gly Ser
465                 470                 475                 480

Thr Gly Ser Pro Gly Ala Pro Thr Ala Ala Arg Thr Leu Val Ser Glu
                485                 490                 495

Pro Ala Ala Asp
            500

<210>  33
<211>  1029
<212>  DNA
<213>  Homo sapiens

<400>  33
atgcaagccg tcgacaatct cacctctgcg cctgggaaca ccagtctgtg caccagagac      60

tacaaaatca cccaggtcct cttcccactg ctctacactg tcctgttttt tgttggactt     120

atcacaaatg gcctggcgat gaggattttc tttcaaatcc ggagtaaatc aaactttatt     180

atttttctta agaacacagt catttctgat cttctcatga ttctgacttt tccattcaaa     240

attcttagtg atgccaaact gggaacagga ccactgagaa cttttgtgtg tcaagttacc     300

tccgtcatat tttatttcac aatgtatatc agtatttcat tcctgggact gataactatc     360

gatcgctacc agaagaccac caggccattt aaaacatcca accccaaaaa tctcttgggg     420

gctaagattc tctctgttgt catctgggca ttcatgttct tactctcttt gcctaacatg     480

attctgacca acaggcagcc gagagacaag aatgtgaaga aatgctcttt ccttaaatca     540

gagttcggtc tagtctggca tgaaatagta aattacatct gtcaagtcat tttctggatt     600

aatttcttaa ttgttattgt atgttataca ctcattacaa aagaactgta ccggtcatac     660

gtaagaacga ggggtgtagg taaagtcccc aggaaaaagg tgaacgtcaa agttttcatt     720

atcattgctg tattctttat ttgttttgtt cctttccatt ttgcccgaat tccttacacc 780

ctgagccaaa cccgggatgt ctttgactgc actgctgaaa atactctgtt ctatgtgaaa 840

gagagcactc tgtggttaac ttccttaaat gcatgcctgg atccgttcat ctattttttc 900

ctttgcaagt ccttcagaaa ttccttgata agtatgctga agtgccccaa ttctgcaaca 960

tctctgtccc aggacaatag gaaaaaagaa caggatggtg gtgacccaaa tgaagagact 1020

ccaatgtaa 1029

<210> 34
<211> 342
<212> PRT
<213> Homo sapiens

<400> 34

<210>  35
<211>  1077
<212>  DNA
<213>  Homo sapiens

<400>  35
atgtcggtct gctaccgtcc cccagggaac gagacactgc tgagctggaa gacttcgcgg      60

gccacaggca cagccttcct gctgctggcg gcgctgctgg ggctgcctgg caacggcttc     120

gtggtgtgga gcttggcggg ctggcggcct gcacgggggc gaccgctggc ggccacgctt     180

gtgctgcacc tggcgctggc cgacggcgcg gtgctgctgc tcacgccgct ctttgtggcc     240

ttcctgaccc ggcaggcctg gccgctgggc caggcgggct gcaaggcggt gtactacgtg     300

tgcgcgctca gcatgtacgc cagcgtgctg ctcaccggcc tgctcagcct gcagcgctgc     360

ctcgcagtca cccgcccctt cctggcgcct cggctgcgca gcccggccct ggcccgccgc     420

ctgctgctgg cggtctggct ggccgccctg ttgctcgccg tcccggccgc cgtctaccgc     480

cacctgtgga gggaccgcgt atgccagctg tgccacccgt cgccggtcca cgccgccgcc     540

cacctgagcc tggagactct gaccgctttc gtgcttcctt tcgggctgat gctcggctgc     600

tacagcgtga cgctggcacg gctgcggggc gcccgctggg gctccgggcg gcacggggcg 660

cgggtgggcc ggctggtgag cgccatcgtg cttgccttcg gcttgctctg ggccccctac 720

cacgcagtca accttctgca ggcggtcgca gcgctggctc caccggaagg ggccttggcg 780

aagctgggcg gagccggcca ggcggcgcga gcgggaacta cggccttggc cttcttcagt 840

tctagcgtca acccggtgct ctacgtcttc accgctggag atctgctgcc ccgggcaggt 900

ccccgtttcc tcacgcggct cttcgaaggc tctggggagg cccgaggggg cggccgctct 960

agggaaggga ccatggagct ccgaactacc cctcagctga aagtggtggg gcagggccgc 1020

ggcaatggag acccgggggg tgggatggag aaggacggtc cggaatggga cctttga 1077

<210> 36
<211> 358
<212> PRT
<213> Homo sapiens

<400> 36

Met Ser Val Cys Tyr Arg Pro Pro Gly Asn Glu Thr Leu Leu Ser Trp
1               5                   10                  15

Lys Thr Ser Arg Ala Thr Gly Thr Ala Phe Leu Leu Leu Ala Ala Leu
            20                  25                  30

Leu Gly Leu Pro Gly Asn Gly Phe Val Val Trp Ser Leu Ala Gly Trp
        35                  40                  45

Arg Pro Ala Arg Gly Arg Pro Leu Ala Ala Thr Leu Val Leu His Leu
    50                  55                  60

Ala Leu Ala Asp Gly Ala Val Leu Leu Leu Thr Pro Leu Phe Val Ala
65                  70                  75                  80

Phe Leu Thr Arg Gln Ala Trp Pro Leu Gly Gln Ala Gly Cys Lys Ala
                85                  90                  95

Val Tyr Tyr Val Cys Ala Leu Ser Met Tyr Ala Ser Val Leu Leu Thr
            100                 105                 110

Gly Leu Leu Ser Leu Gln Arg Cys Leu Ala Val Thr Arg Pro Phe Leu
        115                 120                 125

Ala Pro Arg Leu Arg Ser Pro Ala Leu Ala Arg Arg Leu Leu Leu Ala
    130                 135                 140

Val Trp Leu Ala Ala Leu Leu Leu Ala Val Pro Ala Ala Val Tyr Arg
145                 150                 155                 160

His Leu Trp Arg Asp Arg Val Cys Gln Leu Cys His Pro Ser Pro Val
                165                 170                 175

His Ala Ala Ala His Leu Ser Leu Glu Thr Leu Thr Ala Phe Val Leu
            180                 185                 190

Pro Phe Gly Leu Met Leu Gly Cys Tyr Ser Val Thr Leu Ala Arg Leu
        195                 200                 205

Arg Gly Ala Arg Trp Gly Ser Gly Arg His Gly Ala Arg Val Gly Arg
    210                 215                 220

Leu Val Ser Ala Ile Val Leu Ala Phe Gly Leu Leu Trp Ala Pro Tyr
225                 230                 235                 240

His Ala Val Asn Leu Leu Gln Ala Val Ala Ala Leu Ala Pro Pro Glu
                245                 250                 255

Gly Ala Leu Ala Lys Leu Gly Gly Ala Gly Gln Ala Ala Arg Ala Gly
            260                 265                 270

Thr Thr Ala Leu Ala Phe Phe Ser Ser Ser Val Asn Pro Val Leu Tyr
        275                 280                 285

Val Phe Thr Ala Gly Asp Leu Leu Pro Arg Ala Gly Pro Arg Phe Leu
    290                 295                 300

Thr Arg Leu Phe Glu Gly Ser Gly Glu Ala Arg Gly Gly Gly Arg Ser
305                 310                 315                 320

Arg Glu Gly Thr Met Glu Leu Arg Thr Thr Pro Gln Leu Lys Val Val
                325                 330                 335

Gly Gln Gly Arg Gly Asn Gly Asp Pro Gly Gly Gly Met Glu Lys Asp
            340                 345                 350

Gly Pro Glu Trp Asp Leu
        355

<210> 37
<211> 1005
<212> DNA
<213> Homo sapiens

<400> 37
atgctgggga tcatggcatg gaatgcaact tgcaaaaact ggctggcagc agaggctgcc 60

ctggaaaagt actacctttc cattttttat gggattgagt tcgttgtggg agtccttgga 120

aataccattg ttgtttacgg ctacatcttc tctctgaaga actggaacag cagtaatatt 180

tatctcttta acctctctgt ctctgactta gcttttctgt gcaccctccc catgctgata 240

aggagttatg ccaatggaaa ctggatatat ggagacgtgc tctgcataag caaccgatat 300

gtgcttcatg ccaacctcta taccagcatt ctctttctca cttttatcag catagatcga 360

tacttgataa ttaagtatcc tttccgagaa caccttctgc aaaagaaaga gtttgctatt 420

ttaatctcct tggccatttg ggttttagta accttagagt tactacccat acttcccctt 480

ataaatcctg ttataactga caatggcacc acctgtaatg attttgcaag ttctggagac 540

cccaactaca acctcattta cagcatgtgt ctaacactgt tggggttcct tattcctctt 600

tttgtgatgt gtttctttta ttacaagatt gctctcttcc taaagcagag gaataggcag 660

gttgctactg ctctgcccct tgaaaagcct ctcaacttgg tcatcatggc agtggtaatc 720

ttctctgtgc tttttacacc ctatcacgtc atgcggaatg tgaggatcgc ttcacgcctg 780

gggagttgga agcagtatca gtgcactcag gtcgtcatca actcctttta cattgtgaca 840

cggcctttgg cctttctgaa cagtgtcatc aaccctgtct tctattttct tttgggagat 900

cacttcaggg acatgctgat gaatcaactg agacacaact tcaaatccct tacatccttt 960

agcagatggg ctcatgaact cctactttca ttcagagaaa agtga 1005

<210> 38
<211> 334
<212> PRT
<213> Homo sapiens

<400> 38

Met Leu Gly Ile Met Ala Trp Asn Ala Thr Cys Lys Asn Trp Leu Ala
1               5                   10                  15

Ala Glu Ala Ala Leu Glu Lys Tyr Tyr Leu Ser Ile Phe Tyr Gly Ile
            20                  25                  30

Glu Phe Val Val Gly Val Leu Gly Asn Thr Ile Val Val Tyr Gly Tyr
        35                  40                  45

Ile Phe Ser Leu Lys Asn Trp Asn Ser Ser Asn Ile Tyr Leu Phe Asn
    50                  55                  60

Leu Ser Val Ser Asp Leu Ala Phe Leu Cys Thr Leu Pro Met Leu Ile
65                  70                  75                  80

Arg Ser Tyr Ala Asn Gly Asn Trp Ile Tyr Gly Asp Val Leu Cys Ile
                85                  90                  95

Ser Asn Arg Tyr Val Leu His Ala Asn Leu Tyr Thr Ser Ile Leu Phe
            100                 105                 110

Leu Thr Phe Ile Ser Ile Asp Arg Tyr Leu Ile Ile Lys Tyr Pro Phe
        115                 120                 125

Arg Glu His Leu Leu Gln Lys Lys Glu Phe Ala Ile Leu Ile Ser Leu
    130                 135                 140

Ala Ile Trp Val Leu Val Thr Leu Glu Leu Leu Pro Ile Leu Pro Leu
145                 150                 155                 160

Ile Asn Pro Val Ile Thr Asp Asn Gly Thr Thr Cys Asn Asp Phe Ala
                165                 170                 175

Ser Ser Gly Asp Pro Asn Tyr Asn Leu Ile Tyr Ser Met Cys Leu Thr
            180                 185                 190

Leu Leu Gly Phe Leu Ile Pro Leu Phe Val Met Cys Phe Phe Tyr Tyr
        195                 200                 205

Lys Ile Ala Leu Phe Leu Lys Gln Arg Asn Arg Gln Val Ala Thr Ala
    210                 215                 220

Leu Pro Leu Glu Lys Pro Leu Asn Leu Val Ile Met Ala Val Val Ile
225                 230                 235                 240

Phe Ser Val Leu Phe Thr Pro Tyr His Val Met Arg Asn Val Arg Ile
                245                 250                 255

Ala Ser Arg Leu Gly Ser Trp Lys Gln Tyr Gln Cys Thr Gln Val Val
            260                 265                 270

Ile Asn Ser Phe Tyr Ile Val Thr Arg Pro Leu Ala Phe Leu Asn Ser
        275                 280                 285

Val Ile Asn Pro Val Phe Tyr Phe Leu Leu Gly Asp His Phe Arg Asp
    290                 295                 300

Met Leu Met Asn Gln Leu Arg His Asn Phe Lys Ser Leu Thr Ser Phe
305                 310                 315                 320

Ser Arg Trp Ala His Glu Leu Leu Leu Ser Phe Arg Glu Lys
                325                 330

<210> 39
<211> 1296
<212> DNA
<213> Homo sapiens

<400> 39
atgcaggcgc ttaacattac cccggagcag ttctctcggc tgctgcggga ccacaacctg 60

acgcgggagc agttcatcgc tctgtaccgg ctgcgaccgc tcgtctacac cccagagctg 120

ccgggacgcg ccaagctggc cctcgtgctc accggcgtgc tcatcttcgc cctggcgctc 180

tttggcaatg ctctggtgtt ctacgtggtg acccgcagca aggccatgcg caccgtcacc 240

aacatcttta tctgctcctt ggcgctcagt gacctgctca tcaccttctt ctgcattccc 300

gtcaccatgc tccagaacat ttccgacaac tggctggggg gtgctttcat ttgcaagatg 360

gtgccatttg tccagtctac cgctgttgtg acagaaatgc tcactatgac ctgcattgct 420

gtggaaaggc accagggact tgtgcatcct tttaaaatga agtggcaata caccaaccga 480

agggctttca caatgctagg tgtggtctgg ctggtggcag tcatcgtagg atcacccatg 540

tggcacgtgc aacaacttga gatcaaatat gacttcctat atgaaaagga acacatctgc 600

tgcttagaag agtggaccag ccctgtgcac cagaagatct acaccacctt catccttgtc 660

atcctcttcc tcctgcctct tatggtgatg cttattctgt acagtaaaat tggttatgaa 720

ctttggataa agaaaagagt tggggatggt tcagtgcttc gaactattca tggaaaagaa 780

atgtccaaaa tagccaggaa gaagaaacga gctgtcatta tgatggtgac agtggtggct 840

ctctttgctg tgtgctgggc accattccat gttgtccata tgatgattga atacagtaat 900

tttgaaaagg aatatgatga tgtcacaatc aagatgattt ttgctatcgt gcaaattatt 960

ggattttcca actccatctg taatcccatt gtctatgcat ttatgaatga aaacttcaaa 1020

aaaaatgttt tgtctgcagt ttgttattgc atagtaaata aaaccttctc tccagcacaa 1080

aggcatggaa attcaggaat tacaatgatg cggaagaaag caaagttttc cctcagagag 1140

aatccagtgg aggaaaccaa aggagaagca ttcagtgatg gcaacattga agtcaaattg 1200

tgtgaacaga cagaggagaa gaaaaagctc aaacgacatc ttgctctctt taggtctgaa 1260

ctggctgaga attctccttt agacagtggg cattaa 1296

<210> 40
<211> 431
<212> PRT
<213> Homo sapiens

<400> 40

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu
            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Val
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile
305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

<210>  41
<211>  24
<212>  DNA
<213>  Homo sapiens

<400>  41
ctgtgtacag cagttcgcag agtg                                          24

<210>  42
<211>  24
<212>  DNA
<213>  Homo sapiens

<400>  42
gagtgccagg cagagcaggt agac                                          24

<210>  43
<211>  31
<212>  DNA
<213>  Homo sapiens

<400>  43
cccgaattcc tgcttgctcc cagcttggcc c                                  31

<210> 44
<211> 32
<212> DNA
<213> Homo sapiens

<400> 44
tgtggatcct gctgtcaaag gtcccattcc gg 32

<210> 45
<211> 20
<212> DNA
<213> Homo sapiens

<400> 45
tcacaatgct aggtgtggtc 20

<210> 46
<211> 22
<212> DNA
<213> Homo sapiens

<400> 46
tgcatagaca atgggattac ag 22

<210> 47
<211> 511
<212> DNA
<213> Homo sapiens

<400> 47
tcacaatgct aggtgtggtc tggctggtgg cagtcatcgt aggatcaccc atgtggcacg 60

tgcaacaact tgagatcaaa tatgacttcc tatatgaaaa ggaacacatc tgctgcttag 120

aagagtggac cagccctgtg caccagaaga tctacaccac cttcatcctt gtcatcctct 180

tcctcctgcc tcttatggtg atgcttattc tgtacgtaaa attggttatg aactttggat 240

aaagaaaaga gttggggatg gttcagtgct tcgaactatt catggaaaag aaatgtccaa 300

aatagccagg aagaagaaac gagctgtcat tatgatggtg acagtggtgg ctctctttgc 360

tgtgtgctgg gcaccattcc atgttgtcca tatgatgatt gaatacagta attttgaaaa 420

ggaatatgat gatgtcacaa tcaagatgat ttttgctatc gtgcaaatta ttggattttc 480

caactccatc tgtaatccca ttgtctatgc a 511

<210> 48
<211> 21
<212> DNA
<213> Homo sapiens

<400> 48
ctgcttagaa gagtggacca g 21

<210> 49
<211> 22
<212> DNA
<213> Homo sapiens

<400> 49
ctgtgcacca gaagatctac ac 22

<210> 50
<211> 21
<212> DNA
<213> Homo sapiens

<400> 50
caaggatgaa ggtggtgtag a 21

<210> 51
<211> 23
<212> DNA
<213> Homo sapiens

<400> 51
gtgtagatct tctggtgcac agg 23

<210> 52
<211> 21
<212> DNA
<213> Homo sapiens

<400> 52
gcaatgcagg tcatagtgag c 21

<210> 53
<211> 27
<212> DNA
<213> Homo sapiens

<400> 53
tggagcatgg tgacgggaat gcagaag 27

<210> 54
<211> 27
<212> DNA
<213> Homo sapiens

<400> 54
gtgatgagca ggtcactgag cgccaag 27

<210> 55
<211> 23
<212> DNA
<213> Homo sapiens

<400> 55
gcaatgcagg cgcttaacat tac 23

<210> 56
<211> 22
<212> DNA
<213> Homo sapiens

<400> 56
ttgggttaca atctgaaggg ca 22

<210> 57
<211> 23
<212> DNA
<213> Homo sapiens

<400> 57
actccgtgtc cagcaggact ctg 23

<210> 58
<211> 24
<212> DNA
<213> Homo sapiens

<400> 58
tgcgtgttcc tggaccctca cgtg 24

<210> 59
<211> 29
<212> DNA
<213> Homo sapiens

<400> 59
caggccttgg attttaatgt cagggatgg 29

<210> 60
<211> 27
<212> DNA
<213> Homo sapiens

<400> 60
ggagagtcag ctctgaaaga attcagg 27

<210> 61
<211> 27
<212> DNA
<213> Homo sapiens

<400> 61
tgatgtgatg ccagatacta atagcac 27

<210> 62
<211> 27
<212> DNA
<213> Homo sapiens

<400> 62
cctgattcat ttaggtgaga ttgagac 27

<210> 63
<211> 26
<212> DNA
<213> Homo sapiens

<400> 63
cccaagcttc cccaggtgta tttgat 26

<210> 64
<211> 26
<212> DNA
<213> Homo sapiens

<400> 64
gttggatcca cataatgcat tttctc 26

<210> 65
<211> 1080
<212> DNA
<213> Homo sapiens

<400> 65
atgattctca actcttctac tgaagatggt attaaaagaa tccaagatga ttgtcccaaa 60

gctggaaggc ataattacat atttgtcatg attcctactt tatacagtat catctttgtg 120

gtgggaatat ttggaaacag cttggtggtg atagtcattt acttttatat gaagctgaag 180

actgtggcca gtgtttttct tttgaattta gcactggctg acttatgctt tttactgact 240

ttgccactat gggctgtcta cacagctatg gaataccgct ggccctttgg caattaccta 300

tgtaagattg cttcagccag cgtcagtttc aacctgtacg ctagtgtgtt tctactcacg 360

tgtctcagca ttgatcgata cctggctatt gttcacccaa tgaagtcccg ccttcgacgc 420

acaatgcttg tagccaaagt cacctgcatc atcatttggc tgctggcagg cttggccagt 480

ttgccagcta taatccatcg aaatgtattt ttcattgaga acaccaatat tacagtttgt 540

gctttccatt atgagtccca aaattcaacc cttccgatag ggctgggcct gaccaaaaat 600

atactgggtt tcctgtttcc ttttctgatc attcttacaa gttatactct tatttggaag 660

gccctaaaga aggcttatga aattcagaag aacaaaccaa gaaatgatga tatttttaag 720

ataattatgg caattgtgct tttctttttc ttttcctgga ttccccacca aatattcact 780

tttctggatg tattgattca actaggcatc atacgtgact gtagaattgc agatattgtg 840

gacacggcca tgcctatcac catttgtata gcttatttta acaattgcct gaatcctctt 900

ttttatggct ttctggggaa aaaatttaaa agatattttc tccagcttct aaaatatatt 960

cccccaaaag ccaaatccca ctcaaacctt tcaacaaaaa tgagcacgct ttcctaccgc 1020

ccctcagata atgtaagctc atccaccaag aagcctgcac catgttttga ggttgagtga 1080

<210> 66
<211> 359
<212> PRT
<213> Homo sapiens

<400> 66

<210>  67
<211>  27
<212>  DNA
<213>  Homo sapiens

<400>  67
accatgggca gcccctggaa cggcagc                                          27

<210>  68
<211>  39
<212>  DNA
<213>  Homo sapiens

<400>  68
agaaccacca ccagcaggac gcggacggtc tgccggtgg                              39


<210>  69
<211>  39
<212>  DNA
<213>  Homo sapiens

<400>  69
gtccgcgtcc tgctggtggt ggttctggca tttataatt                              39


<210>  70
<211>  33
<212>  DNA
<213>  Homo sapiens

<400>  70
cctggatcct tatcccatcg tcttcacgtt agc                                    33


<210>  71
<211>  26
<212>  DNA
<213>  Homo sapiens

<400>  71
ctggaattct cctgccagca tggtga                                            26


<210>  72
<211>  30
<212>  DNA
<213>  Homo sapiens

<400>  72
gcaggatcct atattgcgtg ctctgtcccc                                        30

<210> 73
<211> 999
<212> DNA
<213> Homo sapiens

<400> 73
atggtgaact ccacccaccg tgggatgcac acttctctgc acctctggaa ccgcagcagt 60

tacagactgc acagcaatgc cagtgagtcc cttggaaaag gctactctga tggagggtgc 120

tacgagcaac tttttgtctc tcctgaggtg tttgtgactc tgggtgtcat cagcttgttg 180

gagaatatct tagtgattgt ggcaatagcc aagaacaaga atctgcattc acccatgtac 240

tttttcatct gcagcttggc tgtggctgat atgctggtga gcgtttcaaa tggatcagaa 300

accattatca tcaccctatt aaacagtaca gatacggatg cacagagttt cacagtgaat 360

attgataatg tcattgactc ggtgatctgt agctccttgc ttgcatccat ttgcagcctg 420

ctttcaattg cagtggacag gtactttact atcttctatg ctctccagta ccataacatt 480

atgacagtta agcgggttgg gatcagcata agttgtatct gggcagcttg cacggtttca 540

ggcattttgt tcatcattta ctcagatagt agtgctgtca tcatctgcct catcaccatg 600

ttcttcacca tgctggctct catggcttct ctctatgtcc acatgttcct gatggccagg 660

cttcacatta agaggattgc tgtcctcccc ggcactggtg ccatccgcca aggtgccaat 720

atgaagggag cgattacctt gaccatcctg attggcgtct ttgttgtctg ctgggcccca 780

ttcttcctcc acttaatatt ctacatctct tgtcctcaga atccatattg tgtgtgcttc 840

atgtctcact ttaacttgta tctcatactg atcatgtgta attcaatcat cgatcctctg 900

atttatgcac tccggagtca agaactgagg aaaaccttca aagagatcat ctgttgctat 960

cccctgggag gcctttgtga cttgtctagc agatattaa 999

<210>  74
<211>  332
<212>  PRT
<213>  Homo sapiens

<400>  74

<210> 75
<211> 32
<212> DNA
<213> Homo sapiens

<400> 75
ccgaagcttc gagctgagta aggcggcggg ct 32

<210> 76
<211> 31
<212> DNA
<213> Homo sapiens

<400> 76
gtggaattca tttgccctgc ctcaaccccc a 31

<210> 77
<211> 1344
<212> DNA
<213> Homo sapiens

<400> 77
atggagctgc taaagctgaa ccggagcgtg cagggaaccg gacccgggcc gggggcttcc 60

ctgtgccgcc cgggggcgcc tctcctcaac agcagcagtg tgggcaacct cagctgcgag 120

ccccctcgca ttcgcggagc cgggacacga gaattggagc tggccattag aatcactctt 180

tacgcagtga tcttcctgat gagcgttgga ggaaatatgc tcatcatcgt ggtcctggga 240

ctgagccgcc gcctgaggac tgtcaccaat gccttcctcc tctcactggc agtcagcgac 300

ctcctgctgg ctgtggcttg catgcccttc accctcctgc ccaatctcat gggcacattc 360

atctttggca ccgtcatctg caaggcggtt tcctacctca tgggggtgtc tgtgagtgtg 420

tccacgctaa gcctcgtggc catcgcactg gagcgatata gcgccatctg ccgaccactg 480

caggcacgag tgtggcagac gcgctcccac gcggctcgcg tgattgtagc cacgtggctg 540

ctgtccggac tactcatggt gccctacccc gtgtacactg tcgtgcaacc agtggggcct 600

cgtgtgctgc agtgcgtgca tcgctggccc agtgcgcggg tccgccagac ctggtccgta 660

ctgctgcttc tgctcttgtt cttcatccca ggtgtggtta tggccgtggc ctacgggctt 720

atctctcgcg agctctactt agggcttcgc tttgacggcg acagtgacag cgacagccaa 780

agcagggtcc gaaaccaagg cgggctgcca ggggctgttc accagaacgg gcgttgccgg 840

cctgagactg gcgcggttgg caaagacagc gatggctgct acgtgcaact tccacgttcc 900

cggcctgccc tggagctgac ggcgctgacg gctcctgggc cgggatccgg ctcccggccc 960

acccaggcca agctgctggc taagaagcgc gtggtgcgaa tgttgctggt gatcgttgtg 1020

cttttttttc tgtgttggtt gccagtttat agtgccaaca cgtggcgcgc ctttgatggc 1080

ccgggtgcac accgagcact ctcgggtgct cctatctcct tcattcactt gctgagctac 1140

gcctcggcct gtgtcaaccc cctggtctac tgcttcatgc accgtcgctt tcgccaggcc 1200

tgcctggaaa cttgcgctcg ctgctgcccc cggcctccac gagctcgccc cagggctctt 1260

cccgatgagg accctcccac tccctccatt gcttcgctgt ccaggcttag ctacaccacc 1320

atcagcacac tgggccctgg ctga 1344

<210> 78
<211> 447
<212> PRT
<213> Homo sapiens

<400> 78

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu
            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu
145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Val Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

<210>  79
<211>  30
<212>  DNA
<213>  Homo sapiens

<400>  79
tgcaagctta aaaaggaaaa aatgaacagc                                        30

<210>  80
<211>  30
<212>  DNA
<213>  Homo sapiens

<400>  80
taaggatccc ttcccttcaa aacatccttg                                        30

<210>  81
<211>  1014
<212>  DNA
<213>  Homo sapiens

<400>  81
atgaacagca catgtattga agaacagcat gacctggatc actatttgtt tcccattgtt      60

tacatctttg tgattatagt cagcattcca gccaatattg gatctctgtg tgtgtctttc     120

ctgcaaccca agaaggaaag tgaactagga atttacctct tcagtttgtc actatcagat     180

ttactctatg cattaactct ccctttatgg attgattata cttggaataa agacaactgg     240

actttctctc ctgccttgtg caaagggagt gcttttctca tgtacatgaa gttttacagc     300

agcacagcat tcctcacctg cattgccgtt gatcggtatt tggctgttgt ctaccctttg 360

aagttttttt tcctaaggac aagaagaatt gcactcatgg tcagcctgtc catctggata 420

ttggaaacca tcttcaatgc tgtcatgttg tgggaagatg aaacagttgt tgaatattgc 480

gatgccgaaa agtctaattt tactttatgc tatgacaaat accctttaga gaaatggcaa 540

atcaacctca acttgttcag gacgtgtaca ggctatgcaa tacctttggt caccatcctg 600

atctgtaacc ggaaagtcta ccaagctgtg cggcacaata aagccacgga aaacaaggaa 660

aagaagagaa tcataaaact acttgtcagc atcacagtta cttttgtctt atgctttact 720

ccctttcatg tgatgttgct gattcgctgc attttagagc atgctgtgaa cttcgaagac 780

cacagcaatt ctgggaagcg aacttacaca atgtatagaa tcacggttgc attaacaagt 840

ttaaattgtg ttgctgatcc aattctgtac tgttttgtta ccgaaacagg aagatatgat 900

atgtggaata tattaaaatt ctgcactggg aggtgtaata catcacaaag acaaagaaaa 960

cgcatacttt ctgtgtctac aaaagatact atggaattag aggtccttga gtag 1014

<210> 82
<211> 337
<212> PRT
<213> Homo sapiens

<400> 82

Met Asn Ser Thr Cys Ile Glu Glu Gln His Asp Leu Asp His Tyr Leu
1               5                   10                  15

Phe Pro Ile Val Tyr Ile Phe Val Ile Ile Val Ser Ile Pro Ala Asn
            20                  25                  30

Ile Gly Ser Leu Cys Val Ser Phe Leu Gln Pro Lys Lys Glu Ser Glu
        35                  40                  45

Leu Gly Ile Tyr Leu Phe Ser Leu Ser Leu Ser Asp Leu Leu Tyr Ala
    50                  55                  60

Leu Thr Leu Pro Leu Trp Ile Asp Tyr Thr Trp Asn Lys Asp Asn Trp
65                  70                  75                  80

Thr Phe Ser Pro Ala Leu Cys Lys Gly Ser Ala Phe Leu Met Tyr Met
                85                  90                  95

Lys Phe Tyr Ser Ser Thr Ala Phe Leu Thr Cys Ile Ala Val Asp Arg
            100                 105                 110

Tyr Leu Ala Val Val Tyr Pro Leu Lys Phe Phe Phe Leu Arg Thr Arg
        115                 120                 125

Arg Ile Ala Leu Met Val Ser Leu Ser Ile Trp Ile Leu Glu Thr Ile
    130                 135                 140

Phe Asn Ala Val Met Leu Trp Glu Asp Glu Thr Val Val Glu Tyr Cys
145                 150                 155                 160

Asp Ala Glu Lys Ser Asn Phe Thr Leu Cys Tyr Asp Lys Tyr Pro Leu
                165                 170                 175

Glu Lys Trp Gln Ile Asn Leu Asn Leu Phe Arg Thr Cys Thr Gly Tyr
            180                 185                 190

Ala Ile Pro Leu Val Thr Ile Leu Ile Cys Asn Arg Lys Val Tyr Gln
        195                 200                 205

Ala Val Arg His Asn Lys Ala Thr Glu Asn Lys Glu Lys Lys Arg Ile
    210                 215                 220

Ile Lys Leu Leu Val Ser Ile Thr Val Thr Phe Val Leu Cys Phe Thr
225                 230                 235                 240

Pro Phe His Val Met Leu Leu Ile Arg Cys Ile Leu Glu His Ala Val
                245                 250                 255

Asn Phe Glu Asp His Ser Asn Ser Gly Lys Arg Thr Tyr Thr Met Tyr
            260                 265                 270

Arg Ile Thr Val Ala Leu Thr Ser Leu Asn Cys Val Ala Asp Pro Ile
        275                 280                 285

Leu Tyr Cys Phe Val Thr Glu Thr Gly Arg Tyr Asp Met Trp Asn Ile
    290                 295                 300

Leu Lys Phe Cys Thr Gly Arg Cys Asn Thr Ser Gln Arg Gln Arg Lys
305                 310                 315                 320

Arg Ile Leu Ser Val Ser Thr Lys Asp Thr Met Glu Leu Glu Val Leu
                325                 330                 335

Glu

<210>  83
<211>  40
<212>  DNA
<213>  Homo sapiens

<400>  83
caggaagaag aaacgagctg tcattatgat ggtgacagtg                                40

<210>  84
<211>  40
<212>  DNA
<213>  Homo sapiens

<400>  84
cactgtcacc atcataatga cagctcgttt cttcttcctg                              40

<210>  85
<211>  30
<212>  DNA
<213>  Homo sapiens

<400>  85
ggccaccggc agaccaaacg cgtcctgctg                                         30

<210>  86
<211>  31
<212>  DNA
<213>  Homo sapiens

<400>  86
ctccttcggt cctcctatcg ttgtcagaag t                                       31

<210>  87
<211>  37
<212>  DNA
<213>  Homo sapiens

<400>  87
ggaaaagaag agaatcaaaa aactacttgt cagcatc                                 37

<210>  88
<211>  31
<212>  DNA
<213>  Homo sapiens

<400>  88
ctccttcggt cctcctatcg ttgtcagaag t                                       31

<210> 89
<211> 1080
<212> DNA
<213> Homo sapiens

<400> 89
atgattctca actcttctac tgaagatggt attaaaagaa tccaagatga ttgtcccaaa 60

gctggaaggc ataattacat atttgtcatg attcctactt tatacagtat catctttgtg 120

gtgggaatat ttggaaacag cttggtggtg atagtcattt acttttatat gaagctgaag 180

actgtggcca gtgtttttct tttgaattta gcactggctg acttatgctt tttactgact 240

ttgccactat gggctgtcta cacagctatg gaataccgct ggccctttgg caattaccta 300

tgtaagattg cttcagccag cgtcagtttc aacctgtacg ctagtgtgtt tctactcacg 360

tgtctcagca ttgatcgata cctggctatt gttcacccaa tgaagtcccg ccttcgacgc 420

acaatgcttg tagccaaagt cacctgcatc atcatttggc tgctggcagg cttggccagt 480

ttgccagcta taatccatcg aaatgtattt ttcattgaga acaccaatat tacagtttgt 540

gctttccatt atgagtccca aaattcaacc cttccgatag ggctgggcct gaccaaaaat 600

atactgggtt tcctgtttcc ttttctgatc attcttacaa gttatactct tatttggaag 660

gccctaaaga aggcttatga aattcagaag aacaaaccaa gaaatgatga tattaaaaag 720

ataattatgg caattgtgct tttctttttc ttttcctgga ttccccacca aatattcact 780

tttctggatg tattgattca actaggcatc atacgtgact gtagaattgc agatattgtg 840

gacacggcca tgcctatcac catttgtata gcttatttta acaattgcct gaatcctctt 900

ttttatggct ttctggggaa aaaatttaaa agatattttc tccagcttct aaaatatatt 960

cccccaaaag ccaaatccca ctcaaacctt tcaacaaaaa tgagcacgct ttcctaccgc 1020

ccctcagata atgtaagctc atccaccaag aagcctgcac catgttttga ggttgagtga 1080

<210> 90
<211> 359
<212> PRT
<213> Homo sapiens

<400> 90

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Lys Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu
        355

<210>  91
<211>  35
<212>  DNA
<213>  Homo sapiens

<400>  91
ccaagaaatg atgatattaa aaagataatt atggc                                35

<210>  92
<211>  31
<212>  DNA
<213>  Homo sapiens

<400>  92
ctccttcggt cctcctatcg ttgtcagaag t                                    31

<210>  93
<211>  1080
<212>  DNA
<213>  Homo sapiens

<400>  93
atgattctca actcttctac tgaagatggt attaaaagaa tccaagatga ttgtcccaaa      60

gctggaaggc ataattacat atttgtcatg attcctactt tatacagtat catctttgtg     120

gtgggaatat ttggaaacag cttggtggtg atagtcattt acttttatat gaagctgaag     180

actgtggcca gtgtttttct tttgaattta gcactggctg acttatgctt tttactgact     240

ttgccactat gggctgtcta cacagctatg gaataccgct ggccctttgg caattaccta     300

tgtaagattg cttcagccag cgtcagtttc gccctgtacg ctagtgtgtt tctactcacg     360

tgtctcagca ttgatcgata cctggctatt gttcacccaa tgaagtcccg ccttcgacgc     420

acaatgcttg tagccaaagt cacctgcatc atcatttggc tgctggcagg cttggccagt 480

ttgccagcta taatccatcg aaatgtattt ttcattgaga acaccaatat tacagtttgt 540

gctttccatt atgagtccca aaattcaacc cttccgatag ggctgggcct gaccaaaaat 600

atactgggtt tcctgtttcc ttttctgatc attcttacaa gttatactct tatttggaag 660

gccctaaaga aggcttatga aattcagaag aacaaaccaa gaaatgatga tatttttaag 720

ataattatgg caattgtgct tttctttttc ttttcctgga ttccccacca aatattcact 780

tttctggatg tattgattca actaggcatc atacgtgact gtagaattgc agatattgtg 840

gacacggcca tgcctatcac catttgtata gcttatttta acaattgcct gaatcctctt 900

ttttatggct ttctggggaa aaaatttaaa agatattttc tccagcttct aaaatatatt 960

cccccaaaag ccaaatccca ctcaaacctt tcaacaaaaa tgagcacgct ttcctaccgc 1020

ccctcagata atgtaagctc atccaccaag aagcctgcac catgttttga ggttgagtga 1080

<210> 94
<211> 359
<212> PRT
<213> Homo sapiens

<400> 94

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Ala Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Phe Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu
        355

<210>  95
<211>  26
<212>  DNA
<213>  Homo sapiens

<400>  95
cccaagcttc cccaggtgta tttgat                                          26

<210> 96
<211> 29
<212> DNA
<213> Homo sapiens

<400> 96
cctgcaggcg aaactgactc tggctgaag 29

<210> 97
<211> 42
<212> DNA
<213> Homo sapiens

<400> 97
ctgtacgcta gtgtgtttct actcacgtgt ctcagcattg at 42

<210> 98
<211> 26
<212> DNA
<213> Homo sapiens

<400> 98
gttggatcca cataatgcat tttctc 26

<210> 99
<211> 1080
<212> DNA
<213> Homo sapiens

<400> 99
atgattctca actcttctac tgaagatggt attaaaagaa tccaagatga ttgtcccaaa 60

gctggaaggc ataattacat atttgtcatg attcctactt tatacagtat catctttgtg 120

gtgggaatat ttggaaacag cttggtggtg atagtcattt acttttatat gaagctgaag 180

actgtggcca gtgtttttct tttgaattta gcactggctg acttatgctt tttactgact 240

ttgccactat gggctgtcta cacagctatg gaataccgct ggccctttgg caattaccta 300

tgtaagattg cttcagccag cgtcagtttc aacctgtacg ctagtgtgtt tctactcacg 360

tgtctcagca ttgatcgata cctggctatt gttcacccaa tgaagtcccg ccttcgacgc 420

acaatgcttg tagccaaagt cacctgcatc atcatttggc tgctggcagg cttggccagt 480

ttgccagcta taatccatcg aaatgtattt ttcattgaga acaccaatat tacagtttgt 540

gctttccatt atgagtccca aaattcaacc cttccgatag ggctgggcct gaccaaaaat 600

atactgggtt tcctgtttcc ttttctgatc attcttacaa gttattttgg aattcgaaaa 660

cacttactga agacgaatag ctatgggaag aacaggataa cccgtgacca agttaagaag 720

ataattatgg caattgtgct ttttcttttc ttttcctgga ttccccacca aatattcact 780

tttctggatg tattgattca actaggcatc atacgtgact gtagaattgc agatattgtg 840

gacacggcca tgcctatcac catttgtata gcttatttta acaattgcct gaatcctctt 900

ttttatggct ttctggggaa aaaatttaaa agatattttc tccagcttct aaaatatatt 960

cccccaaaag ccaaatccca ctcaaacctt tcaacaaaaa tgagcacgct ttcctaccgc 1020

ccctcagata atgtaagctc atccaccaag aagcctgcac catgttttga ggttgagtga 1080

<210> 100
<211> 359
<212> PRT
<213> Homo sapiens

<400> 100

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Phe Gly Ile Arg Lys His Leu Leu Lys
    210                 215                 220

Thr Asn Ser Tyr Gly Lys Asn Arg Ile Thr Arg Asp Gln Val Lys Lys
225                 230                 235                 240

Ile Ile Met Ala Ile Val Leu Phe Phe Phe Phe Ser Trp Ile Pro His
                245                 250                 255

Gln Ile Phe Thr Phe Leu Asp Val Leu Ile Gln Leu Gly Ile Ile Arg
            260                 265                 270

Asp Cys Arg Ile Ala Asp Ile Val Asp Thr Ala Met Pro Ile Thr Ile
        275                 280                 285

Cys Ile Ala Tyr Phe Asn Asn Cys Leu Asn Pro Leu Phe Tyr Gly Phe
    290                 295                 300

Leu Gly Lys Lys Phe Lys Arg Tyr Phe Leu Gln Leu Leu Lys Tyr Ile
305                 310                 315                 320

Pro Pro Lys Ala Lys Ser His Ser Asn Leu Ser Thr Lys Met Ser Thr
                325                 330                 335

Leu Ser Tyr Arg Pro Ser Asp Asn Val Ser Ser Ser Thr Lys Lys Pro
            340                 345                 350

Ala Pro Cys Phe Glu Val Glu
        355

<210>  101
<211>  37
<212>  DNA
<213>  Homo sapiens

<400>  101
tccgaattcc aaaataactt gtaagaatga tcagaaa                              37

<210> 102
<211> 33
<212> DNA
<213> Homo sapiens

<400> 102
agatcttaag aagataatta tggcaattgt gct 33

<210> 103
<211> 62
<212> DNA
<213> Homo sapiens

<400> 103
aattcgaaaa cacttactga agacgaatag ctatgggaag aacaggataa cccgtgacca 60

ag 62

<210> 104
<211> 62
<212> DNA
<213> Homo sapiens

<400> 104
ttaacttggt cacgggttat cctgttcttc ccatagctat tcgtcttcag taagtgtttt 60

cg 62

<210> 105
<211> 1083
<212> DNA
<213> Homo sapiens

<400> 105
atgattctca actcttctac tgaagatggt attaaaagaa tccaagatga ttgtcccaaa 60

gctggaaggc ataattacat atttgtcatg attcctactt tatacagtat catctttgtg 120

gtgggaatat ttggaaacag cttggtggtg atagtcattt acttttatat gaagctgaag 180

actgtggcca gtgtttttct tttgaattta gcactggctg acttatgctt tttactgact 240

ttgccactat gggctgtcta cacagctatg gaataccgct ggccctttgg caattaccta 300

tgtaagattg cttcagccag cgtcagtttc aacctgtacg ctagtgtgtt tctactcacg 360

tgtctcagca ttgatcgata cctggctatt gttcacccaa tgaagtcccg ccttcgacgc 420

acaatgcttg tagccaaagt cacctgcatc atcatttggc tgctggcagg cttggccagt 480

ttgccagcta taatccatcg aaatgtattt ttcattgaga acaccaatat tacagtttgt 540

gctttccatt atgagtccca aaattcaacc cttccgatag ggctgggcct gaccaaaaat 600

atactgggtt tcctgtttcc ttttctgatc attcttacaa gttatactct tatttggaag 660

gccctaaaga aggcttatga aattcagaag aacaaaccaa gaaatgatga tatttttaag 720

ataattatgg cagcaattgt gcttttcttt ttcttttcct ggattcccca ccaaatattc 780

acttttctgg atgtattgat tcaactaggc atcatacgtg actgtagaat tgcagatatt 840

gtggacacgg ccatgcctat caccatttgt atagcttatt ttaacaattg cctgaatcct 900

cttttttatg gctttctggg gaaaaaattt aaaagatatt ttctccagct tctaaaatat 960

attcccccaa aagccaaatc ccactcaaac ctttcaacaa aaatgagcac gctttcctac 1020

cgcccctcag ataatgtaag ctcatccacc aagaagcctg caccatgttt tgaggttgag 1080

tga 1083

<210> 106
<211> 360
<212> PRT
<213> Homo sapiens

<400> 106

Met Ile Leu Asn Ser Ser Thr Glu Asp Gly Ile Lys Arg Ile Gln Asp
1               5                   10                  15

Asp Cys Pro Lys Ala Gly Arg His Asn Tyr Ile Phe Val Met Ile Pro
            20                  25                  30

Thr Leu Tyr Ser Ile Ile Phe Val Val Gly Ile Phe Gly Asn Ser Leu
        35                  40                  45

Val Val Ile Val Ile Tyr Phe Tyr Met Lys Leu Lys Thr Val Ala Ser
    50                  55                  60

Val Phe Leu Leu Asn Leu Ala Leu Ala Asp Leu Cys Phe Leu Leu Thr
65                  70                  75                  80

Leu Pro Leu Trp Ala Val Tyr Thr Ala Met Glu Tyr Arg Trp Pro Phe
                85                  90                  95

Gly Asn Tyr Leu Cys Lys Ile Ala Ser Ala Ser Val Ser Phe Asn Leu
            100                 105                 110

Tyr Ala Ser Val Phe Leu Leu Thr Cys Leu Ser Ile Asp Arg Tyr Leu
        115                 120                 125

Ala Ile Val His Pro Met Lys Ser Arg Leu Arg Arg Thr Met Leu Val
    130                 135                 140

Ala Lys Val Thr Cys Ile Ile Ile Trp Leu Leu Ala Gly Leu Ala Ser
145                 150                 155                 160

Leu Pro Ala Ile Ile His Arg Asn Val Phe Phe Ile Glu Asn Thr Asn
                165                 170                 175

Ile Thr Val Cys Ala Phe His Tyr Glu Ser Gln Asn Ser Thr Leu Pro
            180                 185                 190

Ile Gly Leu Gly Leu Thr Lys Asn Ile Leu Gly Phe Leu Phe Pro Phe
        195                 200                 205

Leu Ile Ile Leu Thr Ser Tyr Thr Leu Ile Trp Lys Ala Leu Lys Lys
    210                 215                 220

Ala Tyr Glu Ile Gln Lys Asn Lys Pro Arg Asn Asp Asp Ile Phe Lys
225                 230                 235                 240

Ile Ile Met Ala Ala Ile Val Leu Phe Phe Phe Phe Ser Trp Ile Pro
                245                 250                 255

His Gln Ile Phe Thr Phe Leu Asp Val Leu Ile Gln Leu Gly Ile Ile
            260                 265                 270

Arg Asp Cys Arg Ile Ala Asp Ile Val Asp Thr Ala Met Pro Ile Thr
        275                 280                 285

Ile Cys Ile Ala Tyr Phe Asn Asn Cys Leu Asn Pro Leu Phe Tyr Gly
    290                 295                 300

Phe Leu Gly Lys Lys Phe Lys Arg Tyr Phe Leu Gln Leu Leu Lys Tyr
305                 310                 315                 320

Ile Pro Pro Lys Ala Lys Ser His Ser Asn Leu Ser Thr Lys Met Ser
                325                 330                 335

Thr Leu Ser Tyr Arg Pro Ser Asp Asn Val Ser Ser Ser Thr Lys Lys
            340                 345                 350

Pro Ala Pro Cys Phe Glu Val Glu
        355                 360

<210> 107
<211> 26
<212> DNA
<213> Homo sapiens

<400> 107
cccaagcttc cccaggtgta tttgat 26

<210> 108
<211> 38
<212> DNA
<213> Homo sapiens

<400> 108
aagcacaatt gctgcataat tatcttaaaa atatcatc 38

<210> 109
<211> 39
<212> DNA
<213> Homo sapiens

<400> 109
aagataatta tggcagcaat tgtgcttttc tttttcttt 39

<210> 110
<211> 26
<212> DNA
<213> Homo sapiens

<400> 110
gttggatcca cataatgcat tttctc 26

<210> 111
<211> 1344
<212> DNA
<213> Homo sapiens

<400> 111
atggagctgc taaagctgaa ccggagcgtg cagggaaccg gacccgggcc gggggcttcc 60

ctgtgccgcc cgggggcgcc tctcctcaac agcagcagtg tgggcaacct cagctgcgag 120

ccccctcgca ttcgcggagc cgggacacga gaattggagc tggccattag aatcactctt 180

tacgcagtga tcttcctgat gagcgttgga ggaaatatgc tcatcatcgt ggtcctggga 240

ctgagccgcc gcctgaggac tgtcaccaat gccttcctcc tctcactggc agtcagcgac 300

ctcctgctgg ctgtggcttg catgcccttc accctcctgc ccaatctcat gggcacattc 360

atctttggca ccgtcatctg caaggcggtt tcctacctca tgggggtgtc tgtgagtgtg 420

tccacgctaa gcctcgtggc catcgcactg gagcgatata gcgccatctg ccgaccactg 480

caggcacgag tgtggcagac gcgctcccac gcggctcgcg tgattgtagc cacgtggctg 540

ctgtccggac tactcatggt gccctacccc gtgtacactg tcgtgcaacc agtggggcct 600

cgtgtgctgc agtgcgtgca tcgctggccc agtgcgcggg tccgccagac ctggtccgta 660

ctgctgcttc tgctcttgtt cttcatccca ggtgtggtta tggccgtggc ctacgggctt 720

atctctcgcg agctctactt agggcttcgc tttgacggcg acagtgacag cgacagccaa 780

agcagggtcc gaaaccaagg cgggctgcca ggggctgttc accagaacgg gcgttgccgg 840

cctgagactg gcgcggttgg caaagacagc gatggctgct acgtgcaact tccacgttcc 900

cggcctgccc tggagctgac ggcgctgacg gctcctgggc cgggatccgg ctcccggccc 960

acccaggcca agctgctggc taagaagcgc gtgaaacgaa tgttgctggt gatcgttgtg 1020

cttttttttc tgtgttggtt gccagtttat agtgccaaca cgtggcgcgc ctttgatggc 1080

ccgggtgcac accgagcact ctcgggtgct cctatctcct tcattcactt gctgagctac 1140

gcctcggcct gtgtcaaccc cctggtctac tgcttcatgc accgtcgctt tcgccaggcc 1200

tgcctggaaa cttgcgctcg ctgctgcccc cggcctccac gagctcgccc cagggctctt 1260

cccgatgagg accctcccac tccctccatt gcttcgctgt ccaggcttag ctacaccacc 1320

atcagcacac tgggccctgg ctga 1344

<210>  112
<211>  447
<212>  PRT
<213>  Homo sapiens

<400>  112

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu
            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu
145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Lys Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

<210>  113
<211>  34
<212>  DNA
<213>  Homo sapiens

<400>  113
cagcagcatg cgcttcacgc gcttcttagc ccag                                    34

<210>  114
<211>  35
<212>  DNA
<213>  Homo sapiens

<400>  114
agaagcgcgt gaagcgcatg ctgctggtga tcgtt                                   35

<210> 115
<211> 33
<212> DNA
<213> Homo sapiens

<400> 115
atggagaaaa gaatcaaaag aatgttctat ata 33

<210> 116
<211> 33
<212> DNA
<213> Homo sapiens

<400> 116
tatatagaac attcttttga ttcttttctc cat 33

<210> 117
<211> 30
<212> DNA
<213> Homo sapiens

<400> 117
cgctctctgg ccttgaagcg cacgctcagc 30

<210> 118
<211> 30
<212> DNA
<213> Homo sapiens

<400> 118
gctgagcgtg cgcttcaagg ccagagagcg 30

<210> 119
<211> 30
<212> DNA
<213> Homo sapiens

<400> 119
cccaggaaaa aggtgaaagt caaagttttc 30

<210> 120
<211> 30
<212> DNA
<213> Homo sapiens

<400> 120
gaaaactttg actttcacct ttttcctggg 30

<210> 121
<211> 27
<212> DNA
<213> Homo sapiens

<400> 121
ggggcgcggg tgaaacggct ggtgagc 27

<210> 122
<211> 27
<212> DNA
<213> Homo sapiens

<400> 122
gctcaccagc cgtttcaccc gcgcccc 27

<210> 123
<211> 30
<212> DNA
<213> Homo sapiens

<400> 123
ccccttgaaa agcctaagaa cttggtcatc 30

<210> 124
<211> 30
<212> DNA
<213> Homo sapiens

<400> 124
gatgaccaag ttcttaggct tttcaagggg 30

<210>  125
<211>  32
<212>  DNA
<213>  Homo sapiens

<400>  125
gatctctaga atgaacagca catgtattga ag                                    32


<210>  126
<211>  35
<212>  DNA
<213>  Homo sapiens

<400>  126
ctagggtacc cgctcaagga cctctaattc catag                                 35


<210>  127
<211>  1296
<212>  DNA
<213>  Homo sapiens

<400>  127
atgcaggcgc ttaacattac cccggagcag ttctctcggc tgctgcggga ccacaacctg      60

acgcgggagc agttcatcgc tctgtaccgg ctgcgaccgc tcgtctacac cccagagctg     120

ccgggacgcg ccaagctggc cctcgtgctc accggcgtgc tcatcttcgc cctggcgctc     180

tttggcaatg ctctggtgtt ctacgtggtg acccgcagca aggccatgcg caccgtcacc     240

aacatcttta tctgctcctt ggcgctcagt gacctgctca tcaccttctt ctgcattccc     300

gtcaccatgc tccagaacat ttccgacaac tggctggggg gtgctttcat ttgcaagatg     360

gtgccatttg tccagtctac cgctgttgtg acagaaatgc tcactatgac ctgcattgct     420

gtggaaaggc accagggact tgtgcatcct tttaaaatga agtggcaata caccaaccga     480

agggctttca caatgctagg tgtggtctgg ctggtggcag tcatcgtagg atcacccatg     540

tggcacgtgc aacaacttga gatcaaatat gacttcctat atgaaaagga acacatctgc     600

tgcttagaag agtggaccag ccctgtgcac cagaagatct acaccacctt catccttgtc     660

atcctcttcc tcctgcctct tatggtgatg cttattctgt acagtaaaat tggttatgaa 720

ctttggataa agaaaagagt tggggatggt tcagtgcttc gaactattca tggaaaagaa 780

atgtccaaaa tagccaggaa gaagaaacga gctaagatta tgatggtgac agtggtggct 840

ctctttgctg tgtgctgggc accattccat gttgtccata tgatgattga atacagtaat 900

tttgaaaagg aatatgatga tgtcacaatc aagatgattt ttgctatcgt gcaaattatt 960

ggattttcca actccatctg taatcccatt gtctatgcat ttatgaatga aaacttcaaa 1020

aaaaatgttt tgtctgcagt ttgttattgc atagtaaata aaaccttctc tccagcacaa 1080

aggcatggaa attcaggaat tacaatgatg cggaagaaag caaagttttc cctcagagag 1140

aatccagtgg aggaaaccaa aggagaagca ttcagtgatg gcaacattga agtcaaattg 1200

tgtgaacaga cagaggagaa gaaaaagctc aaacgacatc ttgctctctt taggtctgaa 1260

ctggctgaga attctccttt agacagtggg cattaa 1296

<210> 128
<211> 431
<212> PRT
<213> Homo sapiens

<400> 128

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu
            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Lys
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile
305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

<210>  129
<211>  2040
<212>  DNA
<213>  Homo sapiens

<400>  129
atgggcagcc cctggaacgg cagcgacggc cccgaggggg cgcgggagcc gccgtggccc      60

gcgctgccgc cttgcgacga gcgccgctgc tcgccctttc ccctgggggc gctggtgccg     120

gtgaccgctg tgtgcctgtg cctgttcgtc gtcggggtga gcggcaacgt ggtgaccgtg     180

atgctgatcg ggcgctaccg ggacatgcgg accaccacca acttgtacct gggcagcatg     240

gccgtgtccg acctactcat cctgctcggg ctgccgttcg acctgtaccg cctctggcgc     300

tcgcggccct gggtgttcgg gccgctgctc tgccgcctgt ccctctacgt gggcgagggc     360

tgcacctacg ccacgctgct gcacatgacc gcgctcagcg tcgagcgcta cctggccatc     420

tgccgcccgc tccgcgcccg cgtcttggtc acccggcgcc gcgtccgcgc gctcatcgct     480

gtgctctggg ccgtggcgct gctctctgcc ggtcccttct tgttcctggt gggcgtcgag     540

caggaccccg gcatctccgt agtcccgggc ctcaatggca ccgcgcggat cgcctcctcg     600

cctctcgcct cgtcgccgcc tctctggctc tcgcgggcgc caccgccgtc cccgccgtcg     660

gggcccgaga ccgcggaggc cgcggcgctg ttcagccgcg aatgccggcc gagccccgcg     720

cagctgggcg cgctgcgtgt catgctgtgg gtcaccaccg cctacttctt cctgcccttt     780

ctgtgcctca gcatcctcta cgggctcatc gggcgggagc tgtggagcag ccggcggccg     840

ctgcgaggcc cggccgcctc ggggcgggag agaggccacc ggcagaccaa acgcgtcctg     900

cgtaagtgga gccgccgtgg ttccaaagac gcctgcctgc agtccgcccc gccggggacc     960

gcgcaaacgc tgggtcccct tcccctgctc gcccagctct gggcgccgct tccagctccc    1020

tttcctattt cgattccagc ctccacccgc cggtacttcc catcccccga gaaaaccatg    1080

tcctgtcccc caggagctct gggggacccc agggcgcttt gagggtggga tccccggatc 1140

cgattcagta accagcagtg cttttccaga gcctctgaga ccagaaagga gagttggtaa 1200

ttcttaatcc aaccacctgt tagatgccac aaatgaggag tcctcacagt gctcttgaga 1260

agacgaggga gatttcatta agctaaaatt ttttatttaa tgttaagtga tgctgaaggc 1320

taaagtaaac cttgctcgta tcaaaaagta aagattgtgc agacctgttg tagaattctt 1380

ttcaacagag aacagaaaac ttgtctccga agtgggtttg tggaaggaag cctgccaagg 1440

cggcttgttc agagaaattg ctccttctgg tttatgtcca gccttgataa cacatatggg 1500

agcctactat gcagttttaa agcaagtatc catgcagcct gcagcctggt cattttttct 1560

ggggtgagga tctgcctagg tagaagtttt ctctaattta ttttgctgtt acttgttatt 1620

gcagatggtt ccttgtcggg gtggggggtt tatttgcttc ccaatgcttt tgttaatccc 1680

ggtgctgtgt cttatgttgc agtggtggtg gttctggcat ttataatttg ctggttgccc 1740

ttccacgttg gcagaatcat ttacataaac acggaagatt cgcggatgat gtacttctct 1800

cagtacttta acatcgtcgc tctgcaactt ttctatctga gcgcatctat caacccaatc 1860

ctctacaacc tcatttcaaa gaagtacaga gcggcggcct ttaaactgct gctcgcaagg 1920

aagtccaggc cgagaggctt ccacagaagc agggacactg cgggggaagt tgcaggggac 1980

actggaggag acacggtggg ctacaccgag acaagcgcta acgtgaagac gatgggataa 2040

<210> 130
<211> 412
<212> PRT
<213> Homo sapiens

<400> 130

Met Gly Ser Pro Trp Asn Gly Ser Asp Gly Pro Glu Gly Ala Arg Glu
1               5                   10                  15

Pro Pro Trp Pro Ala Leu Pro Pro Cys Asp Glu Arg Arg Cys Ser Pro
            20                  25                  30

Phe Pro Leu Gly Ala Leu Val Pro Val Thr Ala Val Cys Leu Cys Leu
        35                  40                  45

Phe Val Val Gly Val Ser Gly Asn Val Val Thr Val Met Leu Ile Gly
    50                  55                  60

Arg Tyr Arg Asp Met Arg Thr Thr Thr Asn Leu Tyr Leu Gly Ser Met
65                  70                  75                  80

Ala Val Ser Asp Leu Leu Ile Leu Leu Gly Leu Pro Phe Asp Leu Tyr
                85                  90                  95

Arg Leu Trp Arg Ser Arg Pro Trp Val Phe Gly Pro Leu Leu Cys Arg
            100                 105                 110

Leu Ser Leu Tyr Val Gly Glu Gly Cys Thr Tyr Ala Thr Leu Leu His
        115                 120                 125

Met Thr Ala Leu Ser Val Glu Arg Tyr Leu Ala Ile Cys Arg Pro Leu
    130                 135                 140

Arg Ala Arg Val Leu Val Thr Arg Arg Arg Val Arg Ala Leu Ile Ala
145                 150                 155                 160

Val Leu Trp Ala Val Ala Leu Leu Ser Ala Gly Pro Phe Leu Phe Leu
                165                 170                 175

Val Gly Val Glu Gln Asp Pro Gly Ile Ser Val Val Pro Gly Leu Asn
            180                 185                 190

Gly Thr Ala Arg Ile Ala Ser Ser Pro Leu Ala Ser Ser Pro Pro Leu
        195                 200                 205

Trp Leu Ser Arg Ala Pro Pro Pro Ser Pro Pro Ser Gly Pro Glu Thr
    210                 215                 220

Ala Glu Ala Ala Ala Leu Phe Ser Arg Glu Cys Arg Pro Ser Pro Ala
225                 230                 235                 240

Gln Leu Gly Ala Leu Arg Val Met Leu Trp Val Thr Thr Ala Tyr Phe
                245                 250                 255

Phe Leu Pro Phe Leu Cys Leu Ser Ile Leu Tyr Gly Leu Ile Gly Arg
            260                 265                 270

Glu Leu Trp Ser Ser Arg Arg Pro Leu Arg Gly Pro Ala Ala Ser Gly
        275                 280                 285

Arg Glu Arg Gly His Arg Gln Thr Lys Arg Val Leu Leu Val Val Val
    290                 295                 300

Leu Ala Phe Ile Ile Cys Trp Leu Pro Phe His Val Gly Arg Ile Ile
305                 310                 315                 320

Tyr Ile Asn Thr Glu Asp Ser Arg Met Met Tyr Phe Ser Gln Tyr Phe
                325                 330                 335

Asn Ile Val Ala Leu Gln Leu Phe Tyr Leu Ser Ala Ser Ile Asn Pro
            340                 345                 350

Ile Leu Tyr Asn Leu Ile Ser Lys Lys Tyr Arg Ala Ala Ala Phe Lys
        355                 360                 365

Leu Leu Leu Ala Arg Lys Ser Arg Pro Arg Gly Phe His Arg Ser Arg
    370                 375                 380

Asp Thr Ala Gly Glu Val Ala Gly Asp Thr Gly Gly Asp Thr Val Gly
385                 390                 395                 400

Tyr Thr Glu Thr Ser Ala Asn Val Lys Thr Met Gly
                405                 410

<210>  131
<211>  1344
<212>  DNA
<213>  Homo sapiens

<400>  131
atggagctgc taaagctgaa ccggagcgtg cagggaaccg gacccgggcc gggggcttcc      60

ctgtgccgcc cgggggcgcc tctcctcaac agcagcagtg tgggcaacct cagctgcgag     120

ccccctcgca ttcgcggagc cgggacacga gaattggagc tggccattag aatcactctt     180

tacgcagtga tcttcctgat gagcgttgga ggaaatatgc tcatcatcgt ggtcctggga     240

ctgagccgcc gcctgaggac tgtcaccaat gccttcctcc tctcactggc agtcagcgac     300

ctcctgctgg ctgtggcttg catgcccttc accctcctgc ccaatctcat gggcacattc     360

atctttggca ccgtcatctg caaggcggtt tcctacctca tgggggtgtc tgtgagtgtg     420

tccacgctaa gcctcgtggc catcgcactg gagcgatata gcgccatctg ccgaccactg     480

caggcacgag tgtggcagac gcgctcccac gcggctcgcg tgattgtagc cacgtggctg     540

ctgtccggac tactcatggt gccctacccc gtgtacactg tcgtgcaacc agtggggcct     600

cgtgtgctgc agtgcgtgca tcgctggccc agtgcgcggg tccgccagac ctggtccgta     660

ctgctgcttc tgctcttgtt cttcatccca ggtgtggtta tggccgtggc ctacgggctt     720

atctctcgcg agctctactt agggcttcgc tttgacggcg acagtgacag cgacagccaa     780

agcagggtcc gaaaccaagg cgggctgcca ggggctgttc accagaacgg gcgttgccgg     840

cctgagactg gcgcggttgg caaagacagc gatggctgct acgtgcaact tccacgttcc 900

cggcctgccc tggagctgac ggcgctgacg gctcctgggc cgggatccgg ctcccggccc 960

acccaggcca agctgctggc taagaagcgc gtgaaacgaa tgttgctggt gatcgttgtg 1020

cttttttttc tgtgttggtt gccagtttat agtgccaaca cgtggcgcgc ctttgatggc 1080

ccgggtgcac accgagcact ctcgggtgct cctatctcct tcattcactt gctgagctac 1140

gcctcggcct gtgtcaaccc cctggtctac tgcttcatgc accgtcgctt tcgccaggcc 1200

tgcctggaaa cttgcgctcg ctgctgcccc cggcctccac gagctcgccc cagggctctt 1260

cccgatgagg accctcccac tccctccatt gcttcgctgt ccaggcttag ctacaccacc 1320

atcagcacac tgggccctgg ctga 1344

<210> 132
<211> 447
<212> PRT
<213> Homo sapiens

<400> 132

Met Glu Leu Leu Lys Leu Asn Arg Ser Val Gln Gly Thr Gly Pro Gly
1               5                   10                  15

Pro Gly Ala Ser Leu Cys Arg Pro Gly Ala Pro Leu Leu Asn Ser Ser
            20                  25                  30

Ser Val Gly Asn Leu Ser Cys Glu Pro Pro Arg Ile Arg Gly Ala Gly
        35                  40                  45

Thr Arg Glu Leu Glu Leu Ala Ile Arg Ile Thr Leu Tyr Ala Val Ile
    50                  55                  60

Phe Leu Met Ser Val Gly Gly Asn Met Leu Ile Ile Val Val Leu Gly
65                  70                  75                  80

Leu Ser Arg Arg Leu Arg Thr Val Thr Asn Ala Phe Leu Leu Ser Leu
                85                  90                  95

Ala Val Ser Asp Leu Leu Leu Ala Val Ala Cys Met Pro Phe Thr Leu
            100                 105                 110

Leu Pro Asn Leu Met Gly Thr Phe Ile Phe Gly Thr Val Ile Cys Lys
        115                 120                 125

Ala Val Ser Tyr Leu Met Gly Val Ser Val Ser Val Ser Thr Leu Ser
    130                 135                 140

Leu Val Ala Ile Ala Leu Glu Arg Tyr Ser Ala Ile Cys Arg Pro Leu
145                 150                 155                 160

Gln Ala Arg Val Trp Gln Thr Arg Ser His Ala Ala Arg Val Ile Val
                165                 170                 175

Ala Thr Trp Leu Leu Ser Gly Leu Leu Met Val Pro Tyr Pro Val Tyr
            180                 185                 190

Thr Val Val Gln Pro Val Gly Pro Arg Val Leu Gln Cys Val His Arg
        195                 200                 205

Trp Pro Ser Ala Arg Val Arg Gln Thr Trp Ser Val Leu Leu Leu Leu
    210                 215                 220

Leu Leu Phe Phe Ile Pro Gly Val Val Met Ala Val Ala Tyr Gly Leu
225                 230                 235                 240

Ile Ser Arg Glu Leu Tyr Leu Gly Leu Arg Phe Asp Gly Asp Ser Asp
                245                 250                 255

Ser Asp Ser Gln Ser Arg Val Arg Asn Gln Gly Gly Leu Pro Gly Ala
            260                 265                 270

Val His Gln Asn Gly Arg Cys Arg Pro Glu Thr Gly Ala Val Gly Lys
        275                 280                 285

Asp Ser Asp Gly Cys Tyr Val Gln Leu Pro Arg Ser Arg Pro Ala Leu
    290                 295                 300

Glu Leu Thr Ala Leu Thr Ala Pro Gly Pro Gly Ser Gly Ser Arg Pro
305                 310                 315                 320

Thr Gln Ala Lys Leu Leu Ala Lys Lys Arg Val Lys Arg Met Leu Leu
                325                 330                 335

Val Ile Val Val Leu Phe Phe Leu Cys Trp Leu Pro Val Tyr Ser Ala
            340                 345                 350

Asn Thr Trp Arg Ala Phe Asp Gly Pro Gly Ala His Arg Ala Leu Ser
        355                 360                 365

Val Ala Pro Ile Ser Phe Ile His Leu Leu Ser Tyr Ala Ser Ala Cys
    370                 375                 380

Val Asn Pro Leu Val Tyr Cys Phe Met His Arg Arg Phe Arg Gln Ala
385                 390                 395                 400

Cys Leu Glu Thr Cys Ala Arg Cys Cys Pro Arg Pro Pro Arg Ala Arg
                405                 410                 415

Pro Arg Ala Leu Pro Asp Glu Asp Pro Pro Thr Pro Ser Ile Ala Ser
            420                 425                 430

Leu Ser Arg Leu Ser Tyr Thr Thr Ile Ser Thr Leu Gly Pro Gly
        435                 440                 445

<210> 133
<211> 1014
<212> DNA
<213> Homo sapiens

<400> 133
atgaacagca catgtattga agaacagcat gacctggatc actatttgtt tcccattgtt 60

tacatctttg tgattatagt cagcattcca gccaatattg gatctctgtg tgtgtctttc 120

ctgcaagcaa agaaggaaag tgaactagga atttacctct tcagtttgtc actatcagat 180

ttactctatg cattaactct ccctttatgg attgattata cttggaataa agacaactgg 240

actttctctc ctgccttgtg caaagggagt gcttttctca tgtacatgaa tttttacagc 300

agcacagcat tcctcacctg cattgccgtt gatcggtatt tggctgttgt ctaccctttg 360

aagttttttt tcctaaggac aagaagattt gcactcatgg tcagcctgtc catctggata 420

ttggaaacca tcttcaatgc tgtcatgttg tgggaagatg aaacagttgt tgaatattgc 480

gatgccgaaa agtctaattt tactttatgc tatgacaaat accctttaga gaaatggcaa 540

atcaacctca acttgttcag gacgtgtaca ggctatgcaa tacctttggt caccatcctg 600

atctgtaacc ggaaagtcta ccaagctgtg cggcacaata aagccacgga aaacaaggaa 660

aagaagagaa tcaaaaaact acttgtcagc atcacagtta cttttgtctt atgctttact 720

ccctttcatg tgatgttgct gattcgctgc attttagagc atgctgtgaa cttcgaagac 780

cacagcaatt ctgggaagcg aacttacaca atgtatagaa tcacggttgc attaacaagt 840

ttaaattgtg ttgctgatcc aattctgtac tgttttgtta ccgaaacagg aagatatgat 900

atgtggaata tattaaaatt ctgcactggg aggtgtaata catcacaaag acaaagaaaa 960

cgcatacttt ctgtgtctac aaaagatact atggaattag aggtccttga gtag 1014

<210>  134
<211>  337
<212>  PRT
<213>  Homo sapiens

<400>  134

Met Asn Ser Thr Cys Ile Glu Glu Gln His Asp Leu Asp His Tyr Leu
1               5                   10                  15

Phe Pro Ile Val Tyr Ile Phe Val Ile Ile Val Ser Ile Pro Ala Asn
            20                  25                  30

Ile Gly Ser Leu Cys Val Ser Phe Leu Gln Ala Lys Lys Glu Ser Glu
        35                  40                  45

Leu Gly Ile Tyr Leu Phe Ser Leu Ser Leu Ser Asp Leu Leu Tyr Ala
    50                  55                  60

Leu Thr Leu Pro Leu Trp Ile Asp Tyr Thr Trp Asn Lys Asp Asn Trp
65                  70                  75                  80

Thr Phe Ser Pro Ala Leu Cys Lys Gly Ser Ala Phe Leu Met Tyr Met
                85                  90                  95

Asn Phe Tyr Ser Ser Thr Ala Phe Leu Thr Cys Ile Ala Val Asp Arg
            100                 105                 110

Tyr Leu Ala Val Val Tyr Pro Leu Lys Phe Phe Phe Leu Arg Thr Arg
        115                 120                 125

Arg Phe Ala Leu Met Val Ser Leu Ser Ile Trp Ile Leu Glu Thr Ile
    130                 135                 140

Phe Asn Ala Val Met Leu Trp Glu Asp Glu Thr Val Val Glu Tyr Cys
145                 150                 155                 160

Asp Ala Glu Lys Ser Asn Phe Thr Leu Cys Tyr Asp Lys Tyr Pro Leu
                165                 170                 175

Glu Lys Trp Gln Ile Asn Leu Asn Leu Phe Arg Thr Cys Thr Gly Tyr
            180                 185                 190

Ala Ile Pro Leu Val Thr Ile Leu Ile Cys Asn Arg Lys Val Tyr Gln
        195                 200                 205

Ala Val Arg His Asn Lys Ala Thr Glu Asn Lys Glu Lys Lys Arg Ile
    210                 215                 220

Lys Lys Leu Leu Val Ser Ile Thr Val Thr Phe Val Leu Cys Phe Thr
225                 230                 235                 240

Pro Phe His Val Met Leu Leu Ile Arg Cys Ile Leu Glu His Ala Val
                245                 250                 255

Asn Phe Glu Asp His Ser Asn Ser Gly Lys Arg Thr Tyr Thr Met Tyr
            260                 265                 270

Arg Ile Thr Val Ala Leu Thr Ser Leu Asn Cys Val Ala Asp Pro Ile
        275                 280                 285

Leu Tyr Cys Phe Val Thr Glu Thr Gly Arg Tyr Asp Met Trp Asn Ile
    290                 295                 300

Leu Lys Phe Cys Thr Gly Arg Cys Asn Thr Ser Gln Arg Gln Arg Lys
305                 310                 315                 320

Arg Ile Leu Ser Val Ser Thr Lys Asp Thr Met Glu Leu Glu Val Leu
                325                 330                 335

Glu

<210> 135
<211> 999
<212> DNA
<213> Homo sapiens

<400> 135
atggtgaact ccacccaccg tgggatgcac acttctctgc acctctggaa ccgcagcagt 60

tacagactgc acagcaatgc cagtgagtcc cttggaaaag gctactctga tggagggtgc 120

tacgagcaac tttttgtctc tcctgaggtg tttgtgactc tgggtgtcat cagcttgttg 180

gagaatatct tagtgattgt ggcaatagcc aagaacaaga atctgcattc acccatgtac 240

tttttcatct gcagcttggc tgtggctgat atgctggtga gcgtttcaaa tggatcagaa 300

accattatca tcaccctatt aaacagtaca gatacggatg cacagagttt cacagtgaat 360

attgataatg tcattgactc ggtgatctgt agctccttgc ttgcatccat ttgcagcctg 420

ctttcaattg cagtggacag gtactttact atcttctatg ctctccagta ccataacatt 480

atgacagtta agcgggttgg gatcagcata agttgtatct gggcagcttg cacggtttca 540

ggcattttgt tcatcattta ctcagatagt agtgctgtca tcatctgcct catcaccatg 600

ttcttcacca tgctggctct catggcttct ctctatgtcc acatgttcct gatggccagg 660

cttcacatta agaggattgc tgtcctcccc ggcactggtg ccatccgcca aggtgccaat 720

atgaagggaa aaattacctt gaccatcctg attggcgtct ttgttgtctg ctgggcccca 780

ttcttcctcc acttaatatt ctacatctct tgtcctcaga atccatattg tgtgtgcttc 840

atgtctcact ttaacttgta tctcatactg atcatgtgta attcaatcat cgatcctctg 900

atttatgcac tccggagtca agaactgagg aaaaccttca aagagatcat ctgttgctat 960

cccctgggag gcctttgtga cttgtctagc agatattaa 999

<210>  136
<211>  332
<212>  PRT
<213>  Homo sapiens

<400>  136

Met Val Asn Ser Thr His Arg Gly Met His Thr Ser Leu His Leu Trp
1               5                   10                  15

Asn Arg Ser Ser Tyr Arg Leu His Ser Asn Ala Ser Glu Ser Leu Gly
            20                  25                  30

Lys Gly Tyr Ser Asp Gly Gly Cys Tyr Glu Gln Leu Phe Val Ser Pro
        35                  40                  45

Glu Val Phe Val Thr Leu Gly Val Ile Ser Leu Leu Glu Asn Ile Leu
    50                  55                  60

Val Ile Val Ala Ile Ala Lys Asn Lys Asn Leu His Ser Pro Met Tyr
65                  70                  75                  80

Phe Phe Ile Cys Ser Leu Ala Val Ala Asp Met Leu Val Ser Val Ser
                85                  90                  95

Asn Gly Ser Glu Thr Ile Ile Ile Thr Leu Leu Asn Ser Thr Asp Thr
            100                 105                 110

Asp Ala Gln Ser Phe Thr Val Asn Ile Asp Asn Val Ile Asp Ser Val
        115                 120                 125

Ile Cys Ser Ser Leu Leu Ala Ser Ile Cys Ser Leu Leu Ser Ile Ala
    130                 135                 140

Val Asp Arg Tyr Phe Thr Ile Phe Tyr Ala Leu Gln Tyr His Asn Ile
145                 150                 155                 160

Met Thr Val Lys Arg Val Gly Ile Ser Ile Ser Cys Ile Trp Ala Ala
                165                 170                 175

Cys Thr Val Ser Gly Ile Leu Phe Ile Ile Tyr Ser Asp Ser Ser Ala
            180                 185                 190

Val Ile Ile Cys Leu Ile Thr Met Phe Phe Thr Met Leu Ala Leu Met
        195                 200                 205

Ala Ser Leu Tyr Val His Met Phe Leu Met Ala Arg Leu His Ile Lys
    210                 215                 220

Arg Ile Ala Val Leu Pro Gly Thr Gly Ala Ile Arg Gln Gly Ala Asn
225                 230                 235                 240

Met Lys Gly Lys Ile Thr Leu Thr Ile Leu Ile Gly Val Phe Val Val
                245                 250                 255

Cys Trp Ala Pro Phe Phe Leu His Leu Ile Phe Tyr Ile Ser Cys Pro
            260                 265                 270

Gln Asn Pro Tyr Cys Val Cys Phe Met Ser His Phe Asn Leu Tyr Leu
        275                 280                 285

Ile Leu Ile Met Cys Asn Ser Ile Ile Asp Pro Leu Ile Tyr Ala Leu
    290                 295                 300

Arg Ser Gln Glu Leu Arg Lys Thr Phe Lys Glu Ile Ile Cys Cys Tyr
305                 310                 315                 320

Pro Leu Gly Gly Leu Cys Asp Leu Ser Ser Arg Tyr
                325                 330

<210> 137
<211> 33
<212> DNA
<213> Homo sapiens

<400> 137
gccaatatga agggaaaaat taccttgacc atc 33

<210> 138
<211> 31
<212> DNA
<213> Homo sapiens

<400> 138
ctccttcggt cctcctatcg ttgtcagaag t 31

<210> 139
<211> 1842
<212> DNA
<213> Homo sapiens

<400> 139
atggggccca ccctagcggt tcccaccccc tatggctgta ttggctgtaa gctaccccag 60

ccagaatacc caccggctct aatcatcttt atgttctgcg cgatggttat caccatcgtt 120

gtagacctaa tcggcaactc catggtcatt ttggctgtga cgaagaacaa gaagctccgg 180

aattctggca acatcttcgt ggtcagtctc tctgtggccg atatgctggt ggccatctac 240

ccataccctt tgatgctgca tgccatgtcc attgggggct gggatctgag ccagttacag 300

tgccagatgg tcgggttcat cacagggctg agtgtggtcg gctccatctt caacatcgtg 360

gcaatcgcta tcaaccgtta ctgctacatc tgccacagcc tccagtacga acggatcttc 420

agtgtgcgca atacctgcat ctacctggtc atcacctgga tcatgaccgt cctggctgtc 480

ctgcccaaca tgtacattgg caccatcgag tacgatcctc gcacctacac ctgcatcttc 540

aactatctga acaaccctgt cttcactgtt accatcgtct gcatccactt cgtcctccct 600

ctcctcatcg tgggtttctg ctacgtgagg atctggacca aagtgctggc ggcccgtgac 660

cctgcagggc agaatcctga caaccaactt gctgaggttc gcaattttct aaccatgttt 720

gtgatcttcc tcctctttgc agtgtgctgg tgccctatca acgtgctcac tgtcttggtg 780

gctgtcagtc cgaaggagat ggcaggcaag atccccaact ggctttatct tgcagcctac 840

ttcatagcct acttcaacag ctgcctcaac gctgtgatct acgggctcct caatgagaat 900

ttccgaagag aatactggac catcttccat gctatgcggc accctatcat attcttccct 960

ggcctcatca gtgatattcg tgagatgcag gaggcccgta ccctggcccg cgcccgtgcc 1020

catgctcgcg accaagctcg tgaacaagac cgtgcccatg cctgtcctgc tgtggaggaa 1080

accccgatga atgtccggaa tgttccatta cctggtgatg ctgcagctgg ccaccccgac 1140

cgtgcctctg gccaccctaa gccccattcc agatcctcct ctgcctatcg caaatctgcc 1200

tctacccacc acaagtctgt ctttagccac tccaaggctg cctctggtca cctcaagcct 1260

gtctctggcc actccaagcc tgcctctggt caccccaagt ctgccactgt ctaccctaag 1320

cctgcctctg tccatttcaa gggtgactct gtccatttca agggtgactc tgtccatttc 1380

aagcctgact ctgttcattt caagcctgct tccagcaacc ccaagcccat cactggccac 1440

catgtctctg ctggcagcca ctccaagtct gccttcagtg ctgccaccag ccaccctaaa 1500

cccatcaagc cagctaccag ccatgctgag cccaccactg ctgactatcc caagcctgcc 1560

actaccagcc accctaagcc cgctgctgct gacaaccctg agctctctgc ctcccattgc 1620

cccgagatcc ctgccattgc ccaccctgtg tctgacgaca gtgacctccc tgagtcggcc 1680

tctagccctg ccgctgggcc caccaagcct gctgccagcc agctggagtc tgacaccatc 1740

gctgaccttc ctgaccctac tgtagtcact accagtacca atgattacca tgatgtcgtg 1800

gttgttgatg ttgaagatga tcctgatgaa atggctgtgt ga 1842

<210>  140
<211>  613
<212>  PRT
<213>  Homo sapiens

<400>  140

Met Gly Pro Thr Leu Ala Val Pro Thr Pro Tyr Gly Cys Ile Gly Cys
1               5                   10                  15

Lys Leu Pro Gln Pro Glu Tyr Pro Pro Ala Leu Ile Ile Phe Met Phe
            20                  25                  30

Cys Ala Met Val Ile Thr Ile Val Val Asp Leu Ile Gly Asn Ser Met
        35                  40                  45

Val Ile Leu Ala Val Thr Lys Asn Lys Lys Leu Arg Asn Ser Gly Asn
    50                  55                  60

Ile Phe Val Val Ser Leu Ser Val Ala Asp Met Leu Val Ala Ile Tyr
65                  70                  75                  80

Pro Tyr Pro Leu Met Leu His Ala Met Ser Ile Gly Gly Trp Asp Leu
                85                  90                  95

Ser Gln Leu Gln Cys Gln Met Val Gly Phe Ile Thr Gly Leu Ser Val
            100                 105                 110

Val Gly Ser Ile Phe Asn Ile Val Ala Ile Ala Ile Asn Arg Tyr Cys
        115                 120                 125

Tyr Ile Cys His Ser Leu Gln Tyr Glu Arg Ile Phe Ser Val Arg Asn
    130                 135                 140

Thr Cys Ile Tyr Leu Val Ile Thr Trp Ile Met Thr Val Leu Ala Val
145                 150                 155                 160

Leu Pro Asn Met Tyr Ile Gly Thr Ile Glu Tyr Asp Pro Arg Thr Tyr
                165                 170                 175

Thr Cys Ile Phe Asn Tyr Leu Asn Asn Pro Val Phe Thr Val Thr Ile
            180                 185                 190

Val Cys Ile His Phe Val Leu Pro Leu Leu Ile Val Gly Phe Cys Tyr
        195                 200                 205

Val Arg Ile Trp Thr Lys Val Leu Ala Ala Arg Asp Pro Ala Gly Gln
    210                 215                 220

Asn Pro Asp Asn Gln Leu Ala Glu Val Arg Asn Phe Leu Thr Met Phe
225                 230                 235                 240

Val Ile Phe Leu Leu Phe Ala Val Cys Trp Cys Pro Ile Asn Val Leu
                245                 250                 255

Thr Val Leu Val Ala Val Ser Pro Lys Glu Met Ala Gly Lys Ile Pro
            260                 265                 270

Asn Trp Leu Tyr Leu Ala Ala Tyr Phe Ile Ala Tyr Phe Asn Ser Cys
        275                 280                 285

Leu Asn Ala Val Ile Tyr Gly Leu Leu Asn Glu Asn Phe Arg Arg Glu
    290                 295                 300

Tyr Trp Thr Ile Phe His Ala Met Arg His Pro Ile Ile Phe Phe Pro
305                 310                 315                 320

Gly Leu Ile Ser Asp Ile Arg Glu Met Gln Glu Ala Arg Thr Leu Ala
                325                 330                 335

Arg Ala Arg Ala His Ala Arg Asp Gln Ala Arg Glu Gln Asp Arg Ala
            340                 345                 350

His Ala Cys Pro Ala Val Glu Glu Thr Pro Met Asn Val Arg Asn Val
        355                 360                 365

Pro Leu Pro Gly Asp Ala Ala Ala Gly His Pro Asp Arg Ala Ser Gly
    370                 375                 380

His Pro Lys Pro His Ser Arg Ser Ser Ser Ala Tyr Arg Lys Ser Ala
385                 390                 395                 400

Ser Thr His His Lys Ser Val Phe Ser His Ser Lys Ala Ala Ser Gly
                405                 410                 415

His Leu Lys Pro Val Ser Gly His Ser Lys Pro Ala Ser Gly His Pro
            420                 425                 430

Lys Ser Ala Thr Val Tyr Pro Lys Pro Ala Ser Val His Phe Lys Gly
        435                 440                 445

Asp Ser Val His Phe Lys Gly Asp Ser Val His Phe Lys Pro Asp Ser
    450                 455                 460

Val His Phe Lys Pro Ala Ser Ser Asn Pro Lys Pro Ile Thr Gly His
465                 470                 475                 480

His Val Ser Ala Gly Ser His Ser Lys Ser Ala Phe Ser Ala Ala Thr
                485                 490                 495

Ser His Pro Lys Pro Ile Lys Pro Ala Thr Ser His Ala Glu Pro Thr
            500                 505                 510

Thr Ala Asp Tyr Pro Lys Pro Ala Thr Thr Ser His Pro Lys Pro Ala
        515                 520                 525

Ala Ala Asp Asn Pro Glu Leu Ser Ala Ser His Cys Pro Glu Ile Pro
    530                 535                 540

Ala Ile Ala His Pro Val Ser Asp Asp Ser Asp Leu Pro Glu Ser Ala
545                 550                 555                 560

Ser Ser Pro Ala Ala Gly Pro Thr Lys Pro Ala Ala Ser Gln Leu Glu
                565                 570                 575

Ser Asp Thr Ile Ala Asp Leu Pro Asp Pro Thr Val Val Thr Thr Ser
            580                 585                 590

Thr Asn Asp Tyr His Asp Val Val Val Val Asp Val Glu Asp Asp Pro
        595                 600                 605

Asp Glu Met Ala Val
    610

<210> 141
<211> 1842
<212> DNA
<213> Homo sapiens

<400> 141
atggggccca ccctagcggt tcccaccccc tatggctgta ttggctgtaa gctaccccag 60

ccagaatacc caccggctct aatcatcttt atgttctgcg cgatggttat caccatcgtt 120

gtagacctaa tcggcaactc catggtcatt ttggctgtga cgaagaacaa gaagctccgg 180

aattctggca acatcttcgt ggtcagtctc tctgtggccg atatgctggt ggccatctac 240

ccataccctt tgatgctgca tgccatgtcc attgggggct gggatctgag ccagttacag 300

tgccagatgg tcgggttcat cacagggctg agtgtggtcg gctccatctt caacatcgtg 360

gcaatcgcta tcaaccgtta ctgctacatc tgccacagcc tccagtacga acggatcttc 420

agtgtgcgca atacctgcat ctacctggtc atcacctgga tcatgaccgt cctggctgtc 480

ctgcccaaca tgtacattgg caccatcgag tacgatcctc gcacctacac ctgcatcttc 540

aactatctga acaaccctgt cttcactgtt accatcgtct gcatccactt cgtcctccct 600

ctcctcatcg tgggtttctg ctacgtgagg atctggacca aagtgctggc ggcccgtgac 660

cctgcagggc agaatcctga caaccaactt gctgaggttc gcaataaact aaccatgttt 720

gtgatcttcc tcctctttgc agtgtgctgg tgccctatca acgtgctcac tgtcttggtg 780

gctgtcagtc cgaaggagat ggcaggcaag atccccaact ggctttatct tgcagcctac 840

ttcatagcct acttcaacag ctgcctcaac gctgtgatct acgggctcct caatgagaat 900

ttccgaagag aatactggac catcttccat gctatgcggc accctatcat attcttctct 960

ggcctcatca gtgatattcg tgagatgcag gaggcccgta ccctggcccg cgcccgtgcc 1020

catgctcgcg accaagctcg tgaacaagac cgtgcccatg cctgtcctgc tgtggaggaa 1080

accccgatga atgtccggaa tgttccatta cctggtgatg ctgcagctgg ccaccccgac 1140

cgtgcctctg gccaccctaa gccccattcc agatcctcct ctgcctatcg caaatctgcc 1200

tctacccacc acaagtctgt ctttagccac tccaaggctg cctctggtca cctcaagcct 1260

gtctctggcc actccaagcc tgcctctggt caccccaagt ctgccactgt ctaccctaag 1320

cctgcctctg tccatttcaa ggctgactct gtccatttca agggtgactc tgtccatttc 1380

aagcctgact ctgttcattt caagcctgct tccagcaacc ccaagcccat cactggccac 1440

catgtctctg ctggcagcca ctccaagtct gccttcaatg ctgccaccag ccaccctaaa 1500

cccatcaagc cagctaccag ccatgctgag cccaccactg ctgactatcc caagcctgcc 1560

actaccagcc accctaagcc cgctgctgct gacaaccctg agctctctgc ctcccattgc 1620

cccgagatcc ctgccattgc ccaccctgtg tctgacgaca gtgacctccc tgagtcggcc 1680

tctagccctg ccgctgggcc caccaagcct gctgccagcc agctggagtc tgacaccatc 1740

gctgaccttc ctgaccctac tgtagtcact accagtacca atgattacca tgatgtcgtg 1800

gttgttgatg ttgaagatga tcctgatgaa atggctgtgt ga 1842

<210> 142
<211> 613
<212> PRT
<213> Homo sapiens

<400> 142

Met Gly Pro Thr Leu Ala Val Pro Thr Pro Tyr Gly Cys Ile Gly Cys
1               5                   10                  15

Lys Leu Pro Gln Pro Glu Tyr Pro Pro Ala Leu Ile Ile Phe Met Phe
            20                  25                  30

Cys Ala Met Val Ile Thr Ile Val Val Asp Leu Ile Gly Asn Ser Met
        35                  40                  45

Val Ile Leu Ala Val Thr Lys Asn Lys Lys Leu Arg Asn Ser Gly Asn
    50                  55                  60

Ile Phe Val Val Ser Leu Ser Val Ala Asp Met Leu Val Ala Ile Tyr
65                  70                  75                  80

Pro Tyr Pro Leu Met Leu His Ala Met Ser Ile Gly Gly Trp Asp Leu
                85                  90                  95

Ser Gln Leu Gln Cys Gln Met Val Gly Phe Ile Thr Gly Leu Ser Val
            100                 105                 110

Val Gly Ser Ile Phe Asn Ile Val Ala Ile Ala Ile Asn Arg Tyr Cys
        115                 120                 125

Tyr Ile Cys His Ser Leu Gln Tyr Glu Arg Ile Phe Ser Val Arg Asn
    130                 135                 140

Thr Cys Ile Tyr Leu Val Ile Thr Trp Ile Met Thr Val Leu Ala Val
145                 150                 155                 160

Leu Pro Asn Met Tyr Ile Gly Thr Ile Glu Tyr Asp Pro Arg Thr Tyr
                165                 170                 175

Thr Cys Ile Phe Asn Tyr Leu Asn Asn Pro Val Phe Thr Val Thr Ile
            180                 185                 190

Val Cys Ile His Phe Val Leu Pro Leu Leu Ile Val Gly Phe Cys Tyr
        195                 200                 205

Val Arg Ile Trp Thr Lys Val Leu Ala Ala Arg Asp Pro Ala Gly Gln
    210                 215                 220

Asn Pro Asp Asn Gln Leu Ala Glu Val Arg Asn Lys Leu Thr Met Phe
225                 230                 235                 240

Val Ile Phe Leu Leu Phe Ala Val Cys Trp Cys Pro Ile Asn Val Leu
                245                 250                 255

Thr Val Leu Val Ala Val Ser Pro Lys Glu Met Ala Gly Lys Ile Pro
            260                 265                 270

Asn Trp Leu Tyr Leu Ala Ala Tyr Phe Ile Ala Tyr Phe Asn Ser Cys
        275                 280                 285

Leu Asn Ala Val Ile Tyr Gly Leu Leu Asn Glu Asn Phe Arg Arg Glu
    290                 295                 300

Tyr Trp Thr Ile Phe His Ala Met Arg His Pro Ile Ile Phe Phe Ser
305                 310                 315                 320

Gly Leu Ile Ser Asp Ile Arg Glu Met Gln Glu Ala Arg Thr Leu Ala
                325                 330                 335

Arg Ala Arg Ala His Ala Arg Asp Gln Ala Arg Glu Gln Asp Arg Ala
            340                 345                 350

His Ala Cys Pro Ala Val Glu Glu Thr Pro Met Asn Val Arg Asn Val
        355                 360                 365

Pro Leu Pro Gly Asp Ala Ala Ala Gly His Pro Asp Arg Ala Ser Gly
    370                 375                 380

His Pro Lys Pro His Ser Arg Ser Ser Ser Ala Tyr Arg Lys Ser Ala
385                 390                 395                 400

Ser Thr His His Lys Ser Val Phe Ser His Ser Lys Ala Ala Ser Gly
                405                 410                 415

His Leu Lys Pro Val Ser Gly His Ser Lys Pro Ala Ser Gly His Pro
            420                 425                 430

Lys Ser Ala Thr Val Tyr Pro Lys Pro Ala Ser Val His Phe Lys Ala
        435                 440                 445

Asp Ser Val His Phe Lys Gly Asp Ser Val His Phe Lys Pro Asp Ser
    450                 455                 460

Val His Phe Lys Pro Ala Ser Ser Asn Pro Lys Pro Ile Thr Gly His
465                 470                 475                 480

His Val Ser Ala Gly Ser His Ser Lys Ser Ala Phe Asn Ala Ala Thr
                485                 490                 495

Ser His Pro Lys Pro Ile Lys Pro Ala Thr Ser His Ala Glu Pro Thr
            500                 505                 510

Thr Ala Asp Tyr Pro Lys Pro Ala Thr Thr Ser His Pro Lys Pro Ala
        515                 520                 525

Ala Ala Asp Asn Pro Glu Leu Ser Ala Ser His Cys Pro Glu Ile Pro
    530                 535                 540

Ala Ile Ala His Pro Val Ser Asp Asp Ser Asp Leu Pro Glu Ser Ala
545                 550                 555                 560

Ser Ser Pro Ala Ala Gly Pro Thr Lys Pro Ala Ala Ser Gln Leu Glu
                565                 570                 575

Ser Asp Thr Ile Ala Asp Leu Pro Asp Pro Thr Val Val Thr Thr Ser
            580                 585                 590

Thr Asn Asp Tyr His Asp Val Val Val Val Asp Val Glu Asp Asp Pro
        595                 600                 605

Asp Glu Met Ala Val
    610

<210>  143
<211>  33
<212>  DNA
<213>  Homo sapiens

<400>  143
gctgaggttc gcaataaact aaccatgttt gtg                                33

<210>  144
<211>  31
<212>  DNA
<213>  Homo sapiens

<400>  144
ctccttcggt cctcctatcg ttgtcagaag t                                    31


<210>  145
<211>  27
<212>  DNA
<213>  Homo sapiens

<400>  145
ttagatatcg gggcccaccc tagcggt                                         27


<210>  146
<211>  29
<212>  DNA
<213>  Homo sapiens

<400>  146
ggtaccccca cagccatttc atcaggatc                                       29

## Description

This patent application is a continuation-in-part of, and claims priority from, U.S. Serial Number 09/170,496, filed with the United States Patent and Trademark Office on October 13, 1998. This application also claims the benefit of priority from the following provisional applications, all filed via U.S. Express Mail with the United States Patent and Trademark Office on the indicated dates: U.S. Provisional Number 60/110,060, filed November 27, 1998; U.S. Provisional Number 60/120,416, filed February 16, 1999; U.S. Provisional Number 60/121,852, filed February 26, 1999 claiming benefit of U.S. Provisional Number 60/109,213, filed November 20, 1998; U.S. Provisional Number 60/123,944, filed March 12, 1999; U.S. Provisional Number 60/123,945, filed March 12, 1999; U.S. Provisional Number 60/123,948, filed March 12, 1999; U.S. Provisional Number 60/123,951, filed March 12, 1999; U.S. Provisional Number 60/123,946, filed March 12, 1999; U.S. Provisional Number 60/123,949, filed March 12, 1999; U.S. Provisional Number 60/152,524, filed September 3, 1999, claiming benefit of U.S. Provisional Number 60/151,114, filed August 27, 1999 and U.S. Provisional Number 60/108,029, filed November 12, 1998; U.S. Provisional Number 60/136,436, filed May 28, 1999; U.S. Provisional Number 60/136,439, filed May 28, 1999; U.S. Provisional Number 60/136,567, filed May 28, 1999; U.S. Provisional Number 60/137,127, filed May 28, 1999; U.S. Provisional Number 60/137,131, filed May 28, 1999; U.S. Provisional Number 60/141,448, filed June 29, 1999 claiming benefit of U.S. Provisional Number 60/136,437, filed May 28, 1999; U.S. Provisional Number 60/156,633, filed September 29, 1999; U.S. Provisional Number 60/156,555, filed September 29, 1999; U.S. Provisional Number 60/156,634, filed September 29, 1999;U.S. Provisional Number _(Arena Pharmaceuticals, Inc. docket number: CHN10-1), filed September 29, 1999; U.S. Provisional Number _ (Arena Pharmaceuticals, Inc. docket number: RUP6-1), filed October 1, 1999; U.S. Provisional Number _(Arena Pharmaceuticals, Inc. docket number: RUP7-1), filed October 1, 1999; U.S. Provisional Number _(Arena Pharmaceuticals, Inc. docket number: CHN6-1), filed October 1, 1999; U.S. Provisional Number _(Arena Pharmaceuticals, Inc. docket number: RUP5-1), filed October 1, 1999; and U.S. Provisional Number _(Arena Pharmaceuticals, Inc. docket number: CHN9-1), filed October 1, 1999. This application is also related to co-pending U.S. Serial Number _ (Woodcock, Washburn, Kurtz, Makiewicz & Norris, LLP docket number AREN-0050), filed on October 12, 1999 (via U.S. Express Mail) and U.S. Serial Number 09/364,425, filed on July 30, 1999, both incorporated herein by reference. This application also claims priority to U.S. Serial Number _ (Woodcock, Washburn, Kurtz, Makiewicz & Norris, LLP docket number AREN-0054), filed on October 12, 1999 (via U.S. Express Mail), incorporated by reference herein in its entirety. Each of the foregoing applications are incorporated by reference herein in their entirety.

### FIELD OF THE INVENTION

The invention disclosed in this patent document relates to transmembrane receptors, and more particularly to human G protein-coupled receptors, and specifically to GPCRs that have been altered to establish or enhance constitutive activity of the receptor. Preferably, the altered GPCRs are used for the direct identification of candidate compounds as receptor agonists, inverse agonists or partial agonists having potential applicability as therapeutic agents.

### BACKGROUND OF THE INVENTION

Although a number of receptor classes exist in humans, by far the most abundant and therapeutically relevant is represented by the G protein-coupled receptor (GPCR or GPCRs) class. It is estimated that there are some 100,000 genes within the human genome, and of these, approximately 2%, or 2,000 genes, are estimated to code for GPCRs. Receptors, including GPCRs, for which the endogenous ligand has been identified are referred to as "known" receptors, while receptors for which the endogenous ligand has not been identified are referred to as "orphan" receptors. GPCRs represent an important area for the development of pharmaceutical products: from approximately 20 of the 100 known GPCRs, 60% of all prescription pharmaceuticals have been developed.

GPCRs share a common structural motif. All these receptors have seven sequences of between 22 to 24 hydrophobic amino acids that form seven alpha helices, each of which spans the membrane (each span is identified by number, i.e., transmembrane-1 (TM-1), transmebrane-2 (TM-2), etc.). The transmembrane helices are joined by strands of amino acids between transmembrane-2 and transmembrane-3, transmembrane-4 and transmembrane-5, and transmembrane-6 and transmembrane-7 on the exterior, or "extracellular" side, of the cell membrane (these are referred to as "extracellular" regions 1, 2 and 3 (EC-1, EC-2 and EC-3), respectively). The transmembrane helices are also joined by strands of amino acids between transmembrane-1 and transmembrane-2, transmembrane-3 and transmembrane-4, and transmembrane-5 and transmembrane-6 on the interior, or "intracellular" side, of the cell membrane (these are referred to as "intracellular" regions 1, 2 and 3 (IC-1, IC-2 and IC-3), respectively). The "carboxy" ("C") terminus of the receptor lies in the intracellular space within the cell, and the "amino" ("N") terminus of the receptor lies in the extracellular space outside of the cell.

Generally, when an endogenous ligand binds with the receptor (often referred to as "activation" of the receptor), there is a change in the conformation of the intracellular region that allows for coupling between the intracellular region and an intracellular "G-protein." It has been reported that GPCRs are "promiscuous" with respect to G proteins, i. e., that a GPCR can interact with more than one G protein. *See,* Kenakin, T., 43 *Life Sciences* 1095 (1988). Although other G proteins exist, currently, Gq, Gs, Gi, Gz and Go are G proteins that have been identified. Endogenous ligand-activated GPCR coupling with the G-protein begins a signaling cascade process (referred to as "signal transduction"). Under normal conditions, signal transduction ultimately results in cellular activation or cellular inhibition. It is thought that the IC-3 loop as well as the carboxy terminus of the receptor interact with the G protein.

Under physiological conditions, GPCRs exist in the cell membrane in equilibrium between two different conformations: an "inactive" state and an "active" state. A receptor in an inactive state is unable to link to the intracellular signaling transduction pathway to produce a biological response. Changing the receptor conformation to the active state allows linkage to the transduction pathway (via the G-protein) and produces a biological response.

A receptor may be stabilized in an active state by an endogenous ligand or a compound such as a drug. Recent discoveries, including but not exclusively limited to modifications to the amino acid sequence of the receptor, provide means other than endogenous ligands or drugs to promote and stabilize the receptor in the active state conformation. These means effectively stabilize the receptor in an active state by simulating the effect of an endogenous ligand binding to the receptor. Stabilization by such ligand-independent means is termed "constitutive receptor activation."

### SUMMARY OF THE INVENTION

Disclosed herein are non-endogenous versions of endogenous, human GPCRs and uses thereof.

### BRIEF DESCRIPTION OF THE DRAWINGS

**Figure 1** is a representation of 8XCRE-Luc reporter plasmid *(see,* Example 4(c)3.)
**Figures 2A** and **2B** are graphic representations of the results of ATP and ADP binding to endogenous TDAG8 (2A) and comparisons in serum and serum free media (2B).
**Figure 3** is a graphic representation of the comparative signaling results of CMV versus the GPCR Fusion Protein H9(F236K):Gsα.

### DETAILED DESCRIPTION

The scientific literature that has evolved around receptors has adopted a number of terms to refer to ligands having various effects on receptors. For clarity and consistency, the following definitions will be used throughout this patent document. To the extent that these definitions conflict with other definitions for these terms, the following definitions shall control:

**AGONISTS** shall mean materials (e.g., ligands, candidate compounds) that activate the intracellular response when they bind to the receptor, or enhance GTP binding to membranes.

**AMINO ACID ABBREVIATIONS** used herein are set out in Table A:

**TABLE A**

| | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**PARTIAL AGONISTS** shall mean materials (e.g., ligands, candidate compounds) that activate the intracellular response when they bind to the receptor to a lesser degree/extent than do agonists, or enhance GTP binding to membranes to a lesser degree/extent than do agonists.

**ANTAGONIST** shall mean materials (e.g., ligands, candidate compounds) that competitively bind to the receptor at the same site as the agonists but which do not activate the intracellular response initiated by the active form of the receptor, and can thereby inhibit the intracellular responses by agonists or partial agonists. ANTAGONISTS do not diminish the baseline intracellular response in the absence of an agonist or partial agonist.

**CANDIDATE COMPOUND** shall mean a molecule (for example, and not limitation, a chemical compound) that is amenable to a screening technique. Preferably, the phrase "candidate compound" does not include compounds which were publicly known to be compounds selected from the group consisting of inverse agonist, agonist or antagonist to a receptor, as previously determined by an indirect identification process ("indirectly identified compound"); more preferably, not including an indirectly identified compound which has previously been determined to have therapeutic efficacy in at least one mammal; and, most preferably, not including an indirectly identified compound which has previously been determined to have therapeutic utility in humans.

**COMPOSITION** means a material comprising at least one component; a "pharmaceutical composition" is an example of a composition.

**COMPOUND EFFICACY** shall mean a measurement of the ability of a compound to inhibit or stimulate receptor functionality, as opposed to receptor binding affinity. Exemplary means of detecting compound efficacy are disclosed in the Example section of this patent document.

**CODON** shall mean a grouping of three nucleotides (or equivalents to nucleotides) which generally comprise a nucleoside (adenosine (A), guanosine (G), cytidine (C), uridine (U) and thymidine (T)) coupled to a phosphate group and which, when translated, encodes an amino acid.

**CONSTITUTIVELY ACTIVATED RECEPTOR** shall mean a receptor subject to constitutive receptor activation. A constitutively activated receptor can be endogenous or non-endogenous.

**CONSTITUTIVE RECEPTOR ACTIVATION** shall mean stabilization of a receptor in the active state by means other than binding of the receptor with its endogenous ligand or a chemical equivalent thereof.

**CONTACT** or **CONTACTING** shall mean bringing at least two moieties together, whether in an in vitro system or an in vivo system.

**DIRECTLY IDENTIFYING or DIRECTLY IDENTIFIED**, in relationship to the phrase "candidate compound", shall mean the screening of a candidate compound against a constitutively activated receptor, preferably a constitutively activated orphan receptor, and most preferably against a constitutively activated G protein-coupled cell surface orphan receptor, and assessing the compound efficacy of such compound. This phrase is, under no circumstances, to be interpreted or understood to be encompassed by or to encompass the phrase "indirectly identifying" or "indirectly identified."

**ENDOGENOUS** shall mean a material that a mammal naturally produces. ENDOGENOUS in reference to, for example and not limitation, the term "receptor," shall mean that which is naturally produced by a mammal (for example, and not limitation, a human) or a virus. By contrast, the term **NON-ENDOGENOUS** in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not limitation, in a screening approach, the endogenous or non-endogenous receptor may be in reference to an in vitro screening system. As a further example and not limitation, where the genome of a mammal has been manipulated to include a non-endogenous constitutively activated receptor, screening of a candidate compound by means of an in vivo system is viable.

**G PROTEIN COUPLED RECEPTOR FUSION PROTEIN and GPCR FUSION PROTEIN**, in the context of the invention disclosed herein, each mean a non-endogenous protein comprising an endogenous, constitutively activate GPCR or a non-endogenous, constitutively activated GPCR fused to at least one G protein, most preferably the alpha (α) subunit of such G protein (this being the subunit that binds GTP), with the G protein preferably being of the same type as the G protein that naturally couples with endogenous orphan GPCR. For example, and not limitation, in an endogenous state, if the G protein "Gsα" is the predominate G protein that couples with the GPCR, a GPCR Fusion Protein based upon the specific GPCR would be a non-endogenous protein comprising the GPCR fused to Gsα; in some circumstances, as will be set forth below, a non-predominant G protein can be fused to the GPCR. The G protein can be fused directly to the c-terminus of the constitutively active GPCR or there may be spacers between the two.

**HOST CELL** shall mean a cell capable of having a Plasmid and/or Vector incorporated therein. In the case of a prokaryotic Host Cell, a Plasmid is typically replicated as a autonomous molecule as the Host Cell replicates (generally, the Plasmid is thereafter isolated for introduction into a eukaryotic Host Cell); in the case of a eukaryotic Host Cell, a Plasmid is integrated into the cellular DNA of the Host Cell such that when the eukaryotic Host Cell replicates, the Plasmid replicates. Preferably, for the purposes of the invention disclosed herein, the Host Cell is eukaryotic, more preferably, mammalian, and most preferably selected from the group consisting of 293, 293T and COS-7 cells.

**INDIRECTLY IDENTIFYING or INDIRECTLY IDENTIFIED** means the traditional approach to the drug discovery process involving identification of an endogenous ligand specific for an endogenous receptor, screening of candidate compounds against the receptor for determination of those which interfere and/or compete with the ligand-receptor interaction, and assessing the efficacy of the compound for affecting at least one second messenger pathway associated with the activated receptor.

**INHIBIT or INHIBITING**, in relationship to the term "response" shall mean that a response is decreased or prevented in the presence of a compound as opposed to in the absence of the compound.

**INVERSE AGONISTS** shall mean materials (e.g., ligand, candidate compound) which bind to either the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.

**KNOWN RECEPTOR** shall mean an endogenous receptor for which the endogenous ligand specific for that receptor has been identified.

**LIGAND** shall mean an endogenous, naturally occurring molecule specific for an endogenous, naturally occurring receptor.

**MUTANT or MUTATION** in reference to an endogenous receptor's nucleic acid and/or amino acid sequence shall mean a specified change or changes to such endogenous sequences such that a mutated form of an endogenous, non-constitutively activated receptor evidences constitutive activation of the receptor. In terms of equivalents to specific sequences, a subsequent mutated form of a human receptor is considered to be equivalent to a first mutation of the human receptor if (a) the level of constitutive activation of the subsequent mutated form of a human receptor is substantially the same as that evidenced by the first mutation of the receptor; and (b) the percent sequence (amino acid and/or nucleic acid) homology between the subsequent mutated form of the receptor and the first mutation of the receptor is at least about 80%, more preferably at least about 90% and most preferably at least 95%. Ideally, and owing to the fact that the most preferred cassettes disclosed herein for achieving constitutive activation includes a single amino acid and/or codon change between the endogenous and the non-endogenous forms of the GPCR, the percent sequence homology should be at least 98%.

**NON-ORPHAN RECEPTOR** shall mean an endogenous naturally occurring molecule specific for an endogenous naturally occurring ligand wherein the binding of a ligand to a receptor activates an intracellular signaling pathway.

**ORPHAN RECEPTOR** shall mean an endogenous receptor for which the endogenous ligand specific for that receptor has not been identified or is not known.

**PHARMACEUTICAL COMPOSITION** shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan.

**PLASMID** shall mean the combination of a Vector and cDNA. Generally, a Plasmid is introduced into a Host Cell for the purposes of replication and/or expression of the cDNA as a protein.

**STIMULATE or STIMULATING**, in relationship to the term "response" shall mean that a response is increased in the presence of a compound as opposed to in the absence of the compound.

**VECTOR** in reference to cDNA shall mean a circular DNA capable of incorporating at least one cDNA and capable of incorporation into a Host Cell.

The order of the following sections is set forth for presentational efficiency and is not intended, nor should be construed, as a limitation on the disclosure or the claims to follow.

### A. Introduction

The traditional study of receptors has always proceeded from the a priori assumption (historically based) that the endogenous ligand must first be identified before discovery could proceed to find antagonists and other molecules that could affect the receptor. Even in cases where an antagonist might have been known first, the search immediately extended to looking for the endogenous ligand. This mode of thinking has persisted in receptor research even after the discovery of constitutively activated receptors. What has not been heretofore recognized is that it is the active state of the receptor that is most useful for discovering agonists, partial agonists, and inverse agonists of the receptor. For those diseases which result from an overly active receptor or an under-active receptor, what is desired in a therapeutic drug is a compound which acts to diminish the active state of a receptor or enhance the activity of the receptor, respectively, not necessarily a drug which is an antagonist to the endogenous ligand. This is because a compound that reduces or enhances the activity of the active receptor state need not bind at the same site as the endogenous ligand. Thus, as taught by a method of this invention, any search for therapeutic compounds should start by screening compounds against the ligand-independent active state.

### B. Identification of Human GPCRs

The efforts of the Human Genome project has led to the identification of a plethora of information regarding nucleic acid sequences located within the human genome; it has been the case in this endeavor that genetic sequence information has been made available without an understanding or recognition as to whether or not any particular genomic sequence does or may contain open-reading frame information that translate human proteins. Several methods of identifying nucleic acid sequences within the human genome are within the purview of those having ordinary skill in the art. For example, and not limitation, a variety of human GPCRs, disclosed herein, were discovered by reviewing the GenBank™ database, while other GPCRs were discovered by utilizing a nucleic acid sequence of a GPCR, previously sequenced, to conduct a BLAST™ search of the EST database. Table B, below, lists several endogenous GPCRs that we have discovered, along with a GPCR's respective homologous receptor.

**TABLE B**

| **Disclosed Human Orphan GPCRs** | **Accession Number Identified** | **Open Reading Frame (Base Pairs)** | **Per Cent Homology To Designated GPCR** | **Reference To Homologous GPCR (Accession No.)** |
|---|---|---|---|---|
| hARE-3 | AL033379 | 1,260 bp | 52.3% LPA-R | U92642 |
| **hARE-4** | AC006087 | 1,119 bp | 36% P2Y5 | AF000546 |
| **hARE-5** | AC006255 | 1,104 bp | 32% *Oryzias latipes* | D43633 |
| **hGPR27** | AA775870 | 1,128 bp | | |
| **hARE-1** | AI090920 | 999 bp | 43% KIAA0001 | D13626 |
| **hARE-2** | AA359504 | 1,122 bp | 53% GPR27 | |
| **hPPR1** | H67224 | 1,053 bp | 39% EBI1 | L31581 |
| **hG2A** | AA754702 | 1,113 bp | 31% GPR4 | L36148 |
| **hRUP3** | AL035423 | 1,005 bp | 30% *Drosophila melanogaster* | 2133653 |
| **hRUP4** | AI307658 | 1,296 bp | 32% pNPGPR | NP_004876 |
| | | | 28% and 29 % *Zebra fish* Ya and Yb, respectively | AAC41276 and AAB94616 |
| **hRUP5** | AC005849 | 1,413 bp | 25% DEZ | Q99788 |
| | | | 23% FMLPR | P21462 |
| **hRUP6** | AC005871 | 1,245 bp | 48% GPR66 | NP_006047 |
| **hRUP7** | AC007922 | 1,173 bp | 43% H3R | AF140538 |
| **hCHN3** | EST 36581 | 1,113 bp | 53% GPR27 | |
| **hCHN4** | AA804531 | 1,077 bp | 32% thrombin | 4503637 |
| **hCHN6** | EST 2134670 | 1,503 bp | 36% edg-1 | NP_001391 |
| **hCHN8** | EST 764455 | 1,029 bp | 47% KIAA0001 | D13626 |
| **hCHN9** | EST 1541536 | 1,077 bp | 41% LTB4R | NM_000752 |
| **hCHN10** | EST 1365839 | 1,055 bp | 35% P2Y | NM_002563 |

Receptor homology is useful in terms of gaining an appreciation of a role of the receptors within the human body. As the patent document progresses, we will disclose techniques for mutating these receptors to establish non-endogenous, constitutively activated versions of these receptors.

The techniques disclosed herein have also been applied to other human, orphan GPCRs known to the art, as will be apparent as the patent document progresses.

### C. Receptor Screening

Screening candidate compounds against a non-endogenous, constitutively activated version of the human GPCRs disclosed herein allows for the direct identification of candidate compounds which act at this cell surface receptor, without requiring use of the receptor's endogenous ligand. By determining areas within the body where the endogenous version of human GPCRs disclosed herein is expressed and/or over-expressed, it is possible to determine related disease/disorder states which are associated with the expression and/or over-expression of the receptor; such an approach is disclosed in this patent document.

With respect to creation of a mutation that may evidence constitutive activation of the human GPCR disclosed herein is based upon the distance from the proline residue at which is presumed to be located within TM6 of the GPCR; this algorithmic technique is disclosed in co-pending and commonly assigned patent document U.S. Serial Number 09/170,496, incorporated herein by reference. The algorithmic technique is not predicated upon traditional sequence "alignment" but rather a specified distance from the aforementioned TM6 proline residue. By mutating the amino acid residue located 16 amino acid residues from this residue (presumably located in the IC3 region of the receptor) to, most preferably, a lysine residue, such activation may be obtained. Other amino acid residues may be useful in the mutation at this position to achieve this objective.

### D. Disease/Disorder Identification and/or Selection

As will be set forth in greater detail below, most preferably inverse agonists to the non-endogenous, constitutively activated GPCR can be identified by the methodologies ofthis invention. Such inverse agonists are ideal candidates as lead compounds in drug discovery programs for treating diseases related to this receptor. Because of the ability to directly identify inverse agonists to the GPCR, thereby allowing for the development of pharmaceutical compositions, a search for diseases and disorders associated with the GPCR is relevant. For example, scanning both diseased and normal tissue samples for the presence of the GPCR now becomes more than an academic exercise or one which might be pursued along the path of identifying an endogenous ligand to the specific GPCR. Tissue scans can be conducted across a broad range of healthy and diseased tissues. Such tissue scans provide a preferred first step in associating a specific receptor with a disease and/or disorder. *See, for* *example,* co-pending application (docket number ARE-0050) for exemplary dot-blot and RT-PCR results of several of the GPCRs disclosed herein.

Preferably, the DNA sequence of the human GPCR is used to make a probe for (a) dot-blot analysis against tissue-mRNA, and/or (b) RT-PCR identification of the expression of the receptor in tissue samples. The presence of a receptor in a tissue source, or a diseased tissue, or the presence of the receptor at elevated concentrations in diseased tissue compared to a normal tissue, can be preferably utilized to identify a correlation with a treatment regimen, including but not limited to, a disease associated with that disease. Receptors can equally well be localized to regions of organs by this technique. Based on the known functions of the specific tissues to which the receptor is localized, the putative functional role of the receptor can be deduced.

### E. Screening of Candidate Compounds

### 1. Generic GPCR screening assay techniques

When a G protein receptor becomes constitutively active, it binds to a G protein (e.g., Gq, Gs, Gi, Gz, Go) and stimulates the binding of GTP to the G protein. The G protein then acts as a GTPase and slowly hydrolyzes the GTP to GDP, whereby the receptor, under normal conditions, becomes deactivated. However, constitutively activated receptors continue to exchange GDP to GTP. A non-hydrolyzable analog of GTP, [³⁵S]GTPγS, can be used to monitor enhanced binding to membranes which express constitutively activated receptors. It is reported that [³⁵S]GTPγS can be used to monitor G protein coupling to membranes in the absence and presence of ligand. An example of this monitoring, among other examples well-known and available to those in the art, was reported by Traynor and Nahorski in 1995. The preferred use of this assay system is for initial screening of candidate compounds because the system is generically applicable to all G protein-coupled receptors regardless of the particular G protein that interacts with the intracellular domain of the receptor.

### 2. Specific GPCR screening assay techniques

Once candidate compounds are identified using the "generic" G protein-coupled receptor assay (i. e., an assay to select compounds that are agonists, partial agonists, or inverse agonists), further screening to confirm that the compounds have interacted at the receptor site is preferred. For example, a compound identified by the "generic" assay may not bind to the receptor, but may instead merely "uncouple" the G protein from the intracellular domain.

### a. Gs, Gz and Gi.

Gs stimulates the enzyme adenylyl cyclase. Gi (and Gz and Go), on the other hand, inhibit this enzyme. Adenylyl cyclase catalyzes the conversion of ATP to cAMP; thus, constitutively activated GPCRs that couple the Gs protein are associated with increased cellular levels of cAMP. On the other hand, constitutively activated GPCRs that couple Gi (or Gz, Go) protein are associated with decreased cellular levels of cAMP. *See, generally*, "Indirect Mechanisms of Synaptic Transmission," Chpt. 8, From Neuron To Brain (3^{rd} Ed.) Nichols, J.G. et al eds. Sinauer Associates, Inc. (1992). Thus, assays that detect cAMP can be utilized to determine if a candidate compound is, e.g., an inverse agonist to the receptor (i.e., such a compound would decrease the levels of cAMP). A variety of approaches known in the art for measuring cAMP can be utilized; a most preferred approach relies upon the use of anti-cAMP antibodies in an ELISA-based format. Another type of assay that can be utilized is a whole cell second messenger reporter system assay. Promoters on genes drive the expression of the proteins that a particular gene encodes. Cyclic AMP drives gene expression by promoting the binding of a cAMP-responsive DNA binding protein or transcription factor (CREB) that then binds to the promoter at specific sites called cAMP response elements and drives the expression of the gene. Reporter systems can be constructed which have a promoter containing multiple cAMP response elements before the reporter gene, *e*.*g*., β-galactosidase or luciferase. Thus, a constitutively activated Gs-linked receptor causes the accumulation of cAMP that then activates the gene and expression of the reporter protein. The reporter protein such as β-galactosidase or luciferase can then be detected using standard biochemical assays (Chen et al. 1995).

### b. Go and Gq.

Gq and Go are associated with activation of the enzyme phospholipase C, which in turn hydrolyzes the phospholipid PIP₂, releasing two intracellular messengers: diacycloglycerol (DAG) and inistol 1,4,5-triphoisphate (IP₃). Increased accumulation of IP₃ is associated with activation of Gq- and Go-associated receptors. *See, generally*, "Indirect Mechanisms of Synaptic Transmission," Chpt. 8, From Neuron To Brain (3^{rd} Ed.) Nichols, J.G. et al eds. Sinauer Associates, Inc. (1992). Assays that detect IP₃ accumulation can be utilized to determine if a candidate compound is, e.g., an inverse agonist to a Gq- or Go-associated receptor (i.e., such a compound would decrease the levels of IP₃). Gq-associated receptors can also been examined using an AP1 reporter assay in that Gq-dependent phospholipase C causes activation of genes containing AP1 elements; thus, activated Gq-associated receptors will evidence an increase in the expression of such genes, whereby inverse agonists thereto will evidence a decrease in such expression, and agonists will evidence an increase in such expression. Commercially available assays for such detection are available.

### 3. GPCR Fusion Protein

The use of an endogenous, constitutively activate orphan GPCR or a non-endogenous, constitutively activated orphan GPCR, for use in screening of candidate compounds for the direct identification of inverse agonists, agonists and partial agonists provide an interesting screening challenge in that, by definition, the receptor is active even in the absence of an endogenous ligand bound thereto. Thus, in order to differentiate between, e.g., the non-endogenous receptor in the presence of a candidate compound and the non-endogenous receptor in the absence of that compound, with an aim of such a differentiation to allow for an understanding as to whether such compound may be an inverse agonist, agonist, partial agonist or have no affect on such a receptor, it is preferred that an approach be utilized that can enhance such differentiation. A preferred approach is the use of a GPCR Fusion Protein.

Generally, once it is determined that a non-endogenous orphan GPCR has been constitutively activated using the assay techniques set forth above (as well as others), it is possible to determine the predominant G protein that couples with the endogenous GPCR. Coupling of the G protein to the GPCR provides a signaling pathway that can be assessed. Because it is most preferred that screening take place by use of a mammalian expression system, such a system will be expected to have endogenous G protein therein. Thus, by definition, in such a system, the non-endogenous, constitutively activated orphan GPCR will continuously signal. In this regard, it is preferred that this signal be enhanced such that in the presence of, e.g., an inverse agonist to the receptor, it is more likely that it will be able to more readily differentiate, particularly in the context of screening, between the receptor when it is contacted with the inverse agonist.

The GPCR Fusion Protein is intended to enhance the efficacy of G protein coupling with the non-endogenous GPCR. The GPCR Fusion Protein is preferred for screening with a non-endogenous, constitutively activated GPCR because such an approach increases the signal that is most preferably utilized in such screening techniques. This is important in facilitating a significant "signal to noise" ratio; such a significant ratio is import preferred for the screening of candidate compounds as disclosed herein.

The construction of a construct useful for expression of a GPCR Fusion Protein is within the purview of those having ordinary skill in the art. Commercially available expression vectors and systems offer a variety of approaches that can fit the particular needs of an investigator. The criteria of importance for such a GPCR Fusion Protein construct is that the endogenous GPCR sequence and the G protein sequence both be in-frame (preferably, the sequence for the endogenous GPCR is upstream of the G protein sequence) and that the "stop" codon of the GPCR must be deleted or replaced such that upon expression of the GPCR, the G protein can also be expressed. The GPCR can be linked directly to the G protein, or there can be spacer residues between the two (preferably, no more than about 12, although this number can be readily ascertained by one of ordinary skill in the art). We have a preference (based upon convenience) of use of a spacer in that some restriction sites that are not used will, effectively, upon expression, become a spacer. Most preferably, the G protein that couples to the non-endogenous GPCR will have been identified prior to the creation of the GPCR Fusion Protein construct. Because there are only a few G proteins that have been identified, it is preferred that a construct comprising the sequence of the G protein (*i*.*e*., a universal G protein construct) be available for insertion of an endogenous GPCR sequence therein; this provides for efficiency in the context of large-scale screening of a variety of different endogenous GPCRs having different sequences.

As noted above, constitutively activated GPCRs that couple to Gi, Gz and Go are expected to inhibit the formation of cAMP making assays based upon these types of GPCRs challenging (*i*. *e*., the cAMP signal decreases upon activation thus making the direct identification of, *e*.*g*, inverse agonists (which would further decrease this signal), interesting). As will be disclosed herein, we have ascertained that for these types of receptors, it is possible to create a GPCR Fusion Protein that is not based upon the endogenous GPCR's endogenous G protein, in an effort to establish a viable cyclase-based assay. Thus, for example, a Gz coupled receptor such as H9, a GPCR Fusion Protein can be established that utilizes a Gs fusion protein - we believe that such a fusion construct, upon expression, "drives" or "forces" the non-endogenous GPCR to couple with, e.g., Gs rather than the "natural" Gz protein, such that a cyclase-based assay can be established. Thus, for Gi, Gz and Go coupled receptors, we prefer that that when a GPCR Fusion Protein is used and the assay is based upon detection of adenyl cyclase activity, that the fusion construct be established with Gs (or an equivalent G protein that stimulates the formation of the enzyme adenylyl cyclase).

### F. Medicinal Chemistry

Generally, but not always, direct identification of candidate compounds is preferably conducted in conjunction with compounds generated via combinatorial chemistry techniques, whereby thousands of compounds are randomly prepared for such analysis. Generally, the results of such screening will be compounds having unique core structures; thereafter, these compounds are preferably subjected to additional chemical modification around a preferred core structure(s) to further enhance the medicinal properties thereof. Such techniques are known to those in the art and will not be addressed in detail in this patent document.

### G. Pharmaceutical compositions

Candidate compounds selected for further development can be formulated into pharmaceutical compositions using techniques well known to those in the art. Suitable pharmaceutically-acceptable carriers are available to those in the art; for example, see Remington's Pharmaceutical Sciences, 16^{th} Edition, 1980, Mack Publishing Co., (Oslo et al., eds.)

### H. Other Utility

Although a preferred use of the non-endogenous versions the human GPCRs disclosed herein may be for the direct identification of candidate compounds as inverse agonists, agonists or partial agonists (preferably for use as pharmaceutical agents), these versions of human GPCRs can also be utilized in research settings. For example, *in vitro* and *in vivo* systems incorporating GPCRs can be utilized to further elucidate and understand the roles these receptors play in the human condition, both normal and diseased, as well as understanding the role of constitutive activation as it applies to understanding the signaling cascade. The value in non-endogenous human GPCRs is that their utility as a research tool is enhanced in that, because of their unique features, non-endogenous human GPCRs can be used to understand the role of these receptors in the human body before the endogenous ligand therefor is identified. Other uses of the disclosed receptors will become apparent to those in the art based upon, *inter alia*, a review of this patent document.

### EXAMPLES

The following examples are presented for purposes of elucidation, and not limitation, of the present invention. While specific nucleic acid and amino acid sequences are disclosed herein, those of ordinary skill in the art are credited with the ability to make minor modifications to these sequences while achieving the same or substantially similar results reported below. The traditional approach to application or understanding of sequence cassettes from one sequence to another (e.g. from rat receptor to human receptor or from human receptor A to human receptor B) is generally predicated upon sequence alignment techniques whereby the sequences are aligned in an effort to determine areas of commonality. The mutational approach disclosed herein does not rely upon this approach but is instead based upon an algorithmic approach and a positional distance from a conserved proline residue located within the TM6 region of human GPCRs. Once this approach is secured, those in the art are credited with the ability to make minor modifications thereto to achieve substantially the same results *(i. e.,* constitutive activation) disclosed herein. Such modified approaches are considered within the purview of this disclosure

### Example 1

### ENDOGENOUS HUMAN GPCRS

### 1. Identification of Human GPCRs

Certain of the disclosed endogenous human GPCRs were identified based upon a review of the GenBank™ database information. While searching the database, the following cDNA clones were identified as evidenced below (Table C).

**TABLE C**

| **Disclosed Human Orphan GPCRs** | **Accession Number** | **Complete DNA Sequence (Base Pairs)** | **Open Reading Frame (Base Pairs)** | **Nucleic Acid SEQ.ID. NO.** | **Amino Acid SEQ.ID. NO.** |
|---|---|---|---|---|---|
| **hARE-3** | AL033379 | 111,389 bp | 1,260 bp | 1 | 2 |
| **hARE-4** | AC006087 | 226,925 bp | 1,119 bp | 3 | 4 |
| **hARE-5** | AC006255 | 127,605 bp | 1,104 bp | 5 | 6 |
| **hRUP3** | AL035423 | 140,094 bp | 1,005 bp | 7 | 8 |
| **hRUP5** | AC005849 | 169,144 bp | 1,413 bp | 9 | 10 |
| **hRUP6** | AC005871 | 218,807 bp | 1,245 bp | 11 | 12 |
| **hRUP7** | AC007922 | 158,858 bp | 1,173 bp | 13 | 14 |

Other disclosed endogenous human GPCRs were identified by conducting a BLAST™ search of EST database (dbest) using the following EST clones as query sequences. The following EST clones identified were then used as a probe to screen a human genomic library (Table D).

**TABLE D**

| **Disclosed Human Orphan GPCRs** | **Query (Sequence)** | **EST Clone/ Accession No. Identified** | **Open Reading Frame (Base Pairs)** | **Nucleic Acid SEQ.ID.NO.** | **Amino Acid SEQ.ID.NO.** |
|---|---|---|---|---|---|
| **hGPCR27** | Mouse GPCR27 | AA775870 | 1,125 bp | 17 | 18 |
| **hARE-1** | TDAG | 1689643 AI090920 | 999 bp | 19 | 20 |
| **hARE-2** | GPCR27 | 68530 AA359504 | 1,122 bp | 21 | 22 |
| **HPPR1** | Bovine PPR1 | 238667 H67224 | 1,053 bp | 23 | 24 |
| **hG2A** | Mouse 1179426 | *See Example 2(a)*, *below* | 1,113 bp | 25 | 26 |
| **hCHN3** | N.A. | EST 36581 (full length) | 1,113 bp | 27 | 28 |
| **hCHN4** | TDAG | 1184934 AA804531 | 1,077 bp | 29 | 30 |
| **hCHN6** | N.A. | EST2134670 (full length) | 1,503 bp | 31 | 32 |
| **hCHN8** | KIAA0001 | EST 764455 | 1,029 bp | 33 | 34 |
| **hCHN 9** | 1365839 | EST 1541536 | 1,077 bp | 35 | 36 |
| **hCHN10** | Mouse EST 1365839 | Human 1365839 | 1,005 bp | 37 | 38 |
| **hRUP4** | N.A. | AI307658 | 1,296 bp | 39 | 40 |
| *N.A. = "not applicable"*. | | | | | |

### 2. Full Length Cloning

### a. Human G2A

Mouse EST clone 1179426 was used to obtain a human genomic clone containing all but three amino acid G2A coding sequences. The 5'of this coding sequence was obtained by using 5'RACE, and the template for PCR was Clontech's Human Spleen Marathon-Ready™ cDNA. The disclosed human G2A was amplified by PCR using the G2A cDNA specific primers for the first and second round PCR as shown in SEQ.ID.NO.: 41 and SEQ.ID.NO.:42 as follows: 5'-CTGTGTACAGCAGTTCGCAGAGTG-3' (SEQ.ID.NO.: 41; 1st round PCR) 5'-GAGTGCCAGGCAGAGCAGGTAGAC-3' (SEQ.ID.NO.: 42; second round PCR). PCR was performed using Advantage GC Polymerase Kit (Clontech; manufacturing instructions will be followed), at 94°C for 30 sec followed by 5 cycles of 94°C for 5 sec and 72°C for 4 min; and 30 cycles of 94° for 5 sec and 70° for 4 min. An approximate 1.3 Kb PCR fragment was purified from agarose gel, digested with Hind III and Xba I and cloned into the expression vector pRC/CMV2 (Invitrogen). The cloned-insert was sequenced using the T7 Sequenase™ kit (USB Amersham; manufacturer instructions followed) and the sequence was compared with the presented sequence. Expression of the human G2A was detected by probing an RNA dot blot (Clontech; manufacturer instructions followed) with the P³²-labeled fragment.

### b. CHN9

Sequencing of the EST clone 1541536 showed CHN9 to be a partial cDNA clone having only an initiation codon; *i. e.,* the termination codon was missing. When CHN9 was used to blast against data base (nr), the 3' sequence of CHN9 was 100% homologous to the 5' untranslated region of the leukotriene B4 receptor cDNA, which contained a termination codon in the frame with CHN9 coding sequence. To determine whether the 5' untranslated region of LTB4R cDNA was the 3' sequence of CHN9, PCR was performed using primers based upon the 5' sequence flanking the initiation codon found in CHN9 and the 3' sequence around the termination codon found in the LTB4R 5' untranslated region. The 5' primer sequence utilized was as follows: 5'-CCCGAATTCCTGCTTGCTCCCAGCTTGGCCC-3' (SEQ.ID.NO.: 43; sense) and 5'-TGTGGATCCTGCTGTCAAAGGTCCCATTCCGG-3' (SEQ.ID.NO.: 44; antisense). PCR was performed using thymus cDNA as a template and rTth polymerase (Perkin Elmer) with the buffer system provided by the manufacturer, 0.25 uM of each primer, and 0.2 mM of each 4 nucleotides. The cycle condition was 30 cycles of 94°C for 1 min, 65°C for 1min and 72°C for 1 min and 10 sec. A 1.1kb fragment consistent with the predicted size was obtained from PCR. This PCR fragment was subcloned into pCMV *(see* below) and sequenced (*see*, SEQ.ID.NO.: 35).

### c. RUP 4

The full length RUP4 was cloned by RT-PCR with human brain cDNA (Clontech) as templates: 5'-TCACAATGCTAGGTGTGGTC-3' (SEQ.ID.NO.: 45; sense) and 5'-TGCATAGACAATGGGATTACAG-3' (SEQ.ID.NO.: 46; antisense). PCR was performed using TaqPlus Precision™ polymerase (Stratagene; manufacturing instructions followed) by the following cycles: 94°C for 2 min; 94 °C 30 sec; 55 °C for 30 sec, 72°C for 45 sec, and 72°C for 10 min. Cycles 2 through 4 were repeated 30 times.

The PCR products were separated on a 1% agarose gel and a 500 bp PCR fragment was isolated and cloned into the pCRII-TOPO™ vector (Invitrogen) and sequenced using the T7 DNA Sequenase™ kit (Amsham) and the SP6/T7 primers (Stratagene). Sequence analysis revealed that the PCR fragment was indeed an alternatively spliced form of AI307658 having a continuous open reading frame with similarity to other GPCRs. The completed sequence of this PCR fragment was as follows: 
5'-TCACAATGCTAGGTGTGGTCTGGCTGGTGGCAGTCATCGTAGGATCACCCATGTGGCAC
GTGCAACAACTTGAGATCAAATATGACTTCCTATATGAAAAGGAACACATCTGCTGCTTAAGA
GTGGACCAGCCCTGTGCACCAGAAGATCTACACCACCTTCATCCTTGTCATCCTCTTCCTCCTGC
CTCTTATGGTGATGCTTATTCTGTACGTAAAATTGGTTATGAACTTTGGATAAAGAAAAGAGTT
GGGGATGGTTCAGTGCTTCGAACTATTCATGGAAAAGAAATGTCCAAAATAGCCAGGAAGAAG
AAACGAGCTGTCATTATGATGGTGACAGTGGTGGCTCTCTTTGCTGTGTGCTGGGCACCATTCC
ATGTTGTCCATATGATGATTGAATACAGTAATTTTGAAAAGGAATATGATGATGTCACAATCAA
GATGATTTTTGCTATCGTGCAAATTATTGGATTTTCCAACTCCATCTGTAATCCCATTGTCTATGCA-
3' (SEQ.ID.NO.: 47)
 Based on the above sequence, two sense oligonucleotide primer sets: 5'-CTGCTTAGAAGAGTGGACCAG-3' (SEQ.ID.NO.: 48; oligo 1), 5'-CTGTGCACCAGAAGATCTACAC-3' (SEQ.IDNO.: 49; oligo 2) and two antisense oligonucleotide primer sets: 5'-CAAGGATGAAGGTGGTGTAGA-3' (SEQ.ID.NO.: 50; oligo 3) 5'-GTGTAGATCTTCTGGTGCACAGG-3' (SEQ.ID.NO.: 51; oligo 4) were used for 3'- and 5'-RACE PCR with a human brain Marathon-Ready™ cDNA (Clontech, Cat# 7400-1) as template, according to manufacture's instructions. DNA fragments generated by the RACE PCR were cloned into the pCRII-TOPO™ vector (Invitrogen) and sequenced using the SP6/T7 primers (Stratagene) and some internal primers. The 3' RACE product contained a poly(A) tail and a completed open reading frame ending at a TAA stop codon. The 5' RACE product contained an incomplete 5' end; i.e., the ATG initiation codon was not present.

Based on the new 5' sequence, oligo 3 and the following primer: 5'-GCAATGCAGGTCATAGTGAGC -3' (SEQ.ID.NO.: 52; oligo 5) were used for the second round of 5' race PCR and the PCR products were analyzed as above. A third round of 5' race PCR was carried out utilizing antisense primers: 5'-TGGAGCATGGTGACGGGAATGCAGAAG-3' (SEQ.ID.NO.: 53: oligo 6) and 5'-GTGATGAGCAGGTCACTGAGCGCCAAG-3' (SEQ.ID.NO.: 54; oligo7). The sequence of the 5' RACE PCR products revealed the presence of the initiation codon ATG, and further round of 5' race PCR did not generate any more 5' sequence. The completed 5' sequence was confirmed by RT-PCR using sense primer 5'-GCAATGCAGGCGCTTAACATTAC-3' (SEQ.ID.NO.: 55; oligo 8) and oligo 4 as primers and sequence analysis of the 650 bp PCR product generated from human brain and heart cDNA templates (Clontech, Cat# 7404-1). The completed 3' sequence was confirmed by RT-PCR using oligo 2 and the following antisense primer: 5'-TTGGGTTACAATCTGAAGGGCA-3' (SEQ.ID.NO.:56; oligo 9) and sequence analysis of the 670 bp PCR product generated from human brain and heart cDNA templates. (Clontech, Cat# 7404-1).

### d. RUP5

The full length RUP5 was cloned by RT-PCR using a sense primer upstream from ATG, the initiation codon (SEQ.ID.NO.:57), and an antisense primer containing TCA as the stop codon (SEQ.ID.NO.:58), which had the following sequences: 5'-ACTCCGTGTCCAGCAGGACTCTG-3' (SEQ.ID.NO.: 57) 5'-TGCGTGTTCCTGGACCCTCACGTG-3' (SEQ.ID.NO.: 58) and human peripheral leukocyte cDNA (Clontech) as a template. Advantage™ cDNA polymerase (Clontech) was used for the amplification in a 50ul reaction by the following cycle with step 2 through step 4 repeated 30 times: 94°C for 30 sec; 94° for 15 sec; 69° for 40 sec; 72°C for 3 min; and 72°C fro 6 min. A 1.4kb PCR fragment was isolated and cloned with the pCRII-TOPO™ vector (Invitrogen) and completely sequenced using the T7 DNA Sequenase™ kit (Amsham). See, SEQ.ID.NO.: 9.

### e. RUP6

The full length RUP6 was cloned by RT-PCR using primers: 5'-CAGGCCTTGGATTTTAATGTCAGGGATGG-3' (SEQ.ID.NO.: 59) and 5'-GGAGAGTCAGCTCTGAAAGAATTCAGG-3' (SEQ.ID.NO.: 60); and human thymus Marathon-Ready™ cDNA (Clontech) as a template. Advantage cDNA polymerase (Clontech, according to manufacturer's instructions) was used for the amplification in a 50ul reaction by the following cycle: 94°C for 30sec; 94°C for 5 sec; 66°C for 40sec; 72°C for 2.5 sec and 72°C for 7 min. Cycles 2 through 4 were repeated 30 times. A 1.3 Kb PCR fragment was isolated and cloned into the pCRII-TOPO™ vector (Invitrogen) and completely sequenced *(see,* SEQ.ID.NO.: 11) using the ABI Big Dye Terminator™ kit (P.E. Biosystem).

### f. RUP7

The full length RUP7 was cloned by RT-PCR using primers: 5'-TGATGTGATGCCAGATACTAATAGCAC-3' (SEQ.ID.NO.: 61; sense) [?] and 5'-CCTGATTCATTTAGGTGAGATTGAGAC-3' (SEQ.ID.NO.: 62; antisense) and human peripheral leukocyte cDNA (Clontech) as a template. Advantage™ cDNA polymerase (Clontech) was used for the amplification in a 50 ul reaction by the following cycle with step 2 to step 4 repeated 30 times: 94°C for 2 minutes; 94°C for 15 seconds; 60°C for 20 seconds; 72°C for 2 minutes; 72°C for 10 minutes. A 1.25 Kb PCR fragment was isolated and cloned into the pCRII-TOPO™ vector (Invitrogen) and completely sequenced using the ABI Big Dye Terminator™ kit (P.E. Biosystem). See, SEQ.ID.NO.: 13.

### 3. Angiotensin II Type 1 Receptor ("AT1")

The endogenous human angiotensin II type 1 receptor ("AT1") was obtained by PCR using genomic DNA as template and rTth polymerase (Perkin Elmer) with the buffer system provided by the manufacturer, 0.25 µM of each primer, and 0.2 mM of each 4 nucleotides. The cycle condition was 30 cycles of 94°C for 1 min, 55°C for 1min and 72 °C for 1.5 min. The 5' PCR primer contains a HindIII site with the sequence: 5'-CCCAAGCTTCCCCAGGTGTATTTGAT-3' (SEQ.ID.NO.: 63) and the 3' primer contains a BamHI site with the following sequence: 5'-GTTGGATCCACATAATGCATTTTCTC-3' (SEQ.ID.NO.: 64). The resulting 1.3 kb PCR fragment was digested with HindIII and BamHI and cloned into HindIII-BamHI site of pCMV expression vector. The cDNA clone was fully sequenced. Nucleic acid (SEQ.ID.NO.: 65) and amino acid (SEQ.ID.NO.: 66) sequences for human AT1 were thereafter determined and verified.

### 4. GPR38

To obtain GPR38, PCR was performed by combining two PCR fragments, using human genomic cDNA as template and rTth poymerase (Perkin Elmer) with the buffer system provided by the manufacturer, 0.25uM of each primer, and 0.2 mM of each 4 nucleotides. The cycle condition for each PCR reaction was 30 cycles of 94°C for 1 min, 62°C for 1min and 72°C for 2 min.

The first fragment was amplified with the 5' PCR primer that contained an end site with the following sequence: 5'-ACCATGGGCAGCCCCTGGAACGGCAGC-3' (SEQ.ID.NO.:67) and a 3' primer having the following sequence: 5'-AGAACCACCACCAGCAGGACGCGGACGGTCTGCCGGTGG-3' (SEQ.ID.NO.:68). The second PCR fragment was amplified with a 5' primer having the following sequence: 5'-GTCCGCGTCCTGCTGGTGGTGGTTCTGGCATTTATAATT-3' (SEQ.ID.NO.: 69) and a 3' primer that contained a BamHI site and having the following sequence: 5'-CCTGGATCCTTATCCCATCGTCTTCACGTTAGC-3' (SEQ.ID.NO.: 70). The two fragments were used as templates to amplify GPR38, using SEQ.ID.NO.: 67 and SEQ.ID.NO.: 70 as primers (using the above-noted cycle conditions). The resulting 1.44kb PCR fragment was digested with BamHI and cloned into Blunt-BamHI site of pCMV expression vector.

### 5. MC4

To obtain MC4, PCR was performed using human genomic cDNA as template and rTth poymerase (Perkin Elmer) with the buffer system provided by the manufacturer, 0.25uM of each primer, and 0.2 mM of each 4 nucleotides. The cycle condition for each PCR reaction was 30 cycles of 94°C for 1 min, 54°C for 1min and 72°C for 1.5 min. The 5' PCR contained an EcoRI site with the sequence: 5'-CTGGAATTCTCCTGCCAGCATGGTGA-3' (SEQ.ID.NO.: 71) and the 3' primer contained a BamHI site with the sequence: 5'-GCAGGATCCTATATTGCGTGCTCTGTCCCC'-3 (SEQ.ID.NO.: 72). The 1.0 kb PCR fragment was digest with EcoRI and BamHI and cloned into EcoRI-BamHI site of pCMV expression vector. Nucleic acid (SEQ.ID.NO.: 73) and amino acid (SEQ.ID.NO.: 74) sequences for human MC4 were thereafter determined.

### 6. CCKB

To obtain CCKB, PCR was performed using human stomach cDNA as template and rTth poymerase (Perkin Elmer) with the buffer system provided by the manufacturer, 0.25uM of each primer, and 0.2 mM of each 4 nucleotides. The cycle condition for each PCR reaction was 30 cycles of 94°C for 1 min, 65°C for 1min and 72°C for 1 min and 30 sec. The 5' PCR contained a HindIII site with the sequence: 5'-CCGAAGCTTCGAGCTGAGTAAGGCGGCGGGCT-3' (SEQ.ID.NO.: 75) and the 3' primer contained an EcoRI site with the sequence: 5'-GTGGAATTCATTTGCCCTGCCTCAACCCCCA-3 (SEQ.ID.NO.: 76). The resulting 1.44 kb PCR fragment was digest with HindIII and EcoRI and cloned into HindIII-EcoRI site of pCMV expression vector. Nucleic acid (SEQ.ID.NO.: 77) and amino acid (SEQ.ID.NO.: 78) sequences for human CCKB were thereafter determined.

### 7. TDAG8

To obtain TDAG8, PCR was performed using genomic DNA as template and rTth polymerase (Perkin Elmer) with the buffer system provided by the manufacturer, 0.25 µM of each primer, and 0.2 mM of each 4 nucleotides. The cycle condition was 30 cycles of 94°C for 1 min, 56°C for 1min and 72 °C for 1 min and 20 sec. The 5' PCR primer contained a HindIII site with the following sequence: 5'-TGCAAGCTTAAAAGGAAAAAATGAACAGC-3' (SEQ.ID.NO.: 79) and the 3' primer contained a BamHI site with the following sequence: 5'-TAAGGATCCCTTCCCTTCAAAACATCCTTG -3' (SEQ.ID.NO.: 80). The resulting 1.1 kb PCR fragment was digested with HindIII and BamHI and cloned into HindIII-BamHI site of pCMV expression vector. Three resulting clones sequenced contained three potential polymorphisms involving changes of amino acid 43 from Pro to Ala, amino acid 97 from Lys to Asn and amino acid 130 from Ile to Phe. Nucleic acid (SEQ.ID.NO.: 81) and amino acid (SEQ.ID.NO.: 82) sequences for human TDAG8 were thereafter determined.

### 8. H9

To obtain H9, PCR was performed using pituitary cDNA as template and rTth polymerase (Perkin Elmer) with the buffer system provided by the manufacturer, 0.25 µM of each primer, and 0.2 mM of each 4 nucleotides. The cycle condition was 30 cycles of 94°C for 1 min, 62°C for 1 min and 72°C for 2 min. The 5' PCR primer contained a HindIII site with the following sequence: 5'-GGAAAGCTTAACGATCCCCAGGAGCAACAT-3' (SEQ.ID.NO.:15) and the 3' primer contained a BamHI site with the following sequence: 5'-CTGGGATCCTACGAGAGCATTTTTCACACAG-3' (SEQ.ID.NO.:16). The resulting 1.9 kb PCR fragment was digested with HindIII and BamHI and cloned into HindIII-BamHI site of pCMV expression vector. H9 contained three potential polymorphisms involving changes of amino acid P320S, S493N and amino acid G448A. Nucleic acid (SEQ.ID.NO.: 139) and amino acid (SEQ.ID.NO.: 140) sequences for human H9 were thereafter determined and verified.

### Example 2

### PREPARATION OF NON-ENDOGENOUS, CONSTITUTIVELY ACTIVATED GPCRS

Those skilled in the art are credited with the ability to select techniques for mutation of a nucleic acid sequence. Presented below are approaches utilized to create non-endogenous versions of several of the human GPCRs disclosed above. The mutations disclosed below are based upon an algorithmic approach whereby the 16^{th} amino acid (located in the IC3 region of the GPCR) from a conserved proline residue (located in the TM6 region of the GPCR, near the TM6/IC3 interface) is mutated, most preferably to a lysine amino acid residue.

### 1. Tranformer Site-Directed ™ Mutagenesis

Preparation of non-endogenous human GPCRs may be accomplished on human GPCRs using Transformer Site-Directed™ Mutagenesis Kit (Clontech) according to the manufacturer instructions. Two mutagenesis primers are utilized, most preferably a lysine mutagenesis oligonucleotide that creates the lysine mutation, and a selection marker oligonucleotide. For convenience, the codon mutation to be incorporated into the human GPCR is also noted, in standard form (Table E):

**TABLE E**

| **Receptor Identifier** | **Codon Mutation** |
|---|---|
| hARE-3 | F313K |
| hARE-4 | V233K |
| hARE-5 | A240K |
| hGPCR14 | L257K |
| hGPCR27 | C283K |
| hARE-1 | E232K |
| hARE-2 | G285K |
| hPPR1 | L239K |
| hG2A | K232A |
| hRUP3 | L224K |
| hRUP5 | A236K |
| hRUP6 | N267K |
| hRUP7 | A302K |
| hCHN4 | V236K |
| hMC4 | A244K |
| hCHN3 | S284K |
| hCHN6 | L352K |
| hCHN8 | N235K |
| hCHN9 | G223K |
| hCHN10 | L231K |
| hH9 | F236K |

The following GPCRs were mutated according with the above method using the designated sequence primers (Table F).

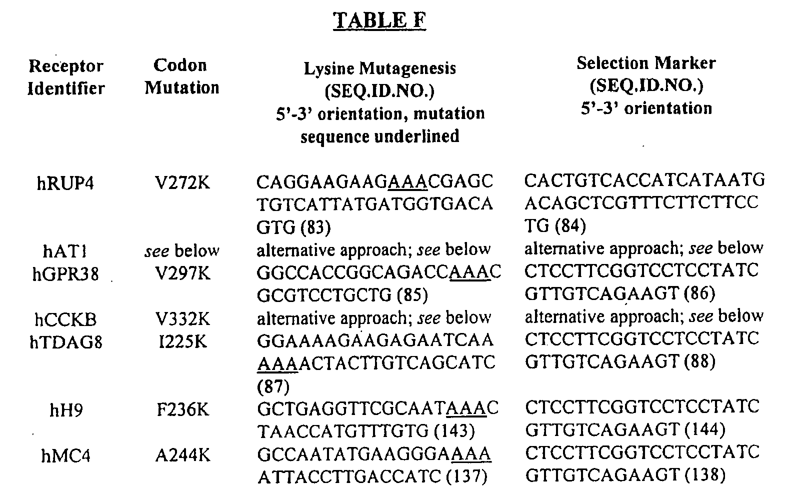

The non-endogenous human GPCRs were then sequenced and the derived and verified nucleic acid and amino acid sequences are listed in the accompanying "Sequence Listing" appendix to this patent document, as summarized in Table G below:

**TABLE G**

| **Non Endogenous Human** | **Nucleic Acid Sequence Listing** | **Amino Acid Sequence** |
|---|---|---|
| GPCR | | **Listing** |
| hRUP4 | SEQ.ID.NO.: 127 | SEQ.ID.NO.: 128 |
| (V272K) | | |
| hATI | (see alternative approaches | (see alternative approaches, |
| (see alternative approaches | below) | below) |
| below) | | |
| hGPR38 | SEQ.ID.NO.: 129 | SEQ.ID.NO.: 130 |
| (V297K) | | |
| hCCKB | SEQ.ID.NO.: 131 | SEQ.ID.NO.: 132 |
| (V332K) | | |
| HTDAG8 | SEQ.ID.NO.: 133 | SEQ.ID.NO.: 134 |
| (I225K) | | |
| hH9 | SEQ.ID.NO.: 141 | SEQ.ID.NO.: 142 |
| (F236K) | | |
| hMC4 | SEQ.ID.NO.: 135 | SEQ.ID.NO.: 136 |
| (A244K) | | |

### 2. Alternative Approaches For Creation of Non-Endogenous Human GPCRs

### a. AT1

### 1. F239K Mutation

Preparation of a non-endogenous, constitutively activated human AT1 receptor was accomplished by creating an F239K mutation (see, SEQ.ID.NO.: 89 for nucleic acid sequence, and SEQ.ID.NO.: 90 for amino acid sequence). Mutagenesis was performed using Transformer Site-Directed Mutagenesis™ Kit (Clontech) according to the to manufacturer's instructions. The two mutagenesis primers were used, a lysine mutagenesis oligonucleotide (SEQ.ID.NO.: 91) and a selection marker oligonucleotide (SEQ.ID.NO.: 92), which had the following sequences: 5'-CCAAGAAATGATGATATTAAAAAGATAATTATGGC-3' (SEQ.ID.NO.: 91) 5'-CTCCTTCGGTCCTCCTATCGTTGTCAGAAGT-3' (SEQ.ID.NO.: 92), respectively.

### 2. N111A Mutation

Preparation of a non-endogenous human AT1 receptor was also accomplished by creating an N111A mutation (see, SEQ.ID.NO.:93 for nucleic acid sequence, and SEQ.ID.NO.: 94 for amino acid sequence). Two PCR reactions were performed using pfu polymerase (Stratagene) with the buffer system provided by the manufacturer, supplemented with 10% DMSO, 0.25 µM of each primer, and 0.5 mM of each 4 nucleotides. The 5' PCR sense primer used had the following sequence: 5'-CCCAAGCTTCCCCAGGTGTATTTGAT-3' (SEQ.ID.NO.: 95) and the antisense primer had the following sequence: 5'-CCTGCAGGCGAAACTGACTCTGGCTGAAG-3' (SEQ.ID.NO.: 96). The resulting 400 bp PCR fragment was digested with HindIII site and subcloned into HindIII-SmaI site of pCMV vector (5' construct). The 3' PCR sense primer used had the following sequence: 5'-CTGTACGCTAGTGTGTTTCTACTCACGTGTCTCAGCATTGAT-3' (SEQ.ID.NO.: 97) and the antisense primer had the following sequence:. 5'-GTTGGATCCACATAATGCATTTTCTC-3' (SEQ.ID.NO.: 98) The resulting 880 bp PCR fragment was digested with BamHI and inserted into Pst (blunted by T4 polymerase) and BamHI site of 5' construct to generated the full length N111A construct. The cycle condition was 25 cycles of 94°C for 1 min, 60°C for 1min and 72 °C for 1 min (5' PCR) or 1.5 min (3' PCR).

### 3. AT2K255IC3 Mutation

Preparation of a non-endogenous, constitutively activated human AT1 was accomplished by creating an AT2K255IC3 "domain swap" mutation (see, SEQ.ID.NO.:99 for nucleic acid sequence, and SEQ.ID.NO.: 100 for amino acid sequence). Restriction sites flanking IC3 of AT 1 were generated to facilitate replacement of the IC3 with corresponding IC3 from angiotensin II type 2 receptor (AT2). This was accomplished by performing two PCR reactions. A 5' PCR fragment (Fragment A) encoded from the 5' untranslated region to the beginning of IC3 was generated by utilizing SEQ.ID.NO.: 63 as sense primer and the following sequence: 5'-TCCGAATTCCAAAATAACTTGTAAGAATGATCAGAAA-3' (SEQ.ID.NO.: 101) as antisense primer. A 3' PCR fragment (Fragment B) encoding from the end of IC3 to the 3' untranslated region was generated by using the following sequence: 5'-AGATCTTAAGAAGATAATTATGGCAATTGTGCT-3' (SEQ.ID.NO.: 102) as sense primer and SEQ.ID.NO.: 64 as antisense primer. The PCR condition was 30 cycles of 94°C for 1 min, 55°C for 1min and 72 °C for 1.5 min using endogenous AT1 cDNA clone as template and pfu polymerase (Stratagene), with the buffer systems provided by the manufacturer, supplemented with 10% DMSO, 0.25 µM of each primer, and 0.5 mM of each 4 nucleotides. Fragment A (720 bp) was digested with HindIII and EcoRI and subcloned. Fragment B was digested with BamHI and subcloned into pCMV vector with an EcoRI site 5' to the cloned PCR fragment.

The DNA fragment (Fragment C) encoding IC3 of AT2 with a L255K mutation and containing an EcoRI cohesive end at 5' and a AfIII cohesive end at 3', was generated by annealing 2 synthetic oligonucleotides having the following sequences:

5'AATTCGAAAACACTTACTGAAGACGAATAGCTATGGGAAGAACAGGATAACCCGTGACCAA
G-3' (sense; SEQ.ID.NO.: 103)

5'TTAACTTGGTCACGGGTTATCCTGTTCTTCCCATAGCTATTCGTCTTCAGT
AAGTGTTTTCG-3' (antisense; SEQ.ID.NO.: 104).

Fragment C was inserted in front of Fragment B through EcoRI and AfIII site. The resulting clone was then ligated with the Fragment A through the EcoRI site to generate AT 1 with AT2K255IC3.

### 4. A243+ Mutation

Preparation of a non-endogenous human AT1 receptor was also accomplished by creating an A243+ mutation (see, SEQ.ID.NO.: 105 for nucleic acid sequence, and SEQ.ID.NO.: 106 for amino acid sequence). An A243+ mutation was constructed using the following PCR based strategy: Two PCR reactions was performed using pfu polymerase (Stratagene) with the buffer system provided by the manufacturer supplemented with 10% DMSO, 0.25 µM of each primer, and 0.5 mM of each 4 nucleotides. The 5' PCR sense primer utilized had the following sequence: 5'-CCCAAGCTTCCCCAGGTGTATTTGAT-3' (SEQ.ID.NO.: 107) and the antisense primer had the following sequence: 5'-AAGCACAATTGCTGCATAATTATCTTAAAAATATCATC-3' (SEQ.ID.NO.: 108). The 3' PCR sense primer utilized had the following sequence: 5'-AAGATAATTATGGCAGCAATTGTGCTTTTCTTTTTCTTT-3' (SEQ.ID.NO.: 109) containing the Ala insertion and antisense primer: 5'-GTTGGATCCACATAATGCATTTTCTC-3'(SEQ.ID.NO.: 110). The cycle condition was 25 cycles of 94°C for 1 min, 54°C for 1min and 72°C for 1.5 min. An aliquot of the 5' and 3' PCR were then used as co-template to perform secondary PCR using the 5' PCR sense primer and 3' PCR antisense primer. The PCR condition was the same as primary PCR except the extention time was 2.5 min. The resulting PCR fragment was digested with HindIII and BamHI and subcloned into pCMV vector. (*See*, SEQ.ID.NO.: 105)

### 4. CCKB

Preparation of the non-endogenous, constitutively activated human CCKB receptor was accomplished by creating a V322K mutation (see, SEQ.ID.NO.: 111 for nucleic acid sequence and SEQ.ID.NO.: 112 for amino acid sequence). Mutagenesis was performed by PCR via amplification using the wildtype CCKB from Example 1.

The first PCR fragment (1kb) was amplified by using SEQ.ID.NO.: 75 and an antisense primer comprising a V322K mutation: 5'-CAGCAGCATGCGCTTCACGCGCTTCTTAGCCCAG-3' (SEQ.ID.NO.: 113). The second PCR fragment (0.44kb) was amplified by using a sense primer comprising the V322K mutation: 
5'-AGAAGCGCGTGAAGCGCATGCTGCTGGTGATCGTT-3' (SEQ.ID.NO.: 114) and SEQ.ID.NO.:
76.
 The two resulting PCR fragments were then used as template for amplifying CCKB comprising V332K, using SEQ.ID.NO.: 75 and SEQ.ID.NO.: 76 and the above-noted system and conditions. The resulting 1.44kb PCR fragment containing the V332K mutation was digested with HindIII and EcoRI and cloned into HindIII-EcoRI site of pCMV expression vector. *(See,* SEQ.ID.NO.: 111).

### 3. QuikChange™ Site-Directed™ Mutagenesis

Preparation of non-endogenous human GPCRs can also be accomplished by using QuikChange™ Site-Directed™ Mutagenesis Kit (Stratagene, according to manufacturer's instructions). Endogenous GPCR is preferably used as a template and two mutagenesis primers utilized, as well as, most preferably, a lysine mutagenesis oligonucleotide and a selection marker oligonucleotide (included in kit). For convenience, the codon mutation incorporated into the human GPCR and the respective oligonucleotides are noted, in standard form (Table H):

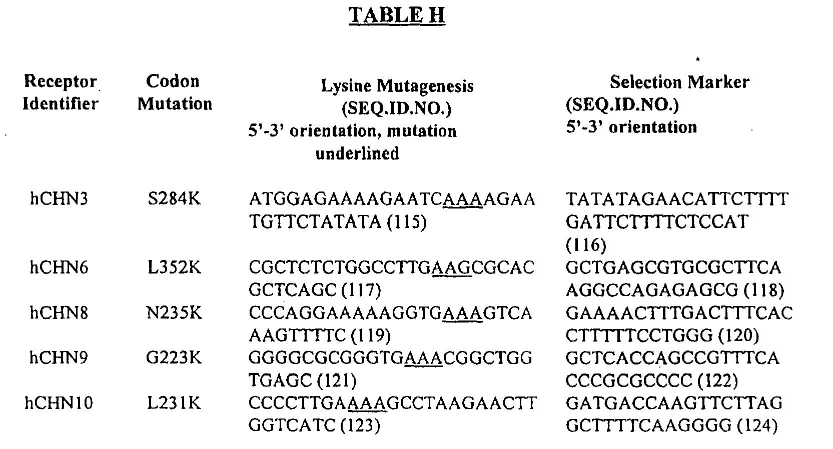

### Example 3

### RECEPTOR EXPRESSION

Although a variety of cells are available to the art for the expression of proteins, it is most preferred that mammalian cells be utilized. The primary reason for this is predicated upon practicalities, *i*.*e*., utilization of, *e*.*g*., yeast cells for the expression of a GPCR, while possible, introduces into the protocol a non-mammalian cell which may not (indeed, in the case of yeast, does not) include the receptor-coupling, genetic-mechanism and secretary pathways that have evolved for mammalian systems - thus, results obtained in non-mammalian cells, while of potential use, are not as preferred as that obtained from mammalian cells. Of the mammalian cells, COS-7, 293 and 293T cells are particularly preferred, although the specific mammalian cell utilized can be predicated upon the particular needs of the artisan.

On day one, 1X10⁷ 293T cells per 150mm plate were plated out. On day two, two reaction tubes were prepared (the proportions to follow for each tube are per plate): tube A was prepared by mixing 20µg DNA (e.g., pCMV vector; pCMV vector with receptor cDNA, etc.) in 1.2ml serum free DMEM (Irvine Scientific, Irvine, CA); tube B was prepared by mixing 120µl lipofectamine (Gibco BRL) in 1.2ml serum free DMEM. Tubes A and B were admixed by inversions (several times), followed by incubation at room temperature for 30-45min. The admixture is referred to as the "transfection mixture". Plated 293T cells were washed with 1XPBS, followed by addition of 10ml serum free DMEM. 2.4ml of the transfection mixture were added to the cells, followed by incubation for 4hrs at 37°C/5% CO₂. The transfection mixture was removed by aspiration, followed by the addition of 25ml of DMEM/10% Fetal Bovine Serum. Cells were incubated at 37°C/5% CO₂. After 72hr incubation, cells were harvested and utilized for analysis.

### Example 4

### ASSAYS FOR DETERMINATION OF CONSTITUTIVE ACTIVITY OF NON-ENDOGENOUS GPCRS

A variety of approaches are available for assessment of constitutive activity of the non-endogenous human GPCRs. The following are illustrative; those of ordinary skill in the art are credited with the ability to determine those techniques that are preferentially beneficial for the needs of the artisan.

### 1. Membrane Binding Assays: [³⁵S]GTPγS Assay

When a G protein-coupled receptor is in its active state, either as a result of ligand binding or constitutive activation, the receptor couples to a G protein and stimulates the release of GDP and subsequent binding of GTP to the G protein. The alpha subunit of the G protein-receptor complex acts as a GTPase and slowly hydrolyzes the GTP to GDP, at which point the receptor normally is deactivated. Constitutively activated receptors continue to exchange GDP for GTP. The non-hydrolyzable GTP analog, [³⁵S]GTPγS, can be utilized to demonstrate enhanced binding of [³⁵S]GTPγS to membranes expressing constitutively activated receptors. The advantage of using [³⁵S]GTPγS binding to measure constitutive activation is that: (a) it is generically applicable to all G protein-coupled receptors; (b) it is proximal at the membrane surface making it less likely to pick-up molecules which affect the intracellular cascade.

The assay utilizes the ability of G protein coupled receptors to stimulate [³⁵S]GTPγS binding to membranes expressing the relevant receptors. The assay can, therefore, be used in the direct identification method to screen candidate compounds to known, orphan and constitutively activated G protein-coupled receptors. The assay is generic and has application to drug discovery at all G protein-coupled receptors.

The [³⁵S]GTPγS assay can be incubated in 20 mM HEPES and between 1 and about 20mM MgCl₂ (this amount can be adjusted for optimization of results, although 20mM is preferred) pH 7.4, binding buffer with between about 0.3 and about 1.2 nM [³⁵S]GTPγS (this amount can be adjusted for optimization of results, although 1.2 is preferred ) and 12.5 to 75 µg membrane protein (e.g, COS-7 cells expressing the receptor; this amount can be adjusted for optimization, although 75µg is preferred) and 1 µM GDP (this amount can be changed for optimization) for 1 hour. Wheatgerm agglutinin beads (25 µl; Amersham) should then be added and the mixture incubated for another 30 minutes at room temperature. The tubes are then centrifuged at 1500 x g for 5 minutes at room temperature and then counted in a scintillation counter.

A less costly but equally applicable alternative has been identified which also meets the needs of large scale screening. Flash plates™ and Wallac™ scintistrips may be utilized to format a high throughput [³⁵S]GTPγS binding assay. Furthermore, using this technique, the assay can be utilized for known GPCRs to simultaneously monitor tritiated ligand binding to the receptor at the same time as monitoring the efficacy via [³⁵S]GTPγS binding. This is possible because the Wallac beta counter can switch energy windows to look at both tritium and ³⁵S-labeled probes. This assay may also be used to detect other types of membrane activation events resulting in receptor activation. For example, the assay may be used to monitor ³²P phosphorylation of a variety of receptors (both G protein coupled and tyrosine kinase receptors). When the membranes are centrifuged to the bottom of the well, the bound [³⁵S]GTPγS or the ³²P-phosphorylated receptor will activate the scintillant which is coated of the wells. Scinti® strips (Wallac) have been used to demonstrate this principle. In addition, the assay also has utility for measuring ligand binding to receptors using radioactively labeled ligands. In a similar manner, when the radiolabeled bound ligand is centrifuged to the bottom of the well, the scintistrip label comes into proximity with the radiolabeled ligand resulting in activation and detection.

### 2. Adenylyl Cyclase

A Flash Plate™ Adenylyl Cyclase kit (New England Nuclear; Cat. No. SMP004A) designed for cell-based assays can be modified for use with crude plasma membranes. The Flash Plate wells contain a scintillant coating which also contains a specific antibody recognizing cAMP. The cAMP generated in the wells was quantitated by a direct competition for binding of radioactive cAMP tracer to the cAMP antibody. The following serves as a brief protocol for the measurement of changes in cAMP levels in membranes that express the receptors.

Transfected cells are harvested approximately three days after transfection. Membranes were prepared by homogenization of suspended cells in buffer containing 20mM HEPES, pH 7.4 and 10mM MgCl₂. Homogenization is performed on ice using a Brinkman Polytron™ for approximately 10 seconds. The resulting homogenate is centrifuged at 49,000 X g for 15 minutes at 4°C. The resulting pellet is then resuspended in buffer containing 20mM HEPES, pH 7.4 and 0.1 mM EDTA, homogenized for 10 seconds, followed by centrifugation at 49,000 X g for 15 minutes at 4°C. The resulting pellet can be stored at - 80°C until utilized. On the day of measurement, the membrane pellet is slowly thawed at room temperature, resuspended in buffer containing 20mM HEPES, pH 7.4 and 10mM MgCL₂ (these amounts can be optimized, although the values listed herein are preferred), to yield a final protein concentration of 0.60mg/ml (the resuspended membranes were placed on ice until use).

cAMP standards and Detection Buffer (comprising 2 µCi of tracer [¹²⁵I cAMP (100 µl] to 11 ml Detection Buffer) are prepared and maintained in accordance with the manufacturer's instructions. Assay Buffer is prepared fresh for screening and contained 20mM HEPES, pH 7.4, 10mM MgCl₂, 20mM (Sigma), 0.1 units/ml creatine phosphokinase (Sigma), 50 µM GTP (Sigma), and 0.2 mM ATP (Sigma); Assay Buffer can be stored on ice until utilized. The assay is initiated by addition of 50ul of assay buffer followed by addition of 50ul of membrane suspension to the NEN Flash Plate. The resultant assay mixture is incubated for 60 minutes at room temperature followed by addition of 100ul of detection buffer. Plates are then incubated an additional 2-4 hours followed by counting in a Wallac MicroBeta™ scintillation counter. Values of cAMP/well are extrapolated from a standard cAMP curve that is contained within each assay plate.

### C. Reporter-Based Assays

### 1. CREB Reporter Assay (Gs-associated receptors)

A method to detect Gs stimulation depends on the known property of the transcription factor CREB, which is activated in a cAMP-dependent manner. A PathDetect™ CREB trans-Reporting System (Stratagene, Catalogue # 219010) can utilized to assay for Gs coupled activity in 293 or 293T cells. Cells are transfected with the plasmids components of this above system and the indicated expression plasmid encoding endogenous or mutant receptor using a Mammalian Transfection Kit (Stratagene, Catalogue #200285) according to the manufacturer's instructions. Briefly, 400 ng pFR-Luc (luciferase reporter plasmid containing Gal4 recognition sequences), 40 ng pFA2-CREB (Gal4-CREB fusion protein containing the Gal4 DNA-binding domain), 80 ng pCMV-receptor expression plasmid (comprising the receptor) and 20 ng CMV-SEAP (secreted alkaline phosphatase expression plasmid; alkaline phosphatase activity is measured in the media of transfected cells to control for variations in transfection efficiency between samples) are combined in a calcium phosphate precipitate as per the Kit's instructions. Half of the precipitate is equally distributed over 3 wells in a 96-well plate, kept on the cells overnight, and replaced with fresh medium the following morning. Forty-eight (48) hr after the start of the transfection, cells are treated and assayed for, e.g., luciferase activity

### 2. AP1 reporter assay (Gq-associated receptors)

A method to detect Gq stimulation depends on the known property of Gq-dependent phospholipase C to cause the activation of genes containing AP1 elements in their promoter. A Pathdetect™ AP-1 cis-Reporting System (Stratagene, Catalogue # 219073) can be utilized following the protocol set forth above with respect to the CREB reporter assay. except that the components of the calcium phosphate precipitate were 410 ng pAP1-Luc. 80 ng pCMV-receptor expression plasmid, and 20 ng CMV-SEAP.

### 3. CRE-LUC Reporter Assay

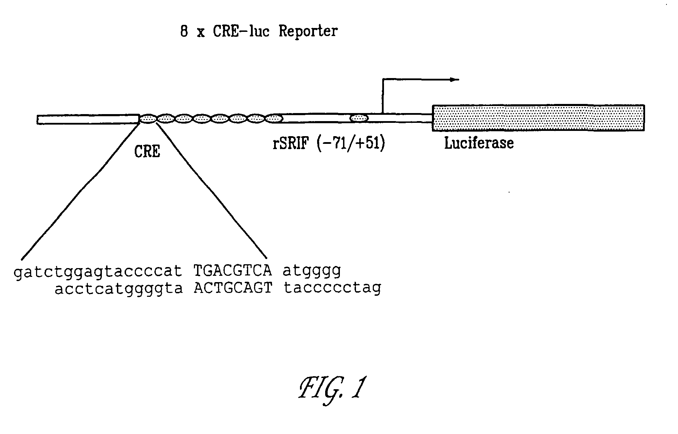

293 and 293T cells are plated-out on 96 well plates at a density of 2 x 10⁴ cells per well and were transfected using Lipofectamine Reagent (BRL) the following day according to manufacturer instructions. A DNA/lipid mixture is prepared for each 6-well transfection as follows: 260ng of plasmid DNA in 100µl of DMEM were gently mixed with 2µl of lipid in 100µl of DMEM (the 260ng of plasmid DNA consisted of 200ng of a 8xCRE-Luc reporter plasmid *(see* below and Figure 1 for a representation of a portion of the plasmid), 50ng of pCMV comprising endogenous receptor or non-endogenous receptor or pCMV alone, and 10ng of a GPRS expression plasmid (GPRS in pcDNA3 (Invitrogen)). The 8XCRE-Luc reporter plasmid was prepared as follows: vector SRIF-β-gal was obtained by cloning the rat somatostatin promoter (-71/+51) at BglV-HindIII site in the pβgal-Basic Vector (Clontech), Eight (8) copies of cAMP response element were obtained by PCR from an adenovirus template AdpCF126CCRE8 *(see, 7 Human Gene Therapy* 1883 (1996)) and cloned into the SRIF-β-gal vector at the Kpn-BglV site, resulting in the 8xCRE-β-gal reporter vector. The 8xCRE-Luc reporter plasmid was generated by replacing the beta-galactosidase gene in the 8xCRE-β-gal reporter vector with the luciferase gene obtained from the pGL3-basic vector (Promega) at the HindIII-BamHI site. Following 30 min. incubation at room temperature, the DNA/lipid mixture was diluted with 400 µl of DMEM and 100µl of the diluted mixture was added to each well. 100 µl of DMEM with 10% FCS were added to each well after a 4hr incubation in a cell culture incubator. The following day the transfected cells were changed with 200 µl/well of DMEM with 10% FCS. Eight (8) hours later, the wells were changed to 100 µl /well of DMEM without phenol red, after one wash with PBS. Luciferase activity were measured the next day using the LucLite™ reporter gene assay kit (Packard) following manufacturer instructions and read on a 1450 MicroBeta™ scintillation and luminescence counter (Wallac).

### 4. SRF-LUC Reporter Assay

One method to detect Gq stimulation depends on the known property ofGq-dependent phospholipase C to cause the activation of genes containing serum response factors in their promoter. A Pathdetect™ SRF-Luc-Reporting System (Stratagene) can be utilized to assay for Gq coupled activity in, e.g., COS7 cells. Cells are transfected with the plasmid components of the system and the indicated expression plasmid encoding endogenous or non-endogenous GPCR using a Mammalian Transfection™ Kit (Stratagene, Catalogue #200285) according to the manufacturer's instructions. Briefly, 410 ng SRF-Luc, 80 ng pCMV-receptor expression plasmid and 20 ng CMV-SEAP (secreted alkaline phosphatase expression plasmid; alkaline phosphatase activity is measured in the media of transfected cells to control for variations in transfection efficiency between samples) are combined in a calcium phosphate precipitate as per the manufacturer's instructions. Half of the precipitate is equally distributed over 3 wells in a 96-well plate, kept on the cells in a serum free media for 24 hours. The last 5 hours the cells are incubated with 1µM Angiotensin, where indicated. Cells are then lysed and assayed for luciferase activity using a Luclite™ Kit (Packard, Cat. # 6016911) and "Trilux 1450 Microbeta" liquid scintillation and luminescence counter (Wallac) as per the manufacturer's instructions. The data can be analyzed using GraphPad Prism™ 2.0a (GraphPad Software Inc.).

### 5. Intracellular IP₃ Accumulation Assay

On day 1, cells comprising the receptors (endogenous and/or non-endogenous) can be plated onto 24 well plates, usually 1x10⁵ cells/well (although his umber can be optimized. On day 2 cells can be transfected by firstly mixing 0.25ug DNA in 50 ul serum free DMEM/well and 2 ul lipofectamine in 50 µl serumfree DMEM/well. The solutions are gently mixed and incubated for 15-30 min at room temperature. Cells are washed with 0.5 ml PBS and 400 µl of serum free media is mixed with the transfection media and added to the cells. The cells are then incubated for 3-4 hrs at 37°C/5%CO₂ and then the transfection media is removed and replaced with 1ml/well of regular growth media. On day 3 the cells are labeled with ³H-myo-inositol. Briefly, the media is removed and the cells are washed with 0.5 ml PBS. Then 0.5 ml inositol-free/serum free media (GIBCO BRL) is added/well with 0.25 µCi of ³H-myo-inositol / well and the cells are incubated for 16-18 hrs o/n at 37°C/5%CO₂. On Day 4 the cells are washed with 0.5 ml PBS and 0.45 ml of assay medium is added containing inositol-free/serum free media 10 µM pargyline 10 mM lithium chloride or 0.4 ml of assay medium and 50 ul of 10x ketanserin (ket) to final concentration of 10µM. The cells are then incubated for 30 min at 37°C. The cells are then washed with 0.5 ml PBSand 200 ul of fresh/icecold stop solution (1M KOH; 18 mM Na-borate; 3.8 mM EDTA) is added/well. The solution is kept on ice for 5-10 min or until cells were lysed and then neutralized by 200 µl of fresh/ice cold neutralization sol. (7.5 % HCL). The lysate is then transferred into 1.5 ml eppendorf tubes and 1 ml of chloroform/methanol (1:2) is added/tube. The solution is vortexed for 15 sec and the upper phase is applied to a Biorad AG1-X8™ anion exchange resin (100-200 mesh). Firstly, the resin is washed with water at 1:1.25 W/V and 0.9 ml of upper phase is loaded onto the column. The column is washed with 10 mls of 5 mM myo-inositol and 10 ml of 5 mM Na-borate/60mM Na-formate. The inositol tris phosphates are eluted into scintillation vials containing 10 ml of scintillation cocktail with 2 ml of 0.1 M formic acid/ 1 M ammonium formate. The columns are regenerated by washing with 10 ml of 0.1 M formic acid/3M ammonium formate and rinsed twice with dd H₂O and stored at 4°C in water.

Exemplary results are presented below in Table I:

**TABLE I**

| **Receptor** | **Mutation** | **Assay Utilized** | **Signal Generated: Endogenous Version** (**Relative Light Units)** | **Signal Generated: Non- Endogenous Version (Relative Light Units)** | **Percent Difference** |
|---|---|---|---|---|---|
| **hAT1** | F239K | SRF-LUC | 34 | 137 | 75%1 |
| | AT2K255IC3 | SRF-LUC | 34 | 127 | 73%1 |
| **hTDAG8** | I225K | CRE-LUC | 2,715 | 14,440 | 81%1 |
| | | (293 cells) | | | |
| | I225K | CRE-LUC | 65,681 | 185,636 | 65%1 |
| | | (293T cells) | | | |
| **hH9** | F236K | CRE-LUC | 1,887 | 6,096 | 69%1 |
| **hCCKB** | V332K | CRE-LUC | 785 | 3,223 | 76%1 |

### C. CELL-BASED DETECTION ASSAY (EXAMPLE -TDAG8)

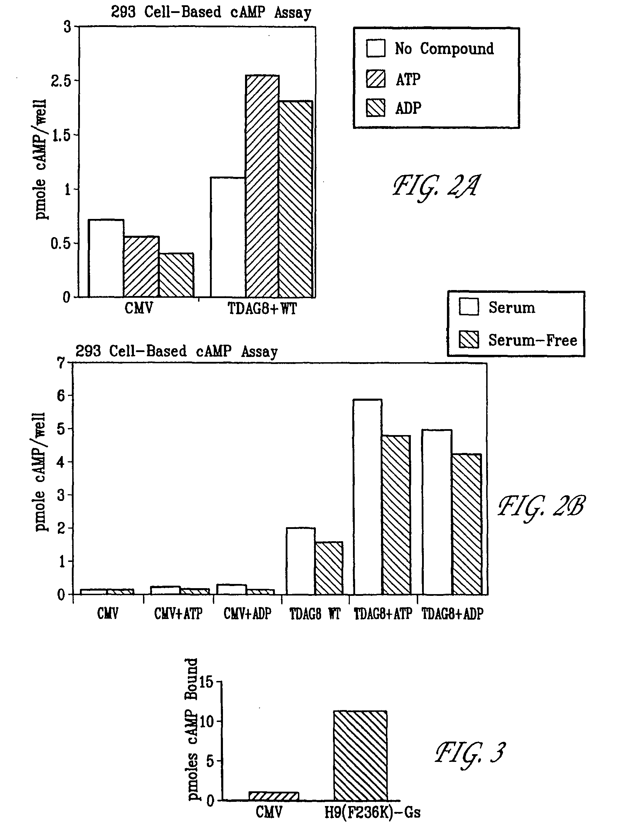

293 cells were plated-out on 150mm plates at a density of 1.3 x 10⁷ cells per plate, and were transfected using 12ug of the respective DNA and 60ul of Lipofectamine Reagent (BRL) per plate. The transfected cells were grown in media containing serum for an assay performed 24 hours post-transfection. For detection assay performed 48 hours post-transfection (assay comparing serum and serum-free media; see Figure 3), the initial media was changed to either serum or serum-free media. The serum-free media was comprised solely of Dulbecco's Modified Eagle's (DME) High Glucose Medium (Irvine Scientific #9024). In addition to the above DME Medium, the media with serum contained the following: 10% Fetal Bovine Serum (Hyclone #SH30071.03), 1% of 100mM Sodium Pyruvate (Irvine Scientific #9334), 1 % of 20mM L-Glutamine (Irvine Scientific #9317), and 1% of Penicillin-Streptomycin solution (Irvine Scientific #9366).

A 96-well Adenylyl Cyclase Activation Flashplate™ was used (NEN: #SMP004A). First, 50ul of the standards for the assay were added to the plate, in duplicate, ranging from concentrations of 50pmol to zero pmol cAMP per well. The standard cAMP (NEN: #SMP004A) was reconstituted in water, and serial dilutions were made using 1xPBS (Irvine Scientific: #9240). Next, 50ul of the stimulation buffer (NEN: #SMP004A) was added to all wells. In the case of using compounds to measure activation or inactivation of cAMP, 10ul of each compound, diluted in water, was added to its respective well, in triplicate. Various final concentrations used range from 1uM up to 1mM. Adenosine 5'-triphosphate, ATP, (Research Biochemicals International: #A-141) and Adenosine 5'-diphosphate, ADP, (Sigma: #A2754) were used in the assay. Next, the 293 cells transfected with the respective cDNA (CMV or TDAG8) were harvested 24 (assay detection in serum media) or 48 hours post-transfection (assay detection comparing serum and serum-free media). The media was aspirated and the cells washed once with 1xPBS. Then 5ml of 1xPBS was added to the cells along with 3ml of cell dissociation buffer (Sigma: #C-1544). The detached cells were transferred to a centrifuge tube and centrifuged at room temperature for five minutes. The supernatant was removed and the cell pellet was resuspended in an appropriate amount of 1xPBS to obtain a final concentration of 2x10⁶ cells per milliliter. To the wells containing the compound, 50ul of the cells in 1xPBS (1x10⁵ cells/well) were added. The plate was incubated on a shaker for 15 minutes at room temperature. The detection buffer containing the tracer cAMP was prepared. In 11ml of detection buffer (NEN: #SMP004A), 50ul (equal to 1uCi) of [¹²⁵I]cAMP (NEN: #SMP004A) was added. Following incubation, 50ul of this detection buffer containing tracer cAMP was added to each well. The plate was placed on a shaker and incubated at room temperature for two hours. Finally, the solution from the wells of the plate were aspirated and the flashplate was counted using the Wallac MicroBeta™ scintillation counter.

In Figure 2A, ATP and ADP bind to endogenous TDAG8 resulting in an increase of cAMP of about 59% and about 55% respectively. Figure 2B evidences ATP and ADP binding to endogenous TDAG8 where endogenous TDAG8 was transfected and grown in serum and serum-free medium. ATP binding to endogenous TDAG8 grown in serum media evidences an increase in cAMP of about 65%, compared to the endogenous TDAG8 with no compounds; in serum-free media there was an increase of about 68%. ADP binding to endogenous TDAG8 in serum evidences about a 61% increase, while in serum-free ADP binding evidences an increase of about 62% increase. ATP and ADP bind to endogenous TDAG8 with an EC50 value of 139.8uM and 120.5uM, respectively (data not shown).

Although the results presented in Figure 2B indicate substantially the same results when serum and serum-free media were compared, our choice is to use a serum based media, although a serum-free media can also be utilized.

### Example 6

### GPCR FUSION PROTEIN PREPARATION

The design of the constitutively activated GPCR-G protein fusion construct was accomplished as follows: both the 5' and 3' ends of the rat G protein Gsα (long form; Itoh, H. et al., 83 *PNAS* 3776 (1986)) were engineered to include a HindIII (5'-AAGCTT-3') sequence thereon. Following confirmation of the correct sequence (including the flanking HindIII sequences), the entire sequence was shuttled into pcDNA3.1(-) (Invitrogen, cat. no. V795-20) by subcloning using the HindIII restriction site of that vector. The correct orientation for the Gsα sequence was determined after subcloning into pcDNA3.1(-). The modified pcDNA3.1 (-) containing the rat Gsα gene at HindIII sequence was then verified; this vector was now available as a "universal" Gsα protein vector. The pcDNA3.1(-) vector contains a variety of well-known restriction sites upstream of the HindIII site, thus beneficially providing the ability to insert, upstream of the Gs protein, the coding sequence of an endogenous, constitutively active GPCR. This same approach can be utilized to create other "universal" G protein vectors, and, of course, other commercially available or proprietary vectors known to the artisan can be utilized - the important criteria is that the sequence for the GPCR be upstream and in-frame with that of the G protein.

TDAG8 couples via Gs, while H9 couples via Gz. For the following exemplary GPCR Fusion Proteins, fusion to Gsα was accomplished.

A TDAG8(I225K)-Gsα Fusion Protein construct was made as follows: primers were designed as follows:

5'-gatcTCTAGAATGAACAGCACATGTATTGAAG-3' (SEQ.ID.NO.: 125; sense)

5'-ctagGGTACCCGCTCAAGGACCTCTAATTCCATAG-3' (SEQ.ID.NO.: 126; antisense).

Nucleotides in lower caps are included as spacers in the restriction sites between the G protein and TDAG8. The sense and anti-sense primers included the restriction sites for XbaI and KpnI, respectively.

PCR was then utilized to secure the respective receptor sequences for fusion within the Gsα universal vector disclosed above, using the following protocol for each: 100ng cDNA for TDAG8 was added to separate tubes containing 2ul of each primer (sense and anti-sense), 3uL of 10mM dNTPs, 10uL of 10XTaqPlus™ Precision buffer, 1uL of TaqPlus™ Precision polymerase (Stratagene: #600211), and 80uL of water. Reaction temperatures and cycle times for TDAG8 were as follows: the initial denaturing step was done it 94°C for five minutes, and a cycle of 94°C for 30 seconds; 55°C for 30 seconds; 72°C for two minutes. A final extension time was done at 72°C for ten minutes. PCR product for was run on a 1% agarose gel and then purified (data not shown). The purified product was digested with XbaI and KpnI (New England Biolabs) and the desired inserts purified and ligated into the Gs universal vector at the respective restriction site. The positive clones was isolated following transformation and determined by restriction enzyme digest; expression using 293 cells was accomplished following the protocol set forth *infra*. Each positive clone for TDAG8:Gs - Fusion Protein was sequenced to verify correctness.

GPCR Fusion Proteins comprising non-endogenous, constitutively activated TDAG8(I225K) were analyzed as above and verified for constitutive activation.

An H9(F236K)-Gsα Fusion Protein construct was made as follows: primers were designed as follows:

5'-TTAgatatcGGGGCCCACCCTAGCGGT-3' (SEQ.ID.NO.: 145; sense)

5'-ggtaccCCCACAGCCATTTCATCAGGATC-3' (SEQ.ID.NO.: 146; antisense).

Nucleotides in lower caps are included as spacers in the restriction sites between the G protein and H9. The sense and anti-sense primers included the restriction sites for EcoRV and KpnI, respectively such that spacers (attributed to the restriction sites) exists between the G protein and H9.

PCR was then utilized to secure the respective receptor sequences for fusion within the Gsα universal vector disclosed above, using the following protocol for each: 80ng cDNA for H9 was added to separate tubes containing 100ng of each primer (sense and anti-sense), and 45uL of PCR Supermix™ (Gibco-Brl, LifeTech) (50ul total reaction volume). Reaction temperatures and cycle times for H9 were as follows: the initial denaturing step was done it 94°C for one, and a cycle of 94°C for 30 seconds: 55°C for 30 seconds: 72°C for two minutes. A final extension time was done at 72°C for seven minutes. PCR product for was run on a 1 % agarose gel and then purified (data not shown). The purified product was cloned into pCRII-TOPO™ System followed by identification of positive clones. Positive clones were isolated, digested with EcoRV and KpnI (New England Biolabs) and the desired inserts were isolated, purified and ligated into the Gs universal vector at the respective restriction site. The positive clones was isolated following transformation and determined by restriction enzyme digest; expression using 293 cells was accomplished following the protocol set forth *infra*. Each positive clone for H9(F236K):Gs - Fusion Protein was sequenced to verify correctness. Membranes were frozen (-80°C) until utilized.

To ascertain the ability of measuring a cAMP response mediated by the Gs protein (even though H9 couples with Gz), the following cAMP membrane assay was utilized, based upon an NEN Adenyl Cyclase Activation Flahplate™ Assay kit (96 well format). "Binding Buffer" consisted of 10mM HEPES, 100mM NaCl and 10mM MgCl (ph 7.4). "Regeneration Buffer" was prepared in Binding Buffer and consisted of 20mM phosphocreatine, 20U creatine phosphokinase, 20uM GTP, 0.2mM ATP, and 0.6mM IBMX. "cAMP Standards" were prepared in Binding Buffer as follows:

| cAMP Stock (5,000 pmol/ml in 2ml H₂O) in ul | | Added to indicted amount of Binding Buffer | Final Assay Concentration (50ul into 100ul) to achieve indicated pmol/well |
|---|---|---|---|
| A | 250 | 1ml | 50 |
| B | 500 of A | 500ul | 25 |
| C | 500 of B | 500ul | 12.5 |
| D | 500 of C | 750ul | 5.0 |
| E | 500 of D | 500ul | 2.5 |
| F | 500 of E | 500ul | 1.25 |
| G | 500 of F | 750ul | 0.5 |

Frozen membranes (both pCMV as control and the non-endogenous H(-Gs Fusion Protein) were thawed (on ice at room temperature until in solution). Membranes were homogenized with a polytron until in suspension (2 x 15 seconds). Membrane protein concentration was determined using the Bradford Assay Protocol (*see infra*). Membrane concentration was diluted to 0.5mg/ml in Regeneration Buffer (final assay concentration - 25ug/well). Thereafter, 50ul of Binding Buffer was added to each well. For control, 50ul/well of cAMP standard was added to wells 11 and 12 A-G, with Binding Buffer alone to 12H (on the 96-well format). Thereafter, 50ul/well of protein was added to the wells and incubated at room temperature (on shaker) for 60min. 100ul[¹²⁵I]cAMP in Detection Buffer (*see infra*) was added to each well (final - 50ul[¹²⁵I]cAMP into 11ml Detection Buffer). These were incubated for 2hrs at room temperature. Plates were aspirated with an 8 channel manifold and sealed with plate covers. Results (pmoles cAMP bound) were read in a Wallac™ 1450 on "prot #15). Results are presented in Figure 3.

The results presented in Figure 3 indicate that the Gs coupled fusion was able to "drive" the cyclase reaction such that measurement of the consitutive activation of H9(F236K) was viable. Based upon these results, the direct identification of candidate compounds that are inverse agonists, agonists and partial agonists is possible using a cyclase-based assay.

### Example 6

### Protocol: Direct Identification of Inverse Agonists and Agonists Using [³⁵S]GTPγS

Although we have utilized endogenous, constitutively active GPCRs for the direct identification of candidate compounds as, e.g., inverse agonists, for reasons that are not altogether understood, intra-assay variation can become exacerbated. Preferably, then, a GPCR Fusion Protein, as disclosed above, is also utilized with a non-endogenous, constitutively activated GPCR. We have determined that when such a protein is used, intra-assay variation appears to be substantially stabilized, whereby an effective signal-to-noise ratio is obtained. This has the beneficial result of allowing for a more robust identification of candidate compounds. Thus, it is preferred that for direct identification, a GPCR Fusion Protein be used and that when utilized, the following assay protocols be utilized.

### Membrane Preparation

Membranes comprising the non-endogenous, constitutively active orphan GPCR Fusion Protein of interest and for use in the direct identification of candidate compounds as inverse agonists, agonists or partial agonists are preferably prepared as follows:

### a. Materials

"Membrane Scrape Buffer" is comprised of 20mM HEPES and 10mM EDTA, pH 7.4; "Membrane Wash Buffer" is comprised of 20 mM HEPES and 0.1 mM EDTA, pH 7.4; "Binding Buffer" is comprised of 20mM HEPES, 100 mM NaCl, and 10 mM MgCl₂, pH 7.4

### b. Procedure

All materials are kept on ice throughout the procedure. Firstly, the media is aspirated from a confluent monolayer of cells, followed by rinse with 10ml cold PBS, followed by aspiration. Thereafter, 5ml of Membrane Scrape Buffer is added to scrape cells; this is followed by transfer of cellular extract into 50ml centrifuge tubes (centrifuged at 20,000 rpm for 17 minutes at 4°C). Thereafter, the supernatant is aspirated and the pellet is resuspended in 30ml Membrane Wash Buffer followed by centrifuge at 20,000 rpm for 17 minutes at 4°C. The supernatant is then aspirated and the pellet resuspended in Binding Buffer. This is then homogenized using a Brinkman polytron™ homogenizer (15-20 second bursts until the all material is in suspension). This is referred to herein as "Membrane Protein".

### Bradford Protein Assay

Following the homogenization, protein concentration of the membranes is determined using the Bradford Protein Assay (protein can be diluted to about 1.5mg/ml, aliquoted and frozen (-80°C) for later use; when frozen, protocol for use is as follows: on the day of the assay, frozen Membrane Protein is thawed at room temperature, followed by vortex and then homogenized with a polytron at about 12 x 1,000 rpm for about 5-10 seconds; it is noted that for multiple preparations, the homogenizor should be thoroughly cleaned between homoginezation of different preparations).

### a. Materials

Binding Buffer (as per above); Bradford Dye Reagent; Bradford Protein Standard are utilized, following manufacturer instructions (Biorad, cat. no. 500-0006).

### b. Procedure

Duplicate tubes are prepared, one including the membrane, and one as a control "blank". Each contained 800ul Binding Buffer. Thereafter, 10ul of Bradford Protein Standard (1mg/ml) is added to each tube, and 10ul of membrane Protein is then added to just one tube (not the blank). Thereafter, 200ul of Bradford Dye Reagent is added to each tube, followed by vortex of each. After five (5) minutes, the tubes were re-vortexed and the material therein is transferred to cuvettes. The cuvettes are then read using a CECIL 3041 spectrophotometer, at wavelength 595.

### Direct Identification Assay

### a. Materials

GDP Buffer consists of 37.5 ml Binding Buffer and 2mg GDP (Sigma, cat. no. G-7127), followed by a series of dilutions in Binding Buffer to obtain 0.2 uM GDP (final concentration of GDP in each well was 0.1 uM GDP); each well comprising a candidate compound, has a final volume of 200ul consisting of 100ul GDP Buffer (final concentration, 0.1uM GDP), 50ul Membrane Protein in Binding Buffer, and 50ul [³⁵S]GTPγS (0.6 nM) in Binding Buffer (2.5 ul [³⁵S]GTPγS per 10ml Binding Buffer).

### b. Procedure

Candidate compounds are preferably screened using a 96-well plate format (these can be frozen at -80°C). Membrane Protein (or membranes with expression vector excluding the GPCR Fusion Protein, as control), are homogenized briefly until in suspension. Protein concentration is then determined using the Bradford Protein Assay set forth above. Membrane Protein (and control) is then diluted to 0.25mg/ml in Binding Buffer (final assay concentration, 12.5ug/well). Thereafter, 100 ul GDP Buffer is added to each well of a Wallac Scintistrip™ (Wallac). A 5ul pin-tool is then used to transfer 5 ul of a candidate compound into such well (*i*.*e*., 5ul in total assay volume of 200 ul is a 1:40 ratio such that the final screening concentration of the candidate compound is 10uM). Again, to avoid contamination, after each transfer step the pin tool should be rinsed in three reservoirs comprising water (1X), ethanol (1X) and water (2X) - excess liquid should be shaken from the tool after each rinse and dried with paper and kimwipes. Thereafter, 50 ul of Membrane Protein is added to each well (a control well comprising membranes without the GPCR Fusion Protein is also utilized), and pre-incubated for 5-10 minutes at room temperature. Thereafter, 50 ul of [³⁵S]GTPγS (0.6 nM) in Binding Buffer is added to each well, followed by incubation on a shaker for 60 minutes at room temperature (again, in this example, plates were covered with foil). The assay is then stopped by spinning of the plates at 4000 RPM for 15 minutes at 22°C. The plates are then aspirated with an 8 channel manifold and sealed with plate covers. The plates are then read on a Wallacc 1450 using setting "Prot. #37" (as per manufacturer instructions).

### Example 7

### Protocol: Confirmation Assay

Using an independent assay approach to provide confirmation of a directly identified candidate compound as set forth above, it is preferred that a confirmation assay then be utilized. In this case, the preferred confirmation assay is a cyclase-based assay.

A modified Flash Plate™ Adenylyl Cyclase kit (New England Nuclear; Cat. No. SMP004A) is preferably utilized for confirmation of candidate compounds directly identified as inverse agonists and agonists to non-endogenous, constitutively activated orphan GPCRs in accordance with the following protocol.

Transfected cells are harvested approximately three days after transfection. Membranes are prepared by homogenization of suspended cells in buffer containing 20mM HEPES, pH 7.4 and 10mM MgCl₂. Homogenization is performed on ice using a Brinkman Polytron™ for approximately 10 seconds. The resulting homogenate is centrifuged at 49,000 X g for 15 minutes at 4°C. The resulting pellet is then resuspended in buffer containing 20mM HEPES, pH 7.4 and 0.1 mM EDTA, homogenized for 10 seconds, followed by centrifugation at 49,000 X g for 15 minutes at 4°C. The resulting pellet can be stored at - 80°C until utilized. On the day of direct identification screening, the membrane pellet is slowly thawed at room temperature, resuspended in buffer containing 20mM HEPES, pH 7.4 and 10mM MgCL2, to yield a final protein concentration of 0.60mg/ml (the resuspended membranes are placed on ice until use).

cAMP standards and Detection Buffer (comprising 2 µCi of tracer [¹²⁵I cAMP (100 µl] to 11 ml Detection Buffer) are prepared and maintained in accordance with the manufacturer's instructions. Assay Buffer is prepared fresh for screening and contained 20mM HEPES, pH 7.4, 10mM MgCl₂, 20mM phospocreatine (Sigma), 0. 1 units/ml creatine phosphokinase (Sigma), 50 µM GTP (Sigma), and 0.2 mM ATP (Sigma); Assay Buffer can be stored on ice until utilized.

Candidate compounds identified as per above (if frozen, thawed at room temperature) are added, preferably, to 96-well plate wells (3*µ*l/well; 12*µ*M final assay concentration), together with 40 *µ*l Membrane Protein (30µg/well) and 50*µ*l of Assay Buffer. This admixture is then incubated for 30 minutes at room temperature, with gentle shaking.

Following the incubation, 100µl of Detection Buffer is added to each well, followed by incubation for 2-24 hours. Plates are then counted in a Wallac MicroBeta™ plate reader using "Prot. #31" (as per manufacturer instructions).

It is intended that each of the patents, applications, and printed publications mentioned in this patent document be hereby incorporated by reference in their entirety.

As those skilled in the art will appreciate, numerous changes and modifications may be made to the preferred embodiments of the invention without departing from the spirit of the invention. It is intended that all such variations fall within the scope of the invention.

Although a variety of expression vectors are available to those in the art, for purposes of utilization for both the endogenous and non-endogenous human GPCRs, it is most preferred that the vector utilized be pCMV. This vector was deposited with the American Type Culture Collection (ATCC) on October 13, 1998 (10801 University Blvd., Manassas, VA 20110-2209 USA) under the provisions of the Budapest Treaty for the International Recognition of the Deposit of Microorganisms for the Purpose of Patent Procedure. The DNA was tested by the ATCC and determined to be. The ATCC has assigned the following deposit number to pCMV: ATCC #203351.

The following numbered paragraphs (paras.) contain statements of broad combinations of the inventive technical features herein disclosed:-
1. A cDNA encoding a non-endogenous, constitutively activated version of a human G protein-coupled receptor comprising hARE-3(F313K).
2. A non-endogenous version of a human G protein-coupled receptor encoded by the cDNA of paragraph 1.
3. A Plasmid comprising a Vector and the cDNA of paragraph 1.
4. A Host Cell comprising the Plasmid of paragraph 3.
5. A cDNA encoding a non-endogenous, constitutively activated version of a human G protein-coupled receptor comprising hARE-4(V233K)
6. A non-endogenous version of a human G protein-coupled receptor encoded by the cDNA of paragraph 5.
7. A Plasmid comprising a Vector and the cDNA of paragraph 5.
8. A Host Cell comprising the Plasmid of paragraph 7.
9. A cDNA encoding a non-endogenous, constitutively activated version of a human G protein-coupled receptor comprising hARE-5(A240K).
10. A non-endogenous version of a human G protein-coupled receptor encoded by the cDNA of paragraph 9.
11. A Plasmid comprising a Vector and the cDNA of paragraph 5.
12. A Host Cell comprising the Plasmid of paragraph 11.
13. A cDNA encoding a non-endogenous, constitutively activated version of a human G protein-coupled receptor comprising hGPCR14(L257K).
14. A non-endogenous version of a human G protein-coupled receptor encoded by the cDNA of paragraph 13.
15. A Plasmid comprising a Vector and the cDNA of paragraph 13.
16. A Host Cell comprising the Plasmid of paragraph 15.
17. A cDNA encoding a non-endogenous, constitutively activated version of a human G protein-coupled receptor comprising hGPCR27(C283K).
18. A non-endogenous version of a human G protein-coupled receptor encoded by the cDNA of paragraph 17.
19. A Plasmid comprising a Vector and the cDNA of paragraph 17.
20. A Host Cell comprising the Plasmid of paragraph 19.
21. A cDNA encoding a non-endogenous, constitutively activated version of a human G protein-coupled receptor comprising hARE-1(E232K).
22. A non-endogenous version of a human G protein-coupled receptor encoded by the cDNA of paragraph 21.
23. A Plasmid comprising a Vector and the cDNA of paragraph 21.
24. A Host Cell comprising the Plasmid of paragraph 23.
25. A cDNA encoding a non-endogenous, constitutively activated version of a human G protein-coupled receptor comprising hARE-2(G285K).
26. A non-endogenous version of a human G protein-coupled receptor encoded by the cDNA of paragraph 25.
27. A Plasmid comprising a Vector and the cDNA of paragraph 25.
28. A Host Cell comprising the Plasmid of paragraph 27.
29. A cDNA encoding a non-endogenous, constitutively activated version of a human G protein-coupled receptor comprising hPPR1(L239K).
30. A non-endogenous version of a human G protein-coupled receptor encoded by the cDNA of paragraph 29.
31. A Plasmid comprising a Vector and the cDNA of paragraph 29.
32. A Host Cell comprising the Plasmid of paragraph 31.
33. A cDNA encoding a non-endogenous, constitutively activated version of a human G protein-coupled receptor comprising hG2A(K232A).
34. A non-endogenous version of a human G protein-coupled receptor encoded by the cDNA of paragraph 33.
35. A Plasmid comprising a Vector and the cDNA of paragraph 33.
36. A Host Cell comprising the Plasmid of paragraph 35.
37. A cDNA encoding a non-endogenous, constitutively activated version of a human G protein-coupled receptor comprising hRUP3(L224K).
38. A non-endogenous version of a human G protein-coupled receptor encoded by the cDNA of paragraph 37.
39. A Plasmid comprising a Vector and the cDNA of paragraph 37.
40. A Host Cell comprising the Plasmid of paragraph 39.
41. A cDNA encoding a non-endogenous, constitutively activated version of a human G protein-coupled receptor comprising hRUP5(A236K).
42. A non-endogenous version of a human G protein-coupled receptor encoded by the cDNA of paragraph 41.
43. A Plasmid comprising a Vector and the cDNA of paragraph 41.
44. A Host Cell comprising the Plasmid of paragraph 42.
45. A cDNA encoding a non-endogenous, constitutively activated version of a human G protein-coupled receptor comprising hRUP6(N267K)
46. A non-endogenous version of a human G protein-coupled receptor encoded by the cDNA of paragraph 45.
47. A Plasmid comprising a Vector and the cDNA of paragraph 45.
48. A Host Cell comprising the Plasmid of paragraph 47.
49. A cDNA encoding a non-endogenous, constitutively activated version of a human G protein-coupled receptor comprising hRUP7(A302K).
50. A non-endogenous version of a human G protein-coupled receptor encoded by the cDNA of paragraph 49.
51. A Plasmid comprising a Vector and the cDNA of paragraph 49.
52. A Host Cell comprising the Plasmid of paragraph 51.
53. A cDNA encoding a non-endogenous, constitutively activated version of a human G protein-coupled receptor comprising hCHN4(V236K).
54. A non-endogenous version of a human G protein-coupled receptor encoded by the cDNA of paragraph 53.
55. A Plasmid comprising a Vector and the cDNA of paragraph 53.
56. A Host Cell comprising the Plasmid of paragraph 55.
57. A cDNA encoding a non-endogenous, constitutively activated version of a human G protein-coupled receptor comprising hMC4(A244K).
58. A non-endogenous version of a human G protein-coupled receptor encoded by the cDNA of paragraph 57.
59. A Plasmid comprising a Vector and the cDNA of paragraph 57.
60. A Host Cell comprising the Plasmid of paragraph 60.
61. A cDNA encoding a non-endogenous, constitutively activated version of a human G protein-coupled receptor comprising hCHN3(S284K).
62. A non-endogenous version of a human G protein-coupled receptor encoded by the cDNA of paragraph 61.
63. A Plasmid comprising a Vector and the cDNA of paragraph 61.
64. A Host Cell comprising the Plasmid of paragraph 63.
65. A cDNA encoding a non-endogenous, constitutively activated version of a human G protein-coupled receptor comprising hCHN6(L352K).
66. A non-endogenous version of a human G protein-coupled receptor encoded by the cDNA of paragraph 65.
67. A Plasmid comprising a Vector and the cDNA of paragraph 65.
68. A Host Cell comprising the Plasmid of paragraph 67.
69. A cDNA encoding a non-endogenous, constitutively activated version of a human G protein-coupled receptor comprising hCHN8(N235K).
70. A non-endogenous version of a human G protein-coupled receptor encoded by the cDNA of paragraph 69.
71. A Plasmid comprising a Vector and the cDNA of paragraph 69.
72. A Host Cell comprising the Plasmid of paragraph 71.
73. A cDNA encoding a non-endogenous, constitutively activated version of a human G protein-coupled receptor comprising hH9(F236K).
74. A non-endogenous version of a human G protein-coupled receptor encoded by the cDNA of paragraph 73.
75. A Plasmid comprising a Vector and the cDNA of paragraph 73.
76. A Host Cell comprising the Plasmid of paragraph 74.
77. A cDNA encoding a non-endogenous, constitutively activated version of a human G protein-coupled AT1 receptor selected from the group consisting of: hAT1(F239K); hAT1(N111A); hAT1(AT2K255IC3); and hAT1(A243+).
78. A non-endogenous version of a human G protein-coupled receptor encoded by a cDNA of paragraph 77.
79. A Plasmid comprising a Vector and the cDNA of paragraph 77.
80. A Host Cell comprising the Plasmid of paragraph 79.

## Claims

1. Use of a G-protein coupled receptor to identify agonists and partial agonists of said receptor for use as pharmaceutical agents, wherein the G-protein coupled receptor comprises an amino acid sequence selected from the group consisting of:
(a) the amino acid sequence of SEQ ID NO:82;
(b) the amino acid sequence of SEQ ID NO:82, wherein the amino acid sequence of SEQ ID NO:82 has a substitution selected from the group consisting of:
(i) a substitution of proline at amino acid position 43 of SEQ ID NO:82 with alanine;
(ii) a substitution of lysine at amino acid position 97 of SEQ ID NO:82 with asparagine; and
(iii) a substitution of isoleucine at amino acid position 130 of SEQ ID NO:82 with phenylalanine;
(c) the amino acid sequence of a G-protein coupled receptor encoded by a polynucleotide comprising a nucleic acid sequence, said nucleic acid sequence being the sequence obtainable by performing PCR on a human DNA sample using specific primers SEQ ID NO:79 and SEQ ID NO:80;
(d) the amino acid sequence of SEQ ID NO:134; and
(e) the amino acid sequence of SEQ ID NO:134, wherein the amino acid sequence of SEQ ID NO:134 has a substitution selected from the group consisting of:
(i') a substitution of alanine at amino acid position 43 of SEQ ID NO:134 with proline;
(ii') a substitution of asparagine at amino acid position 97 of SEQ ID NO:134 with lysine; and
(iii') a substitution of phenylalanine at amino acid position 130 of SEQ ID NO:134 with isoleucine.

2. The use of claim 1, wherein the G-protein coupled receptor forms part of a fusion protein with a G-protein.

3. The use of claim 1 or claim 2, wherein the G-protein coupled receptor is recombinant.

4. The use of any one of claims 1 to 3, wherein the pharmaceutical agent is for increasing a level of intracellular cAMP.

5. A non-endogenous constitutively activated G-protein coupled receptor comprising an amino acid sequence selected from the group consisting of:
(a) the amino acid sequence of SEQ ID NO:134; and
(b) the amino acid sequence of SEQ ID NO:134, wherein the amino acid sequence of SEQ ID NO:134 has a substitution selected from the group consisting of:
(i) a substitution of alanine at amino acid position 43 of SEQ ID NO:134 with proline;
(ii) a substitution of asparagine at amino acid position 97 of SEQ ID NO:134 with lysine; and
(iii) a substitution of phenylalanine at amino acid position 130 of SEQ ID NO:134 with isoleucine.

6. A fusion protein comprising a G-protein and a G-protein coupled receptor, wherein the G-protein coupled receptor is according to claim 5.

7. An isolated polynucleotide encoding a G-protein coupled receptor or a fusion protein according to claim 5 or claim 6.

8. A vector comprising a polynucleotide according to claim 7.

9. A host cell comprising a vector according to claim 8.

10. A method for identifying one or more candidate compounds as an agonist, partial agonist or inverse agonist of a non-endogenous constitutively activated G-protein coupled receptor, wherein the receptor comprises an amino acid sequence selected from the group consisting of:
(a) the amino acid sequence of SEQ ID NO:134; and
(b) the amino acid sequence of SEQ ID NO:134, wherein the amino acid sequence of SEQ ID NO:134 has a substitution selected from the group consisting of:
(i) a substitution of alanine at amino acid position 43 of SEQ ID NO:134 with proline;
(ii) a substitution of asparagine at amino acid position 97 of SEQ ID NO:134 with lysine;
(iii) a substitution of phenylalanine at amino acid position 130 of SEQ ID NO:134 with isoleucine.
comprising the steps of:
(a') contacting said one or more compounds with a host cell or with membrane of a host cell that expresses the receptor; and
(b') measuring the ability of the compound or compounds to inhibit or stimulate functionality of said receptor.

11. A method according to claim 10, wherein the host cell comprises an expression vector, said expression vector comprising a polynucleotide encoding the non-endogenous constitutively activated G-protein coupled receptor.
